(19) Europäisches Patentamt
European Patent Office
Office européen des brevets

(11) EP 2 147 096 B1

(12) **EUROPEAN PATENT SPECIFICATION**

(45) Date of publication and mention of the grant of the patent:
**25.03.2015 Bulletin 2015/13**

(21) Application number: **08742852.0**

(22) Date of filing: **11.04.2008**

(51) Int Cl.:
***C07K 14/745*** *(2006.01)* ***A61K 38/36*** *(2006.01)*
***A61P 7/04*** *(2006.01)*

(86) International application number:
**PCT/US2008/004795**

(87) International publication number:
**WO 2008/127702 (23.10.2008 Gazette 2008/43)**

(54) **Modified factor VII polypeptides and uses thereof**

Modifizierte Faktor-VII-Polypeptide und Verwendungen davon

Polypeptides du facteur VII modifiés et leurs utilisations

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **13.04.2007 US 923512 P**

(43) Date of publication of application:
**27.01.2010 Bulletin 2010/04**

(60) Divisional application:
**12156573.3 / 2 481 797**

(73) Proprietor: **Catalyst Biosciences, Inc.**
**South San Francisco, CA 94080 (US)**

(72) Inventors:
- **MADISON, Edwin, L.**
  **San Francisco, CA 94121 (US)**
- **THANOS, Christopher, D.**
  **San Francisco, CA 94118 (US)**
- **RUGGLES, Sandra, Waugh**
  **Sunnyvale, CA 94086 (US)**
- **COUGHLIN, Shaun**
  **Tiburon, CA 94920 (US)**

(74) Representative: **Boult Wade Tennant**
**Verulam Gardens**
**70 Gray's Inn Road**
**London WC1X 8BT (GB)**

(56) References cited:
**WO-A-2004/083361 WO-A-2006/114105**
**WO-A-2007/031559**

- **GENG J P ET AL: "Properties of a recombinant chimeric protein in which the gamma-carboxyglutamic acid and helical stack domains of human anticoagulant protein C are replaced by those of human coagulation factor VII." THROMBOSIS AND HAEMOSTASIS MAY 1997, vol. 77, no. 5, May 1997 (1997-05), pages 926-933, XP008098458 ISSN: 0340-6245 cited in the application**
- **SHAH A ET AL: "Manipulation of the membrane binding site of vitamin K-dependent proteins: enhanced biological function of human factor VII" PROCEEDINGS OF THE NATIONAL ACADEMY OF SCIENCES OF USA, NATIONAL ACADEMY OF SCIENCE, WASHINGTON, DC.; US, vol. 95, no. 8, 14 April 1998 (1998-04-14), pages 4229-4234, XP002092845 ISSN: 0027-8424 cited in the application**
- **HARVEY S B ET AL: "Mutagenesis of the gamma-Carboxyglutamic Acid Domain of Human Factor VII to Generate Maximum Enhancement of the Membrane Contact Site" JOURNAL OF BIOLOGICAL CHEMISTRY, AMERICAN SOCIETY OF BIOLOCHEMICAL BIOLOGISTS, BIRMINGHAM,; US, vol. 278, no. 10, 7 March 2003 (2003-03-07), pages 8363-8369, XP002306018 ISSN: 0021-9258 cited in the application**
- **RUF W: "Factor VIIa residue Arg290 is required for efficient activation of the macromolecular substrate factor X." BIOCHEMISTRY 27 SEP 1994, vol. 33, no. 38, 27 September 1994 (1994-09-27), pages 11631-11636, XP002493195 ISSN: 0006-2960**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 147 096 B1

Printed by Jouve, 75001 PARIS (FR)

SEQUENCE LISTING

<110> Catalyst Biosciences, Inc. Edwin Madison Christopher Thanos Sandra Waugh Ruggles Shaun Coughlin

<120> MODIFIED FACTOR VII POLYPEPTIDES AND USES THEREOF

<130> 119357-00067/4913PC

<140> Not yet assigned
<141> Herewith

<150> 60/923,512
<151> 2004-04-13

<160> 250

<170> FastSEQ for Windows Version 4.0

<210> 1
<211> 466
<212> PRT
<213> Homo sapiens

<220>
<223> Factor VII precursor isoform a

<400> 1

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
```

```
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 2
<211> 444
<212> PRT
<213> Homo sapiens

<220>
<223> Factor VII precursor isoform b

<400> 2

```
Met Val Ser Gln Ala Leu Arg Leu Leu Cys Leu Leu Leu Gly Leu Gln
1               5                   10                  15
Gly Cys Leu Ala Ala Val Phe Val Thr Gln Glu Glu Ala His Gly Val
            20                  25                  30
```

Leu His Arg Arg Arg Arg Ala Asn Ala Phe Leu Glu Glu Leu Arg Pro
35 40 45
Gly Ser Leu Glu Arg Glu Cys Lys Glu Glu Gln Cys Ser Phe Glu Glu
50 55 60
Ala Arg Glu Ile Phe Lys Asp Ala Glu Arg Thr Lys Leu Phe Trp Ile
65 70 75 80
Ser Tyr Ser Asp Gly Asp Gln Cys Ala Ser Ser Pro Cys Gln Asn Gly
85 90 95
Gly Ser Cys Lys Asp Gln Leu Gln Ser Tyr Ile Cys Phe Cys Leu Pro
100 105 110
Ala Phe Glu Gly Arg Asn Cys Glu Thr His Lys Asp Asp Gln Leu Ile
115 120 125
Cys Val Asn Glu Asn Gly Gly Cys Glu Gln Tyr Cys Ser Asp His Thr
130 135 140
Gly Thr Lys Arg Ser Cys Arg Cys His Glu Gly Tyr Ser Leu Leu Ala
145 150 155 160
Asp Gly Val Ser Cys Thr Pro Thr Val Glu Tyr Pro Cys Gly Lys Ile
165 170 175
Pro Ile Leu Glu Lys Arg Asn Ala Ser Lys Pro Gln Gly Arg Ile Val
180 185 190
Gly Gly Lys Val Cys Pro Lys Gly Glu Cys Pro Trp Gln Val Leu Leu
195 200 205
Leu Val Asn Gly Ala Gln Leu Cys Gly Gly Thr Leu Ile Asn Thr Ile
210 215 220
Trp Val Val Ser Ala Ala His Cys Phe Asp Lys Ile Lys Asn Trp Arg
225 230 235 240
Asn Leu Ile Ala Val Leu Gly Glu His Asp Leu Ser Glu His Asp Gly
245 250 255
Asp Glu Gln Ser Arg Arg Val Ala Gln Val Ile Ile Pro Ser Thr Tyr
260 265 270
Val Pro Gly Thr Thr Asn His Asp Ile Ala Leu Leu Arg Leu His Gln
275 280 285
Pro Val Val Leu Thr Asp His Val Val Pro Leu Cys Leu Pro Glu Arg
290 295 300
Thr Phe Ser Glu Arg Thr Leu Ala Phe Val Arg Phe Ser Leu Val Ser
305 310 315 320
Gly Trp Gly Gln Leu Leu Asp Arg Gly Ala Thr Ala Leu Glu Leu Met
325 330 335
Val Leu Asn Val Pro Arg Leu Met Thr Gln Asp Cys Leu Gln Gln Ser
340 345 350
Arg Lys Val Gly Asp Ser Pro Asn Ile Thr Glu Tyr Met Phe Cys Ala
355 360 365
Gly Tyr Ser Asp Gly Ser Lys Asp Ser Cys Lys Gly Asp Ser Gly Gly
370 375 380
Pro His Ala Thr His Tyr Arg Gly Thr Trp Tyr Leu Thr Gly Ile Val
385 390 395 400
Ser Trp Gly Gln Gly Cys Ala Thr Val Gly His Phe Gly Val Tyr Thr
405 410 415
Arg Val Ser Gln Tyr Ile Glu Trp Leu Gln Lys Leu Met Arg Ser Glu
420 425 430
Pro Arg Pro Gly Val Leu Leu Arg Ala Pro Phe Pro
435 440

<210> 3
<211> 406
<212> PRT
<213> Homo sapiens

<220>
<223> Factor VIIa

<400> 3

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile

    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 4
<211> 447
<212> PRT
<213> Bos taurus

<220>
<223> Factor VII precursor

<400> 4

```
Met Leu Ser Gln Ala Trp Ala Leu Ala Leu Leu Cys Phe Leu Leu Ser
1               5                   10                  15
Leu Trp Gly Ser Leu Pro Ala Val Phe Leu Pro Gln Glu Gln Ala Leu
            20                  25                  30
Ser Ile Leu His Arg Pro Arg Arg Ala Asn Gly Phe Leu Glu Glu Leu
        35                  40                  45
Leu Pro Gly Ser Leu Glu Arg Glu Cys Arg Glu Glu Leu Cys Ser Phe
    50                  55                  60
Glu Glu Ala His Glu Ile Phe Arg Asn Glu Glu Arg Thr Arg Gln Phe
65                  70                  75                  80
Trp Val Ser Tyr Asn Asp Gly Asp Gln Cys Ala Ser Ser Pro Cys Gln
                85                  90                  95
Asn Gly Gly Ser Cys Glu Asp Gln Leu Arg Ser Tyr Ile Cys Phe Cys
            100                 105                 110
Pro Asp Gly Phe Glu Gly Arg Asn Cys Glu Thr Asp Lys Gln Ser Gln
        115                 120                 125
Leu Ile Cys Ala Asn Asp Asn Gly Gly Cys Glu Gln Tyr Cys Gly Ala
    130                 135                 140
Asp Pro Gly Ala Gly Arg Phe Cys Trp Cys His Glu Gly Tyr Ala Leu
145                 150                 155                 160
Gln Ala Asp Gly Val Ser Cys Ala Pro Thr Val Glu Tyr Pro Cys Gly
                165                 170                 175
Lys Ile Pro Val Leu Glu Lys Arg Asn Gly Ser Lys Pro Gln Gly Arg
            180                 185                 190
Ile Val Gly Gly His Val Cys Pro Lys Gly Glu Cys Pro Trp Gln Ala
        195                 200                 205
Met Leu Lys Leu Asn Gly Ala Leu Leu Cys Gly Gly Thr Leu Val Gly
    210                 215                 220
Pro Ala Trp Val Val Ser Ala Ala His Cys Phe Glu Arg Leu Arg Ser
225                 230                 235                 240
Arg Gly Asn Leu Thr Ala Val Leu Gly Glu His Asp Leu Ser Arg Val

                245                 250                 255
Glu Gly Pro Glu Gln Glu Arg Arg Val Ala Gln Ile Ile Val Pro Lys
            260                 265                 270
Gln Tyr Val Pro Gly Gln Thr Asp His Asp Val Ala Leu Leu Gln Leu
        275                 280                 285
Ala Gln Pro Val Ala Leu Gly Asp His Val Ala Pro Leu Cys Leu Pro
    290                 295                 300
```

Asp Pro Asp Phe Ala Asp Gln Thr Leu Ala Phe Val Arg Phe Ser Ala
305                 310                 315                 320
Val Ser Gly Trp Gly Gln Leu Leu Glu Arg Gly Val Thr Ala Arg Lys
                325                 330                 335
Leu Met Val Val Leu Val Pro Arg Leu Leu Thr Gln Asp Cys Leu Gln
            340                 345                 350
Gln Ser Arg Gln Arg Pro Gly Gly Pro Val Val Thr Asp Asn Met Phe
        355                 360                 365
Cys Ala Gly Tyr Ser Asp Gly Ser Lys Asp Ala Cys Lys Gly Asp Ser
    370                 375                 380
Gly Gly Pro His Ala Thr Arg Phe Arg Gly Thr Trp Phe Leu Thr Gly
385                 390                 395                 400
Val Val Ser Trp Gly Glu Gly Cys Ala Ala Ala Gly His Phe Gly Ile
                405                 410                 415
Tyr Thr Arg Val Ser Arg Tyr Thr Ala Trp Leu Arg Gln Leu Met Gly
            420                 425                 430
His Pro Pro Ser Arg Gln Gly Phe Phe Gln Val Pro Leu Leu Pro
        435                 440                 445

<210> 5
<211> 446
<212> PRT
<213> Mus musculus

<220>
<223> Factor VII precursor

<400> 5

Met Val Pro Gln Ala His Gly Leu Leu Leu Leu Cys Phe Leu Leu Gln
1               5                   10                  15
Leu Gln Gly Pro Leu Gly Thr Ala Val Phe Ile Thr Gln Glu Glu Ala
            20                  25                  30
His Gly Val Leu His Arg Gln Arg Arg Ala Asn Ser Leu Leu Glu Glu
        35                  40                  45
Leu Trp Pro Gly Ser Leu Glu Arg Glu Cys Asn Glu Glu Gln Cys Ser
    50                  55                  60
Phe Glu Glu Ala Arg Glu Ile Phe Lys Ser Pro Glu Arg Thr Lys Gln
65                  70                  75                  80
Phe Trp Ile Val Tyr Ser Asp Gly Asp Gln Cys Ala Ser Asn Pro Cys
                85                  90                  95
Gln Asn Gly Gly Thr Cys Gln Asp His Leu Lys Ser Tyr Val Cys Phe
            100                 105                 110
Cys Leu Leu Asp Phe Glu Gly Arg Asn Cys Glu Lys Ser Lys Asn Glu
        115                 120                 125
Gln Leu Ile Cys Ala Asn Glu Asn Gly Asp Cys Asp Gln Tyr Cys Arg
    130                 135                 140
Asp His Val Gly Thr Lys Arg Thr Cys Ser Cys His Glu Asp Tyr Thr
145                 150                 155                 160
Leu Gln Pro Asp Glu Val Ser Cys Lys Pro Lys Val Glu Tyr Pro Cys
                165                 170                 175
Gly Arg Ile Pro Val Val Glu Lys Arg Asn Ser Ser Ser Arg Gln Gly
            180                 185                 190
Arg Ile Val Gly Gly Asn Val Cys Pro Lys Gly Glu Cys Pro Trp Gln
        195                 200                 205

Ala Val Leu Lys Ile Asn Gly Leu Leu Leu Cys Gly Ala Val Leu Leu
210 215 220
Asp Ala Arg Trp Ile Val Thr Ala Ala His Cys Phe Asp Asn Ile Arg
225 230 235 240
Tyr Trp Gly Asn Ile Thr Val Val Met Gly Glu His Asp Phe Ser Glu
245 250 255
Lys Asp Gly Asp Glu Gln Val Arg Arg Val Thr Gln Val Ile Met Pro
260 265 270
Asp Lys Tyr Ile Arg Gly Lys Ile Asn His Asp Ile Ala Leu Leu Arg
275 280 285
Leu His Arg Pro Val Thr Phe Thr Asp Tyr Val Val Pro Leu Cys Leu
290 295 300
Pro Glu Lys Ser Phe Ser Glu Asn Thr Leu Ala Arg Ile Arg Phe Ser
305 310 315 320
Arg Val Ser Gly Trp Gly Gln Leu Leu Asp Arg Gly Ala Thr Ala Leu
325 330 335
Glu Leu Met Ser Ile Glu Val Pro Arg Leu Met Thr Gln Asp Cys Leu
340 345 350
Glu His Ala Lys His Ser Ser Asn Thr Pro Lys Ile Thr Glu Asn Met
355 360 365
Phe Cys Ala Gly Tyr Met Asp Gly Thr Lys Asp Ala Cys Lys Gly Asp
370 375 380
Ser Gly Gly Pro His Ala Thr His Tyr His Gly Thr Trp Tyr Leu Thr
385 390 395 400
Gly Val Val Ser Trp Gly Glu Gly Cys Ala Ala Ile Gly His Ile Gly
405 410 415
Val Tyr Thr Arg Val Ser Gln Tyr Ile Asp Trp Leu Val Arg His Met
420 425 430
Asp Ser Lys Leu Gln Val Gly Val Phe Arg Leu Pro Leu Leu
435 440 445

<210> 6
<211> 466
<212> PRT
<213> Pan paniscus

<220>
<223> Factor VII precursor

<220>
<221> UNSURE
<222> (0) ... (0)
<223> Xaa could be any amino acid

<400> 6

Met Val Ser Gln Ala Leu Arg Leu Leu Cys Leu Leu Leu Gly Leu Gln
1 5 10 15
Gly Cys Leu Ala Ala Gly Gly Val Ala Glu Ala Ser Gly Gly Glu Thr
20 25 30
Arg Asp Met Pro Trp Lys Pro Gly Pro His Arg Val Phe Ile Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu

```
65                  70                  75                  80
Gln Cys Ser Phe Glu Glu Ala Arg Glu Ile Phe Lys Asp Leu Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr Tyr
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Asn Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Xaa Leu Leu Xaa Val Asn Gly Ala Gln Leu Cys Gly Gly
225                 230                 235                 240
Thr Leu Ile Asn Thr Ile Trp Val Ala Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Ile Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Ala Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Ser Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 7
<211> 466
<212> PRT
<213> Pan troglodytes

<220>
<223> Factor VII precursor

<220>

<221> UNSURE
<222> (0)...(0)
<223> Xaa could be any amino acid

<400> 7

```
Met Val Ser Gln Ala Leu Arg Leu Leu Cys Leu Leu Leu Gly Leu Gln
1               5                   10                  15
Gly Cys Leu Ala Ala Gly Gly Val Ala Glu Ala Ser Gly Gly Glu Thr
            20                  25                  30
Arg Asp Xaa Xaa Trp Lys Pro Gly Pro His Arg Val Phe Ile Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80

Gln Cys Ser Phe Glu Glu Ala Arg Glu Ile Phe Lys Asp Leu Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr Tyr
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Ile Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Ala Phe Ser Glu Arg Thr Leu Ala Phe Val
                325                 330                 335
```

Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Ser Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 8
<211> 444
<212> PRT
<213> Oryctolagus cuniculus

<220>
<223> Factor VII precursor

<400> 8

Met Ala Pro Gln Ala Arg Gly Leu Gly Leu Cys Ser Leu Leu Ala Leu
1 5 10 15
Gln Ala Ser Leu Ala Ala Val Phe Ile Thr Gln Glu Glu Ala His Ser
20 25 30
Val Leu Arg Arg Gln Arg Arg Ala Asn Ser Phe Leu Glu Glu Leu Arg
35 40 45
Pro Gly Ser Leu Glu Arg Glu Cys Lys Glu Glu Leu Cys Ser Phe Glu
50 55 60
Glu Ala Arg Glu Val Phe Gln Ser Thr Glu Arg Thr Lys Gln Phe Trp
65 70 75 80
Ile Thr Tyr Asn Asp Gly Asp Gln Cys Ala Ser Asn Pro Cys Gln Asn
85 90 95
Gly Gly Ser Cys Glu Asp Gln Ile Gln Ser Tyr Ile Cys Phe Cys Leu
100 105 110
Ala Asp Phe Glu Gly Arg Asn Cys Glu Lys Asn Lys Asn Asp Gln Leu
115 120 125
Ile Cys Met Tyr Glu Asn Gly Gly Cys Glu Gln Tyr Cys Ser Asp His
130 135 140
Val Gly Ser Gln Arg Ser Cys Arg Cys His Glu Gly Tyr Thr Leu Leu
145 150 155 160
Pro Asn Gly Val Ser Cys Thr Pro Thr Val Asp Tyr Pro Cys Gly Lys
165 170 175
Val Pro Ala Leu Glu Lys Arg Gly Ala Ser Asn Pro Gln Gly Arg Ile
180 185 190
Val Gly Gly Lys Val Cys Pro Lys Gly Glu Cys Pro Trp Gln Ala Ala

```
        195                 200                 205
Leu Met Asn Gly Ser Thr Leu Leu Cys Gly Gly Ser Leu Leu Asp Thr
    210                 215                 220
His Trp Val Val Ser Ala Ala His Cys Phe Asp Lys Leu Ser Ser Leu
225                 230                 235                 240
Arg Asn Leu Thr Ile Val Leu Gly Glu His Asp Leu Ser Glu His Glu
                245                 250                 255
Gly Asp Glu Gln Val Arg His Val Ala Gln Leu Ile Met Pro Asp Lys
            260                 265                 270
Tyr Val Pro Gly Lys Thr Asp His Asp Ile Ala Leu Leu Arg Leu Leu
        275                 280                 285
Gln Pro Ala Ala Leu Thr Asn Asn Val Val Pro Leu Cys Leu Pro Glu
    290                 295                 300
Arg Asn Phe Ser Glu Ser Thr Leu Ala Thr Ile Arg Phe Ser Arg Val
305                 310                 315                 320
Ser Gly Trp Gly Gln Leu Leu Tyr Arg Gly Ala Leu Ala Arg Glu Leu
                325                 330                 335
Met Ala Ile Asp Val Pro Arg Leu Met Thr Gln Asp Cys Val Glu Gln
            340                 345                 350
Ser Glu His Lys Pro Gly Ser Pro Glu Val Thr Gly Asn Met Phe Cys
        355                 360                 365
Ala Gly Tyr Leu Asp Gly Ser Lys Asp Ala Cys Lys Gly Asp Ser Gly
    370                 375                 380
Gly Pro His Ala Thr Ser Tyr His Gly Thr Trp Tyr Leu Thr Gly Val
385                 390                 395                 400
Val Ser Trp Gly Glu Gly Cys Ala Ala Val Gly His Val Gly Val Tyr
                405                 410                 415
Thr Arg Val Ser Arg Tyr Thr Glu Trp Leu Ser Arg Leu Met Arg Ser
            420                 425                 430
Lys Leu His His Gly Ile Gln Arg His Pro Phe Pro
        435                 440
```

<210> 9
<211> 446
<212> PRT
<213> Rattus norvegicus

<220>
<223> Factor VII precursor

<400> 9

```
Met Val Pro Gln Thr His Gly Leu Leu Leu Leu Tyr Phe Leu Leu Gln
 1              5                   10                  15
Leu Gln Gly Pro Leu Gly Ala Val Val Phe Ile Thr Gln Glu Glu Ala
            20                  25                  30
His Gly Val Leu His Arg Gln Arg Arg Ala Asn Ser Leu Leu Glu Glu
        35                  40                  45
Leu Trp Ser Ser Ser Leu Glu Arg Glu Cys Asn Glu Glu Arg Cys Ser
    50                  55                  60
Phe Glu Glu Ala Arg Glu Ile Phe Lys Ser Pro Glu Arg Thr Lys Gln
65                  70                  75                  80
Phe Trp Thr Ile Tyr Ser Asp Gly Asp Gln Cys Ala Ser Asn Pro Cys
                85                  90                  95
```

Gln Asn Gly Gly Thr Cys Gln Asp His Leu Lys Ser Tyr Val Cys Phe
100 105 110
Cys Pro Leu Asp Phe Glu Gly Arg Asn Cys Glu Lys Asn Lys Asn Glu
115 120 125
Gln Leu Ile Cys Ala Asn Glu Asn Gly Asp Cys Asp Gln Tyr Cys Arg
130 135 140
Asp His Val Gly Thr Lys Arg Thr Cys Ser Cys His Glu Asp Tyr Val
145 150 155 160
Leu Gln Pro Asp Glu Val Ser Cys Lys Pro Lys Val Glu Tyr Pro Cys
165 170 175
Gly Arg Ile Pro Val Val Glu Lys Arg Asn Phe Ser Arg Pro Gln Gly
180 185 190
Arg Ile Val Gly Gly Tyr Val Cys Pro Lys Gly Glu Cys Pro Trp Gln
195 200 205
Ala Val Leu Lys Phe Asn Glu Ala Leu Leu Cys Gly Ala Val Leu Leu
210 215 220
Asp Thr Arg Trp Ile Val Thr Ala Ala His Cys Phe Asp Lys Phe Gly
225 230 235 240
Lys Leu Val Asn Ile Thr Val Val Leu Gly Glu His Asp Phe Ser Glu
245 250 255
Lys Glu Gly Thr Glu Gln Val Arg Leu Val Glu Gln Val Ile Met Pro
260 265 270
Asn Lys Tyr Thr Arg Gly Arg Thr Asp His Asp Ile Ala Leu Val Arg
275 280 285
Leu His Arg Pro Val Thr Phe Thr Asp Tyr Val Val Pro Leu Cys Leu
290 295 300
Pro Glu Arg Ala Phe Ser Glu Asn Thr Leu Ala Ser Ile Arg Phe Ser
305 310 315 320
Arg Val Ser Gly Trp Gly Gln Leu Leu Asp Arg Gly Ala Thr Ala Leu
325 330 335
Glu Leu Met Val Ile Glu Val Pro Arg Leu Met Thr Gln Asp Cys Leu
340 345 350
Glu His Ala Lys His Ser Ala Asn Thr Pro Arg Ile Thr Glu Asn Met
355 360 365
Phe Cys Ala Gly Tyr Met Asp Gly Thr Lys Asp Ala Cys Lys Gly Asp
370 375 380
Ser Gly Gly Pro His Ala Thr His Tyr His Gly Thr Trp Tyr Leu Thr
385 390 395 400
Gly Val Val Ser Trp Gly Glu Gly Cys Ala Ala Ile Gly His Ile Gly
405 410 415
Val Tyr Thr Arg Val Ser Gln Tyr Ile Asp Trp Leu Val Lys Tyr Met
420 425 430
Asp Ser Lys Leu Arg Val Gly Ile Ser Arg Val Ser Leu Leu
435 440 445

<210> 10
<211> 469
<212> PRT
<213> Macaca mulatta

<220>
<223> Factor VII precursor

<400> 10

Met Val Ser Arg Ala Leu Gly Leu Leu Cys Leu Leu Leu Gly Leu Gln
1 5 10 15
Gly Cys Leu Ala Ala Ala Thr Phe Leu Pro Gln Ala Gly Ser Leu Arg
20 25 30
Pro Gln Glu Glu Lys Thr Gln Asp Leu Leu Trp Lys Pro Gly Pro His
35 40 45
Arg Val Phe Val Thr Gln Glu Glu Ala His Gly Val Leu His Arg Gln
50 55 60
Arg Arg Ala Asn Ser Phe Leu Glu Glu Leu Arg Pro Gly Ser Leu Glu
65 70 75 80
Arg Glu Cys Lys Glu Glu Gln Cys Ser Phe Glu Glu Ala Arg Glu Ile
85 90 95
Phe Lys Asp Leu Glu Arg Thr Lys Leu Phe Trp Ile Ser Tyr Ser Asp
100 105 110
Gly Asp Gln Cys Ala Ser Asn Pro Cys Gln Asn Gly Gly Ser Cys Lys
115 120 125
Asp Gln Leu Gln Ser Tyr Ile Cys Phe Cys Leu Pro Ser Phe Glu Gly
130 135 140
Arg Asn Tyr Lys Asp Asp Gln Leu Ile Cys Val Asn Glu Asn Gly Gly
145 150 155 160
Cys Glu Gln Tyr Cys Ser Asp His Ala Gly Ala Lys Arg Ser Cys Trp
165 170 175
Cys His Glu Gly Tyr Ser Leu Leu Ala Asp Gly Val Ser Cys Met Pro
180 185 190
Thr Val Glu Tyr Pro Cys Gly Lys Ile Pro Ile Leu Glu Lys Arg Asn
195 200 205
Ala Ser Lys Pro Gln Gly Arg Ile Val Gly Gly Arg Val Cys Pro Lys
210 215 220
Gly Glu Cys Pro Trp Gln Val Leu Leu Leu Val Asn Gly Ala Gln Leu
225 230 235 240
Cys Gly Gly Thr Leu Ile Asn Thr Ile Trp Val Val Ser Ala Ala His
245 250 255
Cys Phe Asp Lys Ile Lys Ser Trp Arg Asn Leu Thr Ala Val Leu Gly
260 265 270
Glu His Asp Leu Ser Glu His Glu Gly Asp Glu Gln Ser Arg Arg Val
275 280 285
Ala Gln Val Ile Ile Pro Ser Thr Tyr Val Leu Gly Ala Thr Asn His
290 295 300
Asp Ile Ala Leu Leu Arg Leu Gln Gln Pro Val Val Leu Thr Asp His
305 310 315 320
Val Val Pro Leu Cys Leu Pro Glu Arg Met Phe Ser Glu Arg Thr Leu
325 330 335
Ala Phe Val Arg Phe Ser Leu Val Ser Gly Trp Gly Gln Leu Leu Asp
340 345 350
Arg Gly Ala Thr Ala Leu Glu Leu Met Ala Leu Asn Val Pro Arg Leu
355 360 365
Met Thr Gln Asp Cys Leu Gln Gln Ser Gln Lys Ala Glu Ala Ser Pro
370 375 380
Asn Ile Thr Glu Tyr Met Phe Cys Ala Gly Tyr Ser Asp Gly Ser Arg
385 390 395 400
Asp Ser Cys Lys Gly Asp Ser Gly Gly Pro His Ala Thr Arg Tyr Arg
405 410 415
Gly Thr Trp Tyr Leu Thr Gly Ile Val Ser Trp Gly Gln Gly Cys Ala
420 425 430
Ala Val Gly His Phe Gly Val Tyr Thr Arg Val Ser Gln Tyr Ile Glu

435 440 445
Trp Leu Gln Lys Leu Met His Ser Glu Pro Arg Pro Gly Val Leu Leu
450 455 460
Arg Ala Pro Phe Pro
465

<210> 11
<211> 445
<212> PRT
<213> Sus scrofa

<220>
<223> Factor VII precursor (isoform b)

<400> 11

```
Met Ala Ser Leu Arg Pro Ala Leu Leu Cys Leu Leu Leu Cys Leu Gln
 1               5                  10                  15
Gly Ser Leu Ala Ala Val Phe Val Gly His Glu Glu Ala His Ser Leu
            20                  25                  30
Leu His Arg Phe Arg Arg Ala Asn Ser Phe Leu Glu Glu Leu Trp Pro
        35                  40                  45
Gly Ser Leu Glu Arg Glu Cys Arg Glu Glu Gln Cys Ser Phe Glu Glu
    50                  55                  60
Ala Arg Glu Ile Phe Lys Ser Glu Glu Arg Thr Arg Gln Phe Trp Val
65                  70                  75                  80
Ser Tyr Asn Asp Gly Asp Gln Cys Ala Ser Asn Pro Cys Leu Asn Gly
                85                  90                  95
Gly Ser Cys Glu Asp Gln Leu Gln Ala Tyr Ile Cys Phe Cys Pro Glu
            100                 105                 110
Gly Phe Glu Gly Arg Asn Cys Glu Thr Asn Lys Lys Ser Gln Leu Ile
        115                 120                 125
Cys Met Asn Asp Asn Gly Gly Cys Glu Gln Tyr Cys Ser Asp His Ala
    130                 135                 140
Glu Ala Gly Arg Ser Cys Trp Cys His Glu Gly Tyr Ala Leu Gln Glu
145                 150                 155                 160
Asp Gly Val Ser Cys Glu Pro Thr Val Glu Tyr Pro Cys Gly Lys Ile
                165                 170                 175
Pro Val Leu Glu Lys Arg Asn Asp Ser Asn Pro Gln Gly Arg Ile Val
            180                 185                 190
Gly Gly Lys Val Cys Pro Lys Gly Glu Cys Pro Trp Gln Ala Met Leu
        195                 200                 205
Lys Leu Lys Gly Ala Leu Leu Cys Gly Gly Thr Leu Leu Asn Thr Ser
    210                 215                 220
Trp Val Val Ser Ala Ala His Cys Phe Asp Arg Ile Arg Ser Trp Lys
225                 230                 235                 240
Asp Leu Thr Val Val Leu Gly Glu His Asp Leu Ser Lys Asp Glu Gly
                245                 250                 255
Asp Glu Gln Glu Arg Pro Val Ala Gln Val Phe Val Pro Asp Lys Tyr
            260                 265                 270
Val Pro Gly Lys Thr Asp His Asp Leu Ala Leu Val Arg Leu Ala Arg
        275                 280                 285
Pro Val Ala Leu Thr Asp His Val Val Pro Leu Cys Leu Pro Glu Arg
    290                 295                 300
Ser Phe Ser Glu Arg Thr Leu Ala Phe Ile Arg Phe Ser Ala Val Ser
```

```
305                 310                 315                 320
Gly Trp Gly Arg Leu Leu Asp Arg Gly Ala Lys Ala Arg Val Leu Met
                325                 330                 335
Ala Ile Gln Val Pro Arg Leu Met Thr Gln Asp Cys Leu Glu Gln Ala
            340                 345                 350
Arg Arg Arg Pro Gly Ser Pro Ser Ile Thr Asp Asn Met Phe Cys Ala
        355                 360                 365
Gly Tyr Leu Asp Gly Ser Lys Asp Ala Cys Lys Gly Asp Ser Gly Gly
    370                 375                 380
Pro His Ala Thr Arg Phe Arg Gly Thr Trp Phe Leu Thr Gly Val Val
385                 390                 395                 400
Ser Trp Gly Glu Gly Cys Ala Ala Thr Gly Arg Phe Gly Val Tyr Thr
                405                 410                 415
Arg Val Ser Arg Tyr Thr Ala Trp Leu Leu Gly Leu Met Ser Ala Pro
            420                 425                 430
Pro Pro Pro Ser Glu Gly Leu Leu Arg Ala Pro Leu Pro
        435                 440                 445
```

<210> 12
<211> 446
<212> PRT
<213> Canis familiaris

<220>
<223> Factor VII precursor

<400> 12

```
Met Val Ala Trp Ala Gly Glu Leu Ala Leu Leu Cys Phe Leu Leu Gly
1               5                   10                  15
Leu Gln Gly Ser Leu Ala Ala Val Phe Leu Thr Gln Glu Glu Ala Gln
            20                  25                  30
Gly Val Leu His Arg Gln Arg Arg Ala Asn Ser Phe Leu Glu Glu Leu
        35                  40                  45
Arg Ala Gly Ser Leu Glu Arg Glu Cys Arg Glu Glu Gln Cys Ser Phe
    50                  55                  60
Glu Glu Ala Arg Glu Ile Phe Gln Asp Val Asp Arg Thr Arg Gln Phe
65                  70                  75                  80
Trp Ile Ser Tyr Lys Asp Gly Asp Gln Cys Ala Ser Asn Pro Cys Gln
                85                  90                  95
Asn Gly Gly Ser Cys Glu Asp Gln Leu Gln Ser Tyr Ile Cys Phe Cys
            100                 105                 110
Pro Asp Asp Phe Gln Gly Arg Asn Cys Glu Thr Asp Lys Lys Asp Gln
        115                 120                 125
Leu Ile Cys Met Asn Glu Asn Gly Gly Cys Gln Gln Tyr Cys Ser Asp
    130                 135                 140
His Ala Glu Ala Arg Arg Ser Cys Trp Cys His Glu Gly Tyr Thr Leu
145                 150                 155                 160
Gln Asp Asp Gly Val Ser Cys Met Pro Ile Val Glu Tyr Pro Cys Gly
                165                 170                 175
Lys Ile Pro Val Leu Glu Lys Arg Ile Gly Ser Asn Pro Gln Gly Arg
            180                 185                 190
Ile Val Gly Gly Lys Val Cys Pro Lys Gly Glu Cys Pro Trp Gln Ala
        195                 200                 205
Ala Val Lys Val Asp Gly Lys Leu Leu Cys Gly Gly Thr Leu Ile Asp
```

```
    210                 215                 220
Ala Ala Trp Val Val Ser Ala Ala His Cys Phe Glu Arg Ile Lys Asn
225                 230                 235                 240
Trp Lys Asn Leu Thr Val Val Leu Gly Glu His Asp Leu Ser Glu Asp
                245                 250                 255
Asp Gly Asp Glu Gln Glu Arg His Val Ala Arg Val Ile Val Pro Asp
            260                 265                 270
Lys Tyr Ile Pro Leu Lys Thr Asn His Asp Ile Ala Leu Leu His Leu
        275                 280                 285
Arg Thr Pro Val Ala Tyr Thr Asp His Val Val Pro Leu Cys Leu Pro
    290                 295                 300
Glu Lys Thr Phe Ser Glu Arg Thr Leu Ala Phe Ile Arg Phe Ser Thr
305                 310                 315                 320
Val Ser Gly Trp Gly Gln Leu Leu Asp Arg Gly Ala Thr Ala Leu Gln
                325                 330                 335
Leu Met Ala Ile Asp Val Pro Arg Val Met Thr Gln Asp Cys Gln Glu
            340                 345                 350
Gln Ser Arg Arg Arg Ser Gly Ser Pro Ala Ile Thr Glu Asn Met Phe
        355                 360                 365
Cys Ala Gly Tyr Leu Asp Gly Ser Lys Asp Ala Cys Gln Gly Asp Ser
    370                 375                 380
Gly Gly Pro His Ala Thr Lys Phe Gln Gly Thr Trp Tyr Leu Thr Gly
385                 390                 395                 400
Val Val Ser Trp Gly Glu Gly Cys Ala Ala Glu Gly His Phe Gly Val
                405                 410                 415
Tyr Thr Arg Val Ser Gln Tyr Ile Glu Trp Leu Arg Gln Leu Met Val
            420                 425                 430
Ser Ser His Thr Leu Arg Gly Leu Leu Arg Ala Pro Leu Pro
        435                 440                 445
```

<210> 13
<211> 433
<212> PRT
<213> Brachydanio rerio

<220>
<223> Factor VII precursor

<400> 13

```
Met Ser Leu Leu Leu Val Phe Ser Val Leu Trp Ser Leu His Tyr Cys
 1               5                  10                  15
His Ser Ala Ala Val Phe Val His Arg Asp Glu Ala His Glu Val Leu
            20                  25                  30
Ile Arg Ser Lys Arg Ala Asn Ser Gly Trp Phe Glu Glu Leu Lys Thr
        35                  40                  45
Gly Asn Leu Glu Arg Glu Cys Leu Glu Glu Lys Cys Ser Tyr Glu Gly
    50                  55                  60
Ala Arg Glu Val Phe Glu His Thr Glu Ala Thr Asn Glu Phe Trp Lys
65                  70                  75                  80
Ile Tyr Asp Val Lys Asp His Cys Ala Ser Ser Pro Cys Glu His Asp
                85                  90                  95
Gly Leu Cys Thr Thr Gln Asn Ala Asp Ser Tyr Met Cys Leu Cys Ala
            100                 105                 110
Pro Gly Phe Ser Gly Arg His Cys Glu Gln Ser Ile Gly Asp Val Pro
```

```
        115                 120                 125
Asp Ser Cys Leu His Asp Asn Gly Gly Cys Glu His Phe Cys Thr Glu
    130                 135                 140
Gln Asp Gly Arg Arg Asn Cys Ser Cys Ala Asp Gly Tyr Tyr Leu Asp
145                 150                 155                 160
Asn Gly Gly Gln Lys Cys Arg Ser His Glu Val Phe Pro Cys Gly Lys
                165                 170                 175
Val Pro Leu Leu Gln Ala Gly Lys Ala Ala Asp His Gln Val Asp Leu
            180                 185                 190
Arg Ser Arg Ile Val Gly Gly Ser Glu Cys Pro Lys Gly His Cys Pro
        195                 200                 205
Trp Gln Val Leu Leu Lys Tyr Gly Glu Lys Gly Phe Cys Gly Gly Val
    210                 215                 220
Ile Tyr Lys Pro Thr Trp Ile Leu Thr Ala Ala His Cys Leu Glu Lys
225                 230                 235                 240
Leu Lys Val Lys Phe Leu Arg Ile Val Ala Gly Glu His Asp Leu Glu
                245                 250                 255
Val Asp Glu Gly Thr Glu Gln Leu Ile Gln Val Asp Gln Met Phe Thr
            260                 265                 270
His Pro Ala Tyr Val Ser Glu Thr Ala Asp Ser Asp Ile Ala Leu Leu
        275                 280                 285
Arg Leu Arg Thr Pro Ile Val Tyr Ser Val Tyr Ala Val Pro Val Cys
    290                 295                 300
Leu Pro Leu Arg Glu Met Ala Glu Arg Glu Leu Trp Ala Val Ser Lys
305                 310                 315                 320
His Thr Val Ser Gly Trp Gly Lys Arg Ser Glu Asp Gly Pro Thr Ser
                325                 330                 335
Arg Leu Leu Arg Arg Leu Leu Val Pro Arg Ile Arg Thr Gln Glu Cys
            340                 345                 350
Val Gln Val Ser Asn Leu Thr Leu Thr Ser Asn Met Phe Cys Ala Gly
        355                 360                 365
Tyr Ile Glu Gly Arg Gln Asp Ser Cys Lys Gly Asp Ser Gly Gly Pro
    370                 375                 380
Leu Val Thr Arg Tyr Arg Asp Thr Ala Phe Leu Leu Gly Ile Val Ser
385                 390                 395                 400
Trp Gly Lys Gly Cys Ala Arg Pro Gly Ser Tyr Gly Ile Tyr Thr Arg
                405                 410                 415
Val Ser Asn Tyr Leu Gln Trp Ile Arg Gln Thr Thr Asn Thr Thr Ile
            420                 425                 430
His
```

<210> 14
<211> 441
<212> PRT
<213> Fugu rubripes

<220>
<223> Factor VII precursor

<400> 14

```
Met Arg Leu Arg Val Phe Phe Thr Leu Val Phe Thr Phe Thr His Cys
1               5                   10                  15
Arg Ala Ala Ser Val Phe Leu Asp Ala Asp Lys Ala His Asp Val Leu
```

```
                20                  25                  30
Val Arg Thr Arg Arg Tyr Asn Ser Gly Trp Leu Glu Glu Leu Gln Lys
            35                  40                  45
Gly Asp Leu Lys Arg Glu Cys Leu Glu Glu Ile Cys Ser Tyr Glu Glu
    50                  55                  60
Ala Arg Glu Val Phe Glu His Thr Lys Thr Thr Asp Glu Phe Trp Lys
65                  70                  75                  80
Ile Tyr Asn Arg Pro Asn Ser Cys Lys Ser Asn Pro Cys Leu Asn Gly
                85                  90                  95
Gly Ser Cys Ser Ala Glu Gly Ser Ser Tyr Thr Cys Phe Cys Leu Pro
            100                 105                 110
Glu Phe Ser Gly Val Asp Cys Glu Leu Glu Tyr Gln Thr Val Pro Asp
        115                 120                 125
Thr Cys Leu Leu Glu Asn Gly Gly Cys Glu His Phe Cys His Glu Asn
    130                 135                 140
Ser Ala Gly Gln Arg Gly Asn Cys Ser Cys Ala Asp Gly Tyr Asp Leu
145                 150                 155                 160
Asp Val Asp Gly Leu Ser Cys Lys Ala Lys Glu Ser Val Ala Cys Gly
                165                 170                 175
Met Val Leu Ser Ala Gln Phe Glu His Asn Gln Leu Asn Pro Arg Ala
            180                 185                 190
Arg Ile Val Gly Gly Asn Glu Cys Pro Lys Gly Glu Cys Pro Trp Gln
        195                 200                 205
Val Leu Leu Val Tyr Lys Gly Lys Gly Phe Cys Gly Gly Val Ile Tyr
    210                 215                 220
Lys Pro Thr Trp Ile Leu Thr Ala Ser His Cys Met Ala Asp Ile Asp
225                 230                 235                 240
Val Gln Phe Leu Lys Val Val Ala Gly Glu His Asn Thr Glu Val Asp
                245                 250                 255
Glu Gly Thr Glu Gln Ile Ile Gln Val Ser Glu Ile Ile Met His Glu
            260                 265                 270
Lys Tyr Val Pro Arg Thr Ala Asp Asn Asp Ile Ala Leu Leu His Leu
        275                 280                 285
Ala Val Pro Ile Thr Tyr Thr Thr Tyr Ala Ile Pro Val Cys Leu Pro
    290                 295                 300
Thr Arg Pro Leu Ala Glu Arg Glu Leu Trp Ala Val Ser Leu His Thr
305                 310                 315                 320
Val Ser Gly Trp Gly Arg Arg Ser Glu Asn Gly Pro Thr Ser His Leu
                325                 330                 335
Leu Arg Gln Leu Lys Val Pro Arg Ile Arg Thr Gln Gln Cys Ile Glu
            340                 345                 350
Glu Ser Gly Val Val Leu Thr Gln Asn Met Phe Cys Ala Gly Tyr Met
        355                 360                 365
Glu Gly Arg Gln Asp Ser Cys Lys Gly Asp Ser Gly Gly Pro Leu Val
    370                 375                 380
Thr Lys Tyr Lys Lys Thr Val Phe Leu Leu Gly Ile Val Ser Trp Gly
385                 390                 395                 400
Lys Gly Cys Ala Arg Pro Gly Asn Tyr Gly Ile Tyr Thr Arg Val Ala
                405                 410                 415
Asn Tyr Leu Glu Trp Ile His Asn Arg Thr Ala Thr Val Asn Gln Pro
            420                 425                 430
Thr Asn Asn Thr Glu Asn Phe Thr Thr
        435                 440
```

<210> 15
<211> 425
<212> PRT
<213> Gallus gallus

<220>
<223> Factor VII precursor

<400> 15

```
Met Val Ser Arg Gln Cys Val Ala Leu Leu Leu Cys Phe Pro Leu Leu
1               5                   10                  15
Val Pro Pro Ser Leu Glu Ala Val Phe Leu Lys Gln Glu Glu Ala Asn
            20                  25                  30
Ser Ile Phe Gln Arg His Arg Arg Ala Asn Ser Phe Phe Glu Glu Ile
        35                  40                  45
Lys Leu Gly Pro Leu Glu Arg Glu Cys Ile Glu Glu Lys Cys Ser Phe
    50                  55                  60
Glu Glu Ala Arg Glu Ile Tyr Arg Asp Asp Glu Arg Thr Lys Glu Phe
65                  70                  75                  80
Trp His Ile Tyr Ser Asp Pro Asn Gln Cys Asp Ser Ser Pro Cys Gln
                85                  90                  95
Asn Gly Gly Ser Cys Asp Asp Gln Phe Gln Asp Tyr Val Cys Arg Cys
            100                 105                 110
Pro Pro Glu Tyr Glu Gly Lys Ser Cys Glu Thr Ala Val Ala Glu Asn
        115                 120                 125
Leu Lys Cys Ile Tyr Asp Asn Gly Gly Cys Glu Gln Tyr Cys Ala Asp
    130                 135                 140
Glu Gln Ser Glu Lys Arg Val Cys Phe Cys Ala Glu Gly Tyr Ala Leu
145                 150                 155                 160
Ala Ser Asp Gly Val Ser Cys Ile Pro Gln Val Lys Tyr Pro Cys Gly
                165                 170                 175
Thr Ile Pro Val Leu Ala Arg Lys Asn Thr Thr Ala Gln Gly Arg Ile
            180                 185                 190
Val Gly Gly Val Thr Cys Pro Pro Gly Glu Cys Pro Trp Gln Ala Leu
        195                 200                 205
Ile Ile Gln Asp Gln Lys Gly Lys Cys Gly Gly Ser Leu Leu Ser Pro
    210                 215                 220
Glu Trp Val Val Thr Ala Ala His Cys Leu Asp Tyr Ala His Ser Lys
225                 230                 235                 240
Gln Leu Arg Val Arg Leu Gly Glu Tyr Ser Val Lys Val Ala Glu Lys
                245                 250                 255
Thr Glu Gln Glu Ser Gly Val Ser Lys Ile Ile Arg His Glu Glu Tyr
            260                 265                 270
Thr Ile Gly Gln Val Asn His Asp Ile Ala Leu Leu Lys Leu Glu Thr
        275                 280                 285
Pro Val Asn Leu Thr Asp Phe Val Val Pro Ile Cys Leu Pro Glu Lys
    290                 295                 300
Arg Phe Ala Val Tyr Glu Leu Ser Ser Ile Lys Phe Ser Met Val Ser
305                 310                 315                 320
Gly Trp Gly Arg Leu Leu Asp Gly Gly Ala Thr Ser Thr Phe Leu Met
                325                 330                 335
Arg Val His Leu Pro Arg Val Lys Thr Gln Glu Cys Glu Lys Gln Ala
            340                 345                 350

Asn Leu Asn Ile Thr Glu Asn Met Phe Cys Ala Gly Asp Leu Thr Gly


        355                 360                 365
Lys Lys Asp Ser Cys Lys Gly Asp Ser Gly Gly Pro His Ala Thr Lys
    370                 375                 380
Tyr Lys Asn Thr Trp Phe Leu Thr Gly Ile Val Ser Trp Gly Lys Gly
385                 390                 395                 400
Cys Ala Val Glu Gly Ser Tyr Gly Val Tyr Thr Arg Val Ser Arg Tyr
                405                 410                 415
Ile Asn Trp Leu Lys Arg His Met Glu
            420                 425
```

<210> 16

<211> 444
<212> PRT
<213> Pongo pygmaeus

<220>
<223> Factor VII (fragment)

<400> 16

```
Gly Val Ala Glu Ala Ser Gly Gly Glu Thr Arg Asp Met Gln Trp Lys
1               5                   10                  15
Leu Gly Pro His Arg Val Phe Ile Thr Gln Glu Glu Ala His Gly Val
            20                  25                  30
Leu His Arg Arg Arg Arg Ala Asn Thr Phe Leu Glu Glu Leu Arg Pro
        35                  40                  45
Gly Ser Leu Glu Arg Glu Cys Lys Glu Glu Gln Cys Ser Phe Glu Glu
    50                  55                  60
Ala Arg Glu Ile Phe Lys Asp Leu Glu Arg Thr Lys Leu Phe Trp Ile
65                  70                  75                  80
Ser Tyr Ser Asp Gly Asp Gln Cys Ala Ser Ser Pro Cys Gln Asn Gly
                85                  90                  95
Gly Ser Cys Lys Asp Gln Leu Gln Ser Tyr Ile Cys Phe Cys Leu Pro
            100                 105                 110
Asp Phe Glu Gly Arg Asn Cys Glu Met Tyr Lys Asp Asp Gln Leu Ile
        115                 120                 125
Cys Val Asn Glu Asn Gly Gly Cys Glu Gln Tyr Cys Ser Asp His Met
    130                 135                 140
Gly Ala Lys Arg Ser Cys Trp Cys His Glu Gly Tyr Ser Leu Leu Ala
145                 150                 155                 160
Asp Gly Val Ser Cys Thr Pro Thr Val Glu Tyr Pro Cys Gly Lys Ile
                165                 170                 175
Pro Ile Leu Glu Lys Arg Asn Ala Ser Lys Pro Gln Gly Arg Ile Val
            180                 185                 190
Gly Gly Lys Val Cys Pro Lys Gly Glu Cys Pro Trp Gln Val Leu Leu
        195                 200                 205
Leu Val Asn Gly Ala Gln Leu Cys Gly Gly Thr Leu Ile Asn Thr Ile
    210                 215                 220
Trp Val Val Ser Ala Ala His Cys Phe Asp Lys Ile Lys Asn Trp Arg
225                 230                 235                 240
Asn Leu Ile Ala Val Leu Gly Glu His Asp Leu Ser Glu Arg Asp Gly
                245                 250                 255
Asp Glu Gln Ser Arg Arg Val Ala Gln Val Ile Ile Pro Ser Thr Tyr
            260                 265                 270
Val Pro Gly Thr Thr Asn His Asp Ile Ala Leu Leu Arg Leu His Gln
```

```
        275                     280                     285
Pro Val Val Leu Thr Asp His Val Val Pro Leu Cys Leu Pro Glu Arg
    290                     295                     300
Thr Phe Ser Glu Arg Thr Leu Ala Phe Val Arg Phe Ser Leu Val Ser
305                 310                     315                 320

Gly Trp Gly Gln Leu Leu Asp Arg Gly Ala Thr Ala Leu Glu Leu Met
                325                     330                 335
Ala Leu Asn Val Pro Arg Leu Met Thr Gln Asp Cys Leu Gln Gln Ser
            340                     345                 350
Arg Lys Val Gly Asp Ser Pro Asn Ile Thr Glu Tyr Met Phe Cys Ala
        355                     360                 365
Gly Tyr Ser Asp Gly Ser Lys Asp Ser Cys Lys Gly Asp Ser Gly Gly
    370                     375                 380
Pro His Ala Thr Arg Tyr Arg Gly Thr Trp Tyr Leu Thr Gly Ile Val
385                 390                     395                 400
Ser Trp Gly Gln Gly Cys Ala Ala Val Gly His Phe Gly Val Tyr Thr
                405                     410                 415
Arg Val Ser Gln Tyr Ile Glu Trp Leu Gln Lys Leu Met Cys Ser Glu
            420                     425                 430
Pro Arg Pro Gly Val Leu Leu Arg Ala Pro Phe Pro
        435                     440
```

<210> 17
<211> 221
<212> PRT
<213> Gorilla gorilla

<220>
<223> Factor VII (fragment)

<220>
<221> UNSURE
<222> (0) ... (0)
<223> Xaa could be any amino acid

<400> 17

```
Ile Trp Val Val Ser Ala Ala His Cys Phe Asp Lys Ile Lys Asn Trp
1               5                   10                  15
Arg Asn Leu Ile Ala Val Leu Gly Glu His Asp Leu Ser Glu His Asp
            20                  25                  30
Gly Asp Glu Gln Ser Arg Arg Val Ala Gln Val Ile Ile Pro Ser Met
        35                  40                  45
Tyr Val Pro Gly Thr Thr Asn His Asp Ile Ala Leu Leu Arg Leu His
    50                  55                  60
Gln Pro Val Val Leu Thr Asp His Val Val Pro Leu Cys Leu Pro Glu
65                  70                  75                  80
Arg Thr Phe Ser Glu Arg Thr Leu Ala Phe Val Arg Phe Ser Leu Val
                85                  90                  95
Ser Gly Trp Gly Gln Leu Leu Asp Arg Gly Ala Thr Ala Leu Glu Leu
            100                 105                 110
Met Val Leu Asn Val Pro Arg Leu Met Thr Gln Asp Cys Leu Gln Gln
        115                 120                 125
Ser Arg Lys Val Gly Asp Ser Pro Asn Ile Thr Glu Tyr Met Phe Cys
```

```
    130                 135                 140
Ala Gly Tyr Ser Asp Gly Ser Lys Asp Ser Cys Lys Gly Asp Ser Gly
145                 150                 155                 160
Gly Pro His Ala Thr His Tyr Arg Gly Thr Trp Xaa Leu Thr Gly Ile
                165                 170                 175
Xaa Ser Trp Gly Gln Gly Cys Ala Thr Val Gly His Phe Gly Val Tyr
            180                 185                 190
Thr Arg Val Ser Gln Tyr Ile Glu Trp Leu Gln Lys Leu Met Arg Ser
        195                 200                 205
Glu Pro Arg Pro Gly Val Leu Leu Arg Ala Pro Phe Pro
    210                 215                 220
```

<210> 18
<211> 406
<212> PRT
<213> Homo sapiens

<220>
<223> factor VIIa variant D60K

<400> 18

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Lys Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
```

```
                260                 265                 270
Leu Ala Phe Val Arg Phe Ser Leu Val Ser Gly Trp Gly Gln Leu Leu
            275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 19
<211> 406
<212> PRT
<213> Homo sapiens

<220>
<223> Factor VIIa variant D60R

<400> 19

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg

    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
```

```
                180                     185                     190
His Cys Phe Arg Lys Ile Lys Asn Trp Arg Asn Leu Ile Ala Val Leu
            195                 200                     205
Gly Glu His Asp Leu Ser Glu His Asp Gly Asp Glu Gln Ser Arg Arg
        210                 215                     220
Val Ala Gln Val Ile Ile Pro Ser Thr Tyr Val Pro Gly Thr Thr Asn
225                 230                     235                 240
His Asp Ile Ala Leu Leu Arg Leu His Gln Pro Val Val Leu Thr Asp
                    245                 250                     255
His Val Val Pro Leu Cys Leu Pro Glu Arg Thr Phe Ser Glu Arg Thr
                260                 265                     270
Leu Ala Phe Val Arg Phe Ser Leu Val Ser Gly Trp Gly Gln Leu Leu
            275                 280                     285
Asp Arg Gly Ala Thr Ala Leu Glu Leu Met Val Leu Asn Val Pro Arg
        290                 295                     300
Leu Met Thr Gln Asp Cys Leu Gln Gln Ser Arg Lys Val Gly Asp Ser
305                 310                     315                 320
Pro Asn Ile Thr Glu Tyr Met Phe Cys Ala Gly Tyr Ser Asp Gly Ser
                    325                 330                     335
Lys Asp Ser Cys Lys Gly Asp Ser Gly Gly Pro His Ala Thr His Tyr
                340                 345                     350
Arg Gly Thr Trp Tyr Leu Thr Gly Ile Val Ser Trp Gly Gln Gly Cys
            355                 360                     365
Ala Thr Val Gly His Phe Gly Val Tyr Thr Arg Val Ser Gln Tyr Ile
        370                 375                     380
Glu Trp Leu Gln Lys Leu Met Arg Ser Glu Pro Arg Pro Gly Val Leu
385                 390                     395                 400
Leu Arg Ala Pro Phe Pro
                    405
```

<210> 20
<211> 406
<212> PRT
<213> Homo sapiens

<220>
<223> Factor VIIa variant D60A

<400> 20

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
                20                  25                  30
Asp Ala Glu Arg Thr Lys Leu Phe Trp Ile Ser Tyr Ser Asp Gly Asp
            35                  40                  45
Gln Cys Ala Ser Ser Pro Cys Gln Asn Gly Gly Ser Cys Lys Asp Gln
        50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
```

115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Ala Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 21
<211> 406
<212> PRT
<213> Homo sapiens

<220>
<223> Factor VIIa variant K60aY

<400> 21

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln

```
        50                      55                      60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Tyr Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 22
<211> 406
<212> PRT
<213> Homo sapiens

<220>
<223> Factor VIIa variant K60aA

<400> 22

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Ala Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 23
<211> 406
<212> PRT
<213> Homo sapiens

<220>
<223> Factor VIIa variant K60aE

<400> 23

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365

Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 24
<211> 406
<212> PRT
<213> Homo sapiens

<220>
<223> Factor VIIa variant K60aD

<400> 24

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Asp Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 25
<211> 406
<212> PRT
<213> Homo sapiens

<220>
<223> Factor VIIa variant K60aL

<400> 25

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Leu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
```

His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 26
<211> 406
<212> PRT
<213> Homo sapiens

<220>
<223> Factor VIIa variant K60aM

<400> 26

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln

```
                    165                     170                     175
Leu Cys Gly Gly Thr Leu Ile Asn Thr Ile Trp Val Val Ser Ala Ala
                180                     185                     190
His Cys Phe Asp Met Ile Lys Asn Trp Arg Asn Leu Ile Ala Val Leu
            195                     200                     205
Gly Glu His Asp Leu Ser Glu His Asp Gly Asp Glu Gln Ser Arg Arg
    210                     215                     220
Val Ala Gln Val Ile Ile Pro Ser Thr Tyr Val Pro Gly Thr Thr Asn
225                     230                     235                     240
His Asp Ile Ala Leu Leu Arg Leu His Gln Pro Val Val Leu Thr Asp
                    245                     250                     255
His Val Val Pro Leu Cys Leu Pro Glu Arg Thr Phe Ser Glu Arg Thr
                260                     265                     270
Leu Ala Phe Val Arg Phe Ser Leu Val Ser Gly Trp Gly Gln Leu Leu
            275                     280                     285
Asp Arg Gly Ala Thr Ala Leu Glu Leu Met Val Leu Asn Val Pro Arg
    290                     295                     300
Leu Met Thr Gln Asp Cys Leu Gln Gln Ser Arg Lys Val Gly Asp Ser
305                     310                     315                     320
Pro Asn Ile Thr Glu Tyr Met Phe Cys Ala Gly Tyr Ser Asp Gly Ser
                    325                     330                     335
Lys Asp Ser Cys Lys Gly Asp Ser Gly Gly Pro His Ala Thr His Tyr
                340                     345                     350
Arg Gly Thr Trp Tyr Leu Thr Gly Ile Val Ser Trp Gly Gln Gly Cys
            355                     360                     365
Ala Thr Val Gly His Phe Gly Val Tyr Thr Arg Val Ser Gln Tyr Ile
    370                     375                     380
Glu Trp Leu Gln Lys Leu Met Arg Ser Glu Pro Arg Pro Gly Val Leu
385                     390                     395                     400
Leu Arg Ala Pro Phe Pro
                    405
```

<210> 27
<211> 406
<212> PRT
<213> Homo sapiens

<220>
<223> Factor VIIa variant K60cA

<400> 27

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
```

```
                100                             105                             110
Arg Cys His Glu Gly Tyr Ser Leu Leu Ala Asp Gly Val Ser Cys Thr
        115                         120                         125
Pro Thr Val Glu Tyr Pro Cys Gly Lys Ile Pro Ile Leu Glu Lys Arg
    130                         135                         140
Asn Ala Ser Lys Pro Gln Gly Arg Ile Val Gly Gly Lys Val Cys Pro
145                         150                         155                     160
Lys Gly Glu Cys Pro Trp Gln Val Leu Leu Leu Val Asn Gly Ala Gln
                    165                         170                         175
Leu Cys Gly Gly Thr Leu Ile Asn Thr Ile Trp Val Val Ser Ala Ala
                180                         185                         190
His Cys Phe Asp Lys Ile Ala Asn Trp Arg Asn Leu Ile Ala Val Leu
        195                         200                         205
Gly Glu His Asp Leu Ser Glu His Asp Gly Asp Glu Gln Ser Arg Arg
    210                         215                         220
Val Ala Gln Val Ile Ile Pro Ser Thr Tyr Val Pro Gly Thr Thr Asn
225                         230                         235                     240
His Asp Ile Ala Leu Leu Arg Leu His Gln Pro Val Val Leu Thr Asp
                    245                         250                         255
His Val Val Pro Leu Cys Leu Pro Glu Arg Thr Phe Ser Glu Arg Thr
                260                         265                         270
Leu Ala Phe Val Arg Phe Ser Leu Val Ser Gly Trp Gly Gln Leu Leu
        275                         280                         285
Asp Arg Gly Ala Thr Ala Leu Glu Leu Met Val Leu Asn Val Pro Arg
    290                         295                         300
Leu Met Thr Gln Asp Cys Leu Gln Gln Ser Arg Lys Val Gly Asp Ser
305                         310                         315                     320
Pro Asn Ile Thr Glu Tyr Met Phe Cys Ala Gly Tyr Ser Asp Gly Ser
                    325                         330                         335
Lys Asp Ser Cys Lys Gly Asp Ser Gly Gly Pro His Ala Thr His Tyr
                340                         345                         350
Arg Gly Thr Trp Tyr Leu Thr Gly Ile Val Ser Trp Gly Gln Gly Cys
        355                         360                         365
Ala Thr Val Gly His Phe Gly Val Tyr Thr Arg Val Ser Gln Tyr Ile
    370                         375                         380
Glu Trp Leu Gln Lys Leu Met Arg Ser Glu Pro Arg Pro Gly Val Leu
385                         390                         395                     400
Leu Arg Ala Pro Phe Pro
                    405
```

<210> 28
<211> 406
<212> PRT
<213> Homo sapiens

<220>
<223> Factor VIIa variant K60cD

<400> 28

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
                20                  25                  30
Asp Ala Glu Arg Thr Lys Leu Phe Trp Ile Ser Tyr Ser Asp Gly Asp
```

```
            35                  40                  45
Gln Cys Ala Ser Ser Pro Cys Gln Asn Gly Gly Ser Cys Lys Asp Gln
        50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Asp Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 29
<211> 406
<212> PRT
<213> Homo sapiens

<220>
<223> Factor VIIa variant K60cE

<400> 29

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Glu Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro

405

<210> 30
<211> 406
<212> PRT
<213> Homo sapiens

<220>
<223> Factor VIIa variant T99A

<400> 30

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Ala Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr

340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 31

<211> 406
<212> PRT
<213> Homo sapiens

<220>
<223> Factor VIIa variant R147A

<400> 31

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
```

```
        275                 280                 285
Asp Ala Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 32
<211> 406
<212> PRT
<213> Homo sapiens

<220>
<223> Factor VIIa variant R147E

<400> 32

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
```

```
    210                     215                     220
Val Ala Gln Val Ile Ile Pro Ser Thr Tyr Val Pro Gly Thr Thr Asn
225                     230                     235                 240
His Asp Ile Ala Leu Leu Arg Leu His Gln Pro Val Val Leu Thr Asp
                245                     250                     255
His Val Val Pro Leu Cys Leu Pro Glu Arg Thr Phe Ser Glu Arg Thr
            260                     265                     270
Leu Ala Phe Val Arg Phe Ser Leu Val Ser Gly Trp Gly Gln Leu Leu
        275                     280                     285
Asp Glu Gly Ala Thr Ala Leu Glu Leu Met Val Leu Asn Val Pro Arg
    290                     295                     300
Leu Met Thr Gln Asp Cys Leu Gln Gln Ser Arg Lys Val Gly Asp Ser
305                     310                     315                 320
Pro Asn Ile Thr Glu Tyr Met Phe Cys Ala Gly Tyr Ser Asp Gly Ser
                325                     330                     335
Lys Asp Ser Cys Lys Gly Asp Ser Gly Gly Pro His Ala Thr His Tyr
            340                     345                     350
Arg Gly Thr Trp Tyr Leu Thr Gly Ile Val Ser Trp Gly Gln Gly Cys
        355                     360                     365
Ala Thr Val Gly His Phe Gly Val Tyr Thr Arg Val Ser Gln Tyr Ile
    370                     375                     380
Glu Trp Leu Gln Lys Leu Met Arg Ser Glu Pro Arg Pro Gly Val Leu
385                     390                     395                 400
Leu Arg Ala Pro Phe Pro
                405
```

<210> 33
<211> 406
<212> PRT
<213> Homo sapiens

<220>
<223> Factor VIIa variant R147D

<400> 33

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
```

```
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu

        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Asp Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 34
<211> 406
<212> PRT
<213> Homo sapiens

<220>
<223> Factor VIIa variant K192E

<400> 34

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
```

Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Glu Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 35
<211> 406
<212> PRT
<213> Homo sapiens

<220>
<223> Factor VIIa variant K192R

<400> 35

Ala Asn Ala Phe Leu Glu Glu Leu Arg Pro Gly Ser Leu Glu Arg Glu
1 5 10 15

```
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Arg Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 36
<211> 406
<212> PRT
<213> Homo sapiens

<220>
<223> Factor VIIa variant D60R/R147E

<400> 36

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Arg Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Glu Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu

385 390 395 400
Leu Arg Ala Pro Phe Pro
405

<210> 37
<211> 406
<212> PRT
<213> Homo sapiens

<220>

<223> Factor VIIa variant D60K/R147E

<400> 37

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Lys Lys Ile Lys Asn Trp Arg Asn Leu Ile Ala Val Leu
        195                 200                 205

Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Glu Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 38

<211> 406
<212> PRT
<213> Homo sapiens

<220>
<223> Factor VIIa variant D60R/R147D

<400> 38

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Arg Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255

His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Asp Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 39
<211> 406
<212> PRT
<213> Homo sapiens

<220>
<223> Factor VIIa variant D60R/K60aE/K60cE

<400> 39

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190

His Cys Phe Arg Glu Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 40
<211> 406
<212> PRT
<213> Homo sapiens

<220>
<223> Factor VIIa variant D60K/K60aE/K60cE

<400> 40

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125

```
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Lys Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 41
<211> 406
<212> PRT
<213> Homo sapiens

<220>
<223> Factor VIIa variant D60R/K60aE/K60cE/R147E

<400> 41

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
```

Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Arg Glu Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Glu Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 42
<211> 406
<212> PRT
<213> Homo sapiens

<220>
<223> Factor VIIa variant D60R/K60aM/K60cE

<400> 42

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Arg Met Ile Glu Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 43
<211> 406
<212> PRT
<213> Homo sapiens

<220>
<223> Factor VIIa variant D60R/K60aM/K60cE/R147E

<400> 43

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Arg Met Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Glu Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile

370 375 380
Glu Trp Leu Gln Lys Leu Met Arg Ser Glu Pro Arg Pro Gly Val Leu
385 390 395 400
Leu Arg Ala Pro Phe Pro
405

<210> 44
<211> 466
<212> PRT
<213> Homo sapiens

<220>
<223> FVII variant L13P

<400> 44

```
Met Val Ser Gln Ala Leu Arg Leu Leu Cys Leu Leu Pro Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
```

```
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 45
<211> 466
<212> PRT
<213> Homo sapiens

<220>
<223> FVII variant (precursor F64L)

<400> 45

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Leu
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
```

```
                180                     185                     190
Tyr Pro Cys Gly Lys Ile Pro Ile Leu Glu Lys Arg Asn Ala Ser Lys
            195                     200                     205
Pro Gln Gly Arg Ile Val Gly Gly Lys Val Cys Pro Lys Gly Glu Cys
    210                     215                     220
Pro Trp Gln Val Leu Leu Leu Val Asn Gly Ala Gln Leu Cys Gly Gly
225                     230                     235                     240
Thr Leu Ile Asn Thr Ile Trp Val Val Ser Ala Ala His Cys Phe Asp
                245                     250                     255
Lys Ile Lys Asn Trp Arg Asn Leu Ile Ala Val Leu Gly Glu His Asp
            260                     265                     270
Leu Ser Glu His Asp Gly Asp Glu Gln Ser Arg Arg Val Ala Gln Val
        275                     280                     285
Ile Ile Pro Ser Thr Tyr Val Pro Gly Thr Thr Asn His Asp Ile Ala
    290                     295                     300
Leu Leu Arg Leu His Gln Pro Val Val Leu Thr Asp His Val Val Pro
305                     310                     315                     320
Leu Cys Leu Pro Glu Arg Thr Phe Ser Glu Arg Thr Leu Ala Phe Val
                325                     330                     335
Arg Phe Ser Leu Val Ser Gly Trp Gly Gln Leu Leu Asp Arg Gly Ala
            340                     345                     350
Thr Ala Leu Glu Leu Met Val Leu Asn Val Pro Arg Leu Met Thr Gln
        355                     360                     365
Asp Cys Leu Gln Gln Ser Arg Lys Val Gly Asp Ser Pro Asn Ile Thr
    370                     375                     380
Glu Tyr Met Phe Cys Ala Gly Tyr Ser Asp Gly Ser Lys Asp Ser Cys
385                     390                     395                     400
Lys Gly Asp Ser Gly Gly Pro His Ala Thr His Tyr Arg Gly Thr Trp
                405                     410                     415
Tyr Leu Thr Gly Ile Val Ser Trp Gly Gln Gly Cys Ala Thr Val Gly
            420                     425                     430
His Phe Gly Val Tyr Thr Arg Val Ser Gln Tyr Ile Glu Trp Leu Gln
        435                     440                     445
Lys Leu Met Arg Ser Glu Pro Arg Pro Gly Val Leu Leu Arg Ala Pro
    450                     455                     460
Phe Pro
465
```

<210> 46
<211> 466
<212> PRT
<213> Homo sapiens

<220>
<223> FVII variant (precursor L73Q)

<400> 46

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
```

```
            50                  55                  60
Leu Glu Glu Leu Arg Pro Gly Ser Gln Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 47
<211> 466
<212> PRT
<213> Homo sapiens

<220>

<223> FVII variant (precursor E79Q)

<400> 47

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Gln Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
```

Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 48
<211> 466
<212> PRT
<213> Homo sapiens

<220>
<223> FVII variant (precursor S120P)

<400> 48

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Pro Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240

```
Thr Leu Ile Asn Thr Ile Trp Val Val Ser Ala Ala His Cys Phe Asp
            245                 250                 255
Lys Ile Lys Asn Trp Arg Asn Leu Ile Ala Val Leu Gly Glu His Asp
        260                 265                 270
Leu Ser Glu His Asp Gly Asp Glu Gln Ser Arg Arg Val Ala Gln Val
    275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 49
<211> 466
<212> PRT
<213> Homo sapiens

<220>
<223> FVII variant (precursor C121F)

<400> 49

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
```

```
Ser Pro Cys Gln Asn Gly Gly Ser Phe Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 50
<211> 466
<212> PRT
<213> Hmo sapiens

<220>
<223> FVII variant (precursor L125P)

<400> 50

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Pro Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415

Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 51
<211> 466
<212> PRT
<213> Homo sapiens

<220>
<223> FVII variant (precursorY128C)

<400> 51

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Cys
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
```

```
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460

Phe Pro
465
```

<210> 52
<211> 466
<212> PRT
<213> Homo sapiens

<220>
<223> FVII variant (precursor R139K)

<400> 52

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Lys Asn Cys Glu Thr His
    130                 135                 140
```

```
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
            165                 170                 175
Gly Tyr Ser Leu Leu Ala Asp Gly Val Ser Cys Thr Pro Thr Val Glu
        180                 185                 190
Tyr Pro Cys Gly Lys Ile Pro Ile Leu Glu Lys Arg Asn Ala Ser Lys
    195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp

            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 53
<211> 466
<212> PRT
<213> Homo sapiens

<220>
<223> FVII variant (precursor R139Q)

<400> 53

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Gln Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
```

```
            435                      440                       445
Lys Leu Met Arg Ser Glu Pro Arg Pro Gly Val Leu Leu Arg Ala Pro
        450                 455                       460
Phe Pro
465
```

<210> 54
<211> 466
<212> PRT
<213> Homo sapiens

<220>
<223> FVII variant (precursor R139W)

<400> 54

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Trp Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
```

Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 55
<211> 466
<212> PRT
<213> Homo sapiens

<220>
<223> FVII variant (precursor C151S)

<400> 55

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Ser Val Asn Glu Asn Gly Gly Cys Glu Gln
145 150 155 160
Tyr Cys Ser Asp His Thr Gly Thr Lys Arg Ser Cys Arg Cys His Glu

```
                    165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 56
<211> 466
<212> PRT
<213> Homo sapiens

<220>
<223> FVII variant (precursor E154K)

<400> 56

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
```

35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Lys Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 57
<211> 466
<212> PRT
<213> Homo sapiens

<220>
<223> FVII variant (precursor G157C)

<400> 57

```
Met Val Ser Gln Ala Leu Arg Leu Leu Cys Leu Leu Leu Gly Leu Gln
1               5                   10                  15

Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Cys Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
```

```
                340                 345                 350
Thr Ala Leu Glu Leu Met Val Leu Asn Val Pro Arg Leu Met Thr Gln

            355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 58
<211> 466
<212> PRT
<213> Homo sapiens

<220>
<223> FVII variant (precursorG157S)

<400> 58

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Ser Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
```

Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 59
<211> 466
<212> PRT
<213> Homo sapiens

<220>
<223> FVII variant (precursorG157V)

<400> 59

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80

```
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Val Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 60
<211> 466
<212> PRT
<213> Homo sapiens

<220>
<223> FVII variant (precursor Q160R)

<400> 60

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Arg
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly

225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
```

Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 61
<211> 466
<212> PRT
<213> Homo sapiens

<220>
<223> FVII variant (precursor P194T)

<400> 61

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Thr Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255

```
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 62
<211> 466
<212> PRT
<213> Homo sapiens

<220>
<223> FVII variant (precursor C195R)

<400> 62

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
```

Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Arg Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 63
<211> 466
<212> PRT
<213> Homo sapiens

<220>
<223> FVII variant (precursor K197E)

<400> 63

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Glu Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 64
<211> 466
<212> PRT
<213> Homo sapiens

<220>
<223> FVII variant (precursor R212Q)

<400> 64

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Gln Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
```

305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 65
<211> 466
<212> PRT
<213> Homo sapiens

<220>
<223> FVII variant (precursor G216D)

<400> 65

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110

Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175

```
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Asp Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro

    450                 455                 460
Phe Pro
465
```

<210> 66
<211> 466
<212> PRT
<213> Homo sapiens

<220>
<223> FVII variant (precursor C238Y)

<400> 66

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
```

Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Tyr Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro

465

<210> 67
<211> 466
<212> PRT

<213> Homo sapiens

<220>
<223> FVII variant (precursor T241N)

<400> 67

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Asn Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
```

```
                340                 345                 350
Thr Ala Leu Glu Leu Met Val Leu Asn Val Pro Arg Leu Met Thr Gln
            355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430

His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 68
<211> 466
<212> PRT
<213> Homo sapiens

<220>
<223> FVII variant (precursor C254Y)

<400> 68

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
```

```
                195                 200                 205
Pro Gln Gly Arg Ile Val Gly Gly Lys Val Cys Pro Lys Gly Glu Cys
        210                 215                 220
Pro Trp Gln Val Leu Leu Leu Val Asn Gly Ala Gln Leu Cys Gly Gly

225                 230                 235                 240
Thr Leu Ile Asn Thr Ile Trp Val Val Ser Ala Ala His Tyr Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 69
<211> 466
<212> PRT
<213> Homo sapiens

<220>
<223> FVII variant (precursor A266T)

<400> 69

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
```

```
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Thr Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 70
<211> 466
<212> PRT
<213> Homo sapiens

<220>
<223> FVII variant (precursor R283W)

<400> 70

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Trp Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
```

```
                355                             360                             365
Asp Cys Leu Gln Gln Ser Arg Lys Val Gly Asp Ser Pro Asn Ile Thr
    370                             375                             380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 71
<211> 466
<212> PRT
<213> Homo sapiens

<220>
<223> FVII variant (precursor V295D)

<400> 71

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
```

```
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Asp Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 72
<211> 466
<212> PRT
<213> Homo sapiens

<220>
<223> FVII variant (precursor D302H)

<400> 72

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
```

```
                    85                      90                      95
Thr Lys Leu Phe Trp Ile Ser Tyr Ser Asp Gly Asp Gln Cys Ala Ser
                100                     105                     110
Ser Pro Cys Gln Asn Gly Gly Ser Cys Lys Asp Gln Leu Gln Ser Tyr
            115                     120                     125
Ile Cys Phe Cys Leu Pro Ala Phe Glu Gly Arg Asn Cys Glu Thr His
    130                     135                     140
Lys Asp Asp Gln Leu Ile Cys Val Asn Glu Asn Gly Gly Cys Glu Gln
145                     150                     155                     160
Tyr Cys Ser Asp His Thr Gly Thr Lys Arg Ser Cys Arg Cys His Glu
                165                     170                     175
Gly Tyr Ser Leu Leu Ala Asp Gly Val Ser Cys Thr Pro Thr Val Glu
            180                     185                     190
Tyr Pro Cys Gly Lys Ile Pro Ile Leu Glu Lys Arg Asn Ala Ser Lys
        195                     200                     205
Pro Gln Gly Arg Ile Val Gly Gly Lys Val Cys Pro Lys Gly Glu Cys
    210                     215                     220
Pro Trp Gln Val Leu Leu Leu Val Asn Gly Ala Gln Leu Cys Gly Gly
225                     230                     235                     240
Thr Leu Ile Asn Thr Ile Trp Val Val Ser Ala Ala His Cys Phe Asp
                245                     250                     255
Lys Ile Lys Asn Trp Arg Asn Leu Ile Ala Val Leu Gly Glu His Asp
            260                     265                     270
Leu Ser Glu His Asp Gly Asp Glu Gln Ser Arg Arg Val Ala Gln Val
        275                     280                     285
Ile Ile Pro Ser Thr Tyr Val Pro Gly Thr Thr Asn His His Ile Ala
    290                     295                     300
Leu Leu Arg Leu His Gln Pro Val Val Leu Thr Asp His Val Val Pro
305                     310                     315                     320
Leu Cys Leu Pro Glu Arg Thr Phe Ser Glu Arg Thr Leu Ala Phe Val
                325                     330                     335
Arg Phe Ser Leu Val Ser Gly Trp Gly Gln Leu Leu Asp Arg Gly Ala
            340                     345                     350
Thr Ala Leu Glu Leu Met Val Leu Asn Val Pro Arg Leu Met Thr Gln
        355                     360                     365
Asp Cys Leu Gln Gln Ser Arg Lys Val Gly Asp Ser Pro Asn Ile Thr
    370                     375                     380
Glu Tyr Met Phe Cys Ala Gly Tyr Ser Asp Gly Ser Lys Asp Ser Cys
385                     390                     395                     400
Lys Gly Asp Ser Gly Gly Pro His Ala Thr His Tyr Arg Gly Thr Trp
                405                     410                     415
Tyr Leu Thr Gly Ile Val Ser Trp Gly Gln Gly Cys Ala Thr Val Gly
            420                     425                     430
His Phe Gly Val Tyr Thr Arg Val Ser Gln Tyr Ile Glu Trp Leu Gln
        435                     440                     445
Lys Leu Met Arg Ser Glu Pro Arg Pro Gly Val Leu Leu Arg Ala Pro
    450                     455                     460
Phe Pro
465
```

<210> 73
<211> 466
<212> PRT
<213> Homo sapiens

<220>
<223> FVII variant (precursor D302N)

<400> 73

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asn Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
```

385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 74
<211> 466
<212> PRT
<213> Homo sapiens

<220>
<223> FVII variant (precursor A304T)

<400> 74

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255

Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Thr
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 75
<211> 466
<212> PRT
<213> Homo sapiens

<220>
<223> FVII variant (precursor A304V)

<400> 75

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr

```
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Val
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 76
<211> 466
<212> PRT
<213> Homo sapiens

<220>
<223> FVII variant (precursor R307C)

<400> 76

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Cys Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415

Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 77
<211> 466
<212> PRT
<213> Homo sapiens

<220>
<223> FVII variant (precursor R307H)

<400> 77

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
```

```
                275                 280                 285
Ile Ile Pro Ser Thr Tyr Val Pro Gly Thr Thr Asn His Asp Ile Ala
    290                 295                 300
Leu Leu His Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 78
<211> 466
<212> PRT
<213> Homo sapiens

<220>
<223> FVII variant (precursor V312M)

<400> 78

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
```

```
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Met Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 79
<211> 466
<212> PRT
<213> Homo sapiens

<220>
<223> FVII variant (precursor E325K)

<400> 79

```
Met Val Ser Gln Ala Leu Arg Leu Leu Cys Leu Leu Leu Gly Leu Gln
```

```
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Lys Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445

Lys Leu Met Arg Ser Glu Pro Arg Pro Gly Val Leu Leu Arg Ala Pro
    450                 455                 460
Phe Pro
465
```

<210> 80
<211> 466
<212> PRT
<213> Homo sapiens

<220>
<223> FVII variant (precursor T332M)

<400> 80

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
```

```
305                 310                 315                 320
Leu Cys Leu Pro Glu Arg Thr Phe Ser Glu Arg Met Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 81
<211> 466
<212> PRT
<213> Homo sapiens

<220>
<223> FVII variant (precursor V341F)

<400> 81

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
```

Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Phe Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 82
<211> 466
<212> PRT
<213> Homo sapiens

<220 >
<223> FVII variant (precursor A352T)

<400> 82

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln

35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Thr
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460

Phe Pro

465

<210> 83
<211> 466
<212> PRT

<213> Homo sapiens

<220>
<223> FVII variant (precursor A354V)

<400> 83

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp

            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
```

```
                        325                             330                             335
Arg Phe Ser Leu Val Ser Gly Trp Gly Gln Leu Leu Asp Arg Gly Ala
                340                             345                             350
Thr Val Leu Glu Leu Met Val Leu Asn Val Pro Arg Leu Met Thr Gln
        355                             360                             365
Asp Cys Leu Gln Gln Ser Arg Lys Val Gly Asp Ser Pro Asn Ile Thr
    370                             375                             380
Glu Tyr Met Phe Cys Ala Gly Tyr Ser Asp Gly Ser Lys Asp Ser Cys
385                             390                             395                             400
Lys Gly Asp Ser Gly Gly Pro His Ala Thr His Tyr Arg Gly Thr Trp
                        405                             410                             415
Tyr Leu Thr Gly Ile Val Ser Trp Gly Gln Gly Cys Ala Thr Val Gly
                420                             425                             430
His Phe Gly Val Tyr Thr Arg Val Ser Gln Tyr Ile Glu Trp Leu Gln
        435                             440                             445
Lys Leu Met Arg Ser Glu Pro Arg Pro Gly Val Leu Leu Arg Ala Pro
    450                             455                             460
Phe Pro
465
```

<210> 84
<211> 466
<212> PRT
<213> Homo sapiens

<220>
<223> FVII variant (precursor M358I)

<400> 84

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
```

Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Ile Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 85
<211> 466
<212> PRT
<213> Homo sapiens

<220>
<223> FVII variant (precursor M358V)

<400> 85

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe

```
        50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Val Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 86
<211> 466
<212> PRT
<213> Homo sapiens

<220>

<223> FVII variant (precursor P363R)

<400> 86

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Arg Arg Leu Met Thr Gln
```

```
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 87
<211> 466
<212> PRT
<213> Homo sapiens

<220>
<223> FVII variant (precursor R364Q)

<400> 87

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
```

Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Gln Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 88
<211> 466
<212> PRT
<213> Homo sapiens

<220>
<223> FVII variant (precursor T367S)

<400> 88

Met Val Ser Gln Ala Leu Arg Leu Leu Cys Leu Leu Leu Gly Leu Gln
1 5 10 15

Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg

```
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Ser Gln

        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 89
<211> 466
<212> PRT
<213> Homo sapiens

<220>
<223> FVII variant (precursor C370F)

<400> 89

Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Phe Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys

```
385                     390                     395                     400
Lys Gly Asp Ser Gly Gly Pro His Ala Thr His Tyr Arg Gly Thr Trp
                405                     410                     415
Tyr Leu Thr Gly Ile Val Ser Trp Gly Gln Gly Cys Ala Thr Val Gly
            420                     425                     430
His Phe Gly Val Tyr Thr Arg Val Ser Gln Tyr Ile Glu Trp Leu Gln
        435                     440                     445
Lys Leu Met Arg Ser Glu Pro Arg Pro Gly Val Leu Leu Arg Ala Pro
    450                     455                     460
Phe Pro
465
```

<210> 90
<211> 466
<212> PRT
<213> Homo sapiens

<220>
<223> FVII variant (precursor C389G)

<400> 90

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
```

```
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335

Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Gly Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 91
<211> 466
<212> PRT
<213> Homo sapiens

<220>
<223> FVII variant (precursor G391S)

<400> 91

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
```

Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Ser Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 92
<211> 466
<212> PRT
<213> Homo sapiens

<220>
<223> FVII variant (precursor G402E)

<400> 92

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Glu Asp Ser Gly Gly Pro His Ala Thr His Tyr Arg Gly Thr Trp


                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 93
<211> 466
<212> PRT
<213> Homo sapiens

<220>
<223> FVII variant (precursor G402R)

<400> 93

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
```

Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Arg Asp Ser Gly Gly Pro His Ala Thr His Tyr Arg Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 94
<211> 466
<212> PRT
<213> Homo sapiens

<220>
<223> FVII variant (precursor D403H)

<400> 94

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His

```
        130                 135                 140
    Lys Asp Asp Gln Leu Ile Cys Val Asn Glu Asn Gly Gly Cys Glu Gln
    145                 150                 155                 160
    Tyr Cys Ser Asp His Thr Gly Thr Lys Arg Ser Cys Arg Cys His Glu
                    165                 170                 175
    Gly Tyr Ser Leu Leu Ala Asp Gly Val Ser Cys Thr Pro Thr Val Glu
                180                 185                 190
    Tyr Pro Cys Gly Lys Ile Pro Ile Leu Glu Lys Arg Asn Ala Ser Lys
            195                 200                 205
    Pro Gln Gly Arg Ile Val Gly Gly Lys Val Cys Pro Lys Gly Glu Cys
        210                 215                 220
    Pro Trp Gln Val Leu Leu Leu Val Asn Gly Ala Gln Leu Cys Gly Gly
    225                 230                 235                 240
    Thr Leu Ile Asn Thr Ile Trp Val Val Ser Ala Ala His Cys Phe Asp
                    245                 250                 255
    Lys Ile Lys Asn Trp Arg Asn Leu Ile Ala Val Leu Gly Glu His Asp
                260                 265                 270
    Leu Ser Glu His Asp Gly Asp Glu Gln Ser Arg Arg Val Ala Gln Val
            275                 280                 285
    Ile Ile Pro Ser Thr Tyr Val Pro Gly Thr Thr Asn His Asp Ile Ala
        290                 295                 300
    Leu Leu Arg Leu His Gln Pro Val Val Leu Thr Asp His Val Val Pro
    305                 310                 315                 320
    Leu Cys Leu Pro Glu Arg Thr Phe Ser Glu Arg Thr Leu Ala Phe Val
                    325                 330                 335
    Arg Phe Ser Leu Val Ser Gly Trp Gly Gln Leu Leu Asp Arg Gly Ala
                340                 345                 350
    Thr Ala Leu Glu Leu Met Val Leu Asn Val Pro Arg Leu Met Thr Gln
            355                 360                 365
    Asp Cys Leu Gln Gln Ser Arg Lys Val Gly Asp Ser Pro Asn Ile Thr
        370                 375                 380
    Glu Tyr Met Phe Cys Ala Gly Tyr Ser Asp Gly Ser Lys Asp Ser Cys
    385                 390                 395                 400
    Lys Gly His Ser Gly Gly Pro His Ala Thr His Tyr Arg Gly Thr Trp
                    405                 410                 415
    Tyr Leu Thr Gly Ile Val Ser Trp Gly Gln Gly Cys Ala Thr Val Gly
                420                 425                 430
    His Phe Gly Val Tyr Thr Arg Val Ser Gln Tyr Ile Glu Trp Leu Gln
            435                 440                 445
    Lys Leu Met Arg Ser Glu Pro Arg Pro Gly Val Leu Leu Arg Ala Pro
        450                 455                 460
    Phe Pro
    465
```

<210> 95
<211> 466
<212> PRT
<213> Homo sapiens

<220>
<223> FVII variant (precursor R413Q)

<400> 95

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu
165 170 175
Gly Tyr Ser Leu Leu Ala Asp Gly Val Ser Cys Thr Pro Thr Val Glu
180 185 190
Tyr Pro Cys Gly Lys Ile Pro Ile Leu Glu Lys Arg Asn Ala Ser Lys
195 200 205
Pro Gln Gly Arg Ile Val Gly Gly Lys Val Cys Pro Lys Gly Glu Cys
210 215 220
Pro Trp Gln Val Leu Leu Leu Val Asn Gly Ala Gln Leu Cys Gly Gly
225 230 235 240
Thr Leu Ile Asn Thr Ile Trp Val Val Ser Ala Ala His Cys Phe Asp
245 250 255
Lys Ile Lys Asn Trp Arg Asn Leu Ile Ala Val Leu Gly Glu His Asp
260 265 270
Leu Ser Glu His Asp Gly Asp Glu Gln Ser Arg Arg Val Ala Gln Val
275 280 285
Ile Ile Pro Ser Thr Tyr Val Pro Gly Thr Thr Asn His Asp Ile Ala
290 295 300
Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Gln Gly Thr Trp
405 410 415
Tyr Leu Thr Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln

435 440 445
Lys Leu Met Arg Ser Glu Pro Arg Pro Gly Val Leu Leu Arg Ala Pro
450 455 460
Phe Pro
465

<210> 96
<211> 466
<212> PRT
<213> Homo sapiens

<220>
<223> FVII variant (precursor T419M)

<400> 96

```
Met Val Ser Gln Ala Leu Arg Leu Leu Cys Leu Leu Leu Gly Leu Gln
 1              5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
```

Leu Leu Arg Leu His Gln Pro Val Val Leu Thr Asp His Val Val Pro
305 310 315 320
Leu Cys Leu Pro Glu Arg Thr Phe Ser Glu Arg Thr Leu Ala Phe Val
325 330 335
Arg Phe Ser Leu Val Ser Gly Trp Gly Gln Leu Leu Asp Arg Gly Ala
340 345 350
Thr Ala Leu Glu Leu Met Val Leu Asn Val Pro Arg Leu Met Thr Gln
355 360 365
Asp Cys Leu Gln Gln Ser Arg Lys Val Gly Asp Ser Pro Asn Ile Thr
370 375 380
Glu Tyr Met Phe Cys Ala Gly Tyr Ser Asp Gly Ser Lys Asp Ser Cys
385 390 395 400
Lys Gly Asp Ser Gly Gly Pro His Ala Thr His Tyr Arg Gly Thr Trp
405 410 415
Tyr Leu Met Gly Ile Val Ser Trp Gly Gln Gly Cys Ala Thr Val Gly
420 425 430
His Phe Gly Val Tyr Thr Arg Val Ser Gln Tyr Ile Glu Trp Leu Gln
435 440 445
Lys Leu Met Arg Ser Glu Pro Arg Pro Gly Val Leu Leu Arg Ala Pro
450 455 460
Phe Pro
465

<210> 97
<211> 466
<212> PRT
<213> Homo sapiens

<220>
<223> FVII variant (precursor G435E)

<400> 97

Met Val Ser Gln Ala Leu Arg Leu Leu Cys Leu Leu Leu Gly Leu Gln
1 5 10 15
Gly Cys Leu Ala Ala Gly Gly Val Ala Lys Ala Ser Gly Gly Glu Thr
20 25 30
Arg Asp Met Pro Trp Lys Pro Gly Pro His Arg Val Phe Val Thr Gln
35 40 45
Glu Glu Ala His Gly Val Leu His Arg Arg Arg Arg Ala Asn Ala Phe
50 55 60
Leu Glu Glu Leu Arg Pro Gly Ser Leu Glu Arg Glu Cys Lys Glu Glu
65 70 75 80
Gln Cys Ser Phe Glu Glu Ala Arg Glu Ile Phe Lys Asp Ala Glu Arg
85 90 95
Thr Lys Leu Phe Trp Ile Ser Tyr Ser Asp Gly Asp Gln Cys Ala Ser
100 105 110
Ser Pro Cys Gln Asn Gly Gly Ser Cys Lys Asp Gln Leu Gln Ser Tyr
115 120 125
Ile Cys Phe Cys Leu Pro Ala Phe Glu Gly Arg Asn Cys Glu Thr His
130 135 140
Lys Asp Asp Gln Leu Ile Cys Val Asn Glu Asn Gly Gly Cys Glu Gln
145 150 155 160
Tyr Cys Ser Asp His Thr Gly Thr Lys Arg Ser Cys Arg Cys His Glu

```
                    165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Glu Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 98
<211> 466
<212> PRT
<213> Homo sapiens

<220>
<223> FVII variant (precursor E445K)

<400> 98

```
Met Val Ser Gln Ala Leu Arg Leu Leu Cys Leu Leu Leu Gly Leu Gln
 1               5                  10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
```

```
                35                  40                  45
Glu Glu Ala His Gly Val Leu His Arg Arg Arg Arg Ala Asn Ala Phe
        50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Arg Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Lys Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 99
<211> 466
<212> PRT
<213> Homo sapiens

<220>
<223> FVII variant (precursor R375W)

<400> 99

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
```

```
Thr Ala Leu Glu Leu Met Val Leu Asn Val Pro Arg Leu Met Thr Gln
        355                 360                 365
Asp Cys Leu Gln Gln Ser Trp Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 100
<211> 466
<212> PRT
<213> Homo sapiens

<220>
<223> FVII variant (precursor R375K)

<400> 100

```
Met Val Ser Gln Ala Leu Arg Leu Leu Cys Leu Leu Leu Gly Leu Gln
1               5                   10                  15
Gly Cys Leu Ala Ala Gly Gly Val Ala Lys Ala Ser Gly Gly Glu Thr
            20                  25                  30
Arg Asp Met Pro Trp Lys Pro Gly Pro His Arg Val Phe Val Thr Gln
        35                  40                  45
Glu Glu Ala His Gly Val Leu His Arg Arg Arg Arg Ala Asn Ala Phe
    50                  55                  60
Leu Glu Glu Leu Arg Pro Gly Ser Leu Glu Arg Glu Cys Lys Glu Glu
65                  70                  75                  80
Gln Cys Ser Phe Glu Glu Ala Arg Glu Ile Phe Lys Asp Ala Glu Arg
                85                  90                  95
Thr Lys Leu Phe Trp Ile Ser Tyr Ser Asp Gly Asp Gln Cys Ala Ser
            100                 105                 110
Ser Pro Cys Gln Asn Gly Gly Ser Cys Lys Asp Gln Leu Gln Ser Tyr
        115                 120                 125
Ile Cys Phe Cys Leu Pro Ala Phe Glu Gly Arg Asn Cys Glu Thr His
    130                 135                 140
Lys Asp Asp Gln Leu Ile Cys Val Asn Glu Asn Gly Gly Cys Glu Gln
145                 150                 155                 160
Tyr Cys Ser Asp His Thr Gly Thr Lys Arg Ser Cys Arg Cys His Glu
                165                 170                 175
Gly Tyr Ser Leu Leu Ala Asp Gly Val Ser Cys Thr Pro Thr Val Glu
            180                 185                 190
Tyr Pro Cys Gly Lys Ile Pro Ile Leu Glu Lys Arg Asn Ala Ser Lys
        195                 200                 205
Pro Gln Gly Arg Ile Val Gly Gly Lys Val Cys Pro Lys Gly Glu Cys
    210                 215                 220
```

```
Pro Trp Gln Val Leu Leu Leu Val Asn Gly Ala Gln Leu Cys Gly Gly
225                 230                 235                 240
Thr Leu Ile Asn Thr Ile Trp Val Val Ser Ala Ala His Cys Phe Asp
                245                 250                 255
Lys Ile Lys Asn Trp Arg Asn Leu Ile Ala Val Leu Gly Glu His Asp
            260                 265                 270
Leu Ser Glu His Asp Gly Asp Glu Gln Ser Arg Arg Val Ala Gln Val
        275                 280                 285
Ile Ile Pro Ser Thr Tyr Val Pro Gly Thr Thr Asn His Asp Ile Ala
    290                 295                 300
Leu Leu Arg Leu His Gln Pro Val Val Leu Thr Asp His Val Val Pro
305                 310                 315                 320
Leu Cys Leu Pro Glu Arg Thr Phe Ser Glu Arg Thr Leu Ala Phe Val
                325                 330                 335
Arg Phe Ser Leu Val Ser Gly Trp Gly Gln Leu Leu Asp Arg Gly Ala
            340                 345                 350
Thr Ala Leu Glu Leu Met Val Leu Asn Val Pro Arg Leu Met Thr Gln
        355                 360                 365
Asp Cys Leu Gln Gln Ser Lys Lys Val Gly Asp Ser Pro Asn Ile Thr
    370                 375                 380
Glu Tyr Met Phe Cys Ala Gly Tyr Ser Asp Gly Ser Lys Asp Ser Cys
385                 390                 395                 400
Lys Gly Asp Ser Gly Gly Pro His Ala Thr His Tyr Arg Gly Thr Trp
                405                 410                 415
Tyr Leu Thr Gly Ile Val Ser Trp Gly Gln Gly Cys Ala Thr Val Gly
            420                 425                 430
His Phe Gly Val Tyr Thr Arg Val Ser Gln Tyr Ile Glu Trp Leu Gln
        435                 440                 445
Lys Leu Met Arg Ser Glu Pro Arg Pro Gly Val Leu Leu Arg Ala Pro
    450                 455                 460
Phe Pro
465
```

<210> 101
<211> 304
<212> PRT
<213> Homo sapiens

<220>
<223> TFPI isoform a precursor

<400> 101

```
Met Ile Tyr Thr Met Lys Lys Val His Ala Leu Trp Ala Ser Val Cys
1               5                   10                  15
Leu Leu Leu Asn Leu Ala Pro Ala Pro Leu Asn Ala Asp Ser Glu Glu
            20                  25                  30
Asp Glu Glu His Thr Ile Ile Thr Asp Thr Glu Leu Pro Pro Leu Lys
        35                  40                  45
Leu Met His Ser Phe Cys Ala Phe Lys Ala Asp Asp Gly Pro Cys Lys
    50                  55                  60
Ala Ile Met Lys Arg Phe Phe Phe Asn Ile Phe Thr Arg Gln Cys Glu
65                  70                  75                  80
Glu Phe Ile Tyr Gly Gly Cys Glu Gly Asn Gln Asn Arg Phe Glu Ser
                85                  90                  95
```

```
Leu Glu Glu Cys Lys Lys Met Cys Thr Arg Asp Asn Ala Asn Arg Ile
            100                 105                 110
Ile Lys Thr Thr Leu Gln Gln Glu Lys Pro Asp Phe Cys Phe Leu Glu
        115                 120                 125
Glu Asp Pro Gly Ile Cys Arg Gly Tyr Ile Thr Arg Tyr Phe Tyr Asn
    130                 135                 140
Asn Gln Thr Lys Gln Cys Glu Arg Phe Lys Tyr Gly Gly Cys Leu Gly
145                 150                 155                 160
Asn Met Asn Asn Phe Glu Thr Leu Glu Glu Cys Lys Asn Ile Cys Glu
                165                 170                 175
Asp Gly Pro Asn Gly Phe Gln Val Asp Asn Tyr Gly Thr Gln Leu Asn
            180                 185                 190
Ala Val Asn Asn Ser Leu Thr Pro Gln Ser Thr Lys Val Pro Ser Leu
        195                 200                 205
Phe Glu Phe His Gly Pro Ser Trp Cys Leu Thr Pro Ala Asp Arg Gly
    210                 215                 220
Leu Cys Arg Ala Asn Glu Asn Arg Phe Tyr Tyr Asn Ser Val Ile Gly
225                 230                 235                 240
Lys Cys Arg Pro Phe Lys Tyr Ser Gly Cys Gly Gly Asn Glu Asn Asn
                245                 250                 255
Phe Thr Ser Lys Gln Glu Cys Leu Arg Ala Cys Lys Lys Gly Phe Ile
            260                 265                 270
Gln Arg Ile Ser Lys Gly Gly Leu Ile Lys Thr Lys Arg Lys Arg Lys
        275                 280                 285
Lys Gln Arg Val Lys Ile Ala Tyr Glu Glu Ile Phe Val Lys Asn Met
    290                 295                 300
```

<210> 102
<211> 276
<212> PRT
<213> Homo sapiens

<220>
<223> TFPI isoform a mature

<400> 102

```
Asp Ser Glu Glu Asp Glu Glu His Thr Ile Ile Thr Asp Thr Glu Leu
1               5                   10                  15
Pro Pro Leu Lys Leu Met His Ser Phe Cys Ala Phe Lys Ala Asp Asp
            20                  25                  30
Gly Pro Cys Lys Ala Ile Met Lys Arg Phe Phe Phe Asn Ile Phe Thr
        35                  40                  45
Arg Gln Cys Glu Glu Phe Ile Tyr Gly Gly Cys Glu Gly Asn Gln Asn
    50                  55                  60
Arg Phe Glu Ser Leu Glu Glu Cys Lys Lys Met Cys Thr Arg Asp Asn
65                  70                  75                  80
Ala Asn Arg Ile Ile Lys Thr Thr Leu Gln Gln Glu Lys Pro Asp Phe
                85                  90                  95
Cys Phe Leu Glu Glu Asp Pro Gly Ile Cys Arg Gly Tyr Ile Thr Arg
            100                 105                 110
Tyr Phe Tyr Asn Asn Gln Thr Lys Gln Cys Glu Arg Phe Lys Tyr Gly
        115                 120                 125
Gly Cys Leu Gly Asn Met Asn Asn Phe Glu Thr Leu Glu Glu Cys Lys
```

```
        130                 135                 140
Asn Ile Cys Glu Asp Gly Pro Asn Gly Phe Gln Val Asp Asn Tyr Gly
145                 150                 155                 160
Thr Gln Leu Asn Ala Val Asn Asn Ser Leu Thr Pro Gln Ser Thr Lys
                165                 170                 175
Val Pro Ser Leu Phe Glu Phe His Gly Pro Ser Trp Cys Leu Thr Pro
            180                 185                 190
Ala Asp Arg Gly Leu Cys Arg Ala Asn Glu Asn Arg Phe Tyr Tyr Asn
        195                 200                 205
Ser Val Ile Gly Lys Cys Arg Pro Phe Lys Tyr Ser Gly Cys Gly Gly
    210                 215                 220
Asn Glu Asn Asn Phe Thr Ser Lys Gln Glu Cys Leu Arg Ala Cys Lys
225                 230                 235                 240
Lys Gly Phe Ile Gln Arg Ile Ser Lys Gly Gly Leu Ile Lys Thr Lys
                245                 250                 255
Arg Lys Arg Lys Lys Gln Arg Val Lys Ile Ala Tyr Glu Glu Ile Phe
            260                 265                 270
Val Lys Asn Met
        275
```

<210> 103
<211> 251
<212> PRT
<213> Homo sapiens

<220>
<223> TFPI isoform b precursor

<400> 103

```
Met Ile Tyr Thr Met Lys Lys Val His Ala Leu Trp Ala Ser Val Cys
1               5                   10                  15
Leu Leu Leu Asn Leu Ala Pro Ala Pro Leu Asn Ala Asp Ser Glu Glu
            20                  25                  30
Asp Glu Glu His Thr Ile Ile Thr Asp Thr Glu Leu Pro Pro Leu Lys
        35                  40                  45
Leu Met His Ser Phe Cys Ala Phe Lys Ala Asp Asp Gly Pro Cys Lys
    50                  55                  60
Ala Ile Met Lys Arg Phe Phe Phe Asn Ile Phe Thr Arg Gln Cys Glu
65                  70                  75                  80
Glu Phe Ile Tyr Gly Gly Cys Glu Gly Asn Gln Asn Arg Phe Glu Ser
                85                  90                  95
Leu Glu Glu Cys Lys Lys Met Cys Thr Arg Asp Asn Ala Asn Arg Ile
            100                 105                 110
Ile Lys Thr Thr Leu Gln Gln Glu Lys Pro Asp Phe Cys Phe Leu Glu
        115                 120                 125
Glu Asp Pro Gly Ile Cys Arg Gly Tyr Ile Thr Arg Tyr Phe Tyr Asn
    130                 135                 140
Asn Gln Thr Lys Gln Cys Glu Arg Phe Lys Tyr Gly Gly Cys Leu Gly
145                 150                 155                 160
Asn Met Asn Asn Phe Glu Thr Leu Glu Glu Cys Lys Asn Ile Cys Glu
                165                 170                 175
Asp Gly Pro Asn Gly Phe Gln Val Asp Asn Tyr Gly Thr Gln Leu Asn
            180                 185                 190
Ala Val Asn Asn Ser Leu Thr Pro Gln Ser Thr Lys Val Pro Ser Leu
```

```
        195                 200                 205
Phe Val Thr Lys Glu Gly Thr Asn Asp Gly Trp Lys Asn Ala Ala His
    210                 215                 220
Ile Tyr Gln Val Phe Leu Asn Ala Phe Cys Ile His Ala Ser Met Phe
225                 230                 235                 240
Phe Leu Gly Leu Asp Ser Ile Ser Cys Leu Cys
                245                 250
```

<210> 104
<211> 222
<212> PRT
<213> Homo sapiens

<220>
<223> TFPI isoform b mature

<400> 104

```
Ser Glu Glu Asp Glu Glu His Thr Ile Ile Thr Asp Thr Glu Leu Pro
1               5                   10                  15
Pro Leu Lys Leu Met His Ser Phe Cys Ala Phe Lys Ala Asp Asp Gly
            20                  25                  30
Pro Cys Lys Ala Ile Met Lys Arg Phe Phe Phe Asn Ile Phe Thr Arg
        35                  40                  45
Gln Cys Glu Glu Phe Ile Tyr Gly Gly Cys Glu Gly Asn Gln Asn Arg
    50                  55                  60
Phe Glu Ser Leu Glu Glu Cys Lys Lys Met Cys Thr Arg Asp Asn Ala
65                  70                  75                  80
Asn Arg Ile Ile Lys Thr Thr Leu Gln Gln Glu Lys Pro Asp Phe Cys
                85                  90                  95
Phe Leu Glu Glu Asp Pro Gly Ile Cys Arg Gly Tyr Ile Thr Arg Tyr
            100                 105                 110
Phe Tyr Asn Asn Gln Thr Lys Gln Cys Glu Arg Phe Lys Tyr Gly Gly
        115                 120                 125
Cys Leu Gly Asn Met Asn Asn Phe Glu Thr Leu Glu Glu Cys Lys Asn
    130                 135                 140
Ile Cys Glu Asp Gly Pro Asn Gly Phe Gln Val Asp Asn Tyr Gly Thr
145                 150                 155                 160
Gln Leu Asn Ala Val Asn Asn Ser Leu Thr Pro Gln Ser Thr Lys Val
                165                 170                 175
Pro Ser Leu Phe Val Thr Lys Glu Gly Thr Asn Asp Gly Trp Lys Asn
            180                 185                 190
Ala Ala His Ile Tyr Gln Val Phe Leu Asn Ala Phe Cys Ile His Ala
        195                 200                 205
Ser Met Phe Phe Leu Gly Leu Asp Ser Ile Ser Cys Leu Cys
    210                 215                 220
```

<210> 105
<211> 213
<212> PRT
<213> Homo sapiens

<220>
<223> TFPI-2 mature

<400> 105

Asp Ala Ala Gln Glu Pro Thr Gly Asn Asn Ala Glu Ile Cys Leu Leu
1 5 10 15
Pro Leu Asp Tyr Gly Pro Cys Arg Ala Leu Leu Leu Arg Tyr Tyr Tyr
20 25 30
Asp Arg Tyr Thr Gln Ser Cys Arg Gln Phe Leu Tyr Gly Gly Cys Glu
35 40 45
Gly Asn Ala Asn Asn Phe Tyr Thr Trp Glu Ala Cys Asp Asp Ala Cys
50 55 60
Trp Arg Ile Glu Lys Val Pro Lys Val Cys Arg Leu Gln Val Ser Val
65 70 75 80
Asp Asp Gln Cys Glu Gly Ser Thr Glu Lys Tyr Phe Phe Asn Leu Ser
85 90 95
Ser Met Thr Cys Glu Lys Phe Phe Ser Gly Gly Cys His Arg Asn Arg
100 105 110
Ile Glu Asn Arg Phe Pro Asp Glu Ala Thr Cys Met Gly Phe Cys Ala
115 120 125
Pro Lys Lys Ile Pro Ser Phe Cys Tyr Ser Pro Lys Asp Glu Gly Leu
130 135 140
Cys Ser Ala Asn Val Thr Arg Tyr Tyr Phe Asn Pro Arg Tyr Arg Thr
145 150 155 160
Cys Asp Ala Phe Thr Tyr Thr Gly Cys Gly Gly Asn Asp Asn Asn Phe
165 170 175
Val Ser Arg Glu Asp Cys Lys Arg Ala Cys Ala Lys Ala Leu Lys Lys
180 185 190
Lys Lys Lys Met Pro Lys Leu Arg Phe Ala Ser Arg Ile Arg Lys Ile
195 200 205
Arg Lys Lys Gln Phe
210

<210> 106
<211> 55
<212> PRT
<213> Bos taurus

<220>
<223> BPTI 5L15 K-1

<400> 106

Ala Pro Asp Phe Cys Leu Glu Pro Pro Tyr Asp Gly Pro Cys Arg Ala
1 5 10 15
Leu His Leu Arg Tyr Phe Tyr Asn Ala Lys Ala Gly Leu Cys Gln Thr
20 25 30
Phe Tyr Tyr Gly Gly Cys Leu Ala Lys Arg Asn Asn Phe Glu Ser Ala
35 40 45
Glu Asp Cys Met Arg Thr Cys
50 55

<210> 107
<211> 245 .
<212> PRT
<213> Homo sapiens

<220>
<223> Chymotrypsinogen B mature

<400> 107

```
Cys Gly Val Pro Ala Ile His Pro Val Leu Ser Gly Leu Ser Arg Ile
1               5                   10                  15
Val Asn Gly Glu Asp Ala Val Pro Gly Ser Trp Pro Trp Gln Val Ser
            20                  25                  30
Leu Gln Asp Lys Thr Gly Phe His Phe Cys Gly Gly Ser Leu Ile Ser
        35                  40                  45
Glu Asp Trp Val Val Thr Ala Ala His Cys Gly Val Arg Thr Ser Asp
    50                  55                  60
Val Val Val Ala Gly Glu Phe Asp Gln Gly Ser Asp Glu Glu Asn Ile
65                  70                  75                  80
Gln Val Leu Lys Ile Ala Lys Val Phe Lys Asn Pro Lys Phe Ser Ile
                85                  90                  95
Leu Thr Val Asn Asn Asp Ile Thr Leu Leu Lys Leu Ala Thr Pro Ala
            100                 105                 110
Arg Phe Ser Gln Thr Val Ser Ala Val Cys Leu Pro Ser Ala Asp Asp
        115                 120                 125
Asp Phe Pro Ala Gly Thr Leu Cys Ala Thr Thr Gly Trp Gly Lys Thr
    130                 135                 140
Lys Tyr Asn Ala Asn Lys Thr Pro Asp Lys Leu Gln Gln Ala Ala Leu
145                 150                 155                 160
Pro Leu Leu Ser Asn Ala Glu Cys Lys Lys Ser Trp Gly Arg Arg Ile
                165                 170                 175
Thr Asp Val Met Ile Cys Ala Gly Ala Ser Gly Val Ser Ser Cys Met
            180                 185                 190
Gly Asp Ser Gly Gly Pro Leu Val Cys Gln Lys Asp Gly Ala Trp Thr
        195                 200                 205
Leu Val Gly Ile Val Ser Trp Gly Ser Asp Thr Cys Ser Thr Ser Ser
    210                 215                 220
Pro Gly Val Tyr Ala Arg Val Thr Lys Leu Ile Pro Trp Val Gln Lys
225                 230                 235                 240
Ile Leu Ala Ala Asn
                245
```

<210> 108
<211> 3141
<212> DNA
<213> Homo sapiens

<220>
<223> FVII transcript variant 1

<400> 108

```
agtcccatgg ggaatgtcaa caggcagggg cagcactgca gagatttcat catggtctcc 60
caggccctca ggctcctctg ccttctgctt gggcttcagg gctgcctggc tgcaggcggg 120
gtcgctaagg cctcaggagg agaaacacgg gacatgccgt ggaagccggg gcctcacaga 180
gtcttcgtaa cccaggagga agcccacggc gtcctgcacc ggcgccggcg cgccaacgcg 240
ttcctggagg agctgcggcc gggctccctg gagagggagt gcaaggagga gcagtgctcc 300
ttcgaggagg cccgggagat cttcaaggac gcggagagga cgaagctgtt ctggatttct 360
tacagtgatg gggaccagtg tgcctcaagt ccatgccaga atgggggctc ctgcaaggac 420
```

```
cagctccagt cctatatctg cttctgcctc cctgccttcg agggccggaa ctgtgagacg 480
cacaaggatg accagctgat ctgtgtgaac gagaacggcg gctgtgagca gtactgcagt 540
gaccacacgg gcaccaagcg ctcctgtcgg tgccacgagg ggtactctct gctggcagac 600
ggggtgtcct gcacacccac agttgaatat ccatgtggaa aaatacctat tctagaaaaa 660
agaaatgcca gcaaacccca aggccgaatt gtgggggggca aggtgtgccc caaaggggag 720
tgtccatggc aggtcctgtt gttggtgaat ggagctcagt tgtgtggggg gaccctgatc 780
aacaccatct gggtggtctc cgcggcccac tgtttcgaca aaatcaagaa ctggaggaac 840
ctgatcgcgg tgctgggcga gcacgacctc agcgagcacg acggggatga gcagagccgg 900
cgggtggcgc aggtcatcat ccccagcacg tacgtcccgg gcaccaccaa ccacgacatc 960
gcgctgctcc gcctgcacca gcccgtggtc ctcactgacc atgtggtgcc cctctgcctg 1020
cccgaacgga cgttctctga gaggacgctg gccttcgtgc gcttctcatt ggtcagcggc 1080
tggggccagc tgctggaccg tggcgccacg gccctggagc tcatggtcct caacgtgccc 1140
cggctgatga cccaggactg cctgcagcag tcacggaagg tgggagactc cccaaatatc 1200
acggagtaca tgttctgtgc cggctactcg gatggcagca aggactcctg caaggggggac 1260
agtggaggcc cacatgccac ccactaccgg ggcacgtggt acctgacggg catcgtcagc 1320
tggggccagg gctgcgcaac cgtgggccac tttggggtgt acaccagggt ctcccagtac 1380
atcgagtggc tgcaaaagct catgcgctca gagccacgcc caggagtcct cctgcgagcc 1440
ccatttccct agcccagcag ccctggcctg tggagagaaa gccaaggctg cgtcgaactg 1500
tcctggcacc aaatcccata tattcttctg cagttaatgg ggtagaggag ggcatgggag 1560
ggagggagag gtggggaggg agacagagac agaaacagag agagacagag acagagagag 1620
actgagggag agactctgag gacatggaga gagactcaaa gagactccaa gattcaaaga 1680
gactaataga gacacagaga tggaatagaa aagatgagag gcagaggcag acaggcgctg 1740
gacagagggg caggggagtg ccaaggttgt cctggaggca gacagcccag ctgagcctcc 1800
ttacctccct tcagccaagc cccacctgcac gtgatctgct ggcctcaggc tgctgctctg 1860
ccttcattgc tggagacagt agaggcatga acacacatgg atgcacacac acacacgcca 1920
atgcacacac acagagatat gcacacacac ggatgcacac acagatggtc acacagagat 1980
acgcaaacac accgatgcac acgcacatag agatatgcac acacagatgc acacacagat 2040
atacacatgg atgcacgcac atgccaatgc acgcacacat cagtgcacac ggatgcacag 2100
agatatgcac acaccgatgt gcgcacacac agatatgcac acacatggat gagcacacac 2160
acaccaatgc gcacacacac cgatgtacac acacagatgc acacacagat gcacacacac 2220
cgatgctgac tccatgtgtg ctgtcctctg aaggcggttg tttagctctc acttttctgg 2280
ttcttatcca ttatcatctt cacttcagac aattcagaag catcaccatg catggtggcg 2340
aatgccccca aactctcccc caaatgtatt tctcccttcg ctgggtgccg ggctgcacag 2400
actattcccc acctgcttcc cagcttcaca ataaacggct gcgtctcctc cgcacacctg 2460
tggtgcctgc cacccactgg gttgcccatg attcattttt ggagcccccg gtgctcatcc 2520
tctgagatgc tcttttcttt cacaattttc aacatcactg aaatgaaccc tcacatggaa 2580
gctatttttt aaaaacaaaa gctgtttgat agatgtttga ggctgtagct cccaggatcc 2640
tgtggaattg gatgttctct ccctgccaca gcccttgtca atgatatttc acagagaccc 2700
tgggagcacc tgctcaagag tcagggacac acgcatcact aaatgcaagt tcccaggccc 2760
tggctgcagt gggaggacct ggcaagctgc actcttgctg agtccccagg gtggtggaag 2820
aagaatgaga aacacatgaa cagagaaatg gggaggtgac aaacagtgcc cccactcaga 2880
ctccggcaag cacggctcag agagtggact cgatgccatc cctgcagggc cgtcctgggc 2940
accactggca ctcacagcag caaggtgggc accattggca ctcacagcag caaggcaggc 3000
accagcaacc cacctcgggg gcactcaggc atcatctact tcagagcaga cagggtctat 3060
gaactacagc cgtgggctgc ttccaaggca ccctgctctt gtaaataaag ttttatggga 3120
acacaaaaaa aaaaaaaaaa a                                           3141
```

<210> 109
<211> 3075
<212> DNA
<213> Homo sapiens

<220>
<223> FVII transcript variant 2

<400> 109

```
agtcccatgg ggaatgtcaa caggcagggg cagcactgca gagatttcat catggtctcc 60
caggccctca ggctcctctg ccttctgctt gggcttcagg gctgcctggc tgcagtcttc 120
gtaacccagg aggaagccca cggcgtcctg caccggcgcc ggcgcgccaa cgcgttcctg 180
gaggagctgc ggccgggctc cctggagagg gagtgcaagg aggagcagtg ctccttcgag 240
gaggcccggg agatcttcaa ggacgcggag aggacgaagc tgttctggat ttcttacagt 300
gatggggacc agtgtgcctc aagtccatgc cagaatgggg gctcctgcaa ggaccagctc 360
cagtcctata tctgcttctg cctccctgcc ttcgagggcc ggaactgtga gacgcacaag 420
gatgaccagc tgatctgtgt gaacgagaac ggcggctgtg agcagtactg cagtgaccac 480
acgggcacca agcgctcctg tcggtgccac gaggggtact ctctgctggc agacggggtg 540
tcctgcacac ccacagttga atatccatgt ggaaaaatac ctattctaga aaaaagaaat 600
gccagcaaac cccaaggccg aattgtgggg ggcaaggtgt gccccaaagg ggagtgtcca 660
tggcaggtcc tgttgttggt gaatggagct cagttgtgtg gggggaccct gatcaacacc 720
atctgggtgg tctccgcggc ccactgtttc gacaaaatca agaactggag gaacctgatc 780
gcggtgctgg gcgagcacga cctcagcgag cacgacgggg atgagcagag ccggcgggtg 840
gcgcaggtca tcatccccag cacgtacgtc ccgggcacca ccaaccacga catcgcgctg 900
ctccgcctgc accagcccgt ggtcctcact gaccatgtgg tgcccctctg cctgcccgaa 960
cggacgttct ctgagaggac gctggccttc gtgcgcttct cattggtcag cggctggggc 1020
cagctgctgg accgtggcgc cacggccctg gagctcatgg tcctcaacgt gccccggctg 1080
atgacccagg actgcctgca gcagtcacgg aaggtgggag actccccaaa tatcacggag 1140
tacatgttct gtgccggcta ctcggatggc agcaaggact cctgcaaggg ggacagtgga 1200
ggcccacatg ccacccacta ccggggcacg tggtacctga cgggcatcgt cagctggggc 1260
cagggctgcg caaccgtggg ccactttggg gtgtacacca gggtctccca gtacatcgag 1320
tggctgcaaa agctcatgcg ctcagagcca cgcccaggag tcctcctgcg agccccattt 1380
ccctagccca gcagccctgg cctgtggaga gaaagccaag gctgcgtcga actgtcctgg 1440
caccaaatcc catatattct tctgcagtta atggggtaga ggagggcatg ggagggaggg 1500
agaggtgggg agggagacag agacagaaac agagagagac agagacagag agagactgag 1560
ggagagactc tgaggacatg gagagagact caaagagact ccaagattca aagagactaa 1620
tagagacaca gagatggaat agaaaagatg agaggcagag gcagacaggc gctggacaga 1680
ggggcagggg agtgccaagg ttgtcctgga ggcagacagc ccagctgagc ctccttacct 1740
cccttcagcc aagcccacct gcacgtgatc tgctggcctc aggctgctgc tctgccttca 1800
ttgctggaga cagtagaggc atgaacacac atggatgcac acacacacac gccaatgcac 1860
acacacagag atatgcacac acacggatgc acacacagat ggtcacacag agatacgcaa 1920
acacaccgat gcacacgcac atagagatat gcacacacag atgcacacac agatatacac 1980
atggatgcac gcacatgcca atgcacgcac acatcagtgc acacggatgc acagagatat 2040
gcacacaccg atgtgcgcac acacagatat gcacacacat ggatgagcac acacacacca 2100
atgcgcacac acaccgatgt acacacacag atgcacacac agatgcacac acaccgatgc 2160
tgactccatg tgtgctgtcc tctgaaggcg gttgtttagc tctcactttt ctggttctta 2220
tccattatca tcttcacttc agacaattca gaagcatcac catgcatggt ggcgaatgcc 2280
cccaaactct cccccaaatg tatttctccc ttcgctgggt gccgggctgc acagactatt 2340
ccccacctgc ttcccagctt cacaataaac ggctgcgtct cctccgcaca cctgtggtgc 2400
ctgccaccca ctgggttgcc catgattcat ttttggagcc cccggtgctc atcctctgag 2460
atgctctttt ctttcacaat tttcaacatc actgaaatga accctcacat ggaagctatt 2520
ttttaaaaac aaaagctgtt tgatagatgt ttgaggctgt agctcccagg atcctgtgga 2580
attggatgtt ctctccctgc cacagcccct gtcaatgata tttcacagag accctgggag 2640
cacctgctca agagtcaggg acacacgcat cactaaatgc aagttcccag gccctggctg 2700
cagtgggagg acctggcaag ctgcactctt gctgagtccc cagggtggtg gaagaagaat 2760
gagaaacaca tgaacagaga aatggggagg tgacaaacag tgccccccact cagactccgg 2820
caagcacggc tcagagagtg gactcgatgc catccctgca gggccgtcct gggcaccact 2880
ggcactcaca gcagcaaggt gggcaccatt ggcactcaca gcagcaaggc aggcaccagc 2940
aacccacctc gggggcactc aggcatcatc tacttcagag cagacagggt ctatgaacta 3000
cagccgtggg ctgcttccaa ggcaccctgc tcttgtaaat aaagttttat gggaacacaa 3060
aaaaaaaaaa aaaaa                                                  3075
```

<210> 110
<211> 46
<212> PRT
<213> Homo sapiens

<220>
<223> factor IX Gla domain

<400> 110

Tyr Asn Ser Gly Lys Leu Glu Glu Phe Val Gln Gly Asn Leu Glu Arg
1               5                   10                  15
Glu Cys Met Glu Glu Lys Cys Ser Phe Glu Glu Ala Arg Glu Val Phe
            20                  25                  30
Glu Asn Thr Glu Arg Thr Thr Glu Phe Trp Lys Gln Tyr Val
        35                  40                  45

<210> 111
<211> 45
<212> PRT
<213> Homo sapiens

<220>
<223> factor X Gla domain

<400> 111

Ala Asn Ser Phe Leu Glu Glu Met Lys Lys Gly His Leu Glu Arg Glu
1               5                   10                  15
Cys Met Glu Glu Thr Cys Ser Tyr Glu Glu Ala Arg Glu Val Phe Glu
            20                  25                  30
Asp Ser Asp Lys Thr Asn Glu Phe Trp Asn Lys Tyr Lys
        35                  40                  45

<210> 112
<211> 46
<212> PRT
<213> Homo sapiens

<220>
<223> prothrombin/thrombin Gla domain

<400> 112

Ala Asn Thr Phe Leu Glu Glu Val Arg Lys Gly Asn Leu Glu Arg Glu
1               5                   10                  15
Cys Val Glu Glu Thr Cys Ser Tyr Glu Glu Ala Phe Glu Ala Leu Glu
            20                  25                  30
Ser Ser Thr Ala Thr Asp Val Phe Trp Ala Lys Tyr Thr Ala
        35                  40                  45

<210> 113
<211> 46
<212> PRT
<213> Homo sapiens

<220>
<223> protein C Gla domain

<400> 113

Ala Asn Ser Phe Leu Glu Glu Leu Arg His Ser Ser Leu Glu Arg Glu
1               5                   10                  15
Cys Ile Glu Glu Ile Cys Asp Phe Glu Glu Ala Lys Glu Ile Phe Gln
            20                  25                  30
Asn Val Asp Asp Thr Leu Ala Phe Trp Ser Lys His Val Asp
        35                  40                  45

<210> 114
<211> 46
<212> PRT
<213> Homo sapiens

<220>
<223> protein S Gla domain

<400> 114

Ala Asn Ser Leu Leu Glu Glu Thr Lys Gln Gly Asn Leu Glu Arg Glu
1               5                   10                  15
Cys Ile Glu Glu Leu Cys Asn Lys Glu Glu Ala Arg Glu Val Phe Glu
            20                  25                  30
Asn Asp Pro Glu Thr Asp Tyr Phe Tyr Pro Lys Tyr Leu Val
        35                  40                  45

<210> 115
<211> 47
<212> PRT
<213> Homo sapiens

<220>
<223> osteocalcin (bone Gla protein) Gla domain

<400> 115

Tyr Leu Tyr Gln Trp Leu Gly Ala Pro Val Pro Tyr Pro Asp Pro Leu
1               5                   10                  15
Glu Pro Arg Arg Glu Val Cys Glu Leu Asn Pro Asp Cys Asp Glu Leu
            20                  25                  30
Ala Asp His Ile Gly Phe Gln Glu Ala Tyr Arg Arg Phe Tyr Gly
        35                  40                  45

<210> 116
<211> 47
<212> PRT
<213> Homo sapiens

<220>
<223> matrix Gla protein Gla domain

<400> 116

Arg Ala Lys Val Gln Glu Arg Ile Arg Glu Arg Ser Lys Pro Val His
1               5                   10                  15
Glu Leu Asn Arg Glu Ala Cys Asp Asp Tyr Arg Leu Cys Glu Arg Tyr
            20                  25                  30
Ala Met Val Tyr Gly Tyr Asn Ala Ala Tyr Asn Arg Tyr Phe Arg
        35                  40                  45

<210> 117
<211> 42
<212> PRT
<213> Homo sapiens

<220>
<223> growth-arrest-specific protein 6 Gla domain

<400> 117

```
Phe Glu Glu Ala Lys Gln Gly His Leu Glu Arg Glu Cys Val Glu Glu
1               5                   10                  15
Leu Cys Ser Arg Glu Glu Ala Arg Glu Val Phe Glu Asn Asp Pro Glu
            20                  25                  30
Thr Asp Tyr Phe Tyr Pro Arg Tyr Leu Asp
        35                  40
```

<210> 118
<211> 46
<212> PRT
<213> Homo sapiens

<220>
<223> protein Z Gla domain

<400> 118

```
Ala Gly Ser Tyr Leu Leu Glu Glu Leu Phe Glu Gly Asn Leu Glu Lys
1               5                   10                  15
Glu Cys Tyr Glu Glu Ile Cys Val Tyr Glu Glu Ala Arg Glu Val Phe
            20                  25                  30
Glu Asn Glu Val Val Thr Asp Glu Phe Trp Arg Arg Tyr Lys
        35                  40                  45
```

<210> 119
<211> 45
<212> PRT
<213> Homo sapiens

<220>
<223> factor VII Gla domain

<400> 119

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser
        35                  40                  45
```

<210> 120
<211> 40
<212> PRT
<213> Notechis scutatus scutatus

<220>
<223> Venom coagulation factor Xa-like protease - Gla domain

<220>
<221> UNSURE
<222> (0)...(0)
<223> Xaa could be any amino acid

<400> 120

```
Ser Asn Ser Leu Phe Glu Glu Ile Arg Pro Gly Asn Ile Glu Arg Glu
1               5                   10                  15
Cys Ile Glu Glu Lys Cys Ser Lys Glu Glu Ala Arg Glu Val Phe Glu
            20                  25                  30
Asp Asn Glu Lys Thr Xaa Xaa Phe
        35                  40
```

<210> 121
<211> 46
<212> PRT
<213> Tropidechis carinatus

<220>
<223> Venom coagulation factor Xa-like protease - Gla domain

<400> 121

```
Ser Asn Ser Leu Phe Glu Glu Ile Arg Pro Gly Asn Ile Glu Arg Glu
1               5                   10                  15
Cys Ile Glu Glu Lys Cys Ser Lys Glu Glu Ala Arg Glu Val Phe Glu
            20                  25                  30
Asp Asn Glu Lys Thr Glu Thr Phe Trp Asn Val Tyr Val Asp
        35                  40                  45
```

<210> 122
<211> 464
<212> PRT
<213> Homo sapiens

<220>
<223> Antithrombin III precursor

<400> 122

Met Tyr Ser Asn Val Ile Gly Thr Val Thr Ser Gly Lys Arg Lys Val
1 5 10 15
Tyr Leu Leu Ser Leu Leu Leu Ile Gly Phe Trp Asp Cys Val Thr Cys
20 25 30
His Gly Ser Pro Val Asp Ile Cys Thr Ala Lys Pro Arg Asp Ile Pro
35 40 45
Met Asn Pro Met Cys Ile Tyr Arg Ser Pro Glu Lys Lys Ala Thr Glu
50 55 60
Asp Glu Gly Ser Glu Gln Lys Ile Pro Glu Ala Thr Asn Arg Arg Val
65 70 75 80
Trp Glu Leu Ser Lys Ala Asn Ser Arg Phe Ala Thr Thr Phe Tyr Gln
85 90 95
His Leu Ala Asp Ser Lys Asn Asp Asn Asp Asn Ile Phe Leu Ser Pro
100 105 110
Leu Ser Ile Ser Thr Ala Phe Ala Met Thr Lys Leu Gly Ala Cys Asn
115 120 125
Asp Thr Leu Gln Gln Leu Met Glu Val Phe Lys Phe Asp Thr Ile Ser
130 135 140
Glu Lys Thr Ser Asp Gln Ile His Phe Phe Phe Ala Lys Leu Asn Cys
145 150 155 160
Arg Leu Tyr Arg Lys Ala Asn Lys Ser Ser Lys Leu Val Ser Ala Asn
165 170 175
Arg Leu Phe Gly Asp Lys Ser Leu Thr Phe Asn Glu Thr Tyr Gln Asp
180 185 190
Ile Ser Glu Leu Val Tyr Gly Ala Lys Leu Gln Pro Leu Asp Phe Lys
195 200 205
Glu Asn Ala Glu Gln Ser Arg Ala Ala Ile Asn Lys Trp Val Ser Asn
210 215 220
Lys Thr Glu Gly Arg Ile Thr Asp Val Ile Pro Ser Glu Ala Ile Asn
225 230 235 240
Glu Leu Thr Val Leu Val Leu Val Asn Thr Ile Tyr Phe Lys Gly Leu
245 250 255
Trp Lys Ser Lys Phe Ser Pro Glu Asn Thr Arg Lys Glu Leu Phe Tyr
260 265 270
Lys Ala Asp Gly Glu Ser Cys Ser Ala Ser Met Met Tyr Gln Glu Gly
275 280 285
Lys Phe Arg Tyr Arg Arg Val Ala Glu Gly Thr Gln Val Leu Glu Leu
290 295 300
Pro Phe Lys Gly Asp Asp Ile Thr Met Val Leu Ile Leu Pro Lys Pro
305 310 315 320
Glu Lys Ser Leu Ala Lys Val Glu Lys Glu Leu Thr Pro Glu Val Leu
325 330 335
Gln Glu Trp Leu Asp Glu Leu Glu Glu Met Met Leu Val Val His Met
340 345 350

Pro Arg Phe Arg Ile Glu Asp Gly Phe Ser Leu Lys Glu Gln Leu Gln
355 360 365
Asp Met Gly Leu Val Asp Leu Phe Ser Pro Glu Lys Ser Lys Leu Pro
370 375 380
Gly Ile Val Ala Glu Gly Arg Asp Asp Leu Tyr Val Ser Asp Ala Phe
385 390 395 400
His Lys Ala Phe Leu Glu Val Asn Glu Glu Gly Ser Glu Ala Ala Ala
405 410 415
Ser Thr Ala Val Val Ile Ala Gly Arg Ser Leu Asn Pro Asn Arg Val
420 425 430
Thr Phe Lys Ala Asn Arg Pro Phe Leu Val Phe Ile Arg Glu Val Pro
435 440 445
Leu Asn Thr Ile Ile Phe Met Gly Arg Val Ala Asn Pro Cys Val Lys
450 455 460

<210> 123

<211> 432
<212> PRT
<213> Homo sapiens

<220>
<223> Antithrombin III mature

<400> 123

```
His Gly Ser Pro Val Asp Ile Cys Thr Ala Lys Pro Arg Asp Ile Pro
1               5                   10                  15
Met Asn Pro Met Cys Ile Tyr Arg Ser Pro Glu Lys Lys Ala Thr Glu
            20                  25                  30
Asp Glu Gly Ser Glu Gln Lys Ile Pro Glu Ala Thr Asn Arg Arg Val
        35                  40                  45
Trp Glu Leu Ser Lys Ala Asn Ser Arg Phe Ala Thr Thr Phe Tyr Gln
    50                  55                  60
His Leu Ala Asp Ser Lys Asn Asp Asn Asp Asn Ile Phe Leu Ser Pro
65                  70                  75                  80
Leu Ser Ile Ser Thr Ala Phe Ala Met Thr Lys Leu Gly Ala Cys Asn
                85                  90                  95
Asp Thr Leu Gln Gln Leu Met Glu Val Phe Lys Phe Asp Thr Ile Ser
            100                 105                 110
Glu Lys Thr Ser Asp Gln Ile His Phe Phe Phe Ala Lys Leu Asn Cys
        115                 120                 125
Arg Leu Tyr Arg Lys Ala Asn Lys Ser Ser Lys Leu Val Ser Ala Asn
    130                 135                 140
Arg Leu Phe Gly Asp Lys Ser Leu Thr Phe Asn Glu Thr Tyr Gln Asp
145                 150                 155                 160
Ile Ser Glu Leu Val Tyr Gly Ala Lys Leu Gln Pro Leu Asp Phe Lys
                165                 170                 175
Glu Asn Ala Glu Gln Ser Arg Ala Ala Ile Asn Lys Trp Val Ser Asn
            180                 185                 190
Lys Thr Glu Gly Arg Ile Thr Asp Val Ile Pro Ser Glu Ala Ile Asn
        195                 200                 205
Glu Leu Thr Val Leu Val Leu Val Asn Thr Ile Tyr Phe Lys Gly Leu
    210                 215                 220

Trp Lys Ser Lys Phe Ser Pro Glu Asn Thr Arg Lys Glu Leu Phe Tyr
```

```
225                 230                 235                 240
Lys Ala Asp Gly Glu Ser Cys Ser Ala Ser Met Met Tyr Gln Glu Gly
                245                 250                 255
Lys Phe Arg Tyr Arg Arg Val Ala Glu Gly Thr Gln Val Leu Glu Leu
            260                 265                 270
Pro Phe Lys Gly Asp Asp Ile Thr Met Val Leu Ile Leu Pro Lys Pro
        275                 280                 285
Glu Lys Ser Leu Ala Lys Val Glu Lys Glu Leu Thr Pro Glu Val Leu
    290                 295                 300
Gln Glu Trp Leu Asp Glu Leu Glu Glu Met Met Leu Val Val His Met
305                 310                 315                 320
Pro Arg Phe Arg Ile Glu Asp Gly Phe Ser Leu Lys Glu Gln Leu Gln
                325                 330                 335
Asp Met Gly Leu Val Asp Leu Phe Ser Pro Glu Lys Ser Lys Leu Pro
            340                 345                 350
Gly Ile Val Ala Glu Gly Arg Asp Asp Leu Tyr Val Ser Asp Ala Phe
        355                 360                 365
His Lys Ala Phe Leu Glu Val Asn Glu Glu Gly Ser Glu Ala Ala Ala
    370                 375                 380
Ser Thr Ala Val Val Ile Ala Gly Arg Ser Leu Asn Pro Asn Arg Val
385                 390                 395                 400
Thr Phe Lys Ala Asn Arg Pro Phe Leu Val Phe Ile Arg Glu Val Pro
                405                 410                 415
Leu Asn Thr Ile Ile Phe Met Gly Arg Val Ala Asn Pro Cys Val Lys
            420                 425                 430
```

<210> 124
<211> 263
<212> PRT
<213> Homo sapiens

<220>
<221> THIOLEST
<222> (0)...(0)
<223> Mature tissue factor

<400> 124

```
Ser Gly Thr Thr Asn Thr Val Ala Ala Tyr Asn Leu Thr Trp Lys Ser
1               5                   10                  15
Thr Asn Phe Lys Thr Ile Leu Glu Trp Glu Pro Lys Pro Val Asn Gln
            20                  25                  30
Val Tyr Thr Val Gln Ile Ser Thr Lys Ser Gly Asp Trp Lys Ser Lys
        35                  40                  45
Cys Phe Tyr Thr Thr Asp Thr Glu Cys Asp Leu Thr Asp Glu Ile Val
    50                  55                  60
Lys Asp Val Lys Gln Thr Tyr Leu Ala Arg Val Phe Ser Tyr Pro Ala
65                  70                  75                  80
Gly Asn Val Glu Ser Thr Gly Ser Ala Gly Glu Pro Leu Tyr Glu Asn
                85                  90                  95
Ser Pro Glu Phe Thr Pro Tyr Leu Glu Thr Asn Leu Gly Gln Pro Thr
            100                 105                 110
Ile Gln Ser Phe Glu Gln Val Gly Thr Lys Val Asn Val Thr Val Glu
        115                 120                 125
Asp Glu Arg Thr Leu Val Arg Arg Asn Asn Thr Phe Leu Ser Leu Arg
```

```
        130                 135                 140
Asp Val Phe Gly Lys Asp Leu Ile Tyr Thr Leu Tyr Tyr Trp Lys Ser
145                 150                 155                 160
Ser Ser Ser Gly Lys Lys Thr Ala Lys Thr Asn Thr Asn Glu Phe Leu
                165                 170                 175
Ile Asp Val Asp Lys Gly Glu Asn Tyr Cys Phe Ser Val Gln Ala Val
            180                 185                 190
Ile Pro Ser Arg Thr Val Asn Arg Lys Ser Thr Asp Ser Pro Val Glu
        195                 200                 205
Cys Met Gly Gln Glu Lys Gly Glu Phe Arg Glu Ile Phe Tyr Ile Ile
    210                 215                 220
Gly Ala Val Val Phe Val Val Ile Ile Leu Val Ile Ile Leu Ala Ile
225                 230                 235                 240
Ser Leu His Lys Cys Arg Lys Ala Gly Val Gly Gln Ser Trp Lys Glu
                245                 250                 255
Asn Ser Pro Leu Asn Val Ser
            260
```

<210> 125
<211> 406
<212> PRT
<213> Homo sapiens

<220>
<223> Factor VIIa variant D60Y

<400> 125

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Tyr Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
```

210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 126
<211> 406
<212> PRT
<213> Homo sapiens

<220>
<223> Factor VIIa variant D60F

<400> 126

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro

```
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Phe Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 127
<211> 406
<212> PRT
<213> Homo sapiens

<220>
<223> Factor VIIa variant D60W

<400> 127

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
```

```
                            85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Trp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 128
<211> 406
<212> PRT
<213> Homno sapiens

<220>
<223> Factor VIIa variant D60L

<400> 128

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
```

20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Leu Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 129
<211> 406
<212> PRT
<213> Homo sapiens

<220>
<223> Factor VIIa variant D60I

<400> 129

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Ile Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400

Leu Arg Ala Pro Phe Pro
                405

<210> 130
<211> 406
<212> PRT
<213> Homo sapiens

<220>

<223> Factor VIIa variant K60al

<400> 130

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Ile Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 131
<211> 406
<212> PRT

<213> Homo sapiens

<220>
<223> Factor VIIa variant K60aV

<400> 131

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys

            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Val Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr


            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 132
<211> 406
<212> PRT
<213> Homo sapiens

<220>
<223> Factor VIIa variant K60aF

<400> 132

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Phe Ile Lys Asn Trp Arg Asn Leu Ile Ala Val Leu
```

```
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro

                405
```

<210> 133
<211> 406
<212> PRT
<213> Homo sapiens

<220>
<223> Factor VIIa variant K60aW

<400> 133

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
```

```
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Trp Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 134
<211> 406
<212> PRT
<213> Homo sapiens

<220>
<223> Factor VIIa variant G97W

<400> 134

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
```

Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Trp Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 135
<211> 406
<212> PRT
<213> Homo sapiens

<220>
<223> Factor VIIa variant G97T

<400> 135

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Thr Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 136
<211> 406
<212> PRT
<213> Homo sapiens

<220>
<223> Factor VIIa variant G97I

<400> 136

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Ile Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile


    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 137
<211> 406
<212> PRT
<213> Homo sapiens

<220>
<223> Factor VIIa variant G97V

<400> 137

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Val Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser

305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 138

<211> 406
<212> PRT
<213> Homo sapiens

<220>
<223> Factor VIIa variant K192Q

<400> 138

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
```

```
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Gln Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 139
<211> 406
<212> PRT
<213> Homo sapiens

<220>
<223> Factor VIIa variant D60K/K60aL

<400> 139

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
```

```
            180                 185                 190
His Cys Phe Lys Leu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                     240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                     320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                     400
Leu Arg Ala Pro Phe Pro
                405
```

<210> 140
<211> 406
<212> PRT
<213> Homo sapiens

<220>
<223> Factor VIIa variant D60F/K60aL

<400> 140

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
```

```
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Phe Leu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 141
<211> 406
<212> PRT
<213> Homo sapiens

<220>
<223> Factor VIIa variant D60L/K60aL

<400> 141

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
```

```
        50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Leu Leu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 142
<211> 406
<212> PRT
<213> Homo sapiens

<220>
<223> Factor VIIa variant D60M/K60aL

<400> 142

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Met Leu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 143
<211> 406
<212> PRT
<213> Homo sapiens

<220>
<223> Factor VIIa variant D60W/K60aL

<400> 143

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Trp Leu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365

Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 144
<211> 406
<212> PRT
<213> Homo sapiens

<220>
<223> Factor VIIa variant D60F/K60aE

<400> 144

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Phe Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300

Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 145

<211> 406
<212> PRT
<213> Homo sapiens

<220>
<223> Factor VIIa variant D60W/K60aE

<400> 145

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Trp Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
```

His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 146
<211> 406
<212> PRT
<213> Homo sapiens

<220>
<223> Factor VIIa variant D60V/K60aE

<400> 146

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175

Leu Cys Gly Gly Thr Leu Ile Asn Thr Ile Trp Val Val Ser Ala Ala
180 185 190
His Cys Phe Val Glu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 147
<211> 406
<212> PRT
<213> Homo sapiens

<220>
<223> Factor VIIa variant L163V/S170bE/K188A/F225Y

<400> 147

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110

```
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Val Met Thr Gln Asp Cys Leu Gln Gln Glu Arg Lys Val Gly Asp Ser
305                 310                 315                 320
Pro Asn Ile Thr Glu Tyr Met Phe Cys Ala Gly Tyr Ser Asp Gly Ser
                325                 330                 335
Ala Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Tyr Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 148
<211> 406
<212> PRT
<213> Homo sapiens

<220>
<223> Factor VIIa variant M156Q

<400> 148

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
```

```
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Gln Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 149
<211> 406
<212> PRT
<213> Homo sapiens

<220>
<223> Factor VIIa variant V21D/E154V/M156Q

<400> 149

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Asp Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Val Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 150
<211> 406
<212> PRT
<213> Homo sapiens

<220>
<223> Factor VIIa variant V21D/E154V/M156Q/K188A

<400> 150

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Asp Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Val Leu Gln Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Ala Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 151
<211> 4278
<212> DNA
<213> Artificial Sequence

<220>
<223> pCMV Script vector

<400> 151

atgcattagt tattaatagt aatcaattac ggggtcatta gttcatagcc catatatgga 60
gttccgcgtt acataactta cggtaaatgg cccgcctggc tgaccgccca acgacccccg 120
cccattgacg tcaataatga cgtatgttcc catagtaacg ccaataggga ctttccattg 180
acgtcaatgg gtggagtatt tacggtaaac tgcccacttg gcagtacatc aagtgtatca 240
tatgccaagt acgcccccta ttgacgtcaa tgacggtaaa tggcccgcct ggcattatgc 300
ccagtacatg accttatggg actttcctac ttggcagtac atctacgtat tagtcatcgc 360
tattaccatg gtgatgcggt tttggcagta catcaatggg cgtggatagc ggtttgactc 420
acggggattt ccaagtctcc accccattga cgtcaatggg agtttgtttt ggcaccaaaa 480
tcaacgggac tttccaaaat gtcgtaacaa ctccgcccca ttgacgcaaa tgggcggtag 540
gcgtgtacgg tgggaggtct atataagcag agctggttta gtgaaccgtc agatccgcta 600
gcgattacgc caagctcgaa attaaccctc actaaaggga acaaaagctg gagctccacc 660
gcggtggcgg ccgctctagc ccgggcggat cccccgggct gcaggaattc gatatcaagc 720

ttatcgatac cgtcgacctc gagggggggc ccggtaccag gtaagtgtac ccaattcgcc 780
ctatagtgag tcgtattaca attcactcga tcgcccttcc caacagttgc gcagcctgaa 840
tggcgaatgg agatccaatt tttaagtgta taatgtgtta aactactgat tctaattgtt 900
tgtgtatttt agattcacag tcccaaggct catttcaggc ccctcagtcc tcacagtctg 960
ttcatgatca taatcagcca taccacattt gtagaggttt tacttgcttt aaaaaacctc 1020
ccacacctcc ccctgaacct gaaacataaa atgaatgcaa ttgttgttgt taacttgttt 1080
attgcagctt ataatggtta caaataaagc aatagcatca caaatttcac aaataaagca 1140
tttttttcac tgcattctag ttgtggtttg tccaaactca tcaatgtatc ttaacgcgta 1200
aattgtaagc gttaatattt tgttaaaatt cgcgttaaat ttttgttaaa tcagctcatt 1260
ttttaaccaa taggccgaaa tcggcaaaat cccttataaa tcaaaagaat agaccgagat 1320
agggttgagt gttgttccag tttggaacaa gagtccacta ttaaagaacg tggactccaa 1380
cgtcaaaggg cgaaaaaccg tctatcaggg cgatggccca ctacgtgaac catcacccta 1440
atcaagtttt ttggggtcga ggtgccgtaa agcactaaat cggaacccta aagggagccc 1500
ccgatttaga gcttgacggg gaaagccggc gaacgtggcg agaaaggaag ggaagaaagc 1560
gaaaggagcg ggcgctaggg cgctggcaag tgtagcggtc acgctgcgcg taaccaccac 1620
acccgccgcg cttaatgcgc cgctacaggg cgcgtcaggt ggcacttttc ggggaaatgt 1680
gcgcggaacc cctatttgtt tatttttcta aatacattca aatatgtatc cgctcatgag 1740
acaataaccc tgataaatgc ttcaataata ttgaaaaagg aagaatcctg aggcggaaag 1800
aaccagctgt ggaatgtgtg tcagttaggg tgtggaaagt ccccaggctc cccagcaggc 1860
agaagtatgc aaagcatgca tctcaattag tcagcaacca ggtgtggaaa gtccccaggc 1920
tccccagcag gcagaagtat gcaaagcatg catctcaatt agtcagcaac catagtcccg 1980
cccctaactc cgcccatccc gcccctaact ccgcccagtt ccgcccattc tccgccccat 2040
ggctgactaa ttttttttat ttatgcagag gccgaggccg cctcggcctc tgagctattc 2100
cagaagtagt gaggaggctt ttttggaggc ctaggctttt gcaaagatcg atcaagagac 2160

aggatgagga tcgtttcgca tgattgaaca agatggattg cacgcaggtt ctccggccgc 2220
ttgggtggag aggctattcg gctatgactg ggcacaacag acaatcggct gctctgatgc 2280
cgccgtgttc cggctgtcag cgcaggggcg cccggttctt tttgtcaaga ccgacctgtc 2340
cggtgccctg aatgaactgc aagacgaggc agcgcggcta tcgtggctgg ccacgacggg 2400
cgttccttgc gcagctgtgc tcgacgttgt cactgaagcg ggaagggact ggctgctatt 2460
gggcgaagtg ccggggcagg atctcctgtc atctcacctt gctcctgccg agaaagtatc 2520
catcatggct gatgcaatgc ggcggctgca tacgcttgat ccggctacct gcccattcga 2580
ccaccaagcg aaacatcgca tcgagcgagc acgtactcgg atggaagccg gtcttgtcga 2640
tcaggatgat ctggacgaag aacatcaggg gctcgcgcca gccgaactgt tcgccaggct 2700
caaggcgagc atgcccgacg gcgaggatct cgtcgtgacc catggcgatg cctgcttgcc 2760
gaatatcatg gtggaaaatg gccgcttttc tggattcatc gactgtggcc ggctgggtgt 2820
ggcggaccgc tatcaggaca tagcgttggc tacccgtgat attgctgaag aacttggcgg 2880
cgaatgggct gaccgcttcc tcgtgcttta cggtatcgcc gctcccgatt cgcagcgcat 2940
cgccttctat cgccttcttg acgagttctt ctgagcggga ctctggggtt cgaaatgacc 3000
gaccaagcga cgcccaacct gccatcacga gatttcgatt ccaccgccgc cttctatgaa 3060
aggttgggct tcggaatcgt tttccgggac gccggctgga tgatcctcca gcgcggggat 3120
ctcatgctgg agttcttcgc ccaccctagg gggaggctaa ctgaaacacg gaaggagaca 3180
ataccggaag gaacccgcgc tatgacggca ataaaaagac agaataaaac gcacggtgtt 3240
gggtcgtttg ttcataaacg cggggttcgg tcccagggct ggcactctgt cgatacccca 3300
ccgagacccc attggggcca atacgcccgc gtttcttcct tttccccacc ccaccccccca 3360
agttcgggtg aaggcccagg gctcgcagcc aacgtcgggg cggcaggccc tgccatagcc 3420
tcaggttact catatatact ttagattgat ttaaaacttc atttttaatt taaaaggatc 3480
taggtgaaga tcctttttga taatctcatg accaaaatcc cttaacgtga gttttcgttc 3540
cactgagcgt cagaccccgt agaaaagatc aaaggatctt cttgagatcc tttttttctg 3600
cgcgtaatct gctgcttgca aacaaaaaaa ccaccgctac cagcggtggt ttgtttgccg 3660
gatcaagagc taccaactct ttttccgaag gtaactggct tcagcagagc gcagatacca 3720
aatactgtcc ttctagtgta gccgtagtta ggccaccact tcaagaactc tgtagcaccg 3780
cctacatacc tcgctctgct aatcctgtta ccagtggctg ctgccagtgg cgataagtcg 3840
tgtcttaccg ggttggactc aagacgatag ttaccggata aggcgcagcg gtcgggctga 3900
acggggggtt cgtgcacaca gcccagcttg gagcgaacga cctacaccga actgagatac 3960
ctacagcgtg agctatgaga aagcgccacg cttcccgaag ggagaaaggc ggacaggtat 4020
ccggtaagcg gcagggtcgg aacaggagag cgcacgaggg agcttccagg gggaaacgcc 4080
tggtatcttt atagtcctgt cgggtttcgc cacctctgac ttgagcgtcg atttttgtga 4140
tgctcgtcag gggggcggag cctatggaaa aacgccagca acgcggcctt tttacggttc 4200
ctggcctttt gctggccttt tgctcacatg ttctttcctg cgttatcccc tgattctgtg 4260
gataaccgta ttaccgcc 4278

<210> 152
<211> 45
<212> DNA
<213> Artificial Sequence

<220>
<223> CBO-125 Factor VII Forward PCR Primer

<400> 152
gcatcatgac gtgacggatc cgccaccatg gtctcccagg ccctc 45

<210> 153
<211> 45
<212> DNA
<213> Artificial Sequence

<220>
<223> CBO-126 Factor VII Reverse PCR Primer

<400> 153
gatcgtacga tacgtgaatt cctagggaaa tggggctcgc aggag 45

<210> 154

<211> 1398
<212> DNA
<213> Homo sapiens

<220>
<223> FVII cDNA (template for PCR)

<400> 154

```
atggtctccc aggccctcag gctcctctgc cttctgcttg ggcttcaggg ctgcctggct 60
gcaggcgggg tcgctaaggc ctcaggagga gaaacacggg acatgccgtg gaagccgggg 120
cctcacagag tcttcgtaac ccaggaggaa gcccacggcg tcctgcaccg gcgccggcgc 180
gccaacgcgt tcctggagga gctgcggccg ggctccctgg agagggagtg caaggaggag 240
cagtgctcct tcgaggaggc ccgggagatc ttcaaggacg cggagaggac gaagctgttc 300
tggatttctt acagtgatgg ggaccagtgt gcctcaagtc catgccagaa tgggggctcc 360
tgcaaggacc agctccagtc ctatatctgc ttctgcctcc ctgccttcga gggccggaac 420
tgtgagacgc acaaggatga ccagctgatc tgtgtgaacg agaacggcgg ctgtgagcag 480
tactgcagtg accacacggg caccaagcgc tcctgtcggt gccacgaggg gtactctctg 540
ctggcagacg gggtgtcctg cacacccaca gttgaatatc catgtggaaa aatacctatt 600
ctagaaaaaa gaaatgccag caaaccccaa ggccgaattg tgggggggcaa ggtgtgcccc 660
aaaggggagt gtccatggca ggtcctgttg ttggtgaatg gagctcagtt gtgtgggggg 720
accctgatca acaccatctg ggtggtctcc gcggcccact gtttcgacaa aatcaagaac 780
tggaggaacc tgatcgcggt gctgggcgag cacgacctca gcgagcacga cggggatgag 840
cagagccggc gggtggcgca ggtcatcatc cccagcacgt acgtcccggg caccaccaac 900
cacgacatcg cgctgctccg cctgcaccag cccgtggtcc tcactgacca tgtggtgccc 960
ctctgcctgc ccgaacggac gttctctgag aggacgctgg ccttcgtgcg cttctcattg 1020
gtcagcggct ggggccagct gctggaccgt ggcgccacgg ccctggagct catggtcctc 1080
aacgtgcccc ggctgatgac ccaggactgc ctgcagcagt cacggaaggt gggagactcc 1140
ccaaatatca cggagtacat gttctgtgcc ggctactcgg atggcagcaa ggactcctgc 1200
aagggggaca gtggaggccc acatgccacc cactaccggg gcacgtggta cctgacgggc 1260
atcgtcagct ggggccaggg ctgcgcaacc gtgggccact ttggggtgta caccagggtc 1320
tcccagtaca tcgagtggct gcaaaagctc atgcgctcag agccacgccc aggagtcctc 1380
ctgcgagccc catttccc                                               1398
```

<210> 155
<211> 33
<212> DNA
<213> Artificial Sequence

<220>
<223> D60 wild-type primer

<400> 155
gcggcccact gtttcgacaa aatcaagaac tgg 33

<210> 156
<211> 33
<212> DNA
<213> Artificial Sequence

<220>
<223> CBO-157 D60K primer

<400> 156
gcggcccact gtttcaagaa aatcaagaac tgg 33

<210> 157
<211> 33
<212> DNA

<213> Artificial Sequence

<220>
<223> CBO-158 D60R primer

<400> 157
gcggcccact gtttcaggaa aatcaagaac tgg 33

<210> 158
<211> 33
<212> DNA
<213> Artificial Sequence

<220>
<223> CBO-159 D60A primer

<400> 158
gcggcccact gtttcgcgaa aatcaagaac tgg 33

<210> 159
<211> 33
<212> DNA
<213> Artificial Sequence

<220>
<223> K60a wild-type primer

<400> 159
gcccactgtt tcgacaaaat caagaactgg agg 33

<210> 160
<211> 33
<212> DNA
<213> Artificial Sequence

<220>
<223> CBO-160 K60aD primer

<400> 160
gcccactgtt tcgacgacat caagaactgg agg 33

<210> 161
<211> 33
<212> DNA
<213> Artificial Sequence

<220>
<223> CBO-161 K60aE primer

<400> 161
gcccactgtt tcgacgagat caagaactgg agg 33

<210> 162
<211> 33
<212> DNA
<213> Artificial Sequence

<220>

<221> allele
<222> (0) ... (0)

<220>
<223> CBO-162 K60aA primer

<400> 162
gcccactgtt tcgacgcgat caagaactgg agg 33

<210> 163
<211> 33
<212> DNA
<213> Artificial Sequence

<220>
<223> CBO-163 K60aL primer

<400> 163
gcccactgtt tcgacctcat caagaactgg agg 33

<210> 164
<211> 33
<212> DNA
<213> Artificial Sequence

<220>
<223> CBO-164 K60aY primer

<400> 164
gcccactgtt tcgactatat caagaactgg agg 33

<210> 165
<211> 33
<212> DNA
<213> Artificial Sequence

<220>
<223> K60c wild-type primer

<400> 165
tgtttcgaca aaatcaagaa ctggaggaac ctg 33

<210> 166
<211> 33
<212> DNA
<213> Artificial Sequence

<220>
<223> CBO-165 K60cD primer

<400> 166
tgtttcgaca aaatcgacaa ctggaggaac ctg 33

<210> 167
<211> 33
<212> DNA
<213> Artificial Sequence

<220>
<223> CBO-156 K60cE primer

<400> 167
tgtttcgaca aaatcgagaa ctggaggaac ctg 33

<210> 168
<211> 33
<212> DNA
<213> Artificial Sequence

<220>
<223> CBO-167 K60cA primer

<400> 168
tgtttcgaca aaatcgcgaa ctggaggaac ctg 33

<210> 169
<211> 33
<212> DNA
<213> Artificial Sequence

<220>
<223> T99 wild-type primer

<400> 169
tacgtcccgg gcaccaccaa ccacgacatc gcg 33

<210> 170
<211> 33
<212> DNA
<213> Artificial Sequence

<220>
<223> CBO-168 T99A primer

<400> 170
tacgtcccgg gcaccgcgaa ccacgacatc gcg 33

<210> 171
<211> 33
<212> DNA
<213> Artificial Sequence

<220>
<223> R147 wild-type primer

<400> 171
ggccagctgc tggaccgtgg cgccacggcc ctg 33

<210> 172
<211> 33
<212> DNA
<213> Artificial Sequence

<220>
<223> CBO-169 R147D primer

<400> 172
ggccagctgc tggacgacgg cgccacggcc ctg 33

<210> 173
<211> 33
<212> DNA
<213> Artificial Sequence

<220>
<223> CBO-170 R147E primer

<400> 173
ggccagctgc tggacgaggg cgccacggcc ctg 33

<210> 174
<211> 33
<212> DNA
<213> Artificial Sequence

<220>
<223> CBO-171 R147A primer

<400> 174
ggccagctgc tggacgcggg cgccacggcc ctg 33

<210> 175
<211> 33
<212> DNA
<213> Artificial Sequence

<220>
<223> K192 wild-type primer

<400> 175
agcaaggact cctgcaaggg ggacagtgga ggc 33

<210> 176
<211> 33
<212> DNA
<213> Artificial Sequence

<220>
<223> CBO-172 K192R primer

<400> 176
agcaaggact cctgccgcgg ggacagtgga ggc 33

<210> 177
<211> 60
<212> DNA
<213> Artificial Sequence

<220>
<223> multiple mutants wild-type primer

<400> 177
gtggtctccg cggcccactg tttcgacaaa atcaagaact ggaggaacct gatcgcggtg 60

<210> 178
<211> 60
<212> DNA
<213> Artificial Sequence

<220>
<223> CBO-177 D60R / K60aE / K60cE primer

<400> 178
gtggtctccg cggcccactg tttcagggag atcgaaaact ggaggaacct gatcgcggtg 60

<210> 179
<211> 60
<212> DNA
<213> Artificial Sequence

<220>
<223> CBO-178 D60 K K60aE / K60cE primer

<400> 179
gtggtctccg cggcccactg tttcaaggag atcgaaaact ggaggaacct gatcgcggtg 60

<210> 180
<211> 60
<212> DNA
<213> Artificial Sequence

<220>
<223> CBO-179 D60R / K60aM / K60cE primer

<404> 180
gtggtctccg cggcccactg tttcaggatg atcgaaaact ggaggaacct gatcgcggtg 60

<210> 181
<211> 33
<212> DNA
<213> Artificial Sequence

<220>
<223> CBOLH-54 K60aI primer

<400> 181
gcccactgtt tcgacatcat caagaactgg agg 33

<210> 182
<211> 33
<212> DNA
<213> Artificial Sequence

<220>
<223> CBOLH-55 K60aV primer

<400> 182
gcccactgtt tcgacgtcat caagaactgg agg 33

<210> 183
<211> 33
<212> DNA

<213> Artificial Sequence

<220>
<223> CBOLH-56 K60aF primer

<400> 183
gcccactgtt tcgacttcat caagaactgg agg 33

<210> 184
<211> 33
<212> DNA
<213> Artificial Sequence

<220>
<223> CBOLH-57 K60aW primer

<400> 184
gcccactgtt tcgactggat caagaactgg agg 33

<210> 185
<211> 33
<212> DNA
<213> Artificial Sequence

<220>
<223> CBOLH-58 K60aM primer

<400> 185
gcccactgtt tcgacatgat caagaactgg agg 33

<210> 186
<211> 33
<212> DNA
<213> Artificial Sequence

<220>
<223> CBOLH-59 D60F primer

<400> 186
gcggcccact gtttctttaa aatcaagaac tgg 33

<210> 187
<211> 33
<212> DNA
<213> Artificial Sequence

<220>
<223> CBOLH-60 D60Y primer

<400> 187
gcggcccact gtttcataaa aatcaagaac tgg 33

<210> 188
<211> 33
<212> DNA
<213> Artificial Sequence

<220>

<223> CBOLH-61 D60W primer

<400> 188
gcggcccact gtttctggaa aatcaagaac tgg 33

<210> 189
<211> 33
<212> DNA
<213> Artificial Sequence

<220>
<223> CBOLH-62 D60L primer

<400> 189
gcggcccact gtttcgagaa aatcaagaac tgg 33

<210> 190
<211> 33
<212> DNA
<213> Artificial Sequence

<220>
<223> CBOLH-63 D60I primer

<400> 190
gcggcccact gtttcatcaa aatcaagaac tgg 33

<210> 191
<211> 33
<212> DNA
<213> Artificial Sequence

<220>
<223> G97 wild-type primer

<400> 191
agcacgtacg tcccgggcac caccaaccac gac 33

<210> 192
<211> 33
<212> DNA
<213> Artificial Sequence

<220>
<223> CBOLH-64 G97W primer

<400> 192
agcacgtacg tcccgtggac caccaaccac gac 33

<210> 193
<211> 33
<212> DNA
<213> Artificial Sequence

<220>
<223> CBOLH-65 G97T primer

<400> 193

agcacgtacg tcccgacgac caccaaccac gac 33

<210> 194
<211> 33
<212> DNA
<213> Artificial Sequence

<220>
<223> CBOLH-66 G97I primer

<400> 194
agcacgtacg tcccgatcac caccaaccac gac 33

<210> 195
<211> 33
<212> DNA
<213> Artificial Sequence

<220>
<223> CBOLH-67 G97V primer

<400> 195
agcacgtacg tcccggtcac caccaaccac gac 33

<210> 196
<211> 33
<212> DNA
<213> Artificial Sequence

<220>
<223> CBOLH-68 K192Q primer

<400> 196
agcaaggact cctgccaggg ggacagtgga ggc 33

<210> 197
<211> 33
<212> DNA
<213> Artificial Sequence

<220>
<223> K60aL/D60 wild-type primer

<400> 197
gcggcccact gtttcgacaa aatcaagaac tgg 33

<210> 198
<211> 33
<212> DNA
<213> Artificial Sequence

<220>
<223> CBOLH-69 K60aL/D60K primer

<400> 198
gcggcccact gtttcaagct catcaagaac tgg 33

<210> 199

<211> 33
<212> DNA
<213> Artificial Sequence

<220>
<223> CBOLH-70 K60aL/D60L primer

<400> 199
gcggcccact gtttcctcct catcaagaac tgg 33

<210> 200
<211> 33
<212> DNA
<213> Artificial Sequence

<220>
<223> CBOLH-71 K60aL/D60F primer

<400> 200
gcggcccact gtttctttct catcaagaac tgg 33

<210> 201
<211> 33
<212> DNA
<213> Artificial Sequence

<220>
<223> CBOLH-72 K60aL/D60M primer

<400> 201
gcggcccact gtttcatgct catcaagaac tgg 33

<210> 202
<211> 33
<212> DNA
<213> Artificial Sequence

<220>
<223> CBOLH-73 K60aL/D60W primer

<400> 202
gcggcccact gtttctggct catcaagaac tgg 33

<210> 203
<211> 33
<212> DNA
<213> Artificial Sequence

<220>
<223> CBOLH-74 D60F primer

<400> 203
gcggcccact gtttctttga gatcaagaac tgg 33

<210> 204
<211> 33
<212> DNA
<213> Artificial Sequence

<220>
<223> CBOLH-75 D60W primer

<400> 204
gcggcccact gtttctggga gatcaagaac tgg 33

<210> 205
<211> 33
<212> DNA
<213> Artificial Sequence

<220>
<223> CBOLH-76 D60V primer

<400> 205
gcggcccact gtttcgtcga gatcaagaac tgg 33

<210> 206
<211> 406
<212> PRT
<213> Homo sapiens

<220>
<223> R290N

<400> 206

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Asn Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 207
<211> 406
<212> PRT
<213> Homo sapiens

<220>

<223> R290Q

<400> 207

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Gln Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 208
<211> 406

<212> PRT
<213> Homo sapiens

<220>
<223> R290K

<400> 208

```
Ala Asn Ala Phe Leu Glu Glu Leu Arg Pro Gly Ser Leu Glu Arg Glu
 1               5                  10                  15

Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
```

```
                180                     185                     190
His Cys Phe Asp Lys Ile Lys Asn Trp Arg Asn Leu Ile Ala Val Leu
            195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Lys Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys

        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 209
<211> 406
<212> PRT
<213> Homo sapiens

<220>
<223> R290M

<400> 209

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
```

```
                100                 105                 110
Arg Cys His Glu Gly Tyr Ser Leu Leu Ala Asp Gly Val Ser Cys Thr
            115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Met Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 210
<211> 406
<212> PRT
<213> Homo sapiens

<220>
<223> R290V

<400> 210

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
```

```
                35                      40                      45
        Gln Cys Ala Ser Ser Pro Cys Gln Asn Gly Gly Ser Cys Lys Asp Gln
            50                      55                      60
        Leu Gln Ser Tyr Ile Cys Phe Cys Leu Pro Ala Phe Glu Gly Arg Asn
        65                      70                      75                  80
        Cys Glu Thr His Lys Asp Asp Gln Leu Ile Cys Val Asn Glu Asn Gly
                            85                      90                      95
        Gly Cys Glu Gln Tyr Cys Ser Asp His Thr Gly Thr Lys Arg Ser Cys
                        100                     105                     110
        Arg Cys His Glu Gly Tyr Ser Leu Leu Ala Asp Gly Val Ser Cys Thr
                115                     120                     125
        Pro Thr Val Glu Tyr Pro Cys Gly Lys Ile Pro Ile Leu Glu Lys Arg
            130                     135                     140
        Asn Ala Ser Lys Pro Gln Gly Arg Ile Val Gly Gly Lys Val Cys Pro
        145                     150                     155                 160
        Lys Gly Glu Cys Pro Trp Gln Val Leu Leu Leu Val Asn Gly Ala Gln
                            165                     170                     175
        Leu Cys Gly Gly Thr Leu Ile Asn Thr Ile Trp Val Val Ser Ala Ala
                        180                     185                     190
        His Cys Phe Asp Lys Ile Lys Asn Trp Arg Asn Leu Ile Ala Val Leu
                195                     200                     205
        Gly Glu His Asp Leu Ser Glu His Asp Gly Asp Glu Gln Ser Arg Arg
            210                     215                     220
        Val Ala Gln Val Ile Ile Pro Ser Thr Tyr Val Pro Gly Thr Thr Asn
        225                     230                     235                 240
        His Asp Ile Ala Leu Leu Arg Leu His Gln Pro Val Val Leu Thr Asp
                            245                     250                     255
        His Val Val Pro Leu Cys Leu Pro Glu Arg Thr Phe Ser Glu Arg Thr
                        260                     265                     270
        Leu Ala Phe Val Arg Phe Ser Leu Val Ser Gly Trp Gly Gln Leu Leu
                275                     280                     285
        Asp Val Gly Ala Thr Ala Leu Glu Leu Met Val Leu Asn Val Pro Arg
            290                     295                     300
        Leu Met Thr Gln Asp Cys Leu Gln Gln Ser Arg Lys Val Gly Asp Ser
        305                     310                     315                 320
        Pro Asn Ile Thr Glu Tyr Met Phe Cys Ala Gly Tyr Ser Asp Gly Ser
                            325                     330                     335
        Lys Asp Ser Cys Lys Gly Asp Ser Gly Gly Pro His Ala Thr His Tyr
                        340                     345                     350
        Arg Gly Thr Trp Tyr Leu Thr Gly Ile Val Ser Trp Gly Gln Gly Cys
                355                     360                     365

        Ala Thr Val Gly His Phe Gly Val Tyr Thr Arg Val Ser Gln Tyr Ile
            370                     375                     380
        Glu Trp Leu Gln Lys Leu Met Arg Ser Glu Pro Arg Pro Gly Val Leu
        385                     390                     395                 400
        Leu Arg Ala Pro Phe Pro
                            405
```

<210> 211
<211> 406
<212> PRT
<213> Homo sapiens

<220>
<223> K341N

<400> 211

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Asn Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro

405

<210> 212
<211> 406
<212> PRT
<213> Homo sapiens

<220>

<223> K341M

<400> 212

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Met Gly Asp Ser Gly Gly Pro His Ala Thr His Tyr

            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 213
<211> 406

<212> PRT
<213> Homo sapiens

<220>
<223> K341D

<400> 213

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
```

```
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Asp Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 214
<211> 407
<212> PRT
<213> Homo sapiens

<220>
<223> G237T238insA

<400> 214

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
```

```
    210                     215                     220
Val Ala Gln Val Ile Ile Pro Ser Thr Tyr Val Pro Gly Ala Thr Thr
225                 230                     235                 240
Asn His Asp Ile Ala Leu Leu Arg Leu His Gln Pro Val Val Leu Thr
                245                     250                 255
Asp His Val Val Pro Leu Cys Leu Pro Glu Arg Thr Phe Ser Glu Arg
            260                     265                 270
Thr Leu Ala Phe Val Arg Phe Ser Leu Val Ser Gly Trp Gly Gln Leu
        275                     280                 285
Leu Asp Arg Gly Ala Thr Ala Leu Glu Leu Met Val Leu Asn Val Pro
    290                     295                 300
Arg Leu Met Thr Gln Asp Cys Leu Gln Gln Ser Arg Lys Val Gly Asp
305                 310                     315                 320
Ser Pro Asn Ile Thr Glu Tyr Met Phe Cys Ala Gly Tyr Ser Asp Gly
                325                     330                 335
Ser Lys Asp Ser Cys Lys Gly Asp Ser Gly Gly Pro His Ala Thr His
            340                     345                 350
Tyr Arg Gly Thr Trp Tyr Leu Thr Gly Ile Val Ser Trp Gly Gln Gly
        355                     360                 365
Cys Ala Thr Val Gly His Phe Gly Val Tyr Thr Arg Val Ser Gln Tyr
    370                     375                 380
Ile Glu Trp Leu Gln Lys Leu Met Arg Ser Glu Pro Arg Pro Gly Val
385                 390                     395                 400
Leu Leu Arg Ala Pro Phe Pro
                405
```

<210> 215
<211> 407
<212> PRT
<213> Homo sapiens

<220>
<223> G237T238insS

<400> 215

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
```

```
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg

    210                 215                 220
Val Ala Gln Val Ile Ile Pro Ser Thr Tyr Val Pro Gly Ser Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro

                405
```

<210> 216
<211> 407
<212> PRT
<213> Homo sapiens

<220>
<223> G237T238insV

<400> 216

```
Ala Asn Ala Phe Leu Glu Glu Leu Arg Pro Gly Ser Leu Glu Arg Glu
 1               5                  10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
```

```
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Val Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 217
<211> 408
<212> PRT
<213> Homo sapiens

<220>
<223> G237T238insAS

<400> 217

```
Ala Asn Ala Phe Leu Glu Glu Leu Arg Pro Gly Ser Leu Glu Arg Glu
```

```
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Ala Ser Thr
225                 230                 235                 240
Thr Asn His Asp Ile Ala Leu Leu Arg Leu His Gln Pro Val Val Leu
                245                 250                 255
Thr Asp His Val Val Pro Leu Cys Leu Pro Glu Arg Thr Phe Ser Glu
            260                 265                 270
Arg Thr Leu Ala Phe Val Arg Phe Ser Leu Val Ser Gly Trp Gly Gln
        275                 280                 285
Leu Leu Asp Arg Gly Ala Thr Ala Leu Glu Leu Met Val Leu Asn Val
    290                 295                 300
Pro Arg Leu Met Thr Gln Asp Cys Leu Gln Gln Ser Arg Lys Val Gly
305                 310                 315                 320
Asp Ser Pro Asn Ile Thr Glu Tyr Met Phe Cys Ala Gly Tyr Ser Asp
                325                 330                 335
Gly Ser Lys Asp Ser Cys Lys Gly Asp Ser Gly Gly Pro His Ala Thr
            340                 345                 350
His Tyr Arg Gly Thr Trp Tyr Leu Thr Gly Ile Val Ser Trp Gly Gln
        355                 360                 365
Gly Cys Ala Thr Val Gly His Phe Gly Val Tyr Thr Arg Val Ser Gln
    370                 375                 380
Tyr Ile Glu Trp Leu Gln Lys Leu Met Arg Ser Glu Pro Arg Pro Gly
385                 390                 395                 400
Val Leu Leu Arg Ala Pro Phe Pro
                405
```

<210> 218
<211> 408
<212> PRT
<213> Homo sapiens

<220>
<223> G237T238insSA

<400> 218

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Ser Ala Thr
225 230 235 240
Thr Asn His Asp Ile Ala Leu Leu Arg Leu His Gln Pro Val Val Leu
245 250 255
Thr Asp His Val Val Pro Leu Cys Leu Pro Glu Arg Thr Phe Ser Glu
260 265 270
Arg Thr Leu Ala Phe Val Arg Phe Ser Leu Val Ser Gly Trp Gly Gln
275 280 285
Leu Leu Asp Arg Gly Ala Thr Ala Leu Glu Leu Met Val Leu Asn Val
290 295 300
Pro Arg Leu Met Thr Gln Asp Cys Leu Gln Gln Ser Arg Lys Val Gly
305 310 315 320
Asp Ser Pro Asn Ile Thr Glu Tyr Met Phe Cys Ala Gly Tyr Ser Asp
325 330 335
Gly Ser Lys Asp Ser Cys Lys Gly Asp Ser Gly Gly Pro His Ala Thr
340 345 350
His Tyr Arg Gly Thr Trp Tyr Leu Thr Gly Ile Val Ser Trp Gly Gln
355 360 365
Gly Cys Ala Thr Val Gly His Phe Gly Val Tyr Thr Arg Val Ser Gln
370 375 380

Tyr Ile Glu Trp Leu Gln Lys Leu Met Arg Ser Glu Pro Arg Pro Gly
385 390 395 400
Val Leu Leu Arg Ala Pro Phe Pro
405

<210> 219
<211> 407
<212> PRT
<213> Homo sapiens

<220>
<223> D196K197insK

<400> 219

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Lys Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320

Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405

<210> 220
<211> 407
<212> PRT
<213> Homo sapiens

<220>
<223> D196K197insR

<400> 220

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Arg Lys Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 221
<211> 407
<212> PRT
<213> Homo sapiens

<220>
<223> D196K197insY

<400> 221

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
```

```
            180                 185                 190
His Cys Phe Asp Tyr Lys Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 222
<211> 407
<212> PRT
<213> Homo sapiens

<220>
<223> D196K197insW

<400> 222

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
```

```
                115                     120                     125
Pro Thr Val Glu Tyr Pro Cys Gly Lys Ile Pro Ile Leu Glu Lys Arg
        130                     135                     140
Asn Ala Ser Lys Pro Gln Gly Arg Ile Val Gly Gly Lys Val Cys Pro
145                     150                     155                     160
Lys Gly Glu Cys Pro Trp Gln Val Leu Leu Leu Val Asn Gly Ala Gln
                        165                     170                     175
Leu Cys Gly Gly Thr Leu Ile Asn Thr Ile Trp Val Val Ser Ala Ala
                180                     185                     190
His Cys Phe Asp Trp Lys Ile Lys Asn Trp Arg Asn Leu Ile Ala Val
        195                     200                     205
Leu Gly Glu His Asp Leu Ser Glu His Asp Gly Asp Glu Gln Ser Arg
    210                     215                     220
Arg Val Ala Gln Val Ile Ile Pro Ser Thr Tyr Val Pro Gly Thr Thr
225                     230                     235                     240
Asn His Asp Ile Ala Leu Leu Arg Leu His Gln Pro Val Val Leu Thr
                        245                     250                     255
Asp His Val Val Pro Leu Cys Leu Pro Glu Arg Thr Phe Ser Glu Arg
                260                     265                     270
Thr Leu Ala Phe Val Arg Phe Ser Leu Val Ser Gly Trp Gly Gln Leu
        275                     280                     285
Leu Asp Arg Gly Ala Thr Ala Leu Glu Leu Met Val Leu Asn Val Pro
    290                     295                     300
Arg Leu Met Thr Gln Asp Cys Leu Gln Gln Ser Arg Lys Val Gly Asp
305                     310                     315                     320
Ser Pro Asn Ile Thr Glu Tyr Met Phe Cys Ala Gly Tyr Ser Asp Gly
                        325                     330                     335
Ser Lys Asp Ser Cys Lys Gly Asp Ser Gly Gly Pro His Ala Thr His
                340                     345                     350
Tyr Arg Gly Thr Trp Tyr Leu Thr Gly Ile Val Ser Trp Gly Gln Gly
        355                     360                     365
Cys Ala Thr Val Gly His Phe Gly Val Tyr Thr Arg Val Ser Gln Tyr
    370                     375                     380
Ile Glu Trp Leu Gln Lys Leu Met Arg Ser Glu Pro Arg Pro Gly Val
385                     390                     395                     400
Leu Leu Arg Ala Pro Phe Pro
                        405
```

<210> 223
<211> 407
<212> PRT
<213> Homo sapiens

<220>
<223> D196K197insA

<400> 223

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
```

```
        50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Ala Lys Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 224
<211> 407
<212> PRT
<213> Homo sapiens

<220>
<223> D196K197insM

<400> 224

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Met Lys Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 225
<211> 407
<212> PRT
<213> Homo sapiens

<220>
<223> K197I198insE

<400> 225

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Glu Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365

Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 226
<211> 407
<212> PRT
<213> Homo sapiens

<220>
<223> K197I198insY

<400> 226

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Tyr Ile Lys Asn Trp Arg Asn Leu Ile Ala Val
195 200 205
Leu Gly Glu His Asp Leu Ser Glu His Asp Gly Asp Glu Gln Ser Arg
210 215 220
Arg Val Ala Gln Val Ile Ile Pro Ser Thr Tyr Val Pro Gly Thr Thr
225 230 235 240
Asn His Asp Ile Ala Leu Leu Arg Leu His Gln Pro Val Val Leu Thr
245 250 255
Asp His Val Val Pro Leu Cys Leu Pro Glu Arg Thr Phe Ser Glu Arg
260 265 270
Thr Leu Ala Phe Val Arg Phe Ser Leu Val Ser Gly Trp Gly Gln Leu
275 280 285
Leu Asp Arg Gly Ala Thr Ala Leu Glu Leu Met Val Leu Asn Val Pro
290 295 300

Arg Leu Met Thr Gln Asp Cys Leu Gln Gln Ser Arg Lys Val Gly Asp
305 310 315 320
Ser Pro Asn Ile Thr Glu Tyr Met Phe Cys Ala Gly Tyr Ser Asp Gly
325 330 335
Ser Lys Asp Ser Cys Lys Gly Asp Ser Gly Gly Pro His Ala Thr His
340 345 350
Tyr Arg Gly Thr Trp Tyr Leu Thr Gly Ile Val Ser Trp Gly Gln Gly
355 360 365
Cys Ala Thr Val Gly His Phe Gly Val Tyr Thr Arg Val Ser Gln Tyr
370 375 380

Ile Glu Trp Leu Gln Lys Leu Met Arg Ser Glu Pro Arg Pro Gly Val
385 390 395 400
Leu Leu Arg Ala Pro Phe Pro
405

<210> 227
<211> 407
<212> PRT
<213> Homo sapiens

<220>
<223> K197I198insA

<400> 227

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ala Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
```

225 230 235 240
Asn His Asp Ile Ala Leu Leu Arg Leu His Gln Pro Val Val Leu Thr
245 250 255
Asp His Val Val Pro Leu Cys Leu Pro Glu Arg Thr Phe Ser Glu Arg
260 265 270
Thr Leu Ala Phe Val Arg Phe Ser Leu Val Ser Gly Trp Gly Gln Leu
275 280 285
Leu Asp Arg Gly Ala Thr Ala Leu Glu Leu Met Val Leu Asn Val Pro
290 295 300
Arg Leu Met Thr Gln Asp Cys Leu Gln Gln Ser Arg Lys Val Gly Asp
305 310 315 320
Ser Pro Asn Ile Thr Glu Tyr Met Phe Cys Ala Gly Tyr Ser Asp Gly
325 330 335
Ser Lys Asp Ser Cys Lys Gly Asp Ser Gly Gly Pro His Ala Thr His
340 345 350
Tyr Arg Gly Thr Trp Tyr Leu Thr Gly Ile Val Ser Trp Gly Gln Gly
355 360 365
Cys Ala Thr Val Gly His Phe Gly Val Tyr Thr Arg Val Ser Gln Tyr
370 375 380
Ile Glu Trp Leu Gln Lys Leu Met Arg Ser Glu Pro Arg Pro Gly Val
385 390 395 400
Leu Leu Arg Ala Pro Phe Pro
405

<210> 228
<211> 407
<212> PRT
<213> Homo sapiens

<220>
<223> K197I198insS

<400> 228

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160

```
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ser Ile Lys Asn Trp Arg Asn Leu Ile Ala Val
        195                 200                 205
Leu Gly Glu His Asp Leu Ser Glu His Asp Gly Asp Glu Gln Ser Arg
    210                 215                 220
Arg Val Ala Gln Val Ile Ile Pro Ser Thr Tyr Val Pro Gly Thr Thr
225                 230                 235                 240
Asn His Asp Ile Ala Leu Leu Arg Leu His Gln Pro Val Val Leu Thr
                245                 250                 255
Asp His Val Val Pro Leu Cys Leu Pro Glu Arg Thr Phe Ser Glu Arg
            260                 265                 270
Thr Leu Ala Phe Val Arg Phe Ser Leu Val Ser Gly Trp Gly Gln Leu
        275                 280                 285
Leu Asp Arg Gly Ala Thr Ala Leu Glu Leu Met Val Leu Asn Val Pro
    290                 295                 300
Arg Leu Met Thr Gln Asp Cys Leu Gln Gln Ser Arg Lys Val Gly Asp
305                 310                 315                 320
Ser Pro Asn Ile Thr Glu Tyr Met Phe Cys Ala Gly Tyr Ser Asp Gly
                325                 330                 335
Ser Lys Asp Ser Cys Lys Gly Asp Ser Gly Gly Pro His Ala Thr His
            340                 345                 350
Tyr Arg Gly Thr Trp Tyr Leu Thr Gly Ile Val Ser Trp Gly Gln Gly
        355                 360                 365
Cys Ala Thr Val Gly His Phe Gly Val Tyr Thr Arg Val Ser Gln Tyr
    370                 375                 380
Ile Glu Trp Leu Gln Lys Leu Met Arg Ser Glu Pro Arg Pro Gly Val
385                 390                 395                 400
Leu Leu Arg Ala Pro Phe Pro
                405
```

<210> 229
<211> 406
<212> PRT
<213> Homo sapiens

<220>
<223> K197E/K341Q

<400> 229

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
```

```
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Gln Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 230
<211> 406
<212> PRT
<213> Homo sapiens

<220>
<223> K197L/K341Q

<400> 230

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
```

Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Leu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 231
<211> 406
<212> PRT
<213> Homo sapiens

<220>
<223> G237V/K341Q

<400> 231

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400

Leu Arg Ala Pro Phe Pro
405

<210> 232
<211> 406
<212> PRT
<213> Homo sapiens

<220>
<223> K197E/K199E

<400> 232

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Glu Ile Glu Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser

                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405

<210> 233

<211> 406
<212> PRT
<213> Homo sapiens

<220>
<223> K197E/G237V

<400> 233

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Val Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
```

```
                260                     265                     270
Leu Ala Phe Val Arg Phe Ser Leu Val Ser Gly Trp Gly Gln Leu Leu
            275                     280                     285
Asp Arg Gly Ala Thr Ala Leu Glu Leu Met Val Leu Asn Val Pro Arg
    290                     295                     300
Leu Met Thr Gln Asp Cys Leu Gln Gln Ser Arg Lys Val Gly Asp Ser
305                     310                     315                     320
Pro Asn Ile Thr Glu Tyr Met Phe Cys Ala Gly Tyr Ser Asp Gly Ser
                    325                     330                     335
Lys Asp Ser Cys Lys Gly Asp Ser Gly Gly Pro His Ala Thr His Tyr
                340                     345                     350
Arg Gly Thr Trp Tyr Leu Thr Gly Ile Val Ser Trp Gly Gln Gly Cys
            355                     360                     365
Ala Thr Val Gly His Phe Gly Val Tyr Thr Arg Val Ser Gln Tyr Ile
    370                     375                     380
Glu Trp Leu Gln Lys Leu Met Arg Ser Glu Pro Arg Pro Gly Val Leu
385                     390                     395                     400
Leu Arg Ala Pro Phe Pro
                    405
```

<210> 234
<211> 406
<212> PRT
<213> Homo sapiens

<220>
<223> K199E/K341Q

<400> 234

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
```

His Cys Phe Asp Lys Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 235
<211> 406
<212> PRT
<213> Homo sapiens

<220>
<223> K197E/G237V/K341Q

<400> 235

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125

Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Glu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 236
<211> 407
<212> PRT
<213> Homo sapiens

<220>
<223> Gla swap FIX

<400> 236

Tyr Asn Ser Gly Lys Leu Glu Glu Phe Val Gln Gly Asn Leu Glu Arg
1 5 10 15
Glu Cys Met Glu Glu Lys Cys Ser Phe Glu Glu Ala Arg Glu Val Phe
20 25 30
Glu Asn Thr Glu Arg Thr Thr Glu Phe Trp Lys Gln Tyr Ser Asp Gly
35 40 45

Asp Gln Cys Ala Ser Ser Pro Cys Gln Asn Gly Gly Ser Cys Lys Asp
50 55 60
Gln Leu Gln Ser Tyr Ile Cys Phe Cys Leu Pro Ala Phe Glu Gly Arg
65 70 75 80
Asn Cys Glu Thr His Lys Asp Asp Gln Leu Ile Cys Val Asn Glu Asn
85 90 95
Gly Gly Cys Glu Gln Tyr Cys Ser Asp His Thr Gly Thr Lys Arg Ser
100 105 110
Cys Arg Cys His Glu Gly Tyr Ser Leu Leu Ala Asp Gly Val Ser Cys
115 120 125
Thr Pro Thr Val Glu Tyr Pro Cys Gly Lys Ile Pro Ile Leu Glu Lys
130 135 140
Arg Asn Ala Ser Lys Pro Gln Gly Arg Ile Val Gly Gly Lys Val Cys
145 150 155 160
Pro Lys Gly Glu Cys Pro Trp Gln Val Leu Leu Leu Val Asn Gly Ala
165 170 175
Gln Leu Cys Gly Gly Thr Leu Ile Asn Thr Ile Trp Val Val Ser Ala
180 185 190
Ala His Cys Phe Asp Lys Ile Lys Asn Trp Arg Asn Leu Ile Ala Val
195 200 205
Leu Gly Glu His Asp Leu Ser Glu His Asp Gly Asp Glu Gln Ser Arg
210 215 220
Arg Val Ala Gln Val Ile Ile Pro Ser Thr Tyr Val Pro Gly Thr Thr
225 230 235 240
Asn His Asp Ile Ala Leu Leu Arg Leu His Gln Pro Val Val Leu Thr
245 250 255
Asp His Val Val Pro Leu Cys Leu Pro Glu Arg Thr Phe Ser Glu Arg
260 265 270
Thr Leu Ala Phe Val Arg Phe Ser Leu Val Ser Gly Trp Gly Gln Leu
275 280 285
Leu Asp Arg Gly Ala Thr Ala Leu Glu Leu Met Val Leu Asn Val Pro
290 295 300
Arg Leu Met Thr Gln Asp Cys Leu Gln Gln Ser Arg Lys Val Gly Asp
305 310 315 320
Ser Pro Asn Ile Thr Glu Tyr Met Phe Cys Ala Gly Tyr Ser Asp Gly
325 330 335
Ser Lys Asp Ser Cys Lys Gly Asp Ser Gly Gly Pro His Ala Thr His
340 345 350
Tyr Arg Gly Thr Trp Tyr Leu Thr Gly Ile Val Ser Trp Gly Gln Gly
355 360 365
Cys Ala Thr Val Gly His Phe Gly Val Tyr Thr Arg Val Ser Gln Tyr
370 375 380
Ile Glu Trp Leu Gln Lys Leu Met Arg Ser Glu Pro Arg Pro Gly Val
385 390 395 400
Leu Leu Arg Ala Pro Phe Pro
405

<210> 237
<211> 406
<212> PRT
<213> Homo sapiens

<220>
<223> Gla swap FX

<400> 237

Ala Asn Ser Phe Leu Glu Glu Met Lys Lys Gly His Leu Glu Arg Glu
1 5 10 15
Cys Met Glu Glu Thr Cys Ser Tyr Glu Glu Ala Arg Glu Val Phe Glu
20 25 30
Asp Ser Asp Lys Thr Asn Glu Phe Trp Asn Lys Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 238
<211> 406
<212> PRT
<213> Homo sapiens

<220>
<223> Gla Swap Prot C

<400> 238

Ala Asn Ser Phe Leu Glu Glu Leu Arg His Ser Ser Leu Glu Arg Glu
1 5 10 15
Cys Ile Glu Glu Ile Cys Asp Phe Glu Glu Ala Lys Glu Ile Phe Gln
20 25 30
Asn Val Asp Asp Thr Leu Ala Phe Trp Ser Lys His Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 239
<211> 406
<212> PRT

<213> Homo sapiens

<220>
<223> Gla Swap Prot S

<400> 239

```
Ala Asn Ser Leu Leu Glu Glu Thr Lys Gln Gly Asn Leu Glu Arg Glu
1               5                   10                  15
Cys Ile Glu Glu Leu Cys Asn Lys Glu Glu Ala Arg Glu Val Phe Glu
            20                  25                  30
Asn Asp Pro Glu Thr Asp Tyr Phe Tyr Pro Lys Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
        275                 280                 285
Asp Arg Gly Ala Thr Ala Leu Glu Leu Met Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 240
<211> 406
<212> PRT
<213> Homo sapiens

<220>
<223> Gla swap Thrombin

<400> 240

```
Ala Asn Thr Phe Leu Glu Glu Val Arg Lys Gly Asn Leu Glu Arg Glu
1               5                   10                  15
Cys Val Glu Glu Thr Cys Ser Tyr Glu Glu Ala Phe Glu Ala Leu Glu
            20                  25                  30
Ser Ser Thr Ala Thr Asp Val Phe Trp Ala Lys Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
```

Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 241
<211> 406
<212> PRT
<213> Homo sapiens

<220>
<223> V158D/G237V/E296V/M298Q

<400> 241

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140

Asn Ala Ser Lys Pro Gln Gly Arg Ile Val Gly Gly Lys Asp Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Val Leu Gln Val Leu Asn Val Pro Arg
290 295 300
Leu Met Thr Gln Asp Cys Leu Gln Gln Ser Arg Lys Val Gly Asp Ser
305 310 315 320

Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 242
<211> 406
<212> PRT
<213> Homo sapiens

<220>
<223> K197E/G237V/M298Q

<400> 242

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn

65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Glu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Lys Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 243
<211> 406
<212> PRT
<213> Homo sapiens

<220>
<223> K197E/G237V/M298Q/K341Q

<400> 243

Ala Asn Ala Phe Leu Glu Glu Leu Arg Pro Gly Ser Leu Glu Arg Glu

1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Glu Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 244
<211> 406
<212> PRT
<213> Homo sapiens

<220>
<223> K197E/K199E/G237V/M298Q/K341Q

<400> 244

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Glu Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile

370 375 380
Glu Trp Leu Gln Lys Leu Met Arg Ser Glu Pro Arg Pro Gly Val Leu
385 390 395 400
Leu Arg Ala Pro Phe Pro
405

<210> 245
<211> 406
<212> PRT
<213> Homo sapiens

<220>
<223> G237V/M298Q

<400> 245

Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
            20                  25                  30
Asp Ala Glu Arg Thr Lys Leu Phe Trp Ile Ser Tyr Ser Asp Gly Asp
        35                  40                  45
Gln Cys Ala Ser Ser Pro Cys Gln Asn Gly Gly Ser Cys Lys Asp Gln
    50                  55                  60
Leu Gln Ser Tyr Ile Cys Phe Cys Leu Pro Ala Phe Glu Gly Arg Asn
65                  70                  75                  80
Cys Glu Thr His Lys Asp Asp Gln Leu Ile Cys Val Asn Glu Asn Gly
                85                  90                  95
Gly Cys Glu Gln Tyr Cys Ser Asp His Thr Gly Thr Lys Arg Ser Cys
            100                 105                 110
Arg Cys His Glu Gly Tyr Ser Leu Leu Ala Asp Gly Val Ser Cys Thr
        115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Lys Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Val Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Gln Val Leu Asn Val Pro Arg
    290                 295                 300
Leu Met Thr Gln Asp Cys Leu Gln Gln Ser Arg Lys Val Gly Asp Ser

305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405

<210> 246
<211> 406
<212> PRT
<213> Homo sapiens

<220>
<223> G237V/M298Q/K341Q

<400> 246

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Val Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 247
<211> 407

<212> PRT
<213> Homo sapiens

<220>
<223> M298Q/Gla Swap FIX

<400> 247

```
Tyr Asn Ser Gly Lys Leu Glu Glu Phe Val Gln Gly Asn Leu Glu Arg
1               5                   10                  15
Glu Cys Met Glu Glu Lys Cys Ser Phe Glu Glu Ala Arg Glu Val Phe
            20                  25                  30
Glu Asn Thr Glu Arg Thr Thr Glu Phe Trp Lys Gln Tyr Ser Asp Gly
        35                  40                  45
Asp Gln Cys Ala Ser Ser Pro Cys Gln Asn Gly Gly Ser Cys Lys Asp
    50                  55                  60
Gln Leu Gln Ser Tyr Ile Cys Phe Cys Leu Pro Ala Phe Glu Gly Arg
65                  70                  75                  80
Asn Cys Glu Thr His Lys Asp Asp Gln Leu Ile Cys Val Asn Glu Asn
                85                  90                  95
Gly Gly Cys Glu Gln Tyr Cys Ser Asp His Thr Gly Thr Lys Arg Ser
            100                 105                 110
Cys Arg Cys His Glu Gly Tyr Ser Leu Leu Ala Asp Gly Val Ser Cys
        115                 120                 125
Thr Pro Thr Val Glu Tyr Pro Cys Gly Lys Ile Pro Ile Leu Glu Lys
    130                 135                 140
Arg Asn Ala Ser Lys Pro Gln Gly Arg Ile Val Gly Gly Lys Val Cys
145                 150                 155                 160
Pro Lys Gly Glu Cys Pro Trp Gln Val Leu Leu Leu Val Asn Gly Ala
                165                 170                 175
Gln Leu Cys Gly Gly Thr Leu Ile Asn Thr Ile Trp Val Val Ser Ala
```

180 185 190
Ala His Cys Phe Asp Lys Ile Lys Asn Trp Arg Asn Leu Ile Ala Val
195 200 205
Leu Gly Glu His Asp Leu Ser Glu His Asp Gly Asp Glu Gln Ser Arg
210 215 220
Arg Val Ala Gln Val Ile Ile Pro Ser Thr Tyr Val Pro Gly Thr Thr
225 230 235 240
Asn His Asp Ile Ala Leu Leu Arg Leu His Gln Pro Val Val Leu Thr
245 250 255
Asp His Val Val Pro Leu Cys Leu Pro Glu Arg Thr Phe Ser Glu Arg
260 265 270
Thr Leu Ala Phe Val Arg Phe Ser Leu Val Ser Gly Trp Gly Gln Leu
275 280 285
Leu Asp Arg Gly Ala Thr Ala Leu Glu Leu Gln Val Leu Asn Val Pro
290 295 300
Arg Leu Met Thr Gln Asp Cys Leu Gln Gln Ser Arg Lys Val Gly Asp
305 310 315 320
Ser Pro Asn Ile Thr Glu Tyr Met Phe Cys Ala Gly Tyr Ser Asp Gly
325 330 335
Ser Lys Asp Ser Cys Lys Gly Asp Ser Gly Gly Pro His Ala Thr His
340 345 350
Tyr Arg Gly Thr Trp Tyr Leu Thr Gly Ile Val Ser Trp Gly Gln Gly
355 360 365

Cys Ala Thr Val Gly His Phe Gly Val Tyr Thr Arg Val Ser Gln Tyr
370 375 380
Ile Glu Trp Leu Gln Lys Leu Met Arg Ser Glu Pro Arg Pro Gly Val
385 390 395 400
Leu Leu Arg Ala Pro Phe Pro
405

<210> 248
<211> 406
<212> PRT
<213> Homo sapiens

<220>
<223> K197E/M298Q

<400> 248

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys

```
                100                 105                 110
Arg Cys His Glu Gly Tyr Ser Leu Leu Ala Asp Gly Val Ser Cys Thr
            115                 120                 125
Pro Thr Val Glu Tyr Pro Cys Gly Lys Ile Pro Ile Leu Glu Lys Arg
    130                 135                 140
Asn Ala Ser Lys Pro Gln Gly Arg Ile Val Gly Gly Lys Val Cys Pro
145                 150                 155                 160
Lys Gly Glu Cys Pro Trp Gln Val Leu Leu Leu Val Asn Gly Ala Gln
                165                 170                 175
Leu Cys Gly Gly Thr Leu Ile Asn Thr Ile Trp Val Val Ser Ala Ala
            180                 185                 190
His Cys Phe Asp Glu Ile Lys Asn Trp Arg Asn Leu Ile Ala Val Leu
        195                 200                 205
Gly Glu His Asp Leu Ser Glu His Asp Gly Asp Glu Gln Ser Arg Arg
    210                 215                 220
Val Ala Gln Val Ile Ile Pro Ser Thr Tyr Val Pro Gly Thr Thr Asn
225                 230                 235                 240
His Asp Ile Ala Leu Leu Arg Leu His Gln Pro Val Val Leu Thr Asp
                245                 250                 255
His Val Val Pro Leu Cys Leu Pro Glu Arg Thr Phe Ser Glu Arg Thr
            260                 265                 270
Leu Ala Phe Val Arg Phe Ser Leu Val Ser Gly Trp Gly Gln Leu Leu
        275                 280                 285
Asp Arg Gly Ala Thr Ala Leu Glu Leu Gln Val Leu Asn Val Pro Arg
    290                 295                 300
Leu Met Thr Gln Asp Cys Leu Gln Gln Ser Arg Lys Val Gly Asp Ser
305                 310                 315                 320
Pro Asn Ile Thr Glu Tyr Met Phe Cys Ala Gly Tyr Ser Asp Gly Ser
                325                 330                 335
Lys Asp Ser Cys Lys Gly Asp Ser Gly Gly Pro His Ala Thr His Tyr
            340                 345                 350
Arg Gly Thr Trp Tyr Leu Thr Gly Ile Val Ser Trp Gly Gln Gly Cys
        355                 360                 365
Ala Thr Val Gly His Phe Gly Val Tyr Thr Arg Val Ser Gln Tyr Ile
    370                 375                 380
Glu Trp Leu Gln Lys Leu Met Arg Ser Glu Pro Arg Pro Gly Val Leu
385                 390                 395                 400
Leu Arg Ala Pro Phe Pro
                405
```

<210> 249
<211> 406
<212> PRT
<213> Homo sapiens

<220>
<223> M298Q/K341D

<400> 249

```
Ala Asn Ala Phe Leu Glu Glu Leu Arg Pro Gly Ser Leu Glu Arg Glu
1               5                   10                  15
Cys Lys Glu Glu Gln Cys Ser Phe Glu Glu Ala Arg Glu Ile Phe Lys
```

20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Lys Ile Lys Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Gln Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Asp Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro
405

<210> 250
<211> 406
<212> PRT
<213> Homo sapiens

<220>
<223> K197E/K199E/K341Q

<400> 250

Ala Asn Ala Phe Leu Glu Glu Leu Arg Pro Gly Ser Leu Glu Arg Glu
1 5 10 15
Cys Lys Glu Glu Gln Cys Ser Phe Glu Glu Ala Arg Glu Ile Phe Lys
20 25 30
Asp Ala Glu Arg Thr Lys Leu Phe Trp Ile Ser Tyr Ser Asp Gly Asp
35 40 45
Gln Cys Ala Ser Ser Pro Cys Gln Asn Gly Gly Ser Cys Lys Asp Gln
50 55 60
Leu Gln Ser Tyr Ile Cys Phe Cys Leu Pro Ala Phe Glu Gly Arg Asn
65 70 75 80
Cys Glu Thr His Lys Asp Asp Gln Leu Ile Cys Val Asn Glu Asn Gly
85 90 95
Gly Cys Glu Gln Tyr Cys Ser Asp His Thr Gly Thr Lys Arg Ser Cys
100 105 110
Arg Cys His Glu Gly Tyr Ser Leu Leu Ala Asp Gly Val Ser Cys Thr
115 120 125
Pro Thr Val Glu Tyr Pro Cys Gly Lys Ile Pro Ile Leu Glu Lys Arg
130 135 140
Asn Ala Ser Lys Pro Gln Gly Arg Ile Val Gly Gly Lys Val Cys Pro
145 150 155 160
Lys Gly Glu Cys Pro Trp Gln Val Leu Leu Leu Val Asn Gly Ala Gln
165 170 175
Leu Cys Gly Gly Thr Leu Ile Asn Thr Ile Trp Val Val Ser Ala Ala
180 185 190
His Cys Phe Asp Glu Ile Glu Asn Trp Arg Asn Leu Ile Ala Val Leu
195 200 205
Gly Glu His Asp Leu Ser Glu His Asp Gly Asp Glu Gln Ser Arg Arg
210 215 220
Val Ala Gln Val Ile Ile Pro Ser Thr Tyr Val Pro Gly Thr Thr Asn
225 230 235 240
His Asp Ile Ala Leu Leu Arg Leu His Gln Pro Val Val Leu Thr Asp
245 250 255
His Val Val Pro Leu Cys Leu Pro Glu Arg Thr Phe Ser Glu Arg Thr
260 265 270
Leu Ala Phe Val Arg Phe Ser Leu Val Ser Gly Trp Gly Gln Leu Leu
275 280 285
Asp Arg Gly Ala Thr Ala Leu Glu Leu Met Val Leu Asn Val Pro Arg
290 295 300
Leu Met Thr Gln Asp Cys Leu Gln Gln Ser Arg Lys Val Gly Asp Ser
305 310 315 320
Pro Asn Ile Thr Glu Tyr Met Phe Cys Ala Gly Tyr Ser Asp Gly Ser
325 330 335
Lys Asp Ser Cys Gln Gly Asp Ser Gly Gly Pro His Ala Thr His Tyr
340 345 350
Arg Gly Thr Trp Tyr Leu Thr Gly Ile Val Ser Trp Gly Gln Gly Cys
355 360 365
Ala Thr Val Gly His Phe Gly Val Tyr Thr Arg Val Ser Gln Tyr Ile
370 375 380
Glu Trp Leu Gln Lys Leu Met Arg Ser Glu Pro Arg Pro Gly Val Leu
385 390 395 400
Leu Arg Ala Pro Phe Pro

405

**Description**

**FIELD OF THE INVENTION**

Modified therapeutic proteins are provided. In particular modified Factor VII polypeptides, which includes Factor VIIa and other forms of Factor VII, and uses thereof are provided.

**BACKGROUND**

Hemostasis is the complex physiological process that leads to the cessation of bleeding. Platelets, plasma proteins, blood vessels and endothelial cells are the three components of this process that each play an important role in the events that immediately follow tissue injury and which, under normal circumstances, results in the rapid formation of a clot. Central to this is the coagulation cascade, a series of proteolytic events in which certain plasma proteins (or coagulation factors) are sequentially activated in a "cascade" by another previously activated coagulation factor, leading to the rapid generation of thrombin. The large quantities of thrombin produced in this cascade then function to cleave fibrinogen into the fibrin peptides that are required for clot formation.

The coagulation factors circulate as inactive single-chain zymogens, and are activated by cleavage at one or more positions to generate a two-chain activated form of the protein. Factor VII (FVII), a vitamin K-dependent plasma protein, initially circulates in the blood as a zymogen. The FVII zymogen is activated by proteolytic cleavage at a single site, $Arg^{152}$-$Ile^{153}$, resulting is a two-chain protease linked by a single disulphide bond (FVIIa). FVIIa binds its cofactor, tissue factor (TF), to form a complex in which FVIIa can efficiently activate factor X (FX) to FXa, thereby initiating the series of events that result in fibrin formation and hemostasis.

While normal hemostasis is achieved in most cases, defects in the process can lead to bleeding disorders in which the time taken for clot formation is prolonged. Such disorders can be congenital or acquired. For example, hemophilia A and B are inherited diseases characterized by deficiencies in factor VIII (FVIII) and factor IX (FIX), respectively. Replacement therapy is the traditional treatment for hemophilia A and B, and involves intravenous administration of FVIII or FIX, either prepared from human plasma or as recombinant proteins. In many cases, however, patients develop antibodies (also known as inhibitors) against the infused proteins, which reduces or negates the efficacy of the treatment. Recombinant FVIIa (Novoseven®) has been approved for the treatment of hemophilia A or B patients that have inhibitors to FVIII or FIX, and also is used to stop bleeding episodes or prevent bleeding associated with trauma and/or surgery. Recombinant FVIIa also has been approved for the treatment of patients with congenital FVII deficiency, and is increasingly being utilized in off-label uses, such as the treatment of bleeding associated with other congenital or acquired bleeding disorders, trauma, and surgery in non-hemophilic patients.

The use of recombinant FVIIa to promote clot formation underlines its growing importance as a therapeutic agent. FVIIa therapy leaves significant unmet medical need. For example, based on clinical trial data, an average of 3 doses of FVIIa over a 6 hour or more time period are required to manage acute bleeding episodes in hemophilia patients. More efficacious variants of FVIIa are needed to reduce these requirements. Therefore, among the objects herein, it is an object to provide modified FVII polypeptides that are designed to have improved therapeutic properties.

WO 2004/083661 provides variants of FVII or FVIIa comprising at least one amino acid modification in position 196, 237 or 341 relative to hFVII or hFVIIa. The variants exhibit an increased clotting activity, i.e. reduced clotting time, compared to rhFVIIa.

WO 2004/111242 provides Gla domain variants of human Factor VII or human Factor VIIa, comprising 1-15 amino acid modifications relative to human Factor VII or human Factor VIIa, wherein a hydrophobic amino acid residue has been introduced by substitution in position 34, or having an amino acid substitution in position 36, or having amino acid substitutions in positions 10 and 32 and at least one further amino acid substitution in a position selected from 74, 77 and 116.

SUMMARY

In a first embodiment the invention provides a modified factor VII (FVII) polypeptide, comprising: a heterologous Gla domain, or a sufficient portion thereof to effect phospholipid binding, whereby the modified FVII polypeptide exhibits increased phospholipid affinity or binding compared with an unmodified FVII polypeptide that does not contain the heterologous Gla domain, wherein: the sufficient portion contains 30 or more contiguous amino acids from the heterologous Gla domain; and the heterologous Gla domain is selected from among a Gla domain in Factor IX (FIX), Factor X (FX), prothrombin, protein C, protein S, osteocalcin, Growth-arrest-specific protein 6 (Gas6) and protein Z; andone or more further amino acid modification(s) that increases resistance to antithrombin-III (AT-III), increases affinity for tissue factor (TF), increases intrinsic activity, increases TF-dependent catalytic or coagulant activity, increases coagulant activity, alters the conformation of the polypeptide to alter zymogenicity, increases catalytic or coagulant activity by shifting the equilibrium between highly active and less active FVIIa conformations in favor of the highly active conformations, increases resistance to proteases, decreases glycosylation, increases glycosylation, reduces immunogenicity, increases stability, and/or facilitates chemical group linkage.

In a further embodiment the invention provides a nucleic acid molecule comprising a sequence of nucleotides encoding such a modified FVII polypeptide, a vector comprising such a nucleotide and a cell comprising such a vector.

In a further embodiment the invention provides a pharmaceutical composition comprising a therapeutically effective concentration or amount of such a modified FVII polypeptide, nucleic acid molecule, vector or cell in a pharmaceutically acceptable vehicle.

In a further embodiment the invention provides such a modified FVII polypeptide for use in treating a disease or condition that is treated by administration of FVII or a pro-coagulant

In a further embodiment the invention provides use of such a pharmaceutical composition in the preparation of a medicament for treatment of a disease or condition that is treated by administration of FVII or a pro-coagulant.

Provided herein are modified Factor VII (FVII) polypeptides. In particular, provided herein are modified FVII polypeptides that exhibit procoagulant activities. The FVII polypeptides are modified in primary sequence compared to an unmodified FVII polypeptide, and can include, amino acid insertions, deletions and replacements. Modified FVII polypeptides provided herein include FVII polypeptides that exhibit increased resistance to the inhibitory affects of tissue factor pathway inhibitor (TFPI), increased resistance to the inhibitory affects antithrombin-III (AT-III), decreased $Zn^{2+}$ binding, improved pharmacokinetic properties, such as increased half-life, increased catalytic activity in the presence and/or or absence of TF, and/or increased binding to activated platelets. The modified FVII polypeptides can contain any combination of modifications provided herein, whereby one or more activities or properties of the polypeptide are altered compared to an unmodified FVII polypeptide. Typically the modified FVII polypeptide retains procoagulant activity. Also provided herein are nucleic acid molecules, vectors and cells that encode/express modified FVII polypeptides. Pharmaceutical compositions, articles of manufacture, kits and methods of treatment also are provided herein. FVII polypeptides include allelic and species variants and polypeptides and other variants that have modifications that affect other activities and/or properties. Also included are active fragments of the FVII polypeptides that include a modification provided herein. Exemplary of FVII polypeptides are those that include the sequence of amino acids set forth in SEQ ID NO:3, as well as variants thereof having 60%, 65%, 70%, 75%, 80%, 85%, 88%, 90%, 91%, 92%, 93%, 94%. 95%, 96%, 97%, 98%, 99% or more sequence identity therewith.

The factor VII (FVII) polypeptides are modified in primary sequence compared to an unmodified FVII polypeptide, and can include, amino acid insertions, deletions and replacements. Modified FVII polypeptides provided herein include FVII polypeptides that exhibit increased resistance to the inhibitory affects of TFPI, increased resistance to the inhibitory affects AT-III, decreased $Zn^{2+}$ binding, improved pharmacokinetic properties, such as increased half-life, increased catalytic activity in the presence and/or or absence of (tissue factor) TF, and/or increased binding to activated platelets. The modified FVII polypeptides can contain any combination of modifications provided herein, whereby one or more activities or properties of the polypeptide are altered compared to an unmodified FVII polypeptide. The FVII polypeptides can include other modifications that alter other properties and/or activities. Typically the modified FVII polypeptide retains procoagulant activity. Also provided herein are nucleic acid molecules, vectors and cells that encode/express modified FVII polypeptides. Pharmaceutical compositions, articles of manufacture, kits and methods of treatment also are provided herein.

In particular, provided are modified factor VII (FVII) polypeptides allelic and species variant thereof or active fragments or other variants thereof. Provided herein are FVII polypeptides, including allelic and species variant thereof or active fragments thereof, that contain a modification that is at a position corresponding to position D196, K197 or K199 in a FVII polypeptide with a sequence of amino acids set forth in SEQ ID NO:3, or in corresponding residues in a FVII polypeptide, including allelic and species variants thereof, active fragments, and other FVII polypeptides modified for other activities or properties. The FVII polypeptides contain at least one modification at a position corresponding to positione D196, K197 or K199 in a FVII polypeptide containing a sequence of amino acids as set forth in SEQ ID NO:3 or in corresponding residues in a FVII polypeptide. The modification is an insertion and/or replacement by a hydrophobic or acidic amino acid selected from among Val (V), Leu (L), Ile (I), Phe (F), Trp (W), Met (M), Tyr (Y), Cys (C), Asp (D) and Glu (E). When an active fragment is provided, the active fragment includes such modification. For example, provided are modified factor VII (FVII) polypeptides, allelic and species variants thereof or active fragments or other variants thereof that also include a replacement selected from among D196F, D196W, D196L, D196I, D196Y, K197E, K197D, K197L, K197M, K197I, K197V, K197F, K197W, K199D, K199E and K197Y.

Additionally, modified FVII polypeptides provided can contain a further modification, including an amino acid replacement, insertion or deletion, at another position in the FVII polypeptide. In some examples, the further modification is an amino acid replacement at a position corresponding to a position selected from among D196, K197, K199, G237, T239, R290 and K341, wherein the first modification and second modification are at different amino acids. These modification can include, but are not limited to D196K, D196R, D196A, D196Y, D196F, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341R, K341N, K341M, K341D and K341Q. Other examples modifications that are amino acid insertions, such, as, but not limited to, any of the following insertions: G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA and K197I198insS. Other exemplary modified FVII polypeptides include the modifications as follows: D196R/K197E/K199E, D196K/K197E/K199E, D196R/K197E/K199E/R290E, D196R/K197M/K199E, D196R/K197M/ K199E/R290E, D196K/K197L, D196F/ K197L, D196L/K197L, D196M/K197L, D196W/K197L, D196F/K197E, D196W/K197E, K196V/K197E, K197E/K341Q, K197L/K341Q, K197E/G237V/K341Q, K197E/K199E, K197E/G237V, K199E/K341Q or K197E/K199E/K341Q.

Also provided are modified FVII polypeptides, including allelic and species variant thereof or active fragments or other variants thereof, that also can contain amino acid modifications corresponding to any one or more of D196R, D196Y, D196F, D196W, D196L, D196I, K197Y, K197E, K197D, K197L, K197M, K197M, K197I, K197V, K197F, K197W, K199D, K199E, G237W, G237I, G237V, R290M, R290V, K341M, K341D, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA or K197I198insS in a FVII polypeptide having a sequence of amino acids set forth in SEQ ID NO:3 or in corresponding residues in a FVII polypeptide. Additionally, these modified FVII polypeptides can contain a further modification, including an amino acid replacement, insertion or deletion, at another position in the FVII polypeptide. In some examples, the further modification can be an amino acid replacement at a position corresponding to a position D196, K197, K199, G237, T239, R290 and K341, wherein the further modification is a different position from the first modification. For example, the modified FVII polypeptide can additionally contain an amino acid replacement selected from among D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341R, K341N, K341M, K341D, and K341Q. Exemplary of such modified FVII polypeptide are those that include modifications selected from among D196R/R290E, D196R/R290D, D196R/K197E/K199E, D196K/K197E/K199E, D196E/K197E/K199E/R290E, D196R/K197M/K199E, D196R/K197M/K199E/R290E, D196K/K197L, D196F/K197L, D196L/K197L, D196M/K197L, D196W/K197L, D196F/K197E, D196W/K197E, K197L/K341Q, G237V/K341Q, K197E/G237V/K341Q, K197E/K199E, K197E/G237V, K199E/K341Q, K197E/K199E/K341Q and K196V/K197E.

In some instances, a modified factor VII (FVII) polypeptide , including allelic and species variant thereof or active fragments thereof or other variants thereof can include two or more modifications in a FVII polypeptide, where at least two of the amino acid modifications correspond to D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341 R, K341N, K341M, K341D, K341Q, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA or K197I198insS in a FVII polypeptide having or including a sequence of amino acids set forth in SEQ ID NO:3 or in corresponding residues in a FVII polypeptide. The polypeptide can include more than two modifications, such as 2, 3, 4, 5, 6 or 7 modifications.

Exemplary of these are FVII polypeptides that include comprising modifications selected from among D196R/R290E, D196K/R290E, D196R/R290D, D196R/ K197E/K199E, D196K/K197E/K199E, D196R/ K197E/K199E/R290E, D196R/K197M/K199E, D196R/K197M/K199E/R290E, D196K/K197L, D196F/ K197L, D196L/K197L, D196M/K197L, D196W/ K197L, D196F/K197E, D196W/K197E, D196V/K197E, K197E/K341Q, K197L/K341Q, G237V/K341Q, K197E/G237V/K341Q, K197E/K199E, K197E/G237V, K199E/K341Q, K197E/K199E/K341Q and K197E/G237V/M298Q.

Any of the above-mentioned modified FVII polypeptides can exhibit increased resistance to tissue factor pathway inhibitor (TFPI) compared with the unmodified FVII polypeptide. In some examples, the modified FVII polypeptide is at least about or is 1%, 2%, 3%, 4%, 5%, 6%, 7%, 8%, 9%, 10%, 20%, 30%, 40%, 50%, 60%, 70%, 80%, 90%, 91%, 92%, 93%, 94%, 95%, 96%, 97%, 98%, 99%, 100%, 200%, 300%, 400%, 500% or more resistant to TFPI. Additionally, these modified FVII polypeptides also can contain a heterologous Gla domain, or a sufficient portion thereof to effect phospholipid binding.

Also provided herein are modified FVII polypeptides that contain a heterologous Gla domain, or a sufficient portion thereof, such as 30%, 40%, 50%, 60%, 70%, 80%, 90%, 91%, 92%, 93%, 94%, 95%, 96%, 97%, 98%, 99%, or more of the heterologous Gla domain, to effect phospholipid binding. Any and all of the above-noted modified FVII polypeptides also can include a Gla swap, including Gla swaps as exemplified and described herein.

A heterologous Gla domain can be selected from the Gla domains of Factor IX (FIX), Factor X (FX), prothrombin, protein C, protein S, osteocalcin, matrix Gla protein, Growth-arrest-specific protein 6 (Gas6) or protein Z. In some examples, the heterologous Gla domain in the modified FVII polypeptide provided herein has a sequence of amino acids set forth in any of SEQ ID NOS: 110-115, 117 and 118, or a sufficient portion thereof to effect phospholipid binding. The modifications to the FVII polypeptide can be effected by removing all or a contiguous portion of the native FVII Gla domain, which can include amino acids 1-45 in a FVII polypeptide having or including a sequence of amino acids set forth in SEQ ID NO:3, or in corresponding residues in a FVII polypeptide, and replacing it with the heterologous Gla domain, or a sufficient portion thereof to affect phospholipid binding, such as to increase it. Such modifications can result in the modified FVII polypeptide exhibiting increased phospholipid binding compared with the unmodified FVII polypeptide. For example, the modified FVII polypeptide can exhibit at least about or 1%, 2%, 3%, 4%, 5%, 6%, 7%, 8%, 9%, 10%, 20%, 30%, 40%, 50%, 60%, 70%, 80%, 90%, 91%, 92%, 93%, 94%, 95%, 96%, 97%, 98%, 99%, 100%, 200%, 300%, 400%, 500% or more increased phospholipid binding.

The modified FVII polypeptides can have all or a contiguous portion of the native FVII Gla domain removed and replaced with the heterologous Gla domain, or a sufficient portion thereof to effect phospholipid binding. Exemplary of such polypeptides are those in which the native FVII Gla domain includes amino acids 1-45 in a FVII polypeptide having or including a sequence of amino acids set forth in SEQ ID NO:3, or in corresponding residues in a FVII polypeptide. Gla modifications include, but are not limited to, modifications among a Gla Swap FIX, Gla Swap FX, Gla Swap Prot C, Gla Swap Prot S and Gla Swap Thrombin.

By virtue of a Gla swap the modified FVII polypeptide can exhibit increased phospholipid binding. Such increase can be at least about or 0.5%, 1%, 2%, 3%, 4%, 5%, 6%, 7%, 8%, 9%, 10%, 20%, 30%, 40%, 50%, 60%, 70%, 80%, 90%, 91%, 92%, 93%, 94%, 95%, 96%, 97%, 98%, 99%, 100%, 200%, 300%, 400%, 500% or more increased phospholipid binding.

The modified FVII polypeptides containing heterologous Gla domains can contain further modifications at positions that result in or exhibit increased resistance to tissue factor pathway inhibitor (TFPI) compared to an unmodified FVII polypeptide. Exemplary of such modifications are one or more amino acid modification(s) at positions selected from among D196, K197, K199, G237, T239, R290, and K341 in a FVII polypeptide having or including a sequence of amino acids set forth in SEQ ID NO:3 or in corresponding residues in a FVII polypeptide. Specifically, such modifications can include one or more amino acid modification(s) are selected from among D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341 R, K341N, K341M, K341D, K341Q, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA and K197I198insS. For example, the modified FVII polypeptides containing a heterologous Gla domain also can contain the amino acid replacements and/or insertions such as, but not limited to, D196R/R290E, D196K/R290E, D196R/R290D, D196R/K197E/K199E, D196K/K197E/K199E, D196R/K197E/K199E/R290E, D196R/K197M/ K199E, and D196R/K197M/K199E/R290E.

In some instances, such further modifications result in an increased resistance to TFPI of the modified polypeptide compared to the FVII polypeptide not containing the modification, i.e. the uninodified FVII polypeptide.

Any of the modified FVII polypeptides described above, including those that exhibit increased resistance to TFPI or increased binding to phospholipids, or any combination thereof, can additionally contain other modifications, including any described in the art. Such further amino acid modifications can increase resistance to antithrombin-III (AT-III), increase binding and/or affinity to phospholipids, increase affinity for tissue factor (TF), increase intrinsic activity, alter the conformation of the polypeptides to alter zymogenicity, including altering the conformation to a more zymogen-like shape or a less zymogen-like shape, increase resistance to proteases, decrease glycosylation, increase glycosylation, reduce immunogenicity, increase stability, and/or facilitate chemical group linkage. For example, the modified FVII polypeptides provided herein can contain modification(s) at position Q176, M298 or E296 in a FVII polypeptide having or including a sequence of amino acids set forth in SEQ ID NO:3 or in corresponding residues in a FVII polypeptide. In some examples, the modified FVII polypeptides can additionally contain amino acid modifications from among Q176A, M298Q, E296V and/or E296A. In other examples, the modified FVII polypeptides can additionally contain, in addition to the Gla swap and/or other noted modifications, one or more of the following further amino acid modification(s): S278C/V302C, L279C/N301C, V280C/V301C, S281C/V299C, insertion of a tyrosine at position 4, F4S, F4T, P10Q, P10E, P10D, P10N, Q21N, R28F, R28E, I30C, I30D, I30E, K32D, K32Q, K32E, K32G, K32H, K32T, K32C, K32A, K32S, D33C, D33F, D33E, D33K, A34C, A34E, A34D, A34I, A34L, A34M, A34V, A34F, A34W, A34Y, R36D, R36E, T37C, T37D, T37E, K38C, K38E, K38T, K38D, K38L, K38G, K38A, K38S, K38N, K38H, L39E, L39Q, L39H, W41N, W41C, W41E, W41D, I42R, I42N, I42S, I42A, I42Q, I42N, I42S, I42A, I42Q, I42K, S43Q, S43N, Y44K, Y44C, Y44D, Y44E, S45C, S45D, S45E, D46C, A51N, S53N, G58N, G59S, G59T, K62E, K62R, K62D, K62N, K62Q, K62T, L65Q, L65S, L65N, F71D, F71Y, F71E, F71Q, F71N, P74S, P74A, A75E, A75D, E77A, E82Q, E82N, E82S, E82T T83K, N95S, N95T, G97S, G97T, Y101N, D104N, T106N, K109N, E116D, G117N, G124N, S126N, T128N, LY41C, L141D, L141E, E142D, E142C, K143C, K143D, K143E, R144E, R144C, R144D, N145Y, N145G, N145F, N145M, N145S, N145I, N145L, N145T, N145V, N145P, N145K, N145H, N145Q, N145E, N145R, N145W, N145D, N145C, K157V, K157L, K157I, K157M, K157F, K157W, K157P, K157G, K157S, K157T, K157C, K157Y, K157N, K157E, K157R, K157H, K157D, K157Q, V158L, V158I, V158M, V158F, V158W, V158P, V158G, V158S, V158T, V158C, V158Y, V158N, V158E, V158R, V158K, V158H, V158D, V158Q, A175S, A175T, G179N, I186S, I186T, V188N, R202S, R202T, I205S, I205T, D212N, E220N, I230N, P231N, P236N, G237N, Q250C, V253N, E265N, T267N, E270N, A274M, A274L, A274K, A274R, A274D, A274V, A274I, A274F, A274W, A274P, A274G, A274T, A274C, A274Y, A274N, A274E, A274H, A274S, A274Q, F275H, R277N, F278S, F278A, F278N, F278Q, F278G, L280N, L288K, L288C, L288D, D289C, D289K, L288E, R290C, R290G, R290A, R290S, R290T, R290K, R290D, R290E, G291E, G291D, G291C, G291N, G291K, A292C, A292K, A292D, A292E, T293K, E296V, E296L, E296I, E296M, E296F, E296W, E296P, E296G, E296S, E296T, E296C, E296Y, E296N, E296K, E296R, E296H, E296D, E296Q, M298Q, M298V, M298L, M298I, M298F, M298W, M298P, M298G, M298S, M298T, M298C, M298Y, M298N, M298K, M298R, M298H, M298E, M298D, P303S, P303T, R304Y, R304F, R304L, R304M, R304G, R304T, R304A, R304S, R304N, L305V, L305Y, L305I, L305F, L305A, L305M, L305W, L305P, L305G, L305S, L305T, L305C, L305N, L305E, L305K, L305R, L305H, L305D, L305Q, M306D, M306N, D309S, D309T, Q312N, Q313K, Q313D, Q313E, S314A, S314V, S314I, S314M, S314F, S314W, S314P, S314G, S314L, S314T, S314C, S314Y, S314N, S314E, S314K, S314R, S314H, S314D, S314Q, R315K, R315G, R315A, R315S, R315T, R315Q, R315C, R315D, R315E, K316D, K316C, K316E, V317C, V317K, V317D, V317E, G318N, N322Y, N322G, N322F, N322M, N322S, N322I, N322L, N322T, N322V, N322P, N322K, N322H, N322Q, N322E, N322R, N322W, N322C, G331N, Y332S, Y332A, Y332N, Y332Q, Y332G, D334G, D334E, D334A, D334V, D334I, D334M, D334F, D334W, D334P, D334L, D334T, D334C, D334Y, D334N, D334K, D334R, D334H, D334S, D334Q, S336G, S336E, S336A, S336V, S336I, S336M, S336F, S336W, S336P, S336L, S336T, S336C, S336Y, S336N, S336K, S336R, S336H, S336D, S336Q, K337L, K337V, K337I, K337M, K337F, K337W, K337P, K337G, K337S, K337T, K337C, K337Y, K337N, K337E, K337R, K337H, K337D, K337Q, K341E, K341Q, K341G, K341T, K341A, K341S, G342N, H348N, R353N, Y357N, I361N, F374P, F374A, F374V, F374I, F374L, F374M, F374W, F374G, F374S, F374T, F374C, F374Y, F374N, F374E, F374K, F374R, F374H, F374D, F374Q, V376N, R379N, L390C, L390K, L390D, L390E, M391D, M391C, M391K, M391N, M391E, R392C, R392D, R392E, S393D, S393C, S393K, S393E, E394K, P395K, E394C, P395D, P395C, P395E, R396K, R396C, R396D, R396E, P397D, P397K, P397C, P397E, G398K, G398C, G398D, G398E, V399C, V399D, V399K, V399E, L400K, L401K, L401C, L401D, L401E, R402D, R402C, R402K, R402E, A403K, A403C, A403D, A403E, P404E, P404D, P404C, P404K, F405K, P406C, K32N/A34S, K32N/A34T, F31N/D33S, F31N/D33T, I30N/K32S, I30N/K32T, A34N/R36S, A34N/R36T, K38N/F40S, K38N/F40T, T37N/L39S, T37N/L39T, R36N/K38S, R36N/K38T, L39N/W41S, L39N/W41T, F40N/I42S, F40N/I42T, I42N/Y44S, I42N/Y44T, Y44N/D46S, Y44N/ D46T, D46N/D48S, D46N/D48T, G47N/Q49S, G47N/Q49T, K143N/N145S, K143N/N145T, E142N/R144S, E142N/R144T, L141N/K143S, L141N/K143T, I140N/E142S/, I140N/E142T, R144N/A146S, R144N/A146T, A146N/K148S, A146N/K148T, S147N/P149S/, S147N/P149T, R290N/A292S, R290N/A292T, D289N/G291S, D289N/G291T, L288N/R290S, L288N/R290T, L287N/D289S, L287N/D289, A292N/A294S, A292N/A294T, T293N/L295S, T293N/L295T, R315N/V317S, R315N/V317T, S314N/ K316S, S314N/ K316T, Q313N/R315S, Q313N/R315T, K316N/G318S, K316N/G318T, V317N/D319S, V317N/D319T, K341N/D343S, K341N/ D343T, S339N/K341S, S339N/K341T, D343N/G345S, D343N/G345T, R392N/E394S, R392N/E394T, L390N/ R392S, L390N/ R392T, K389N/M391S, K389N/M391T, S393N/P395S, S393N/P395T, E394N/R396S, E394N/R396T, P395N/P397S, P395N/P397T, R396N/G398S, R396N/G398T, P397N/V399S, P397N/V399T, G398N/L400S, G398N/L400T, V399N/L401S, V399N/L401T, L400N/R402S, L400N/R402T, L401N/A403S, L401N/A403T, R402N/P404S, R402N/P404T, A403N/F405S, A403N/F405T, P404N/P406S and P404N/P406T, V1858D/G237V/E296V/M298Q, K197E/G237V/M298Q, K197E/G237V/M298Q/K341Q, K197E/K199E/G237V/M298Q/K341Q, G237V/M298Q, G237V/M298Q/K341Q, M298Q/Gla Swap FIX, K197E/M298Q and M298Q/K341D

Any of the modified FVII polypeptides provided herein can contain one or more further amino acid modification(s) that increases resistance to antithrombin-III (AT-III), increases binding and/or affinity to phospholipids, increases affinity for tissue factor (TF), increases intrinsic activity, alters the conformation of the polypeptide to alter zymogenicity, increases resistance to proteases, decreases glycosylation, increases glycosylation, reduces immunogenicity, increases stability, and/or facilitates chemical group linkage. For example, altered zymogenicity can confer a more zymogen-like shape or a less zymogen-like shape. Such modified FVII polypeptides, for example, can include substitution of positions 300-322, 305-322, 300-312, or 305-312 with the corresponding amino acids from trypsin, thrombin or FX, or substitution of positions 310-329, 311-322 or 233-329 with the corresponding amino acids from trypsin.

Modified polypeptides provided herein include those in which the unmodified FVII polypeptide contains only or includes a sequence of amino acids set forth in SEQ ID NO:3. Exemplary modified FVII polypeptides are polypeptides having or including a sequence of amino acids set forth in any of SEQ ID NOS: 18-43, 125-150 and 206-250 or allelic or species variants thereof or other variants thereof. The allelic or species variant or other variant can have 40%, 50%, 60%, 65%, 70%, 75%, 80%, 85%, 88%, 90%, 91%, 92%, 93%, 94%, 95%, 96%, 97%, 98%, 99% or more sequence identity to the polypeptide set forth in SEQ ID NO: 3, excluding the amino acid modification(s). Sources of FVII polypeptides include humans and other non-human species. The polypeptides can be mature polypeptides or precursor polypeptides. In some embodiments, only the primary sequence of the FVII polypeptide is modified. In addition, the FVII polypeptides can include a chemical modification or a post-translational modification, such as, but not limited to, glycosylation, carboxylation, hydroxylation, sulfonation, phosphorylation, albumination, or conjugation to another moiety, such as a polyethylene glycol (PEG) moiety.

The modified FVII polypeptides can be provided as single-chain polypeptides or as mixtures of single-chain and two-chain or multiple chain forms, or as two-chain, three-chain or other multiple chain forms. The modified FVII polypeptides can be provided as inactive or as activated polypeptides. Activation can be effected, for example, by proteolytic cleavage by autoactivation, cleavage by Factor IX (FIXa), cleavage by Factor X (FXa), cleavage by Factor XII (FXIIa), or cleavage by thrombin.

The modified FVII polypeptides typically retain one or more activities or properties of the unmodified FVII polypeptide. Modifications can include modifications at 1, 2, 3, 4, 5, 6, 7, 8, 9, 10, 20, 30, 40, 50 or 60 amino acid positions so long as the polypeptide retains at least one FVII activity of the unmodified FVII polypeptide. Retention of activity can be at least about or 1%, 2%, 3%, 4%, 5%, 10%, 20%, 30%, 40%, 50%, 60%, 70%, 80%, 90%, 95%, 96%, 97%, 98%, 99%, 100%, 200%, 300%, 400%, 500%, or more of the activity of the unmodified FVII polypeptide. Activities include, for example, tissue factor (TF) binding, factor X (FX) activation, Factor IX (FIX) activation, phospholipid binding, and coagulation activity. Activities can be increased or decreased. Among the modified polypeptides provided herein are those in which coagulation activity is increased. Activity can be assessed *in vitro* or *in vivo.*

Also provided are nucleic acid molecules that include a sequence of nucleotides that encodes any of the modified FVII polypeptides. Also provided are vectors, such as prokaryotic vectors or eukaryotic vectors, including mammalian vectors, and viral vectors. Exemplary viral vectors include an adenovirus, an adeno-associated-virus, a retrovirus, a herpes virus, a lentivirus, a poxvirus, and a cytomegalovirus vectors. Also provided are cells containing the nucleic acid molecules or the vectors. The cells can be eukaryotic, such as mammalian or yeast cells, or prokaryotic cells. Mammalian cells include, for example, baby hamster kidney cells (BHK-21) or 293 cells and CHO cells. The cells can be grown under conditions whereby the modified FVII polypeptide is expressed. It can be secreted. Provided are the modified FVII polypeptide produced by such cells.

Also provided are compositions that contain the modified FVII polypeptides provided herein. In particular, provided are pharmaceutical compositions that contain such polypeptides. The compositions can contain a the therapeutically effective concentration or amount of a modified FVII polypeptide, or a nucleic acid molecule or a vector or a cell provided herein in a pharmaceutically acceptable vehicle. Pharmaceutical compositions can be formulated for single dosage or multiple dosage administration. The pharmaceutical composition can be in any form, such as a liquid, gel or solid, and provided in capsules, containers and other suitable vehicles. They can be formulated for dilution prior to administration or in any suitable form. The amount can depend upon the disorder treated and/or individual treated and, if necessary, can be determined empirically. The pharmaceutical composition can be formulated for any route of administration, including, for example, local, systemic, or topical administration, or formulated for oral, nasal, pulmonary buccal, transdermal, subcutaneous, intraduodenal, enteral, parenteral, intravenous, or intramuscular administration. The pharmaceutical compositions can be formulated for controlled release.

Also provided are methods of treatment and uses of the compositions for treatment. The pharmaceutical compositions are administered or formulated for administration to a subject who has a disease or condition that is treated by administration of FVII, including treatment by administration of active FVII (FVIIa). Treatment with the pharmaceutical composition ameliorates or alleviates the symptoms associated with the disease or condition. Administration can be followed by or accompanied by monitoring a subject for changes in the symptoms associated with the FVII-mediated disease or condition. Diseases or conditions treated, include, but are not limited to, blood coagulation disorders, hematologic disorders, hemorrhagic disorders, hemophilias, factor VII deficiency and bleeding disorders. Exemplary of these are hemophilia A or hemophilia B or hemophilia C. The hemophilia can be congenital or acquired, such as due to a bleeding complication due to surgery or trauma. The bleeding can be manifested as acute haemarthroses, chronic haemophilic arthropathy, haematomas, haematuria, central nervous system bleedings, gastrointestinal bleedings, or cerebral haemorrhage, ad can result from, for example, dental extraction or surgery, such as, for example, angioplasty, lung surgery, abdominal surgery, spinal surgery, brain surgery, vascular surgery, dental surgery, or organ transplant surgery, such as transplantation of bone marrow, heart, lung, pancreas, and liver. The subject can have autoantibodies to factor VIII or factor IX. The treatment can be accompanied by or administered sequentially or intermittently with one or more additional coagulation factors, such as, for example, plasma purified or recombinant coagulation factors, procoagulants, such as vitamin K, vitamin K derivative and protein C inhibitors, plasma, platelets, red blood cells and corticosteroids. The pharmaceutical compositions can be used with compositions that contain such other coagulation factors.

Provided are articles of manufacture that contain packaging material and a pharmaceutical composition provided contained within the packaging material, and optionally, instructions for administration. For example, the modified FVII polypeptide in the pharmaceutical composition can be for treatment of a FVII-mediated disease or disorder, and the packaging material can includes a label that indicates that the modified FVII polypeptide is used for treatment of a FVII -mediated disease or disorder. Also provided are kits containing the pharmaceutical compositions and a device for administration of the composition and, optionally, instructions for administration.

## BRIEF DESCRIPTION OF THE FIGURES

**Figure 1** depicts the coagulation cascade. The figure shows the intrinsic pathway and the extrinsic pathway of coagulation for the independent production of FXa and convergence of the pathways to a common pathway to generate thrombin and fibrin for the formation of a clot. These pathways are interconnected. The figure depicts the order of molecules involved in the activation cascade in which a zymogen is converted to an activated protease by cleavage of one or more peptide bonds. The activated protease then serves as the activating protease for the next zymogen molecule in the cascade, ultimately resulting in clot formation.

**Figure 2** depicts the cell based model of coagulation (*see e.g.* Hoffman et al. (2001) Thromb Haemost 85:958-965. The figure depicts the coagulation events as being separated into three phases, where initiation of coagulation is effected by the activation of FX to FXa by the TF/FVIIa complex on the TF-bearing cell, resulting in the generation of a small amount of thrombin after activation by FXa/FVa. Amplification takes place when thrombin binds to and activates the platelets, and initiates the activation of sufficient quantities of the appropriate coagulation factors to form the FVIIIa/FIXa and FVa/FXa complexes. Propagation of coagulation occurs on the surface of large numbers of activated platelets at the site of injury, resulting in a burst of thrombin generation that is sufficiently large to generate enough fibrin from fibrinogen to establish a clot at the site of injury.

**Figure 3** depicts the mechanisms by which FVIIa can initiate thrombin formation. The figure illustrates the TF-dependent pathway of FVIIa thrombin generation, which acts at the surface of a TF-bearing cell and involves complexing of FVIIa with TF prior to activation of FX to FXa. The figure also depicts the TF-independent pathway of FVIIa thrombin generation, during which FVIIa binds to phospholipids on the activated platelet and activates FX to FXa, which in turn complexes with FVa to cleave prothrombin into thrombin.

**Figure 4** depicts the quaternary inhibitory complex that results when the TFPI/FXa complex binds to the TF/FVIIa complex. TFPI contains three Kunitz domains. The Kunitz domain 2 (K-2) interacts with and inhibits FXa, while the Kunitz domain (K-1) interacts with and inhibits FVIIa.

**Figure 5** depicts the alignment of the amino acid sequence of the first Kunitz domains of (a) $BPTI^{5L15}$ (amino acid positions 1 to 55 of SEQ ID NO:106) and TFPI-2 (amino acid positions 14-65 of SEQ ID NO:105), and (b) TFPI-1 (amino acid positions 26-76 of SEQ ID NO:102) and TFPI-2 (amino acid positions 14-65 of SEQ ID NO:105), and shows conserved amino acids.

**Figure 6** depicts the homology model used to determine the contact resides at the interface of the interaction between FVIIa and TFPI. The structure of Kunitz domain 1 (K1) of TFPI-2 was taken from the trypsin/TFPI complex crystal structure and modeled onto $BPTI^{5L15}$ on the TF/FVIIa/ $BPTI^{5L15}$ crystal structure. *In silico* mutagenesis was performed to fit the corresponding amino acids of TFPI-1 K1 to the model. The contact residues of FVIIa that are likely involved in interaction with TFPI at the protein-protein interface were identified and established as candidates for mutagenesis in the development of TFPI-resistant FVII polypeptides.

**Figure 7** depicts the modeled interaction between FVIIa and TFPI. In particular, the figure depicts the FVII contact residues that are present at the interface of the FVIIa and TFPI interaction, and the corresponding TFPI contact residues, that form complementary electrostatic contacts.

## DETAILED DESCRIPTION

### Outline

**A. Definitions**
**B. Hemostasis Overview**

- **1. Platelet adhesion and aggregation**
- **2. Coagulation cascade**
  - **a. Initiation**
  - **b. Amplification**
  - **c. Propagation**
- **3. Regulation of Coagulation**

C. Factor VII (FVII)

1. FVII structure and organization
2. Post-translational modifications
3. FVII processing
4. FVII activation
5. FVII function

a. Tissue factor-dependent FVIIa activity
b. Tissue factor-independent FVIIa activity

6. FVII as a biopharmaceutical

D. Modified FVII polypeptides

1. Resistance to inhibitors

a. TFPI
Modifications to effect increased resistance to TFPI
b. Antithrombin III (AT-III)
Modifications to effect increased resistance to AT-III

2. Binding to Activated Platelets
Modification by introduction of a heterologous Gla domain
3. Combinations and additional modifications

a. Modifications that increase intrinsic activity
b. Modifications that increase resistance to proteases
c. Modifications that increase binding to phospholipids
d. Modifications that alter glycosylation
e. Modifications that facilitate chemical group linkage
f. Exemplary FVII combination mutants

E. Design and methods for modifying FVII

1. Rational
2. Emperical (i.e. screening)

a. random mutagenesis
b. Focused mutagenesis
c. Screening

3. Selecting FVII variants

F. Production of FVII polypeptides

1. Vectors and cells
2. Expression systems

a. Prokaryotic expression
b. Yeast
c. Insects and insect cells
d. Mammalian cells
e. Plants

2. Purification
3. Fusion proteins
4. Polypeptide modifications

**5. Nucelotide sequences**

**G. Assessing modified FVII polypeptide activities**

**1. *In vitro* assays**

**a. Post-translational modification**
**b. Proteolytic activity**
**c. Coagulation activity**
**d. Binding to and/or inhibition by other proteins**
**e. Phospholipid binding**

**2. Non-human animal models**
**3. Clinical assays**

**H. Formulation and administration**

**1. Formulations**

**a. Dosages**
**b. Dosage forms**

**2. Administration of modified FVII polypeptides**
**3. Administration of nucleic acids encoding modified FVII polypeptides (gene therapy)**

**I. Therapeutic Uses**

**1. Congenital bleeding disorders**

**a. Hemophilia**
**b. FVII deficiency**
**c. Others**

**2. Acquired bleeding disorders**

**a. Chemotherapy-acquired thrombocytopenia**
**b. Other coagulopathies**
**c. Transplant-acquired bleeding**
**d. Anticoagulant therapy-induced bleeding**
**e. Aquired hemophilia**

**3. Trauma and surgical bleeding**

**J. Combination Therapies**
**K. Articles of manufacture and kits**
**L. Examples**

## A. DEFINITIONS

Unless defined otherwise, all technical and scientific terms used herein have the same meaning as is commonly understood by one of skill in the art to which the invention(s) belong. In the event that there are a plurality of definitions for terms herein, those in this section prevail. Where reference is made to a URL or other such identifier or address, it understood that such identifiers can change and particular information on the internet can come and go, but equivalent information can be found by searching the internet. Reference thereto evidences the availability and public dissemination of such information.

As used herein, coagulation pathway or coagulation cascade refers to the series of activation events that leads to the formation of an insoluble fibrin clot. In the coagulation cascade or pathway, an inactive protein of a serine protease (also called a zymogen) is converted to an active protease by cleavage of one or more peptide bonds, which then serves as the activating protease for the next zymogen molecule in the cascade. In the final proteolytic step of the cascade, fibrinogen is proteolytically cleaved by thrombin to fibrin, which is then crosslinked at the site of injury to form a clot.

As used herein, "hemostasis" refers to the stopping of bleeding or blood flow in an organ or body part. The term hemostasis can encompass the entire process of blood clotting to prevent blood loss following blood vessel injury to subsequent dissolution of the blood clot following tissue repair.

As used herein, "clotting" or "coagulation" refers to the formation of an insoluble fibrin clot, or the process by which the coagulation factors of the blood interact in the coagulation cascade, ultimately resulting in the formation of an insoluble fibrin clot.

As used herein, a "protease" is an enzyme that catalyzes the hydrolysis of covalent peptidic bonds. These designations include zymogen forms and activated single-, two- and multiple-chain forms thereof. For clarity, reference to proteases refer to all forms. Proteases include, for example, serine proteases, cysteine proteases, aspartic proteases, threonine and metallo-proteases depending on the catalytic activity of their active site and mechanism of cleaving peptide bonds of a target substrate.

As used herein, serine proteases or serine endopeptidases refers to a class of peptidases, which are characterized by the presence of a serine residue in the active site of the enzyme. Serine proteases participate in a wide range of functions in the body, including blood clotting and inflammation, as well as functioning as digestive enzymes in prokaryotes and eukaryotes. The mechanism of cleavage by serine proteases is based on nucleophilic attack of a targeted peptidic bond by a serine. Cysteine, threonine or water molecules associated with aspartate or metals also can play this role. Aligned side chains of serine, histidine and aspartate form a catalytic triad common to most serine proteases. The active site of serine proteases is shaped as a cleft where the polypeptide substrate binds.

As used herein, Factor VII (FVII, F7; also referred to as Factor 7, coagulation factor VII, serum factor VII, serum prothrombin conversion accelerator, SPCA, proconvertin and eptacog alpha) refers to a serine protease that is part of the coagulation cascade. FVII includes a Gla domain, two EGF domains (EGF-1 and EGF-2), and a serine protease domain (or peptidase S1 domain) that is highly conserved among all members of the peptidase S1 family of serine proteases, such as for example with chymotrypsin. The sequence of an exemplary precursor FVII having a signal peptide and propeptide is set forth in SEQ ID NO: 1. An exemplary mature FVII polypeptide is set forth in SEQ ID NO:3. FVII occurs as a single chain zymogen, a zymogen-like two-chain polypeptide and a fully activated two-chain form. Full activation, which occurs upon conformational change from a zymogen-like form, occurs upon binding to is co-factor tissue factor. Also, mutations can be introduced that result in the conformation change in the absence of tissue factor. Hence, reference to FVII includes single-chain and two-chain forms thereof, including zymogen-like and fully activated two-chain forms.

Reference to FVII polypeptide also includes precursor polypeptides and mature FVII polypeptides in single-chain or two-chain forms, truncated forms thereof that have activity, and includes allelic variants and species variants, variants encoded by splice variants, and other variants, including polypeptides that have at least 40%, 45%, 50%, 55%, 65%, 70%, 75%, 80%, 85%, 90%, 95%, 96%, 97%, 98%, 99% or more sequence identity to the precursor polypeptide set forth in SEQ ID NO: 1 or the mature form thereof. Included are modified FVII polypeptides, such as those of SEQ ID NOS:18-43, 125-150 or 206-150 and variants thereof. Also included are those that retain at least an activity of a FVII, such as TF binding, factor X binding, phospholipid binding, and/or coagulant activity of a FVII. By retaining activity, the activity can be altered, such as reduced or increased, as compared to a wild-type FVII so long as the level of activity retained is sufficient to yield a detectable effect. FVII polypeptides include, but are not limited to, tissue-specific isoforms and allelic variants thereof, synthetic molecules prepared by translation of nucleic acids, proteins generated by chemical synthesis, such as syntheses that include ligation of shorter polypeptides, through recombinant methods, proteins isolated from human and non-human tissue and cells, chimeric FVII polypeptides and modified forms thereof. FVII polypeptides also include fragments or portions of FVII that are of sufficient length or include appropriate regions to retain at least one activity (upon activation if needed) of a full-length mature polypeptide. FVII polypeptides also include those that contain chemical or posttranslational modifications and those that do not contain chemical or posttranslational modifications. Such modifications include, but are not limited to, pegylation, albumination, glycosylation, farnysylation, carboxylation, hydroxylation, phosphorylation, and other polypeptide modifications known in the art.

Exemplary FVII polypeptides are those of mammalian, including human, origin. Exemplary amino acid sequences of FVII of human origin are set forth in SEQ ID NOS: 1, 2, and 3. Exemplary variants of such a human FVII polypeptide, include any of the precursor polypeptides set forth in SEQ ID NOS: 44-100. FVII polypeptides also include any of non-human origin including, but not limited to, murine, canine, feline, leporine, avian, bovine, ovine, porcine, equine, piscine, ranine, and other primate factor VII polypeptides. Exemplary FVII polypeptides of non-human origin include, for example, cow (*Bos taurus,* SEQ ID NO:4), mouse (*Mus musculus,* SEQ ID NO:5), pygmy chimpanzee (*Pan paniscus,* SEQ ID NO:6), chimpanzee (*Pan troglodytes,* SEQ ID NO:7), rabbit (*Oryctolagus cuniculus,* SEQ ID NO:8), rat (*Rattus norvegicus,* SEQ ID NO: 9), rhesus macaque (*Macaca mulatta*, SEQ ID NO:10), pig (*Sus scrofa,* SEQ ID NO:11), dog (*Canis familiaris,* SEQ ID NO:12), zebrafish (*Brachydanio rerio,* SEQ ID NO:13), Japanese pufferfish (*Fugu rubripes,* SEQ ID NO:14), chicken (*Gallus gallus,* SEQ ID NO:15), orangutan (*Pongo pygmaeus,* SEQ ID NO: 16) and gorilla (*Gorilla gorilla,* SEQ ID NO:17).

One of skill in the art recognizes that the referenced positions of the mature factor VII polypeptide (SEQ ID NO: 3) differ by 60 amino acid residues when compared to the isoform a precursor FVII polypeptide set forth in SEQ ID NO: 1, which is the isoform a factor VII polypeptide containing the signal peptide and propeptide sequences. Thus, the first amino acid residue of SEQ ID NO: 3 "corresponds to" the sixty first (61st) amino acid residue of SEQ ID NO: 1. One of skill in the art also recognizes that the referenced positions of the mature factor VII polypeptide (SEQ ID NO: 3) differ by 38 amino acid residues when compared to the precursor FVII polypeptide set forth in SEQ ID NO:2, which is the isoform b factor VII polypeptide containing the signal peptide and propeptide sequences. Thus, the first amino acid residue of SEQ ID NO: 3 "corresponds to" the thirty-nineth ($39^{th}$) amino acid residue of SEQ ID NO:2.

As used herein, corresponding residues refers to residues that occur at aligned loci. Related or variant polypeptides are aligned by any method known to those of skill in the art. Such methods typically maximize matches, and include methods such as using manual alignments and by using the numerous alignment programs available (for example, BLASTP) and others known to those of skill in the art. By aligning the sequences of polypeptides, one skilled in the art can identify corresponding residues, using conserved and identical amino acid residues as guides. For example, by aligning the sequences of factor VII polypeptides, one of skill in the art can identify corresponding residues, using conserved and identical amino acid residues as guides. For example, the alanine in amino acid position 1 (A1) of SEQ ID NO:3 (mature factor VII) corresponds to the alanine in amino acid position 61 (A61) of SEQ ID NO:1, and the alanine in amino acid position 39 (A39) of SEQ ID NO:2. In other instances, corresponding regions can be identified. For example, the Gla domain corresponds to amino acid positions A1 through F45 of SEQ ID NO:3, to amino acid positions A61 through S105 of SEQ ID NO:1 and to amino acid positions A39 to S83 of SEQ ID NO:2. One skilled in the art also can employ conserved amino acid residues as guides to find corresponding amino acid residues between and among human and non-human sequences. For example, amino acid residues S43 and E163 of SEQ ID NO:3 (human) correspond to S83 and E203 of SEQ ID NO: 4 (bovine). Corresponding positions also can be based on structural alignments, for example by using computer simulated alignments of protein structure. In other instances, corresponding regions can be identified.

As used herein, a "proregion," "propeptide," or "pro sequence," refers to a region or a segment that is cleaved to produce a mature protein. This can include segments that function to suppress proteolytic activity by masking the catalytic machinery and thus preventing formation of the catalytic intermediate (*i.e.*, by sterically occluding the substrate binding site). A proregion is a sequence of amino acids positioned at the amino terminus of a mature biologically active polypeptide and can be as little as a few amino acids or can be a multidomain structure.

As used herein, "mature factor VII" refers to a FVII polypeptide that lacks a signal sequence and a propeptide sequence. Typically, a signal sequence targets a protein for secretion via the endoplasmic reticulum (ER)-golgi pathway and is cleaved following insertion into the ER during translation. A propeptide sequence typically functions in post-translational modification of the protein and is cleaved prior to secretion of the protein from the cell. Thus, a mature FVII polypeptide is typically a secreted protein. In one example, a mature human FVII polypeptide is set forth in SEQ ID NO:3. The amino acid sequence set forth in SEQ ID NO:3 differs from that of the precursor polypeptides set forth in SEQ ID NOS:1 and 2 in that SEQ ID NO:3 is lacking the signal sequence, which corresponds to amino acid residues 1-20 of SEQ ID NOS:1 and 2; and also lacks the propeptide sequence, which corresponds to amino acid residues 21-60 of SEQ ID NO:1 and amino acid residues 21-38 of SEQ ID NO:2. Reference to a mature FVII polypeptide encompasses the single-chain zymogen form and the two-chain form.

As used herein, "wild-type" or "native" with reference to FVII refers to a FVII polypeptide encoded by a native or naturally occurring FVII gene, including allelic variants, that is present in an organism, including a human and other animals, in nature. Reference to wild-type factor VII without reference to a species is intended to encompass any species of a wild-type factor VII. Included among wild-type FVII polypeptides are the encoded precursor polypeptide, fragments thereof, and processed forms thereof, such as a mature form lacking the signal peptide as well as any pre- or post-translationally processed or modified forms thereof. Also included among native FVII polypeptides are those that are post-translationally modified, including, but not limited to, modification by glycosylation, carboxylation and hydroxylation. Native FVII polypeptides also include single-chain and two-chain forms. For example, humans express native FVII. The amino acid sequence of exemplary wild-type human FVII are set forth in SEQ ID NOS: 1, 2, 3 and allelic variants set forth in SEQ ID NOS:44-100 and the mature forms thereof. Other animals produce native FVII, including, but not limited to, cow (*Bos Taurus,* SEQ ID NO:4), mouse (*Mus musculus,* SEQ ID NO:5), pygmy chimpanzee (*Pan paniscus,* SEQ ID NO:6), chimpanzee (*Pan troglodytes,* SEQ ID NO:7), rabbit (*Oryctolagus cuniculus,* SEQ ID NO:8), rat (*Rattus norvegicus,* SEQ ID NO: 9), rhesus macaque (*Macaca mulatta*, SEQ ID NO:10), pig (*Sus scrofa*, SEQ ID NO:11), dog (*Canis familiaris,* SEQ ID NO:12), zebrafish (*Brachydanio rerio,* SEQ ID NO:13) Japanese pufferfish (*Fugu rubripes*, SEQ ID NO:14), chicken (*Gallus gallus,* SEQ ID NO:15), orangutan (*Pongo pygmaeus,* SEQ ID NO:16) and gorilla (*Gorilla gorilla,* SEQ ID NO:17).

As used herein, species variants refer to variants in polypeptides among different species, including different mammalian species, such as mouse and human.

As used herein, allelic variants refer to variations in proteins among members of the same species.

As used herein, a splice variant refers to a variant produced by differential processing of a primary transcript of genomic DNA that results in more than one type ofmRNA.

As used herein, a zymogen refers to a protease that is activated by proteolytic cleavage, including maturation cleavage, such as activation cleavage, and/or complex formation with other protein(s) and/or cofactor(s). A zymogen is an inactive precursor of a proteolytic enzyme. Such precursors are generally larger, although not necessarily larger, than the active form. With reference to serine proteases, zymogens are converted to active enzymes by specific cleavage, including catalytic and autocatalytic cleavage, or by binding of an activating co-factor, which generates an active enzyme. For example, generally, zymogens are present in a single-chain form. Zymogens, generally, are inactive and can be converted to mature active polypeptides by catalytic or autocatalytic cleavage at one or more proteolytic sites to generate a multi-chain, such as a two-chain, polypeptide. A zymogen, thus, is an enzymatically inactive protein that is converted to a proteolytic enzyme by the action of an activator. Cleavage can be effected by autoactivation. A number of coagulation proteins are zymogens; they are inactive, but become cleaved and activated upon the initiation of the coagulation system following vascular damage. With reference to FVII polypeptides exist in the blood plasma as zymogens until cleavage by aproteases, such as for example, activated factor IX (FIXa), activated factor X (FXa), activated factor XII (FXIIa), thrombin, or by autoactivation to produce a zymogen-like two-chain form, which then requires further conformation change for full activity.

As used herein, a "zymogen-like" protein or polypeptide refers to a protein that has been activated by proteolytic cleavage, but still exhibits properties that are associated with a zymogen, such as, for example, low or no activity, or a conformation that resembles the conformation of the zymogen form of the protein. For example, when it is not bound to tissue factor, the two-chain activated form of FVII is a zymogen-like protein; it retains a conformation similar to the uncleaved FVII zymogen, and, thus, exhibits very low activity. Upon binding to tissue factor, the two-chain activated form of FVII undergoes conformational change and acquires its full activity as a coagulation factor.

As used herein, an activation sequence refers to a sequence of amino acids in a zymogen that is the site required for activation cleavage or maturation cleavage to form an active protease. Cleavage of an activation sequence can be catalyzed autocatalytically or by activating partners.

As used herein, activation cleavage is a type of maturation cleavage, which induces a conformation change that is required for the development of full enzymatic activity. This is a classical activation pathway, for example, for serine proteases in which a cleavage generates a new N-terminus that interacts with the conserved regions of the protease, such as Asp194 in chymotrypsin, to induce conformational changes required for activity. Activation can result in production of multi-chain forms of the proteases. In some instances, single chain forms of the protease can exhibit proteolytic activity.

As used herein, "activated Factor VII" or "FVIIa" refers to any two-chain form of a FVII polypeptide. A two-chain form typically results from proteolytic cleavage, but can be produced synthetically. Activated Factor VII, thus, includes the zymogen-like two-chain form with low coagulant activity, a fully activated form (about 1000-fold more activity) that occurs upon binding to tissue factor, and mutated forms that exist in a fully activated two-chain form or undergo conformation change to a fully activated form. For example, a single-chain form of FVII polypeptide (see, *e.g.,* SEQ ID NO:3) is proteolytically cleaved between amino acid residues R152 and I153 of the mature FVII polypeptide. The cleavage products, FVII heavy chain and FVII light chain, which are held together by a disulfide bond (between amino acid residues 135C and 162C in the FVII of SEQ ID NO:3), form the two-chain activated FVII enzyme. Proteolytic cleavage can be carried out, for example, by activated factor IX (FIXa), activated factor X (FXa), activated factor XII (FXIIa), thrombin, or by autoactivation.

As used herein, a "property" of a FVII polypeptide refers to a physical or structural property, such three-dimensional structure, pI, half-life, conformation and other such physical characteristics.

As used herein, an "activity" of a FVII polypeptide refers to any activity exhibited by a factor VII polypeptide. Such activities can be tested *in vitro* and/or in *vivo* and include, but are not limited to, coagulation or coagulant activity, pro-coaguant activity, proteolytic or catalytic activity such as to effect factor X (FX) activation or Factor IX (FIX) activation; antigenicity (ability to bind to or compete with a polypeptide for binding to an anti-FVII antibody); ability to bind tissue factor, factor X or factor IX; and/or ability to bind to phospholipids. Activity can be assessed *in vitro* or *in vivo* using recognized assays, for example, by measuring coagulation in *vitro* or *in vivo.* The results of such assays indicate that a polypeptide exhibits an activity that can be correlated to activity of the polypeptide *in vivo,* in which *in vivo* activity can be referred to as biological activity. Assays to determine functionality or activity of modified forms of FVII are known to those of skill in the art. Exemplary assays to assess the activity of a FVII polypeptide include prothromboplastin time (PT) assay or the activated partial thromboplastin time (aPTT) assay to assess coagulant activity, or chromogenic assays using synthetic substrates, such as described in Examples 4, 5 and 11, to assess catalytic or proteolytic activity.

As used herein, "exhibits at least one activity" or "retains at least one activity" refers to the activity exhibited by a modified FVII polypeptide as compared to an unmodified FVII polypeptide of the same form and under the same conditions. For example, a modified FVII polypeptide in a two-chain form is compared with an unmodified FVII polypeptide in a two-chain form, under the same experimental conditions, where the only difference between the two polypeptides is the modification under study. In another example, a modified FVII polypeptide in a single-chain form is compared with an unmodified FVII polypeptide in a single-chain form, under the same experimental conditions, where the only difference between the two polypeptides is the modification under study. Typically, a modified FVII polypeptide that retains or exhibits at least one activity of an unmodified FVII polypeptide of the same form retains a sufficient amount of the activity such that, when administered *in vivo,* the modified FVII polypeptide is therapeutically effective as a procoagulant therapeutic. Generally, for a modified FVII polypeptide to retain therapeutic efficacy as a procoagulant, the amount of activity that is retained is or is about 0.5%, 1%, 2%, 3%, 4%, 5%, 6%, 7%, 8%, 9%, 10%, 11%, 12%, 13%, 14%, 15%, 16%, 17%, 18%, 19%, 20%, 30%, 40%, 50%, 60%, 70%, 80%, 90%, 100%, 200%, 300%, 400%, 500% or more of the activity of an unmodified FVII polypeptide of the same form that displays therapeutic efficacy as a procoagulant. The amount of activity that is required to maintain therapeutic efficacy as a procoagulant can be empirically determined, if necessary. Typically, retention of 0.5% to 20%, 0.5% to 10%, 0.5% to 5% of an activity is sufficient to retain therapeutic efficacy as a procoagulant *in vivo.*

It is understood that the activity being exhibited or retained by a modified FVII polypeptide can be any activity, including, but not limited to, coagulation or coagulant activity, pro-coagulant activity; proteolytic or catalytic activity such as to effect factor X (FX) activation or Factor IX (FIX) activation; antigenicity (ability to bind to or compete with a polypeptide for binding to an anti-FVII antibody); ability to bind tissue factor, factor X or factor IX; and/or ability to bind to phospholipids. In some instances, a modified FVII polypeptide can retain an activity that is increased compared to an unmodified FVII polypeptide. In some cases, a modified FVII polypeptide can retain an activity that is decreased compared to an unmodified FVII polypeptide. Activity of a modified FVII polypeptide can be any level of percentage of activity of the unmodified polypeptide, where both polypeptides are in the same form, including but not limited to, 1% of the activity, 2%, 3%, 4%, 5%, 10%, 20%, 30%, 40%, 50%, 60%, 70%, 80%, 90%, 95%, 96%, 97%, 98%, 99%, 100%, 200%, 300%, 400%, 500%, or more activity compared to the polypeptide that does not contain the modification at issue. For example, a modified FVII polypeptide can exhibit increased or decreased activity compared to the unmodified FVII polypeptide in the same form. For example, it can retain at least about or 1%, 2%, 3%, 4%, 5%, 6%, 7%, 8%, 9%, 10%, 20%, 30%, 40%, 50%, 60%, 70%, 80%, 90%, 95%, 96%, 97%, 98% or at least 99% of the activity of the unmodified FVII polypeptide. In other embodiments, the change in activity is at least about 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more times greater than unmodified FVII. The particular level to be retained is a function of the intended use of the polypeptide and can be empirically determined. Activity can be measured, for example, using *in vitro* or *in vivo* assays such as those described herein or in the Examples below.

As used herein, "coagulation activity" or "coagulant activity" or "pro-coagulant activity" refers to the ability of a polypeptide to effect coagulation. Assays to assess coagulant activity are known to those of skill in the art, and include prothromboplastin time (PT) assay or the activated partial thromboplastin time (aPTT) assay.

As used herein, "catalytic activity" or "proteolytic activity" with reference to FVII refers to the ability of a FVII protein to catalyze the proteolytic cleavage of a substrate, and are used interchangeably. Assays to assess such activities are known in the art. For example, the proteolytic activity of FVII can be measured using chromogenic substrates such as Spectrozyme FVIIa (CH3SO2-D-CHA-But-Arg-pNA), where cleavage of the substrate is monitored by absorbance and the rate of substrate hydrolysis determined by linear regression.

As used herein, "intrinsic activity" with reference to FVII refers to the catalytic, proteolytic, and/or coagulant activity of a FVII protein in the absence of tissue factor.

As used herein, domain (typically a sequence of three or more, generally 5 or 7 or more amino acids) refers to a portion of a molecule, such as proteins or the encoding nucleic acids, that is structurally and/or functionally distinct from other portions of the molecule and is identifiable. For example, domains include those portions of a polypeptide chain that can form an independently folded structure within a protein made up of one or more structural motifs and/or that is recognized by virtue of a functional activity, such as proteolytic activity. A protein can have one, or more than one, distinct domains. For example, a domain can be identified, defined or distinguished by homology of the sequence therein to related family members, such as homology to motifs that define a protease domain or a gla domain. In another example, a domain can be distinguished by its function, such as by proteolytic activity, or an ability to interact with a biomolecule, such as DNA binding, ligand binding, and dimerization. A domain independently can exhibit a biological function or activity such that the domain independently or fused to another molecule can perform an activity, such as, for example proteolytic activity or ligand binding. A domain can be a linear sequence of amino acids or a non-linear sequence of amino acids. Many polypeptides contain a plurality of domains. Such domains are known, and can be identified by, those of skill in the art. For exemplification herein, definitions are provided, but it is understood that it is well within the skill in the art to recognize particular domains by name. If needed appropriate software can be employed to identify domains.

As used herein, a protease domain is the catalytically active portion of a protease. Reference to a protease domain of a protease includes the single, two- and multi-chain forms of any of these proteins. A protease domain of a protein contains all of the requisite properties of that protein required for its proteolytic activity, such as for example, the catalytic center. In reference to FVII, the protease domain shares homology and structural feature with the chymotrypsin/trypsin family protease domains, including the catalytic triad. For example, in the mature FVII polypeptide set forth in SEQ ID NO:3, the protease domain corresponds to amino acid positions 153 to 392.

As used herein, a gamma-carboxyglutamate (Gla) domain refers to the portion of a protein, for example a vitamin K-dependent protein, that contains post-translational modifications of glutamate residues, generally most, but not all of the glutamate residues, by vitamin K-dependent carboxylation to form Gla. The Gla domain is responsible for the high-affinity binding of calcium ions and binding to negatively-charged phospholipids. Typically, the Gla domain starts at the N-terminal extremity of the mature form of vitamin K-dependent proteins and ends with a conserved aromatic residue. In a mature FVII polypeptide the Gla domain corresponds to amino acid positions 1 to 45 of the exemplary polypeptide set forth in SEQ ID NO:3. Gla domains are well known and their locus can be identified in particular polypeptides. The Gla domains of the various vitamin K-dependent proteins share sequence, structural and functional homology, including the clustering of N-terminal hydrophobic residues into a hydrophobic patch that mediates interaction with negatively charged phospholipids on the cell surface membrane. Exemplary other Gla-containing polypeptides include, but are not limited to, FIX, FX, prothrombin, protein C, protein S, osteocalcin, matrix Gla protein, Growth-arrest-specific protein 6 (Gas6), and protein Z. The Gla domains of these and other exemplary proteins are set forth in any of SEQ ID NOS: 110-121.

As used herein, "native" or "endogenous" with reference to a Gla domain refers to the naturally occurring Gla domain associated with all or a part of a polypeptide having a Gla domain. For purposes herein, a native Gla domain is with reference to a FVII polypeptide. For example, the native Gla domain of FVII, set forth in SEQ ID NO:119, corresponds to amino acids 1-45 of the sequence of amino acids set forth in SEQ ID NO:3.

As used herein, a heterologous Gla domain refers to the Gla domain from a polypeptide, from the same or different species, that is not a FVII Gla domain. Exemplary of heterologous Gla domains are the Gla domains from Gla-containing polypeptides including, but are not limited to, FIX, FX, prothrombin, protein C, protein S, osteocalcin, matrix Gla protein, Growth-arrest-specific protein 6 (Gas6), and protein Z. The Gla domains of these and other exemplary proteins are set forth in any of SEQ ID NOS: 110-118, 120 and 121.

As used herein, a contiguous portion of a Gla domain refers to at least two or more adjacent amino acids, typically 2, 3, 4, 5, 6, 8, 10, 15, 20, 30, 40 or more up to all amino acids that make up a Gla domain.

As used herein, "a sufficient portion of a Gla domain to effect phospholipid binding" includes at least one amino acid, typically, 2, 3, 4, 5, 6, 8, 10, 15 or more amino acids of the domain, but fewer than all of the amino acids that make up the domain so long as the polypeptide that contains such portion exhibits phospholipid binding.

As used herein, "replace" with respect to a Gla domain or "Gla domain swap" refers to the process by which the endogenous Gla domain of a protein is replaced, using recombinant, synthetic or other methods, with the Gla domain of another protein. In the context of a "Gla domain swap", a "Gla domain" is any selection of amino acids from a Gla domain and adjacent regions that is sufficient to retain phospholipid binding activity. Typically, a Gla domain swap will involve the replacement of between 40 and 50 amino acids of the endogenous protein with between 40 and 50 amino acids of another protein, but can involve fewer or more amino acids.

As used herein, an epidermal growth factor (EGF) domain (EGF-1 or EGF-2) refers to the portion of a protein that shares sequence homology to a specific 30 to 40 amino acid portion of the epidermal growth factor (EGF) sequence. The EGF domain includes six cysteine residues that have been shown (in EGF) to be involved in disulfide bonds. The main structure of an EGF domain is a two-stranded beta-sheet followed by a loop to a C-terminal short two-stranded sheet. FVII contains two EGF domains: EGF-1 and EGF-2. These domains correspond to amino acid positions 46-82, and 87-128, respectively, of the mature FVII polypeptide set forth in SEQ ID NO:3.

As used herein, "unmodified polypeptide" or "unmodified FVII" and grammatical variations thereof refer to a starting polypeptide that is selected for modification as provided herein. The starting polypeptide can be a naturally-occurring, wild-type form of a polypeptide. In addition, the starting polypeptide can be altered or mutated, such that it differs from a native wild type isoform but is nonetheless referred to herein as a starting unmodified polypeptide relative to the subsequently modified polypeptides produced herein. Thus, existing proteins known in the art that have been modified to have a desired increase or decrease in a particular activity or property compared to an unmodified reference protein can be selected and used as the starting unmodified polypeptide. For examples, a protein that has been modified from its native form by one or more single amino acid changes and possesses either an increase or decrease in a desired property, such as a change in a amino acid residue or residues to alter glycosylation, can be a target protein, referred to herein as unmodified, for further modification of either the same or a different property. Exemplary modified FVII polypeptides known in the art include any FVII polypeptide described in, for example, U.S. Patent Nos. 5580560, 6017882, 6693075, 6762286 and 6806063, U.S. Patent Publication Nos. 20030100506 and 20040220106 and International Patent Publication Nos. WO1988010295, WO200183725, WO2003093465, WO200338162, WO2004083361, WO2004108763, WO2004029090, WO2004029091, WO2004111242 and WO2005123916.

As used herein, "modified factor VII polypeptides" and "modified factor VII" refer to a FVII polypeptide that has one or more amino acid differences compared to an unmodified factor VII polypeptide. The one or more amino acid differences can be amino acid mutations such as one or more amino acid replacements (substitutions), insertions or deletions, or can be insertions or deletions of entire domains, and any combinations thereof. Typically, a modified FVII polypeptide has one or more modifications in primary sequence compared to an unmodified FVII polypeptide. For example, a modified FVII polypeptide provided herein can have 1, 2, 3, 4, 5, 6, 7, 8, 9, 10, 11, 12, 13, .14, 15, 16, 17, 18, 19, 20, 30, 40, 50 or more amino acid differences compared to an unmodified FVII polypeptide. Any modification is contemplated as long as the resulting polypeptide exhibits at least one FVII activity associated with a native FVII polypeptide, such as, for example, catalytic activity, proteolytic activity, the ability to bind TF or the ability to bind activated platelets.

As used herein, "inhibitors of coagulation" refer to proteins or molecules that act to inhibit or prevent coagulation or clot formation. The inhibition or prevention of coagulation can be observed *in vivo* or *in vitro,* and can be assayed using any method known in the art including, but not limited to, prothromboplastin time (PT) assay or the activated partial thromboplastin time (aPTT) assay.

As used herein, tissue factor pathway inhibitor (TFPI, also referred to as TFPI-1) is a Kunitz-type inhibitor that is involved in the formation of a quaternary TF/FVIIa/TFPI/FXa inhibitory complex in which the activity of FVIIa is inhibited. TFPI is expressed as two different precursor forms following alternative splicing, TFPIa (SEQ ID NO:101) and TFPIβ (SEQ ID NO:103) precursors, which are cleaved during secretion to generate a 276 amino acid (SEQ ID NO:102) and a 223 amino acid (SEQ ID NO:104) mature protein, respectively. TFPI contains 3 Kunitz domains, of which the Kunitz-1 domain is responsible for binding and inhibition of FVIIa.

As used herein, TFPI-2 (also is known as placental protein 5 (PP5) and matrix-associated serine protease inhibitor (MSPI)) refers to a homolog of TFPI. The 213 amino acid mature TFPI-2 protein (SEQ ID NO:105) contains three Kunitz-type domains that exhibit 43%, 35% and 53% primary sequence identity with TFPI-1 Kunitz-type domains 1, 2, and 3, respectively. TFPI-2 plays a role in the regulation of extracellular matrix digestion and remodeling, and is not thought to be an important factor in the coagulation pathway.

As used herein, antithrombin III (AT-III) is a serine protease inhibitor (serpin). AT-III is synthesized as a precursor protein containing 464 amino acid residues (SEQ ID NO:122) that is cleaved during secretion to release a 432 amino acid mature antithrombin (SEQ ID NO:123).

As used herein, cofactors refer to proteins or molecules that bind to other specific proteins or molecules to form an active complex. In some examples, binding to a cofactor is required for optimal proteolytic activity. For example, tissue factor (TF) is a cofactor of FVIIa. Binding of FVIIa to TF induces conformational changes that result in increased proteolytic activity of FVIIa for its substrates, FX and FIX.

As used herein, tissue factor (TF) refers to a 263 amino acids transmembrane glycoprotein (SEQ ID NO:124) that functions as a cofactor for FVIIa. It is constitutively expressed by smooth muscle cells and fibroblasts, and helps to initiate coagulation by binding FVII and FVIIa when these cells come in contact with the bloodstream following tissue injury.

As used herein, activated platelet refers to a platelet that has been triggered by the binding of molecules such as collagen, thromboxane A2, ADP and thrombin to undergo various changes in morphology, phenotype and function that ultimately promote coagulation. For example, an activated platelet changes in shape to a more amorphous form with projecting fingers. Activated platelets also undergo a "flip" of the cell membrane such that phosphatidylserine and other negatively charged phospholipids that are normally present in the inner leaflet of the cell membrane are translocated to the outer, plasma-oriented surface. These membranes of the activated platelets provide the surface on which many of the reactions of the coagulation cascade are effected. Activated platelets also secrete vesicles containing such pro-coagulant factors as vWF, FV, thrombin, ADP and thromboxane A2, and adhere to one another to form a platelet plug which is stabilized by fibrin to become a clot.

As used herein, increased binding and/or affinity for activated platelets, and any grammatical variations thereof, refers to an enhanced ability of a polypeptide or protein, for example a FVII polypeptide, to bind to the surface of an activated platelet, as compared with a reference polypeptide or protein. For example, the ability of a modified FVII polypeptide to bind to activated platelets can be greater than the ability of the unmodified FVII polypeptide to bind to activated platelets. The binding and/or affinity of a polypeptide for activated platelets can be increased by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the binding and/or affinity of an unmodified polypeptide. Assays to determine the binding and/or affinity of a polypeptide for activated platelets are known in the art. Binding of a FVII polypeptide to activated platelets is mediated through the interaction of amino acids in the Gla domain of the FVII polypeptide and negatively charged phospholipids, such as phosphatidylserine, on the activated platelet. As such, methods to assay for binding of polypeptides, such as FVII polypeptides, to activated platelets use membranes and vesicles that contain phospholipids, such as phosphatidylserine. For example, the ability of a polypeptide to bind to an activated platelet is reflected by the ability of the polypeptide to bind to phospholipid vesicles, which can be measured by light scattering techniques.

As used herein, increased binding and/or affinity for phospholipids, and any grammatical variations thereof, refers to an enhanced ability of a polypeptide or protein to bind to phospholipids as compared with a reference polypeptide or protein. Phospholipids can include any phospholipids, but particularly include phosphatidylserine. The binding and/or affinity of a polypeptide for phospholipids can be increased by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the binding and/or affinity of an unmodified polypeptide. Assays to determine the affinity and/or binding of a polypeptide to phospholipids are known in the art. For example, FVII polypeptide binding to phospholipid vesicles can be determined by relative light scattering at 90° to the incident light. The intensity of the light scatter with the phospholipid vesicles alone and with phospholipid vesicles with FVII is measured to determine the dissociation constant. Surface plasma resonance, such as on a BIAcore biosensor instrument, also can be used to measure the affinity of FVII polypeptides for phospholipid membranes.

As used herein, increased resistance to inhibitors or "increased resistance to TFPI" or "increased resistance to AT-III" refers to any amount of decreased sensitivity of a polypeptide to the inhibitory effects of an inhibitor, such as TFPI or AT-III, compared with a reference polypeptide, such as an unmodified FVII polypeptide. For example, TFPI, complexed with FXa, binds to the TF/FVIIa complex. In doing so, it inhibits the activity of FVIIa. Hence, a modified FVII polypeptide that reduces or prevents the binding of TFPI to the TF/FVIIa complex, and therefore reduces or prevents the TFPI-mediated inhibition of FVIIa activity, displays increased resistance to TFPI. Increased resistance to an inhibitor, such as TFPI, can be assayed by assessing the binding of a modified FVII polypeptide to an inhibitor. Increased resistance to an inhibitor, such as TFPI, also can be assayed by measuring the intrinsic activity or coagulant activity of a FVII polypeptide in the presence of TFPI. Assays to determine the binding of a polypeptide to an inhibitor, such as TFPI or AT-III, are known in the art. For non-covalent inhibitors, such as, for example, TFPI, a $k_i$ can be measured. For covalent inhibitors, such as, for example, AT-III, a second order rate constant for inhibition can be measured. In addition, surface plasma resonance, such as on a BIAcore biosensor instrument, also can be used to measure the binding of FVII polypeptides to TFPI, AT-III or other inhibitors. However, for covalent inhibitors such as AT-III, only an on-rate can be measured using BIAcore. Assays to determine the inhibitory effect of, for example, TFPI on FVII coagulant activity or intrinsic activity also are known in the art. For example, the ability of a modified FVII polypeptide to cleave its substrate FX in the presence or absence of TFPI can be measured, and the degree to which TFPI inhibits the reaction determined. This can be compared to the ability of an unmodified FVII polypeptide to cleave its substrate FX in the presence or absence of TFPI. A modified polypeptide that exhibits increased resistance to an inhibitor exhibits, for example, an increase of 1%, 2%, 3%, 4%, 5%, 6%, 7%, 8%, 9%, 10%, 20%, 30%, 40%, 50%, 60%, 70%, 80%, 90%, 91%, 92%, 93%, 94%, 95%, 96%, 97%, 98%, 99%, 100%, 200%, 300%, 400%, 500%, or more resistance to the effects of an inhibitor compared to an unmodified polypeptide.

As used herein, "biological activity" refers to the *in vivo* activities of a compound or physiological responses that result upon *in vivo* administration of a compound, composition or other mixture. Biological activity, thus, encompasses therapeutic effects and pharmaceutical activity of such compounds, compositions and mixtures. Biological activities can be observed in *in vitro* systems designed to test or use such activities. Thus, for purposes herein a biological activity of a FVII polypeptide encompasses the coagulant activity.

As used herein the term "assess", and grammatical variations thereof, is intended to include quantitative and qualitative determination in the sense of obtaining an absolute value for the activity of a polypeptide, and also of obtaining an index, ratio, percentage, visual or other value indicative of the level of the activity. Assessment can be direct or indirect. For example, detection of cleavage of a substrate by a polypeptide can be by direct measurement of the product, or can be indirectly measured by determining the resulting activity of the cleaved substrate.

As used herein, "chymotrypsin numbering" refers to the amino acid numbering of a mature chymotrypsin polypeptide of SEQ ID NO:107. Alignment of a protease domain of another protease, such as for example the protease domain of factor VII, can be made with chymotrypsin. In such an instance, the amino acids of factor VII that correspond to amino acids of chymotrypsin are given the numbering of the chymotrypsin amino acids. Corresponding positions can be determined by such alignment by one of skill in the art using manual alignments or by using the numerous alignment programs available (for example, BLASTP). Corresponding positions also can be based on structural alignments, for example by using computer simulated alignments of protein structure. Recitation that amino acids of a polypeptide correspond to amino acids in a disclosed sequence refers to amino acids identified upon alignment of the polypeptide with the disclosed sequence to maximize identity or homology (where conserved amino acids are aligned) using a standard alignment algorithm, such as the GAP algorithm. The corresponding chymotrypsin numbers of amino acid positions 153 to 406 of the FVII polypeptide set forth in SEQ ID NO:3 are provided in Table 1. The amino acid positions relative to the sequence set forth in SEQ ID NO:3 are in normal font, the amino acid residues at those positions are in bold, and the corresponding chymotrypsin numbers are in italics. For example, upon alignment of the mature factor VII (SEQ ID NO:3) with mature chymotrypsin (SEQ ID NO:107), the isoleucine (I) at amino acid position 153 in factor VII is given the chymotrypsin numbering of 116. Subsequent amino acids are numbered accordingly. In one example, a glutamic acid (E) at amino acid position 210 of the mature factor VII (SEQ ID NO:3) corresponds to amino acid position E70 based on chymotrypsin numbering. Where a residue exists in a protease, but is not present in chymotrypsin, the amino acid residue is given a letter notation. For example, residues in chymotrypsin that are part of a loop with amino acid 60 based on chymotrypsin numbering, but are inserted in the factor VII sequence compared to chymotrypsin, are referred to for example as K60a,160b, K60c or N60d. These residues correspond to K197, I198, K199 and N200, respectively, by numbering relative to the mature factor VII sequence (SEQ ID NO:3).

**Table 1. Chymotryspin numbering of factor VII**

| | | | | | | | | | | | | | | |
|---|---|---|---|---|---|---|---|---|---|---|---|---|---|---|
| 153 | 154 | 155 | 156 | 157 | 158 | 159 | 160 | 161 | 162 | 163 | 164 | 165 | 166 | 167 |
| **I** | **V** | **G** | **G** | **K** | **V** | **C** | **P** | **K** | **G** | **E** | **C** | **P** | **W** | **Q** |
| *16* | *17* | *18* | *19* | *20* | *21* | *22* | *23* | *24* | *25* | *26* | *27* | *28* | *29* | *30* |
| | | | | | | | | | | | | | | |
| 168 | 169 | 170 | 171 | 172 | 173 | 174 | 175 | 176 | 177 | 178 | 179 | 180 | 181 | 182 |
| **V** | **L** | **L** | **L** | **V** | **N** | **G** | **A** | **Q** | **L** | **C** | **G** | **G** | **T** | **L** |
| *31* | *32* | *33* | *34* | *35* | *37* | *38* | *39* | *40* | *41* | *42* | *43* | *44* | *45* | *46* |
| | | | | | | | | | | | | | | |
| 183 | 184 | 185 | 186 | 187 | 188 | 189 | 190 | 191 | 192 | 193 | 194 | 195 | 196 | 197 |
| **I** | **N** | **T** | **I** | **W** | **V** | **V** | **S** | **A** | **A** | **H** | **C** | **F** | **D** | **K** |
| *47* | *48* | *49* | *50* | *51* | *52* | *53* | *54* | *55* | *56* | *57* | *58* | *59* | *60* | *60A* |
| | | | | | | | | | | | | | | |
| 198 | 199 | 200 | 201 | 202 | 203 | 204 | 205 | 206 | 207 | 208 | 209 | 210 | 211 | 212 |
| **I** | **K** | **N** | **W** | **R** | **N** | **L** | **I** | **A** | **V** | **L** | **G** | **E** | **H** | **D** |
| *60B* | *60C* | *60D* | *61* | *62* | *63* | *64* | *65* | *66* | *67* | *68* | *69* | *70* | *71* | *72* |
| | | | | | | | | | | | | | | |
| 213 | 214 | 215 | 216 | 217 | 218 | 219 | 220 | 221 | 222 | 223 | 224 | 225 | 226 | 227 |
| **L** | **S** | **E** | **H** | **D** | **G** | **D** | **E** | **Q** | **S** | **R** | **R** | **V** | **A** | **Q** |
| *73* | *74* | *75* | *76* | *77* | *78* | *79* | *80* | *81* | *82* | *83* | *84* | *85* | *86* | *87* |
| | | | | | | | | | | | | | | |
| 228 | 229 | 230 | 231 | 232 | 233 | 234 | 235 | 236 | 237 | 238 | 239 | 240 | 241 | 242 |
| **V** | **I** | **I** | **P** | **S** | **T** | **Y** | **V** | **P** | **G** | **T** | **T** | **N** | **H** | **D** |
| *88* | *89* | *90* | *91* | *92* | *93* | *94* | *95* | *96* | *97* | *98* | *99* | *100* | *101* | *102* |
| | | | | | | | | | | | | | | |
| 243 | 244 | 245 | 246 | 247 | 248 | 249 | 250 | 251 | 252 | 253 | 254 | 255 | 256 | 257 |
| **I** | **A** | **L** | **L** | **R** | **L** | **H** | **Q** | **P** | **V** | **V** | **L** | **T** | **D** | **H** |
| *103* | *104* | *105* | *106* | *107* | *108* | *109* | *110* | *111* | *112* | *113* | *114* | *115* | *116* | *117* |
| | | | | | | | | | | | | | | |
| 258 | 259 | 260 | 261 | 262 | 263 | 264 | 265 | 266 | 267 | 268 | 269 | 270 | 271 | 272 |
| **V** | **V** | **P** | **L** | **C** | **L** | **P** | **E** | **R** | **T** | **F** | **S** | **E** | **R** | **T** |
| *118* | *119* | *120* | *121* | *122* | *123* | *124* | *125* | *126* | *127* | *128* | *129* | *129A* | *129B* | *129C* |
| | | | | | | | | | | | | | | |
| 273 | 274 | 275 | 276 | 277 | 278 | 279 | 280 | 281 | 282 | 283 | 284 | 285 | 286 | 287 |
| **L** | **A** | **F** | **V** | **R** | **F** | **S** | **L** | **V** | **S** | **G** | **W** | **G** | **Q** | **L** |
| *129D* | *129E* | *129F* | *129G* | *134* | *135* | *136* | *137* | *138* | *139* | *140* | *141* | *142* | *143* | *144* |
| | | | | | | | | | | | | | | |

(continued)

| | | | | | | | | | | | | | | |
|---|---|---|---|---|---|---|---|---|---|---|---|---|---|---|
| 288 | 289 | 290 | 291 | 292 | 293 | 294 | 295 | 296 | 297 | 298 | 299 | 300 | 301 | 302 |
| **L** | **D** | **R** | **G** | **A** | **T** | **A** | **L** | **E** | **L** | **M** | **V** | **L** | **N** | **V** |
| *145* | *146* | *147* | *149* | *150* | *151* | *152* | *153* | *154* | *155* | *156* | *157* | *158* | *159* | *160* |
| | | | | | | | | | | | | | | |
| 303 | 304 | 305 | 306 | 307 | 308 | 309 | 310 | 311 | 312 | 313 | 314 | 315 | 316 | 317 |
| **P** | **R** | **L** | **M** | **T** | **Q** | **D** | **C** | **L** | **Q** | **Q** | **S** | **R** | **K** | **V** |
| *161* | *162* | *163* | *164* | *165* | *166* | *167* | *168* | *169* | *170* | *170A* | *170B* | *170C* | *170D* | *170E* |
| | | | | | | | | | | | | | | |
| 318 | 319 | 320 | 321 | 322 | 323 | 324 | 325 | 326 | 327 | 328 | 329 | 330 | 331 | 332 |
| **G** | **D** | **S** | **P** | **N** | **I** | **T** | **E** | **Y** | **M** | **F** | **C** | **A** | **G** | **Y** |
| *170F* | *170G* | *170H* | *1701* | *175* | *176* | *177* | *178* | *179* | *180* | *181* | *182* | *183* | *184A* | *184* |
| | | | | | | | | | | | | | | |
| 333 | 334 | 335 | 336 | 337 | 338 | 339 | 340 | 341 | 342 | 343 | 344 | 345 | 346 | 347 |
| **S** | **D** | **G** | **S** | **K** | **D** | **S** | **C** | **K** | **G** | **D** | **S** | **G** | **G** | **P** |
| *185* | *186* | *187* | *188A* | *188* | *189* | *190* | *191* | *192* | *193* | *194* | *195* | *196* | *197* | *198* |
| | | | | | | | | | | | | | | |
| 348 | 349 | 350 | 351 | 352 | 353 | 354 | 355 | 356 | 357 | 358 | 359 | 360 | 361 | 362 |
| **H** | **A** | **T** | **H** | **Y** | **R** | **G** | **T** | **W** | **Y** | **L** | **T** | **G** | **I** | **V** |
| *199* | *200* | *201* | *202* | *203* | *204* | *205* | *206* | *207* | *208* | *209* | *210* | *211* | *212* | *213* |
| | | | | | | | | | | | | | | |
| 363 | 364 | 365 | 366 | 367 | 368 | 369 | 370 | 371 | 372 | 373 | 374 | 375 | 376 | 377 |
| **S** | **W** | **G** | **Q** | **G** | **C** | **A** | **T** | **V** | **G** | **H** | **F** | **G** | **V** | **Y** |
| *214* | *215* | *216* | *217* | *219* | *220* | *221A* | *221* | *222* | *223* | *224* | *225* | *226* | *227* | *228* |
| | | | | | | | | | | | | | | |
| 378 | 379 | 380 | 381 | 382 | 383 | 384 | 385 | 386 | 387 | 388 | 389 | 390 | 391 | 392 |
| **T** | **R** | **V** | **S** | **Q** | **Y** | **I** | **E** | **W** | **L** | **Q** | **K** | **L** | **M** | **R** |
| *229* | *230* | *231* | *232* | *233* | *234* | *235* | *236* | *237* | *238* | *239* | *240* | *241* | *242* | *243* |
| | | | | | | | | | | | | | | |
| 393 | 394 | 395 | 396 | 397 | 398 | 399 | 400 | 401 | 402 | 403 | 404 | 405 | 406 | |
| **S** | **E** | **P** | **R** | **P** | **G** | **V** | **L** | **L** | **R** | **A** | **P** | **F** | **P** | |
| *244* | *245* | *246* | *247* | *248* | *249* | *250* | *251* | *252* | *253* | *254* | *255* | *256* | *257* | |

As used herein, nucleic acids include DNA, RNA and analogs thereof, including peptide nucleic acids (PNA) and mixtures thereof. Nucleic acids can be single or double-stranded. When referring to probes or primers, which are optionally labeled, such as with a detectable label, such as a fluorescent or radiolabel, single-stranded molecules are contemplated. Such molecules are typically of a length such that their target is statistically unique or of low copy number (typically less than 5, generally less than 3) for probing or priming a library. Generally a probe or primer contains at least 14, 16 or 30 contiguous nucleotides of sequence complementary to or identical to a gene of interest. Probes and primers can be 10, 20, 30, 50, 100 or more nucleic acids long.

As used herein, a peptide refers to a polypeptide that is from 2 to 40 amino acids in length.

As used herein, the amino acids that occur in the various sequences of amino acids provided herein are identified according to their known, three-letter or one-letter abbreviations (Table 1). The nucleotides which occur in the various nucleic acid fragments are designated with the standard single-letter designations used routinely in the art.

As used herein, an "amino acid" is an organic compound containing an amino group and a carboxylic acid group. A polypeptide contains two or more amino acids. For purposes herein, amino acids include the twenty naturally-occurring amino acids, non-natural amino acids and amino acid analogs (i.e., amino acids wherein the $\alpha$-carbon has a side chain).

In keeping with standard polypeptide nomenclature described in J. Biol. Chem., 243: 3552-3559 (1969), and adopted 37 C.F.R.. §§ 1.821-1.822, abbreviations for the amino acid residues are shown in Table 1:

**Table 2 - Table of Correspondence**

| **SYMBOL** | | |
|---|---|---|
| **1-Letter** | **3-letter** | **AMINO ACID** |
| Y | Tyr | Tyrosine |
| G | Gly | Glycine |
| F | Phe | Phenylalanine |
| M | Met | Methionine |
| A | Ala | Alanine |
| S | Ser | Serine |
| I | Ile | Isoleucine |
| L | Leu | Leucine |
| T | Thr | Threonine |
| V | Val | Valine |
| P | Pro | proline |
| K | Lys | Lysine |
| H | His | Histidine |
| Q | Gln | Glutamine |
| E | Glu | glutamic acid |
| Z | Glx | Glu and/or Gin |
| W | Trp | Tryptophan |
| R | Arg | Arginine |
| D | Asp | aspartic acid |
| N | Asn | asparagines |
| B | Asx | Asn and/or Asp |
| **1-Letter** | **3-Letter** | **AMINO ACID** |
| C | Lys | Cysteine |
| X | Xaa | Unknown or other |

It should be noted that all amino acid residue sequences represented herein by formulae have a left to right orientation in the conventional direction of amino-terminus to carboxyl-terminus. In addition, the phrase "amino acid residue" is broadly defined to include the amino acids listed in the Table of Correspondence (Table 2) and modified and unusual amino acids, such as those referred to in 37 C.F.R. §§ 1.821-1.82. Furthermore, it should be noted that a dash at the beginning or end of an amino acid residue sequence indicates a peptide bond to a further sequence of one or more amino acid residues, to an amino-terminal group such as $NH_2$ or to a carboxyl-terminal group such as COOH.

As used herein, a "hydrophobic amino acid" includes any one of the amino acids determined to be hydrophobic using the Eisenberg hydrophobicity consensus scale. Exemplary are the naturally occurring hydrophobic amino acids, such as isoleucine, phenylalanine, valine, leucine, tryptophan, methionine, alanine, glycine, cysteine and tyrosine (Eisenberg et al., (1982) Faraday Symp. Chem. Soc. 17:109-120). Non-naturally-occurring hydrophobic amino acids also are included.

As used herein, an "acidic amino acid" includes among the naturally-occurring amino acids aspartic acid and glutamic acid residues. Non-naturally-occurring acidic amino acids also are included.

As used herein, "naturally occurring amino acids" refer to the 20 L-amino acids that occur in polypeptides.

As used herein, "non-natural amino acid" refers to an organic compound containing an amino group and a carboxylic acid group that is not one of the naturally-occuning amino acids listed in Table 2. Non-naturally occurring amino acids thus include, for example, amino acids or analogs of amino acids other than the 20 naturally-occurring amino acids and include, but are not limited to, the D-isostereomers of amino acids. Exemplary non-natural amino acids are known to those of skill in the art and can be included in a modified factor VII polypeptide.

As used herein, a DNA construct is a single or double stranded, linear or circular DNA molecule that contains segments of DNA combined and juxtaposed in a manner not found in nature. DNA constructs exist as a result of human manipulation, and include clones and other copies of manipulated molecules.

As used herein, a DNA segment is a portion of a larger DNA molecule having specified attributes. For example, a DNA segment encoding a specified polypeptide is a portion of a longer DNA molecule, such as a plasmid or plasmid fragment, which, when read from the 5' to 3' direction , encodes the sequence of amino acids of the specified polypeptide.

As used herein, the term polynucleotide means a single- or double-stranded polymer of deoxyribonucleotides or ribonucleotide bases read from the 5' to the 3' end. Polynucleotides include RNA and DNA, and can be isolated from natural sources, synthesized *in vitro,* or prepared from a combination of natural and synthetic molecules. The length of a polynucleotide molecule is given herein in terms of nucleotides (abbreviated "nt") or base pairs (abbreviated "bp"). The term nucleotides is used for single- and double-stranded molecules where the context permits. When the term is applied to double-stranded molecules it is used to denote overall length and will be understood to be equivalent to the term base pairs. It will be recognized by those skilled in the art that the two strands of a double-stranded polynucleotide can differ slightly in length and that the ends thereof can be staggered; thus all nucleotides within a double-stranded polynucleotide molecule can not be paired. Such unpaired ends will, in general, not exceed 20 nucleotides in length.

As used herein, "primary sequence" refers to the sequence of amino acid residues in a polypeptide.

As used herein, "similarity" between two proteins or nucleic acids refers to the relatedness between the sequence of amino acids of the proteins or the nucleotide sequences of the nucleic acids. Similarity can be based on the degree of identity and/or homology of sequences of residues and the residues contained therein. Methods for assessing the degree of similarity between proteins or nucleic acids are known to those of skill in the art. For example, in one method of assessing sequence similarity, two amino acid or nucleotide sequences are aligned in a manner that yields a maximal level of identity between the sequences. "Identity" refers to the extent to which the amino acid or nucleotide sequences are invariant. Alignment of amino acid sequences, and to some extent nucleotide sequences, also can take into account conservative differences and/or frequent substitutions in amino acids (or nucleotides). Conservative differences are those that preserve the physico-chemical properties of the residues involved. Alignments can be global (alignment of the compared sequences over the entire length of the sequences and including all residues) or local (the alignment of a portion of the sequences that includes only the most similar region or regions).

As used herein, the terms "homology" and "identity"" are used interchangeably, but homology for proteins can include conservative amino acid changes. In general to identify corresponding positions the sequences of amino acids are aligned so that the highest order match is obtained (see, *e.g.*: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carillo et al. (1988) SIAM J Applied Math 48:1073).

As use herein, "sequence identity" refers to the number of identical amino acids (or nucleotide bases) in a comparison between a test and a reference polypeptide or polynucleotide. Homologous polypeptides refer to a pre-determined number of identical or homologous amino acid residues. Homology includes conservative amino acid substitutions as well identical residues. Sequence identity can be determined by standard alignment algorithm programs used with default gap penalties established by each supplier. Homologous nucleic acid molecules refer to a pre-determined number of identical or homologous nucleotides. Homology includes substitutions that do not change the encoded amino acid (*i.e*., "silent substitutions") as well identical residues. Substantially homologous nucleic acid molecules hybridize typically at moderate stringency or at high stringency all along the length of the nucleic acid or along at least about 70%, 80% or 90% of the full-length nucleic acid molecule of interest. Also contemplated are nucleic acid molecules that contain degenerate codons in place of codons in the hybridizing nucleic acid molecule. (For determination of homology of proteins, conservative amino acids can be aligned as well as identical amino acids; in this case, percentage of identity and percentage homology varies). Whether any two nucleic acid molecules have nucleotide sequences (or any two polypeptides have amino acid sequences) that are at least 80%, 85%, 90%, 95%, 96%, 97%, 98% or 99% "identical" can be determined using known computer algorithms such as the "FAST A" program, using for example, the default parameters as in Pearson et al. Proc. Natl. Acad. Sci. USA 85: 2444 (1988) (other programs include the GCG program package (Devereux, J., et al., Nucleic Acids Research 12(I): 387 (1984)), BLASTP, BLASTN, FASTA (Atschul, S.F., et al., J. Molec. Biol. 215:403 (1990); Guide to Huge Computers, Martin J. Bishop, ed., Academic Press, San Diego (1994), and Carillo et al. SIAM J Applied Math 48: 1073 (1988)). For example, the BLAST function of the National Center for Biotechnology Information database can be used to determine identity. Other commercially or publicly available programs include DNAStar "MegAlign" program (Madison, WI) and the University of Wisconsin Genetics Computer Group (UWG) "Gap" program (Madison WI)). Percent homology or identity of proteins and/or nucleic acid molecules can be determined, for example, by comparing sequence information using a GAP computer program (*e.g.*, Needleman et al. J. Mol. Biol. 48: 443 (1970), as revised by Smith and Waterman (Adv. Appl. Math. 2: 482 (1981)). Briefly, a GAP program defines similarity as the number of aligned symbols (*i.e.*, nucleotides or amino acids) which are similar, divided by the total number of symbols in the shorter of the two sequences. Default parameters for the GAP program can include: (1) a unary comparison matrix (containing a value of 1 for identities and 0 for non identities) and the weighted comparison matrix of Gribskov et al. Nucl. Acids Res. 14: 6745 (1986), as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps.

Therefore, as used herein, the term "identity" represents a comparison between a test and a reference polypeptide or polynucleotide. In one non-limiting example, "at least 90% identical to" refers to percent identities from 90 to 100% relative to the reference polypeptides. Identity at a level of 90% or more is indicative of the fact that, assuming for exemplification purposes a test and reference polynucleotide length of 100 amino acids are compared, no more than 10% (*i.e.*, 10 out of 100) of amino acids in the test polypeptide differs from that of the reference polypeptides. Similar comparisons can be made between a test and reference polynucleotides. Such differences can be represented as point mutations randomly distributed over the entire length of an amino acid sequence or they can be clustered in one or more locations of varying length up to the maximum allowable, *e.g.*, 10/100 amino acid difference (approximately 90% identity). Differences are defined as nucleic acid or amino acid substitutions, insertions or deletions. At the level of homologies or identities above about 85-90%, the result should be independent of the program and gap parameters set; such high levels of identity can be assessed readily, often without relying on software.

As used herein, it also is understood that the terms "substantially identical" or "similar" varies with the context as understood by those skilled in the relevant art, but that those of skill can assess such.

As used herein, an aligned sequence refers to the use of homology (similarity and/or identity) to align corresponding positions in a sequence of nucleotides or amino acids. Typically, two or more sequences that are related by 50% or more identity are aligned. An aligned set of sequences refers to 2 or more sequences that are aligned at corresponding positions and can include aligning sequences derived from RNAs, such as ESTs and other cDNAs, aligned with genomic DNA sequence.

As used herein, "specifically hybridizes" refers to annealing, by complementary base-pairing, of a nucleic acid molecule (e.g. an oligonucleotide) to a target nucleic acid molecule. Those of skill in the art are familiar with *in vitro* and in *vivo* parameters that affect specific hybridization, such as length and composition of the particular molecule. Parameters particularly relevant to *in vitro* hybridization further include annealing and washing temperature, buffer composition and salt concentration. Exemplary washing conditions for removing non-specifically bound nucleic acid molecules at high stringency are 0.1 x SSPE, 0.1% SDS, 65°C, and at medium stringency are 0.2 x SSPE, 0.1% SDS, 50°C. Equivalent stringency conditions are known in the art. The skilled person can readily adjust these parameters to achieve specific hybridization of a nucleic acid molecule to a target nucleic acid molecule appropriate for a particular application.

As used herein, isolated or purified polypeptide or protein or biologically-active portion thereof is substantially free of cellular material or other contaminating proteins from the cell of tissue from which the protein is derived, or substantially free from chemical precursors or other chemicals when chemically synthesized. Preparations can be determined to be substantially free if they appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification would not detectably alter the physical and chemical properties, such as proteolytic and biological activities, of the substance. Methods for purification of the compounds to produce substantially chemically pure compounds are known to those of skill in the art. A substantially chemically pure compound, however, can be a mixture of stereoisomers. In such instances, further purification might increase the specific activity of the compound.

The term substantially free of cellular material includes preparations of proteins in which the protein is separated from cellular components of the cells from which it is isolated or recombinantly-produced. The term substantially free of cellular material may include preparations of protease proteins having less that about 30% (by dry weight) of non-protease proteins (also referred to herein as a contaminating protein), generally less than about 20% of non-protease proteins or 10% of non-protease proteins or less that about 5% of non-protease proteins. When the protease protein or active portion thereof is recombinantly produced, it also is substantially free of culture medium, *i.e.*, culture medium represents less than, about, or equal to 20%, 10% or 5% of the volume of the protease protein preparation.

As used herein, the term substantially free of chemical precursors or other chemicals includes preparations of protease proteins in which the protein is separated from chemical precursors or other chemicals that are involved in the synthesis of the protein. The term includes preparations of protease proteins having less than about 30% (by dry weight), 20%, 10%, 5% or less of chemical precursors or non-protease chemicals or components.

As used herein, production by recombinant methods by using recombinant DNA methods refers to the use of the well known methods of molecular biology for expressing proteins encoded by cloned DNA.

As used herein, vector (or plasmid) refers to discrete elements that are used to introduce heterologous nucleic acid into cells for either expression or replication thereof. The vectors typically remain episomal, but can be designed to effect integration of a gene or portion thereof into a chromosome of the genome. Also contemplated are vectors that are artificial chromosomes, such as bacterial artificial chromosomes, yeast artificial chromosomes and mammalian artificial chromosomes. Selection and use of such vehicles are well known to those of skill in the art.

As used herein, expression refers to the process by which nucleic acid is transcribed into mRNA and translated into peptides, polypeptides, or proteins. If the nucleic acid is derived from genomic DNA, expression can, if an appropriate eukaryotic host cell or organism is selected, include processing, such as splicing of the mRNA.

As used herein, an expression vector includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, vector also includes "virus vectors" or "viral vectors." Viral vectors are engineered viruses that are operatively linked to exogenous genes to transfer (as vehicles or shuttles) the exogenous genes into cells.

As used herein, an adenovirus refers to any of a group of DNA-containing viruses that cause conjunctivitis and upper respiratory tract infections in humans.

As used herein, naked DNA refers to histone-free DNA that can be used for vaccines and gene therapy. Naked DNA is the genetic material that is passed from cell to cell during a gene transfer processed called transformation or transfection. In transformation or transfection, purified or naked DNA that is taken up by the recipient cell will give the recipient cell a new characteristic or phenotype.

As used herein, operably or operatively linked when referring to DNA segments means that the segments are arranged so that they function in concert for their intended purposes, e.g., transcription initiates in the promoter and proceeds through the coding segment to the terminator.

As used herein, an agent that modulates the activity of a protein or expression of a gene or nucleic acid either decreases or increases or otherwise alters the activity of the protein or, in some manner, up- or down-regulates or otherwise alters expression of the nucleic acid in a cell.

As used herein, a "chimeric protein" or "fusion protein" refers to a polypeptide operatively-linked to a different polypeptide. A chimeric or fusion protein provided herein can include one or more FVII polypeptides, or a portion thereof, and one or more other polypeptides for any one or more of a transcriptional/translational control signals, signal sequences, a tag for localization, a tag for purification, part of a domain of an immunoglobulin G, and/or a targeting agent. A chimeric FVII polypeptide also includes those having their endogenous domains or regions of the polypeptide exchanged with another polypeptide. These chimeric or fusion proteins include those produced by recombinant means as fusion proteins, those produced by chemical means, such as by chemical coupling, through, for example, coupling to sulfhydryl groups, and those produced by any other method whereby at least one polypeptide (*i.e.* FVII), or a portion thereof, is linked, directly or indirectly via linker(s) to another polypeptide.

As used herein, operatively-linked when referring to a fusion protein refers to a protease polypeptide and a non-protease polypeptide that are fused in-frame to one another. The non-protease polypeptide can be fused to the N-terminus or C-terminus of the protease polypeptide.

As used herein, a targeting agent, is any moiety, such as a protein or effective portion thereof, that provides specific binding to a cell surface molecule, such a cell surface receptor, which in some instances can internalize a bound conjugate or portion thereof. A targeting agent also can be one that promotes or facilitates, for example, affinity isolation or purification of the conjugate; attachment of the conjugate to a surface; or detection of the conjugate or complexes containing the conjugate.

As used herein, derivative or analog of a molecule refers to a portion derived from or a modified version of the molecule.

As used herein, "disease or disorder" refers to a pathological condition in an organism resulting from cause or condition including, but not limited to, infections, acquired conditions, genetic conditions, and characterized by identifiable symptoms. Diseases and disorders of interest herein are those involving coagulation, including those mediated by coagulation proteins and those in which coagulation proteins play a role in the etiology or pathology. Diseases and disorders also include those that are caused by the absence of a protein such as in hemophilia, and of particular interest herein are those disorders where coagulation does not occur due to a deficiency of defect in a coagulation protein.

As used herein, "procoagulant" refers to any substance that promotes blood coagulation.

As used herein, "anticoagulant" refers to any substance that inhibits blood coagulation

As used herein, "hemophilia" refers to a bleeding disorder caused by a deficiency in a blood clotting factors. Hemophilia can be the result, for example, of absence, reduced expression, or reduced function of a clotting factor. The most common type of hemophilia is hemophilia A, which results from a deficiency in factor VIII. The second most common type of hemophilia is hemophilia B, which results from a deficiency in factor IX. Hemophilia C, also called FXI deficiency, is a milder and less common form of hemophila.

As used herein, "congenital hemophilia" refers to types of hemophilia that are inherited. Congenital hemophilia results from mutation, deletion, insertion, or other modification of a clotting factor gene in which the production of the clotting factor is absent, reduced, or non-functional. For example, hereditary mutations in clotting factor genes, such as factor VIII and factor IX result in the congenital hemophilias, Hemophilia A and B, respectively.

As used herein, "acquired hemophilia" refers to a type of hemophilia that develops in adulthood from the production of autoantibodies that inactivate FVIII.

As used herein, "bleeding disorder" refers to a condition in which the subject has a decreased ability to control bleeding. Bleeding disorders can be inherited or acquired, and can result from, for example, defects or deficiencies in the coagulation pathway, defects or deficiencies in platelet activity, or vascular defects.

As used herein, "acquired bleeding disorder" refers to bleeding disorders that results from clotting deficiencies caused by conditions such as liver disease, vitamin K deficiency, or coumadin (warfarin) or other anti-coagulant therapy.

As used herein, "treating" a subject having a disease or condition means that a polypeptide, composition or other product provided herein is administered to the subject.

As used herein, a therapeutic agent, therapeutic regimen, radioprotectant, or chemotherapeutic mean conventional drugs and drug therapies, including vaccines, which are known to those skilled in the art. Radiotherapeutic agents are well known in the art.

As used herein, treatment means any manner in which the symptoms of a condition, disorder or disease are ameliorated or otherwise beneficially altered. Hence treatment encompasses prophylaxis, therapy and/or cure. Treatment also encompasses any pharmaceutical use of the compositions herein. Treatment also encompasses any pharmaceutical use of a modified FVII and compositions provided herein.

As used herein, amelioration of the symptoms of a particular disease or disorder by a treatment, such as by administration of a pharmaceutical composition or other therapeutic, refers to any lessening, whether permanent or temporary, lasting or transient, of the symptoms that can be attributed to or associated with administration of the composition or therapeutic.

As used herein, prevention or prophylaxis refers to methods in which the risk of developing disease or condition is reduced. Prophylaxis includes reduction in the risk of developing a disease or condition and/or a prevention of worsening of symptoms or progression of a disease or reduction in the risk of worsening of symptoms or progression of a disease.

As used herein an effective amount of a compound or composition for treating a particular disease is an amount that is sufficient to ameliorate, or in some manner reduce the symptoms associated with the disease. Such amount can be administered as a single dosage or can be administered according to a regimen, whereby it is effective. The amount can cure the disease but, typically, is administered in order to ameliorate the symptoms of the disease. Typically, repeated administration is required to achieve a desired amelioration of symptoms.

As used herein, "therapeutically effective amount" or "therapeutically effective dose" refers to an agent, compound, material, or composition containing a compound that is at least sufficient to produce a therapeutic effect. An effective amount is the quantity of a therapeutic agent necessary for preventing, curing, ameliorating, arresting or partially arresting a symptom of a disease or disorder.

As used herein, "patient" or "subject" to be treated includes humans and or non-human animals, including mammals. Mammals include primates, such as humans, chimpanzees, gorillas and monkeys; domesticated animals, such as dogs, horses, cats, pigs, goats, cows; and rodents such as mice, rats, hamsters and gerbils.

As used herein, a combination refers to any association between two or among more items. The association can be spatial or refer to the use of the two or more items for a common purpose.

As used herein, a composition refers to any mixture of two or more products or compounds (e.g., agents, modulators, regulators, etc.). It can be a solution, a suspension, liquid, powder, a paste, aqueous or non-aqueous formulations or any combination thereof.

As used herein, an "article of manufacture" is a product that is made and sold. As used throughout this application, the term is intended to encompass modified protease polypeptides and nucleic acids contained in articles of packaging.

As used herein, fluid refers to any composition that can flow. Fluids thus encompass compositions that are in the form of semi-solids, pastes, solutions, aqueous mixtures, gels, lotions, creams and other such compositions.

As used herein, a "kit" refers to a packaged combination, optionally including reagents and other products and/or components for practicing methods using the elements of the combination. For example, kits containing a modified protease polypeptide or nucleic acid molecule provided herein and another item for a purpose including, but not limited to, administration, diagnosis, and assessment of a biological activity or property are provided. Kits optionally include instructions for use.

As used herein, antibody includes antibody fragments, such as Fab fragments, which are composed of a light chain and the variable region of a heavy chain.

As used herein, a receptor refers to a molecule that has an affinity for a particular ligand. Receptors can be naturally-occurring or synthetic molecules. Receptors also can be referred to in the art as anti-ligands.

As used herein, animal includes any animal, such as, but not limited to; primates including humans, gorillas and monkeys; rodents, such as mice and rats; fowl, such as chickens; ruminants, such as goats, cows, deer, sheep; ovine, such as pigs and other animals. Non-human animals exclude humans as the contemplated animal. The proteases provided herein are from any source, animal, plant, prokaryotic and fungal.

As used herein, gene therapy involves the transfer of heterologous nucleic acid, such as DNA, into certain cells, target cells, of a mammal, particularly a human, with a disorder or condition for which such therapy is sought. The nucleic acid, such as DNA, is introduced into the selected target cells, such as directly or in a vector or other delivery vehicle, in a manner such that the heterologous nucleic acid, such as DNA, is expressed and a therapeutic product encoded thereby is produced. Alternatively, the heterologous nucleic acid, such as DNA, can in some manner mediate expression of DNA that encodes the therapeutic product, or it can encode a product, such as a peptide or RNA that in some manner mediates, directly or indirectly, expression of a therapeutic product. Genetic therapy also can be used to deliver nucleic acid encoding a gene product that replaces a defective gene or supplements a gene product produced by the mammal or the cell in which it is introduced. The introduced nucleic acid can encode a therapeutic compound, such as a protease or modified protease, that is not normally produced in the mammalian host or that is not produced in therapeutically effective amounts or at a therapeutically useful time. The heterologous nucleic acid, such as DNA, encoding the therapeutic product can be modified prior to introduction into the cells of the afflicted host in order to enhance or otherwise alter the product or expression thereof. Genetic therapy also can involve delivery of an inhibitor or repressor or other modulator of gene expression.

As used herein, heterologous nucleic acid is nucleic acid that is not normally produced *in vivo* by the cell in which it is expressed or that is produced by the cell but is at a different locus or expressed differently or that mediates or encodes mediators that alter expression of endogenous nucleic acid, such as DNA, by affecting transcription, translation, or other regulatable biochemical processes. Heterologous nucleic acid is generally not endogenous to the cell into which it is introduced, but has been obtained from another cell or prepared synthetically. Heterologous nucleic acid can be endogenous, but is nucleic acid that is expressed from a different locus or altered in its expression. Generally, although not necessarily, such nucleic acid encodes RNA and proteins that are not normally produced by the cell or in the same way in the cell in which it is expressed. Heterologous nucleic acid, such as DNA, also can be referred to as foreign nucleic acid, such as DNA. Thus, heterologous nucleic acid or foreign nucleic acid includes a nucleic acid molecule not present in the exact orientation or position as the counterpart nucleic acid molecule, such as DNA, is found in a genome. It also can refer to a nucleic acid molecule from another organism or species (*i.e.*, exogenous).

Any nucleic acid, such as DNA, that one of skill in the art would recognize or consider as heterologous or foreign to the cell in which the nucleic acid is expressed is herein encompassed by heterologous nucleic acid; heterologous nucleic acid includes exogenously added nucleic acid that also is expressed endogenously. Examples of heterologous nucleic acid include, but are not limited to, nucleic acid that encodes traceable marker proteins, such as a protein that confers drug resistance, nucleic acid that encodes therapeutically effective substances, such as anti-cancer agents, enzymes and hormones, and nucleic acid, such as DNA, that encodes other types of proteins, such as antibodies. Antibodies that are encoded by heterologous nucleic acid can be secreted or expressed on the surface of the cell in which the heterologous nucleic acid has been introduced.

As used herein, a therapeutically effective product for gene therapy is a product that is encoded by heterologous nucleic acid, typically DNA, that, upon introduction of the nucleic acid into a host, a product is expressed that ameliorates or eliminates the symptoms, manifestations of an inherited or acquired disease or that cures the disease. Also included are biologically active nucleic acid molecules, such as RNAi and antisense.

As used herein, recitation that a polypeptide "consists essentially" of a recited sequence of amino acids means that only the recited portion, or a fragment thereof, of the full-length polypeptide is present. The polypeptide can optionally, and generally will, include additional amino acids from another source or can be inserted into another polypeptide

As used here , the singular forms "a," "an" and "the" include plural referents unless the context clearly dictates otherwise. Thus, for example, reference to compound, comprising "an extracellular domain"" includes compounds with one or a plurality of extracellular domains.

As used herein, ranges and amounts can be expressed as "about" a particular value or range. About also includes the exact amount. Hence "about 5 bases" means "about 5 bases" and also "5 bases.'

As used herein, "optional" or "optionally" means that the subsequently described event or circumstance does or does not occur, and that the description includes instances where said event or circumstance occurs and instances where it does not. For example, an optionally substituted group means that the group is unsubstituted or is substituted.

As used herein, the abbreviations for any protective groups, amino acids and other compounds, are, unless indicated otherwise, in accord with their common usage, recognized abbreviations, or the IUPAC-IUB Commission on Biochemical Nomenclature (see, (1972) Biochem. 11:726).

B. Hemostasis Overview

Provided herein are modified Factor VII (FVII) polypeptides. Such FVII polypeptides are designed to have increased coagulant activity. Accordingly, these polypeptides have a variety of uses and applications, for example, as therapeutics for modulating hemostasis, and other related biological processes. To appreciate the modifications provided herein and the use of such modified FVII molecules, an understanding of the haemostatic system and the blood coagulation cascade is advantageous. The following discussion provides such background, prefatory to a discussion of factor VII, and modifications thereof.

Hemostasis is the physiological mechanism that stems the bleeding that results from injury to the vasculature. Normal hemostasis depends on cellular components and soluble plasma proteins, and involves a series of signaling events that ultimately leads to the formation of a blood clot. Coagulation is quickly initiated after an injury occurs to the blood vessel and endothelial cells are damaged. In the primary phase of coagulation, platelets are activated to form a haemostatic plug at the site of injury. Secondary hemostasis follows involving plasma coagulation factors, which act in a proteolytic cascade resulting in the formation of fibrin strands which strengthen the platelet plug.

Upon vessel injury, the blood flow to the immediate injured area is restricted by vascular constriction allowing platelets to adhere to the newly-exposed fibrillar collagen on the subendothelial connective tissue. This adhesion is dependent upon the von Willebrand factor (vWF), which binds to the endothelium within three seconds of injury, thereby facilitating platelet adhesion and aggregation. Activation of the aggregated platelets results in the secretion of a variety of factors, including ADP, ATP, thromboxane and serotonin. Adhesion molecules, fibrinogen, vWF, thrombospondin and fibronectin also are released. Such secretion promotes additional adhesion and aggregation of platelets, increased platelet activation and blood vessel constriction, and exposure of anionic phospholipids on the platelet surface that serve as platforms for the assembly of blood coagulation enzyme complexes. The platelets change shape leading to pseudopodia formation, which further facilitates aggregation to other platelets resulting in a loose platelet plug.

A clotting cascade of peptidases (the coagulation cascade) is simultaneously initiated. The coagulation cascade involves a series of activation events involving proteolytic cleavage. In such a cascade, an inactive protein of a serine protease (also called a zymogen) is converted to an active protease by cleavage of one or more peptide bonds, which then serves as the activating protease for the next zymogen molecule in the cascade, ultimately resulting in clot formation by the cross-linking of fibrin. For example, the cascade generates activated molecules such as thrombin (from cleavage of prothrombin), which further activates platelets, and also generates fibrin from cleavage of fibrinogen. Fibrin then forms a cross-linked polymer around the platelet plug to stabilize the clot. Upon repair of the injury, fibrin is digested by the fibrinolytic system, the major components of which are plasminogen and tissue-type plasminogen activator (tPA). Both of these proteins are incorporated into polymerizing fibrin, where they interact to generate plasmin, which, in turn, acts on fibrin to dissolve the preformed clot. During clot formation, coagulation factor inhibitors also circulate through the blood to prevent clot formation beyond the injury site.

The interaction of the system, from injury to clot formation and subsequent fibrinolysis, is described below.

**1. Platelet adhesion and aggregation**

The clotting of blood is actively circumvented under normal conditions. The vascular endothelium supports vasodilation, inhibits platelet adhesion and activation, suppresses coagulation, enhances fibrin cleavage and is anti-inflammatory in character. Vascular endothelial cells secrete molecules such as nitrous oxide (NO) and prostacylin, which inhibit platelet aggregation and dilate blood vessels. Release of these molecules activates soluble guanylate cyclases (sGC) and cGMP-dependent protein kinase I (cGKI) and increases cyclic guanosine monophosphate (cGMP) levels, which cause relaxation of the smooth muscle in the vessel wall. Furthermore, endothelial cells express cell-surface ADPases, such as CD39, which control platelet activation and aggregation by converting ADP released from platelets into adenine nucleotide platelet inhibitors. The endothelium also plays an important role in the regulation of the enzymes in the fibrinolytic cascade. Endothelial cells directly promote the generation of plasmin through the expression of receptors of plasminogen (annexin II) and urokinase, as well as the secretion of tissue-type and urokinase plasminogen activators, all of which promote clot clearance. In a final layer of prothrombotic regulation, endothelial cells play an active role in inhibiting the coagulation cascade by producing heparan sulfate, which increases the kinetics of antithrombin III inhibition of thrombin and other coagulation factors.

Under acute vascular trauma, however, vasoconstrictor mechanisms predominate and the endothelium becomes prothrombotic, procoagulatory and proinflammatory in nature. This is achieved by a reduction of endothelial dilating agents: adenosine, NO and prostacyclin; and the direct action of ADP, serotonin and thromboxane on vascular smooth muscle cells to elicit their contraction (Becker, Heindl *et al*.. 2000). The chief trigger for the change in endothelial function that leads to the formation of haemostatic thrombus is the loss of the endothelial cell barrier between blood and extracellular matrix (ECM) components (Ruggeri (2002) Nat Med 8:1227-1234). Circulating platelets identify and discriminate areas of endothelial lesions and adhere to the exposed sub endothelium. Their interaction with the various thrombogenic substrates and locally-generated or released agonists results in platelet activation. This process is described as possessing two stages, 1) adhesion: the initial tethering to a surface, and 2) aggregation: the platelet-platelet cohesion (Savage et al. (2001) Curr Opin Hematol 8:270-276).

Platelet adhesion is initiated when the circulating platelets bind to exposed collagen through interaction with collagen binding proteins on the cell surface, and through interaction with vWF, also present on the endothelium. vWF protein is a multimeric structure of variable size, secreted in two directions by the endothelium; basolaterally and into the bloodstream. vWF also binds to factor VIII, which is important in the stabilization of factor VIII and its survival in the circulation.

Platelet adhesion and subsequent activation is achieved when vWF binds via its A1 domain to GPIb (part of the platelet glycoprotein receptor complex GPIb-IX-V). The interaction between vWF and GPIb is regulated by shear force such that an increase in the shear stress results in a corresponding increase in the affinity of vWF for GPIb. Integrin $\alpha 1\beta 2$, also known on leukocytes as VLA-2, is the major collagen receptor on platelets, and engagement through this receptor generates the intracellular signals that contribute to platelet activation. Binding through $\alpha 1\beta 2$ facilitates the engagement of the lower-affinity collagen receptor, GP VI. This is part of the immunoglobulin superfamily and is the receptor that generates the most potent intracellular signals for platelet activation. Platelet activation results in the release of adenosine diphosphate (ADP), which is converted to thromboxane A2.

Platelet activation also results in the surface expression of platelet glycoprotein IIb-IIIa (GP IIb-IIIa) receptors, GP IIb-IIIa receptors allow the adherence of platelets to each other (*i.e.* aggregation) by virtue of fibrinogen molecules linking the platelets through these receptors. This results in the formation of a platelet plug at the site of injury to help prevent further blood loss, while the damaged vascular tissue releases factors that initiate the coagulation cascade and the formation of a stabilizing fibrin mesh around the platelet plug.

### 2. Coagulation cascade

The coagulation pathway is a proteolytic pathway where each enzyme is present in the plasma as a zymogen, or inactive form. Cleavage of the zymogen is regulated to release the active form from the precursor molecule. Cofactors of the activated proteases, such as the glycoproteins FVIII and FV, also are activated in the cascade reaction and play a role in clot formation. The pathway functions as a series of positive and negative feedback loops which control the activation process, where the ultimate goal is to produce thrombin, which can then convert soluble fibrinogen into fibrin to form a clot. The factors in the coagulation are typically given a roman numeral number, with a lower case "a" appended to indicate an activated form. Table 3 below sets forth an exemplary list of the factors, including their common name, and their role in the coagulation cascade. Generally, these proteins participate in blood coagulation through one or more of the intrinsic, extrinsic or common pathway of coagulation (see Figure 1). As discussed below, these pathways are interconnected, and blood coagulation is believed to occur through a cell-based model of activation with Factor VII (FVII) being the primary initiator of coagulation.

**Table 3**

| Coagulation Factors | | | |
|---|---|---|---|
| **Factor** | **Common Name** | **Pathway** | **Characteristic** |
| I | Fibrinogen | Both | - |
| II | Prothrombin | Both | Contains N-terminal Gla domain |
| III | Tissue Factor | Extrinsic | - |
| IV | Calcium | Both | - |
| V | Proaccelerin, labile factor, Accelerator globulin | Both | Protein cofactor |

(continued)

| Coagulation Factors | | | |
|---|---|---|---|
| Factor | Common Name | Pathway | Characteristic |
| VI (Va) | Accelerin | - | (Redundant to factor V) |
| VII | Proconvertin, serum prothrombin conversion accelerator (SPCA) cothromboplastin | Extrinsic | Endopeptidase with Gla domain |
| VIII | Antihemophiliac factor A, antihemophiliac globulin (AHG) | Intrinsic | Protein cofactor |
| IX | Christmas factor, antihemophiliac factor B, plasma thromboplastin component (PTC) | Intrinsic | Endopeptidase with Gla domain |
| X | Stuart-prower factor | Both | Endopeptidase with Gla domain |
| XI | Plasma thromboplastin antecedent (PTA) | Intrinsic | Endopeptidase |
| XII | Hageman factor | Intrinsic | Endopeptidase |
| XIII | Protransglutamidase, fibrin stabilizing factor (FSF), fibrinoligase | Both | Transpeptidase |
| *Table adapted from M.W.King (2006) at med.unibs.it/-marchesi/blood.html | | | |

The generation of thrombin has historically been divided into three pathways, the intrinsic (suggesting that all components of the pathway are intrinsic to plasma) and extrinsic (suggesting that one or more components of the pathway are extrinsic to plasma) pathways that provide alternative routes for the generation of activated factor X (FXa), and the final common pathway which results in thrombin formation (Figure 1). These pathways participate together in an interconnected and interdependent process to effect coagulation. A cell-based model of coagulation was developed that describes these pathways (Figure 2) (Hoffman et al. (2001) Thromb Haemost 85:958-965). In this model, the "extrinsic" and "intrinsic" pathways are effected on different cell surfaces, the tissue factor (TF)-bearing cell and the platelet, respectively. The process of coagulation is separated into distinct phases, initiation, amplification and propagation, during which the extrinsic and intrinsic pathways function at various stages to produce the large burst of thrombin required to convert sufficient quantities of fibrinogen to fibrin for clot formation.

**a. Initiation**

FVII is considered to be the coagulation factor responsible for initiating the coagulation cascade, which initiation is dependent on its interaction with TF. TF is a transmembrane glycoprotein expressed by a variety of cells such as smooth muscle cells, fibroblasts, monocytes, lymphocytes, granulocytes, platelets and endothelial cells. Myeloid cells and endothelial cells only express TF when they are stimulated, such as by proinflammatory cytokines. Smooth muscle cells and fibroblasts, however, express TF constitutively. Accordingly, once these cells come in contact with the bloodstream following tissue injury, the coagulation cascade is rapidly initiated by the binding of TF with factor VII or FVIIa in the plasma.

As discussed below, the majority of FVII in the blood is in the zymogen form with a small amount, approximately 1 %, present as FVIIa. In the absence of TF binding, however, even FVIIa has zymogen-like characteristics and does not display significant activity until it is complexed with TF. Thus, plasma FVII requires activation by proteolytic cleavage, and additional conformational change through interaction with TF, for full activity. A range of proteases, including factors IXa, Xa, XIIa, and thrombin, have been shown to be capable of FVII cleavage *in vitro,* a process which is accelerated in the presence of TF. FVIIa itself also can activate FVII in the presence of TF, a process termed autoactivation. The small amounts of FVIIa in the blood are likely due to activation by FXa and/or FIXa (Wildgoose et al. (1992) Blood 80:25-28, and Butenas et al. (1996) Biochemistry 35:1904-1910). TF/FVIIa complexes can thus be formed by the direct binding of FVIIa to TF, or by the binding of FVII to TF and then the subsequent activation of FVII to FVIIa by a plasma protease, such as FXa, FIXa, FXIIa, or FVIIa itself. The TF/FVIIa complex remains anchored to the TF-bearing cell where it activates small amounts FX into FXa in what is known as the "extrinsic pathway" of coagulation.

The TF/FVIIa complex also cleaves small amounts of FIX into FIXa. FXa associates with its cofactor FVa to also form a complex on the TF-bearing cell that can then covert prothrombin to thrombin. The small amount of thrombin produced is, however, inadequate to support the required fibrin formation for complete clotting. Additionally, any active FXa and FIXa are inhibited in the circulation by antithrombin III (AT-III) and other serpins, which are discussed in more detail below. This would normally prevent clot formation in the circulation. In the presence of injury, however, damage to the vasculature results in platelet aggregation and activation at this site of thrombin formation, thereby allowing for amplification of the coagulation signal.

### b. Amplification

Amplification takes place when thrombin binds to and activates the platelets. The activated platelets release FV from their alpha granules, which is activated by thrombin to FVa. Thrombin also releases and activates FVIII from the FVIII/vWF complex on the platelet membrane, and cleaves FXI into FXIa. These reactions generate activated platelets that have FVa, FVIIIa and FIXa on their surface, which set the stage for a large burst of thrombin generation during the propagation stage.

### c. Propagation

Propagation of coagulation occurs on the surface of large numbers of platelets at the site of injury. As described above, the activated platelets have FXIa, FVIIIa and FVa on their surface. It is here that the extrinsic pathway is effected. FXIa activates FIX to FIXa, which can then bind with FVIIIa. This process, in addition to the small amounts of FIXa that is generated by cleavage of FIX by the TF/FVIIa complex on the TF-bearing cell, generates large numbers of FXIa/FVIIIa complexes which in turn can activate significant amounts of FX to FXa. The FXa molecules bind to FVa to generate the prothrombinase complexes that activate prothrombin to thrombin. Thrombin acts in a positive feedback loop to activate even more platelets and again initiates the processes described for the amplification phase.

Very shortly, there are sufficient numbers of activated platelets with the appropriate complexes to generate the burst of thrombin that is large enough to generate sufficient amounts of fibrin from fibrinogen to form a hemostatic fibrin clot. Fibrinogen is a dimer soluble in plasma which, when cleaved by thrombin, releases fibrinopeptide A and fibrinopeptide B. Fibrinopeptide B is then cleaved by thrombin, and the fibrin monomers formed by this second proteolytic cleavage spontaneously forms an insoluble gel. The polymerized fibrin is held together by noncovalent and electrostatic forces and is stabilized by the transamidating enzyme factor XIIIa (FXIIIa), produced by the cleavage of FXIII by thrombin. Thrombin also activates TAFI, which inhibits fibrinolysis by reducing plasmin generation at the clot surface. Additionally, thrombin itself is incorporated into the structure of the clot for further stabilization. These insoluble fibrin aggregates (clots), together with aggregated platelets (thrombi), block the damaged blood vessel and prevent further bleeding.

## 3. Regulation of Coagulation

During coagulation, the cascade is regulated by constitutive and stimulated processes to inhibit further clot formation. There are several reasons for such regulatory mechanisms. First, regulation is required to limit ischemia of tissues by fibrin clot formation. Second, regulation prevents widespread thrombosis by localizing the clot formation only to the site of tissue injury.

Regulation is achieved by the cations of several inhibitory molecules. For example, antithrombin III (AT-III) and tissue factor pathway inhibitor (TFPI) work constitutively to inhibit factors in the coagulation cascade. AT-III inhibits thrombin, FIXa, and FXa, whereas TFPI inhibits FXa and FVIIa/TF complex. An additional factor, Protein C, which is stimulated via platelet activation, regulates coagulation by proteolytic cleavage and inactivation of FVa and FVIIIa. Protein S enhances the activity of Protein C. Further, another factor which contributes to coagulation inhibition is the integral membrane protein thrombomodulin, which is produced by vascular endothelial cells and serves as a receptor for thrombin. Binding of thrombin to thrombomodulin inhibits thrombin procoagulant activities and also contributes to protein C activation.

Fibrinolysis, the breakdown of the fibrin clot, also provides a mechanism for regulating coagulation. The crosslinked fibrin multimers in a clot are broken down to soluble polypeptides by plasmin, a serine protease. Plasmin can be generated from its inactive precursor plasminogen and recruited to the site of a fibrin clot in two ways: by interaction with tissue plasminogen activator (tPA) at the surface of a fibrin clot, and by interaction with urokinase plasminogen activator (uPA) at a cell surface. The first mechanism appears to be the major one responsible for the dissolution of clots within blood vessels. The second, although capable of mediating clot dissolution, can play a major role in tissue remodeling, cell migration, and inflammation.

Clot dissolution also is regulated in two ways. First, efficient plasmin activation and fibrinolysis occur only in complexes formed at the clot surface or on a cell membrane, while proteins free in the blood are inefficient catalysts and are rapidly inactivated. Second, plasminogen activators and plasmin are inactivated by molecules such as plasminogen activator inhibitor type 1 (PAI-1) and PAI-2 which act on the plasminogen activators, and $\alpha$2-antiplasmin and $\alpha$2-macroglobulin that inactivate plasmin. Under normal circumstances, the timely balance between coagulation and fibrinolysis results in the efficient formation and clearing of clots following vascular injury, while simultaneously preventing unwanted thrombotic or bleeding episodes.

A summary of exemplary coagulation factors, cofactors and regulatory proteins, and their activities, are set forth in Table 4 below.

**Table 4**

| Coagulation Factor Zymogens and Cofactors | |
|---|---|
| **Name of Factor** | **Activity** |
| **Zymogens of Serine Proteases** | |
| Factor XII | Binds exposed collagen at site of vessel wall injury, activated by high-MW kininogen and kallikrein |
| Factor XI | Activated by factor XIIa |
| Factor IX | Activated by factor XIa + $Ca^{2+}$ |
| Factor VII | Activated by thrombin, factor X, factor IXa or factor XIIa + $Ca^{2+}$, or autoactivation |
| Factor X | Activated on platelet surface by tenase complex (FIXa/FVIIIa); Also activated by factor VIIa + tissue factor + $Ca^{2+}$, or factor VIIa + $Ca^{2+}$ |
| Factor II | Activated on platelet surface by prothrombinase complex (FXa/FVa) |
| **Cofactors** | |
| Factor VIII | Activated by thrombin; factor VIIIa acts as cofactor for factor IXa in activation of factor X |
| Factor V | Activated by thrombin; factor Va acts as cofactor for factor Xa in activation of prothrombin |
| Factor III (Tissue factor) | Acts as cofactor for factor VIIa |
| **Fibrinogen** | |
| Factor I (Fibrinogen) | Cleaved by thrombin to form fibrin |
| **Transglutaminase** | |
| Factor XIII | Activated by thrombin + $Ca^{2+}$; promotes covalent cross-linking of fibrin |
| **Regulatory and other proteins** | |
| von Willebrand factor (vWF) | Acts as bridge between GPIb-V-IX complex and collagen |
| Protein C | Activated by thrombin bound to thrombomodulin; Ca degrades factors VIIIa and Va |
| Protein S | Acts as cofactor of protein C |
| Thrombomodulin | Endothelial cell surface protein; binds thrombin, which activates protein C |
| Antithrombin III | Coagulation inhibitor, primarily of thrombin and factor Xa, but also factors IXa, XIa, and XIIa, and factor VIIa complexed with TF |
| Tissue Factor Pathway Inhibitor (TFPI) | Binds FXa and then forms a quaternary structure with TF/FVIIa to inhibit TF/FVIIa activity |
| *Table adapted from M.W.King (2006) med.unibs.it/-marchesi/blood.html | |

**C. Factor VII (FVII)**

Factor VII is a vitamin K-dependent serine protease glycoprotein that is synthesized in animals, including mammals, as a single-chain zymogen in the liver and secreted into the blood stream. As described above, FVII is the coagulation protease responsible for initiating the cascade of proteolytic events that lead to thrombin generation and fibrin deposition. It is part of the extrinsic pathway, although the downstream effects of its activity also impact greatly on the intrinsic pathway. This integral role in clot formation has attracted significant interest in FVII as a target for clinical anti-coagulant and haemostatic therapies. For example, recombinant activated FVII (rFVIIa) has been developed as a haemostatic agent for use in hemophilic subjects, and subjects with other bleeding conditions. Provided herein are modified FVII polypeptides that are designed to have increased coagulation activity upon activation, and that can serve as improved therapeutics to treat diseases and conditions amenable to factor VII therapy.

**1. FVII structure and organization**

The human FVII gene (F7) is located on chromosome 13 at 13q34 and is 12.8 kb long with 9 exons. The FVII gene shares significant organizational similarity with genes coding for other vitamin-K dependent proteins, such as prothrombin, factor IX, factor X and protein C. The mRNA for FVII undergoes alternative splicing to produce two transcripts: variant 1 (Genbank Accession No. NM_000131, set forth in SEQ ID NO: 108) and variant 2 (Genbank Accession No. NM_019616, set forth in SEQ ID NO: 109). Transcript variant 2, which is the more abundant form in the liver, does not include exon 1b and thus encodes a shorter precursor polypeptide of 444 amino acids (FVII isoform b precursor; SEQ ID NO:2), compared with the 466 amino acid precursor polypeptide encoded by transcript variant 1 (FVII isoform a precursor; SEQ ID NO:1). The amino acids that are not present in the FVII isoform b precursor polypeptide correspond to amino acid positions 22 to 43 of the FVII isoform a precursor. These amino acids are part of the propeptide sequence, resulting in truncated FVII isoform b propeptide. The precursor polypeptides are made up of the following segments and domains: a hydrophobic signal peptide (aa 1-20 of SEQ ID NO:1 and 2), a propeptide (aa 21-60 of SEQ ID NO:1, and aa 21-38 of SEQ ID NO:2), a Gla domain (aa 39-83 of SEQ ID NO:1, and aa 61-105 of SEQ ID NO: 2), a type B epidermal growth factor domain (EGF-like 1, aa 84-120 of SEQ ID NO: 1, and aa 106-142 of SEQ ID NO: 2), a type A epidermal growth factor domain (EGF-like 2, aa 125-166 of SEQ ID NO: 1; and aa 147-188 of SEQ ID NO: 2), and a serine protease domain (aa 191-430 of SEQ ID NO: 1, and aa 213-452 of SEQ ID NO: 2).

The 406 amino acid mature form of the FVII polypeptide (SEQ ID NO: 3) lacks the signal peptide and propeptide sequences, and is identical in length and sequence regardless of the isoform precursor from which it originated. In the mature form of the FVII polypeptide the corresponding amino acid positions for the above mentioned domains are as follows: Gla domain (aa 1-45 of SEQ ID NO: 3), EGF-like 1 (aa 46-82 of SEQ ID NO: 3), EGF-like 2 (aa 87-128 of SEQ ID NO: 3), and serine protease domain (aa 153-392 of SEQ ID NO: 3).

The Gla domain of FVII is a membrane binding motif which, in the presence of calcium ions, interacts with phospholipid membranes that include phosphatidylserine. The Gla domain also plays a role in binding to the FVIIa cofactor, tissue factor (TF). Complexed with TF, the Gla domain of FVIIa is loaded with seven $Ca^{2+}$ ions, projects three hydrophobic side chains in the direction of the cell membrane for interaction with phospholipids on the cell surface, and has significant contact with the C-terminal domain of TF. The Gla domain is conserved among vitamin K-dependent proteins, such as prothrombin, coagulation factors VII, IX and X, proteins C, S, and Z. These proteins require vitamin K for the posttranslational synthesis of $\gamma$-carboxyglutamic acid, an amino acid clustered in the N-terminal Gla domain of these proteins. All glutamic residues present in the domain are potential carboxylation sites and many of them are therefore modified by carboxylation.

In addition to the Gla domain, the mature FVII protein also contains two EGF-like domains. The first EGF-like domain (EGF-like 1 or EGF1) is a calcium-binding EGF domain, in which six conserved core cysteines form three disulfide bridges. The EGF1 domain of FVII binds just one $Ca^{2+}$ ion, but with significantly higher affinity than that observed with the Gla domain (Banner et al. (1996) Nature 380:41-46). This bound $Ca^{2+}$ ion promotes the strong interaction between the EGF1 domain of FVII and TF (Osterbund et al. (2000) Eur J Biochem 267:6204-6211.) The second EGF-like domain (EGF-like 2 or EGF2) is not a calcium-binding domain, but also forms 3 disulphide bridges. Like the other domains in FVII, the EGF2 domain interacts with TF. It also is disulphide-bonded together with the protease domain, with which it shares a large contact interface.

Finally, the serine protease domain of FVII is the domain responsible for the proteolytic activity of FVIIa. The sequence of amino acids of FVII in its catalytic domain displays high sequence identity and tertiary structure similarity with other serine proteases such as trypsin and chymotrypsin (Jin et al.(2001) J Mol Biol, 307: 1503-1517). For example, these serine proteases share a common catalytic triad H57, D102, S195, based on chymotrypsin numbering. Unlike other serine proteases, however, cleavage of FVIIa is not sufficient to complete the conversion of the zymogen to a fully active enzyme. Instead, as discussed below, FVIIa is allosterically activated in its catalytic function by binding to the cell-surface receptor TF, which induces a conformational change in the FVIIa protease domain switching it from a zymogen-like inactive state to a catalytically active enzyme. A helix loop region between the cofactor binding site and the active site (*i.e.* amino acid residue positions 305-321, corresponding to residues 163-170 based on chymotrypsin numbering) of FVIIa is important for the allostery and zymogenicity of FVIIa (Persson et al. (2004) Biochem J., 379: 497-503). This region is composed of a short a helix (amino acid residue positions 307 to 312) followed by a loop. The N-terminal portion of the helix forms part of the interface between the protease domain and TF, and contains a number of residues that are important for proteolytic function and optimal binding to TF. A comparison of the crystal structure of FVIIa alone and FVIIa complexed with TF indicates that the a helix undergoes significant conformational change when FVIIa binds TF. The a helix of FVIIa alone appears distorted, shortened and oriented differently. This affects adjacent loop structures, moving them away from the active site. In contrast, the a helix of FVIIa when complexed with TF is stabilized, and the neighboring loops are positioned closer to the active site. This stabilization is effected through mechanisms that involve at least the methionine at amino acid position 306 (amino acid residue $Met^{164}$ by chymotrypsin numbering) of FVII (Pike et al. (1999) PNAS 8925-8930).

### 2. Post-Translational Modifications

The FVII precursor polypeptide (either isoform of the Factor VII gene) is targeted to the cellular secretory pathway by the hydrophobic signal peptide, which inserts into the endoplasmic reticulum (ER) to initiate translocation across the membrane. While the protein is translocated through the ER membrane, the 20 amino acid signal peptide is cleaved off by a signal peptidase within the ER lumen, after which the polypeptide undergoes further post-translational modifications, including N- and O-glycosylation, vitamin K-dependent carboxylation of N-terminal glutamic acids to γ-carboxyglutamic acids, and hydroxylation of aspartic acid to *β*-hydroxyaspartic acid.

The propeptide provides a binding site for a vitamin K-dependent carboxylase which recognizes a 10-residue amphipathic α-helix in the FVII propeptide. After binding, the carboxylase γ-carboxylates 10 glutamic acid residues within the Gla domain of the FVII polypeptide, producing γ-carboxyglutamyl residues at positions E66, E67, E74, E76, E79, E80, E85, E86, E89 and E95 relative to the FVII precursor amino acid sequence set forth in SEQ ID NO: 2. These positions correspond to positions E6, E7, E14, E19, E20, E25, E26, E29 and E35 of the mature FVII polypeptide set forth in SEQ ID NO: 3. For optimal activity, the FVII molecule requires calcium, which binds the polypeptide and facilitates the conformational changes needed for binding of FVIIa with TF and lipids. The γ-carboxylated Gla domain binds seven $Ca^{2+}$ ions with variable affinity, which induces the conformational change that enables the Gla domain to interact with the C-terminal domain of TF, and also phosphatidylserines or other negatively charged phospholipids on the platelet membrane.

N-linked glycosylation is carried out by transfer of $Glc_3Man_9$ (GlcNAc) to two asparagine residues in the FVII polypeptide, at positions that correspond to amino acid residues145 and 262 of the mature protein (SEQ ID NO:3). O-linked glycosylation occurs at amino acid residues 52 and 60 of the mature polypeptide, and hydroxylation to a *β*-hydroxyaspartic acid accurs at the aspartic acid residue at position 63. These O-glycosylated serine residues and the *β*-hydroxylated aspartic acid residue are in the EGF-1 domain of FVII. These modifications are effected in the ER and Golgi complex before final processing of the polypeptide to its mature form.

### 3. FVII Processing

The modified pro-FVII polypeptide is transported through the Golgi lumen to the *trans*-Golgi compartment where the propeptide is cleaved by a propeptidase just prior to secretion of the protein from the cell. PACE/ furin (where PACE is an acronym for Paired basic Amino acid Cleaving Enzyme) is an endopeptidase localized to the Golgi membrane that cleaves many proteins on the carboxyterminal side of the sequence motif Arg-[any residue]-(Lys or Arg)-Arg. This propeptidase cleaves vitamin K-dependent glycoproteins such as the pro-factor IX and pro-vWF polypeptides (Himmelspach et al. (2000) Thromb Research 97; 51-67), releasing the propeptide from the mature protein. Inclusion of an appropriate PACE/furin recognition site into recombinant Factor VII precursors facilitates correct processing and secretion of the recombinant polypeptide (Margaritas et al. (2004) Clin Invest 113(7): 1025-1031). PACE/furin, or another subtilising-like propeptidase enzyme, is likely responsible for the proteolytic processing of pro-FVII to FVII. It can recognize and bind to the -Arg-Arg-Arg-Arg- consensus motif at amino acid positions 35-38 of the sequences set forth in SEQ ID NO:1, and 57-60 of the sequence set forth in SEQ ID NO:2, cleaving the propeptide and releasing the mature protein for secretion.

### 4. FVII activation

The vast majority of FVII in the blood is in the form of an unactivated single-chain zymogen, although a small amount is present in a two-chain activated form. Activation of FVII occurs upon proteolytic cleavage of the $Arg^{152}$-$Ile^{153}$ bond (positions relative to the mature FVII polypeptide, set forth in SEQ ID NO:3), giving rise to a two-chain polypeptide containing a 152 amino acid light chain (approximately 20 kDa) linked by a disulphide bridge to a 254 amino acid heavy chain (approximately 30 kDa). The light chain of FVIIa contains the Gla domain and EGF-like domains, while the heavy chain contains the catalytic or serine-protease portion of the molecule. Conversion of the single chain FVII into the two-chain FVIIa is mediated by cleavage by FIXa, FXa, FXIIa, thrombin, or in an autocatalytic manner by endogenous FVIIa (Butenas et al. (1996) Biochem 35:1904-1910; Nakagaki et al. (1991) Biochem 30:10819-10824). The trace amount of FVIIa that does occur in circulation likely arises from the action of FXa and FIXa.

As discussed above, cleavage of FVII from its zymogen form to FVIIa is not sufficient for full activity. FVIIa requires association with TF for full activity (Higashi et al. (1996) J Biol Chem 271:26569-26574). Because of this requirement, FVIIa alone has been ascribed zymogen-like features, displaying zymogen folding and shape, and exhibiting relatively low activity. This zymogen-like characteristic of FVIIa in the absence of its association with TF makes it relatively resistant to antithrombin III (AT-III) and other serpins, which generally act primarily on the active forms of serine proteases rather than the zymogen form. In addition, TFPI, the principal inhibitor of TF/FVIIa activity, also does not bind efficiently to the "inactive" uncomplexed form of FVIIa.

Upon complexation with TF, FVIIa undergoes a conformational change that permits full activity of the molecule. All of the FVII domains are involved in the interaction with TF, but the conformational changes that occur are localized to the protease domain of FVIIa. For example, the conformational changes that occur in upon allosteric interaction of FVIIa and TF include the creation of an extended macromolecular substrate binding exosite. This extended binding site greatly enhances the FVII-mediated proteolytic activation of factor X.

The activity of FVIIa is further increased (i.e. a thousand-fold) when the interaction of FVIIa is with cell surface-expressed TF. This is because phospholipid membranes containing negatively-charged phospholipids, such as phosphatidylserine, are a site of interaction of other vitamin-K dependent coagulation factors such as FIX and FX, which bind via their Gla domains. Thus, the local concentration of these vitamin K-dependent proteins is high at the cell surface, promoting their interaction with the TF/FVIIa complex.

**5. FVII Function**

Although FVIIa exhibits increased activity following allosteric activation by TF, there is evidence that mechanisms exist in which FVIIa alone can initiate coagulation. Hence, FVII can function in a TF-dependent and a TF-independent manner. This latter pathway can play a much smaller role in normal hemostasis, although it could be significant when it is considered in the context of bleeding disorders, and the treatment thereof.

**a. Tissue factor-dependent FVIIa activity**

Circulating FVII binds cell-surface TF and is activated by FIXa, FXa, thrombin, or in an autocatalytic manner by endogenous FVIIa as described above. Alternatively, the very small amount of circulating FVIIa can directly bind TF. The TF/FVIIa complex then binds a small fraction of plasma FX and the FVIIa catalytic domain cleaves FX to produce FXa. Thrombin is thus formed via the extrinsic pathway on the surface of the TF-bearing cell, when FXa complexes with FVa and activates prothrombin to thrombin (Figure 3). FIX also is activated by the TF/FVIIa complex, providing a link to the intrinsic pathway that operates on the surface of the activated platelet. The positive feedback systems in the coagulation cascade described above provide the means by which large amounts of thrombin are produced, which cleaves fibrinogen into fibrin to form a clot.

**b. Tissue factor-independent FVIIa activity**

In addition to the TF-dependent mechanism for the activation of FX to FXa, there is evidence that FVIIa also can activate FX in the absence of TF. Activated platelets translocate phosphatidylserines and other negatively charged phospholipids to the outer, plasma-oriented surface. (Hemker et al. (1983) Blood Cells 9:303-317). These provide alternative "receptors" through which FVIIa can bind, albeit with a relatively low affinity that is 1000-fold less than the binding affinity of FVIIa to TF (Monroe et al. (1997) Br J Haematol 99:542-7). This interaction is mediated through residues in the Gla domain (Harvey *et al.* (2003) 278:8363-8369). FVIIa can then convert FX to FXa and FIX to FIXa on the activated platelet surface (Hoffman et al. (1998) Blood Coagul Fibrinolysis 9:S61-S65). The FXa remains associated with the platelet surface, where it can bind to FVa and generate sufficient thrombin from prothrombin, while the newly formed FIXa assembles with FVIIIa to catalyze the activation of more FX to FXa (Figure 3). Hemostasis in the absence of TF can then achieved by the positive feedback and propagation mechanisms described above. It is notable, however, that while FVIIIa can contribute to the coagulation process on the activated platelet, its presence is not required for thrombin generation in the TF-independent mechanism (Figure 3). Thus, in the absence of FVIII, such as in hemophilia patients, there is evidence that FVIIa can initiate and/or amplify thrombin generation through this secondary mechanism, and effect clot formation.

**6. FVII as a biopharmaceutical**

FVII functions to initiate blood coagulation. Recombinant FVIIa (NovoSeven®; rFVIIa) is approved for treatment of bleeding episodes or prevention of bleeding in surgical or invasive procedures in patients having hemophilia A or B with inhibitors to Factor VIII or Factor IX, and in patients with congenital Factor VII deficiency. Novoseven® is a genetically engineered preparation of factor VIIa that is produced in a mammalian expression system using baby hamster kidney (BHK) cells. The agent is nearly identical to plasma-derived factor VIIa in its structure and function (Ratko et al. (2004), P & T, 29: 712-720).

Administration of recombinant FVIIa (rFVIIa) has been shown to promote blood clotting in patients suffering from hemophilia, and treatment with doses of FVIIa have been found to be safe and well-tolerated in human subjects.

Typically, the use of rFVIIa has been in patients who have developed inhibitors (*i.e.* alloantibodies) to Factor VIII or Factor IX. The use of rFVIIa as a coagulant has been extended to treatment of other bleeding disorders, for example Glanzmann's thrombasthenia; other events associated with extensive bleeding, such as a result of trauma or surgery including, but not limited to, liver transplants, prostate surgery and hemorrhaging trauma; neonatal coagulophathies, severe hepatic disease; bone marrow transplantation, thrombocytopenias and platelet function disorders; urgent reversal of oral anticoagulation; congenital deficiencies of factors V, VII, X, and XI; and von Willebrand disease with inhibitors to von Willebrand factor.

A high-dose of rFVII is required to achieve a therapeutic effect. The dose and dosing regime required for rFVII administration varies depending on the clinical indication. For example, the typical dosage of rFVII for hemorrhagic episodes in patients with hemophilia A or hemophilia B having alloantibodies is 90 μg/kg administered by intravenous (IV) injection. Since rFVII has a half-life of 2 hours, repeat dosing is required. Additional dosing can be given every two hours until hemostasis is achieved. The dose range can be altered depending on the severity of the condition. For example, doses ranging from 35 - 120 μg/kg have been efficacious. Also, the dose and dosing regime can vary with other indications. For example, hemophilia A or hemophilia B patients undergoing surgery can be administered with an initial dose of 90μg/kg immediately before surgery, with repeat dosing given every two hours during and following surgery. Depending on the severity of the surgery and bleeding episode, the bolus IV infusion can continue every two to six hours until healing is achieved. In congenital FVII deficient patients, rFVII is typically administered to prevent bleeding in surgery or other invasive procedures at 15 - 30 μg/kg every 4- 6 hours until hemostatsis is achieved.

The mechanism of action of rFVIIa to initiate hemostasis explains the high-dose requirement. Hemophilia patients have a normal initiation phase of coagulation, where the TF/FVIIa complex activates FX to FXa and leads to thrombin production at the site of the TF-bearing cell. Thereafter, however, the coagulation process breaks down as hemophilia patients lack FVIII (hemophilia A) or FIX (hemophilia B), and are therefore unable to form the FVIIIa/FIXa complexes on the surface of the activated platelet, which normally serve to activate large amounts of FX to FXa in the amplification and propagation phases described previously. Due to the presence of inhibitors, such as TFPI and AT-III, the FXa that is produced on the TF-bearing cell following cleavage by TF/FVIIa is unable to easily diffuse between cell surfaces. As a result, large-scale thrombin generation on the surface of the activated platelet does not occur, and a clot is not formed.

There is evidence that the hemostatic effect of high doses of rFVIIa can be achieved using TF-dependent and/or TF-independent generation of FXa by rFVIIa on the activated platelets (Figure 3). TF-dependent thrombin generation can be maximized very quickly with the saturation of TF molecules with endogenous FVIIa and rFVIIa. In some instances, the high dose rFVIIa can bind activated platelets and convert FX to FXa. The surface-associated FXa activates FVa to generate sufficient thrombin for hemostasis. Since rFVII binds to the platelet surface with low affinity, a higher dose of rFVII can be required for thrombin generation. The activation of FXa on activated platelets ensures that rFVIIa-mediated hemostasis is localized to the site of injury.

A means to achieve reduced dosage of rFVII can improve its utility and efficiency as a drug. Provided herein are modified FVII polypeptides. Among these are modified FVII polypeptides that exhibit increased resistance to TFPI, increased resistance to AT-III, improved pharmacokinetic properties, such as increased half-life, increased catalytic activity in the presence and/or absence of TF, and/or increased binding to activated platelets. These FVII polypeptides can exhibit increased coagulant activity. FVII polypeptides provided herein can be used in treatments to initiate hemostasis in a TF-dependent and/or a TF-independent mechanism such that FXa is produced and thrombin generated.

## D. Modified FVII polypeptides

Provided herein are modified FVII polypeptides. The FVII polypeptides exhibit alterations in one or more activities or properties compared with an unmodified FVII polypeptide. The activities or properties that can be altered as a result of modification include, but are not limited to, coagulation or coagulant activity; pro-coagulant activity; proteolytic or catalytic activity such as to effect factor X (FX) activation or Factor IX (FIX) activation; antigenicity (ability to bind to or compete with a polypeptide for binding to an anti-FVII antibody); ability to bind tissue factor, factor X or factor IX; ability to bind to the cell surface of activated platelets; ability to bind to phospholipids; half-life; three-dimensional structure; pI; and/or conformation. Typically, the modified FVII polypeptides exhibit procoagulant activity. Provided herein are modified FVII polypeptides that exhibit increased coagulant activity upon activation from their single-chain zymogen form. Such modified FVII polypeptides can be used in the treatment of bleeding disorders or events, such as hemophilias or injury, where FVII polypeptides can function to promote blood coagulation. Included among such modified FVII polypeptides are those that have increased resistance to inhibitors such as tissue factor pathway inhibitor (TFPI) and antithrombin III (AT-III), those that have increased catalytic activity in the presence and/or absence of TF, those that have improved pharmacokinetic properties, such as increased half-life and those that have increased binding and/or affinity for the platelet surface. In particular, such modified FVII polypeptides can be used in diseases or conditions to provide coagulant activity while at the same time bypassing the requirements for FVIIIa and FIXa. In one example, modified FVII polypeptides provided herein can be used in hemophiliac patients having autoantibodies to FVIIIa and FIXa. Hence, the modified FVII polypeptides provided herein offer advantages including a decrease in the amount of administered FVII that is required to maintain a sufficient concentration of active FVII in the serum for hemostasis. This can lead to, for example, lower doses and/or dosage frequency necessary to achieve comparable biological effects, faster onset of therapeutic benefit, faster amelioration of acute bleeding episodes, higher comfort and acceptance by subjects, and attenuation of secondary, non-beneficial effects.

Modifications in a FVII polypeptide can be made to any form of a FVII polypeptide, including allelic and species variants, splice variants, variants known in the art, or hybrid or chimeric FVII molecules. For example, the modifications provided herein can be made in a precursor FVII polypeptide set forth in SEQ ID NOS:1 or 2, a mature FVII polypeptide set forth in SEQ ID NO:3, or any species, allelic or modified variants and active fragments thereof, that has 40%, 50%, 60%, 70%, 80%, 90%, 91%, 92%, 93%, 94%, 95%, 96%, 97%, 98%, or 99% sequence identity to any of the FVII polypeptides set forth in SEQ ID NOS: 1-3. Allelic variants of FVII include, but are not limited to, any of those precursor polypeptides having a sequence of amino acids set forth in any of SEQ ID NOS: 18-74. Exemplary species variants for modification herein include, but are not limited to, human and non-human polypeptides including FVII polypeptides from cow, mouse, pygmy chimpanzee, chimpanzee, rabbit, rat, rhesus macaque, pig, dog, zebra fish, pufferfish, chicken, orangutan and gorilla FVII polypeptides, whose sequences are set forth in SEQ ID NOS: 4-17 respectively. Modifications in a FVII polypeptide can be made to a FVII polypeptide that also contains other modifications, such as those described in the art, including modifications of the primary sequence and modifications not in the primary sequence of the polypeptide.

Modification of FVII polypeptides also include modification of polypeptides that are hybrids of different FVII polypeptides and also synthetic FVII polypeptides prepared recombinantly or synthesized or constructed by other methods known in the art based upon the sequence of known polypeptides. For example, based on alignment of FVII with other coagulation factor family members, such as factor IX (FIX) or factor X (FX), homologous domains among the family members are readily identified. Chimeric variants of FVII polypeptides can be constructed where one or more amino acids or entire domains are replaced in the FVII amino acid sequence using the amino acid sequence of the corresponding family member. Additionally, chimeric FVII polypeptides include those where one or more amino acids or entire domains are replaced in the human FVII amino acid sequence using the amino acid sequence of a different species (*see, e.g.,* Williamson et al. (2005) J Thromb Haemost 3:1250-6). Such chimeric proteins can be used as the starting, unmodified FVII polypeptide herein.

Modifications provided herein of a starting, unmodified reference polypeptide include amino acid replacements or substitution, additions or deletions of amino acids, or any combination thereof. For example, modified FVII polypeptides include those with 1, 2, 3, 4, 5, 6, 7, 8, 9, 10, 11, 12, 13, 14, 15, 16, 17, 18, 19, 20, 30, 40, 50 or more modified positions. Also provided herein are modified FVII polypeptides with two or more modifications compared to a starting reference FVII polypeptide. Modified FVII polypeptides include those with 2, 3, 4, 5, 6, 7, 8, 9, 10, 11, 12, 13, 14, 15, 16, 17, 18, 19, 20, 30, 40, 50 or more modified positions. Any modification provided herein can be combined with any other modification known to one of skill in the art so long as the resulting modified FVII polypeptide exhibits increased coagulation activity when it is in its two-chain or activated form. Typically, the modified FVII polypeptides exhibit increased coagulant activity. The activities or properties that can be altered as a result of modification include, but are not limited to, coagulation or coagulant activity; pro-coagulant activity; proteolytic or catalytic activity such as to effect factor X (FX) activation or Factor IX (FIX) activation; antigenicity (ability to bind to or compete with a polypeptide for binding to an anti-FVII antibody); ability to bind tissue factor, tissue factor inhibitory factor (TFPI), antithrombin III, factor X or factor IX; ability to the surface of activated platelets; ability to bind to phospholipids; serum half-life; three-dimensional structure; pI; and/or conformation. Included among the modified FVII polypeptides provided herein are those that have increased resistance to tissue factor pathway inhibitor (TFPI), increased resistance to antithrombin III (AT-III), increased catalytic activity in the presence and/or absence of TF, improved pharmacokinetic properties, such as increased serum half-life, increased intrinsic activity and/or increased affinity and/or binding for activated platelets.

In some examples, a modification can affect two or more properties or activities of a FVII polypeptide. For example, a modification can result in increased TFPI resistance and increased catalytic activity of the modified FVII polypeptide compared to an unmodified FVII polypeptide. Modified FVII polypeptides provided herein can be assayed for each property and activity to identify the range of effects of a modification. Such assays are known in the art and described below. Modified FVII polypeptides provided herein also include FVII polypeptides that are additionally modified by the cellular machinery and include, for example, glycosylated, γ-carboxylated and β-hydroxylated polypeptides.

The modifications provided herein to a FVII polypeptide are made to increase TFPI resistance, increase AT-III resistance, improve pharmacokinetic properties, such as increase serum half-life, increase catalytic activity in the presence and/or absence of TF and/or increase affinity and/or binding for activated platelets. For example, a FVII polypeptide can include modification(s) that increase one or both of TFPI resistance and binding to platelets. In other examples, any modification provided herein can be combined with any other modification known to one of skill in the art so long as the resulting modified FVII polypeptide exhibits increased coagulation activity when it is in its two-chain form.

Typically, such increased coagulation activity is due to increased resistance to TFPI, improved pharmacokinetic properties, such as increased serum half-life, increased resistance to AT-III, altered glycosylation, increased catalytic activity, and/or increased binding and/or affinity for phospholipids. In some examples, modifications that are introduced into a FVII polypeptide to alter a specific activity or property also, or instead, can affect another activity or property. Thus, the modifications provided herein can affect the property or activity that they were designed to affect and one or more other properties or activities. For example, modifications made to a FVII polypeptide to increase TFPI resistance also can improve pharmacokinetic properties, such as serum half-life. In some examples, a single modification, such as single amino acid substitution, alters 2, 3,4 or more properties or activities of a FVII polypeptide. Modified FVII polypeptides provided herein can be assayed for each property and activity to identify the range of effects of a modification. Such assays are known in the art and described below. Modified FVII polypeptides provided herein also include FVII polypeptides that are additionally modified by the cellular machinery and include, for example, glycosylated, γ-carboxylated and β-hydroxylated polypeptides.

The modifications provided herein can be made by standard recombinant DNA techniques such as are routine to one of skill in the art. Any method known in the art to effect mutation of any one or more amino acids in a target protein can be employed. Methods include standard site-directed mutagenesis (using *e.g.*, a kit, such as kit such as QuikChange available from Stratagene) of encoding nucleic acid molecules, or by solid phase polypeptide synthesis methods. In addition, modified chimeric proteins provided herein (*i.e*. Gla domain swap) can be generated by routine recombinant DNA techniques. For example, chimeric polypeptides can be generated using restriction enzymes and cloning methodologies for routine subcloning of the desired chimeric polypeptide components.

Other modifications that are or are not in the primary sequence of the polypeptide also can be included in a modified FVII polypeptide, or conjugate thereof, including, but not limited to, the addition of a carbohydrate moiety, the addition of a polyethylene glycol (PEG) moiety, the addition of an Fc domain, etc. For example, such additional modifications can be made to increase the stability or half-life of the protein.

The resulting modified FVII polypeptides include those that are single-chain zymogen or active polypeptides or those that are one-chain or two-chain zymogen-like polypeptides. For example, any modified polypeptide provided herein that is a single-chain zymogen polypeptide can be autoactivated or activated by other coagulation factors to generate a modified FVII that is a two-chain form (i.e. FVIIa). The activities of a modified FVII polypeptide are typically exhibited in its two-chain form.

The modified FVII polypeptides provided herein can exhibit increased TFPI resistance, increased AT-III resistance, increased catalytic activity in the presence and/or absence of TF, improved pharmacokinetic properties, such as increased serum half-life, and/or increased binding and/or affinity for phospholipids. Typically, such properties and/or activities of the modified FVII polypeptides provided herein are made while retaining other FVII activities or properties, such as, but not limited to, binding to TF and/or binding and activation of FX. Hence, modified FVII polypeptides provided herein retain TF binding and/or FX binding and activation as compared to a wild-type or starting form of the FVII polypeptide. Typically, such activity is substantially unchanged (less than 1%, 5%, 10%, 20% or 30% changed) compared to a wild-type or starting protein. In other examples, the activity of a modified FVII polypeptide is increased or is decreased as compared to a wild-type or starting FVII polypeptide. Activity can be assessed *in vitro, ex vivo* or *in vivo* and can be compared to that of the unmodified FVII polypeptide, such as for example, the mature, wild-type native FVII polypeptide (SEQ ID NO: 3), the wild-type precursor FVII polypeptide (SEQ ID NO: 1 or 2), or any other FVII polypeptide known to one of skill in the art that is used as the starting material.

Hence, by virtue of the modifications provided herein, the modified FVII polypeptides can exhibit increased coagulation activity in a TF-dependent and/or TF-independent manner. Typically, the increased coagulation activity of the modified FVII polypeptides provided herein can be observed *in vitro* in appropriate assays, or *in vivo,* such as upon administration to a subject, such as a human or non-human subject. The increased activity of the modified FVII polypeptides can be enhanced by at least or about 1%, 2%, 3%, 4%, 5%, 6%, 7%, 8%, 9%, 10%, 20%, 30%, 40%, 50%, 60%, 70%, 80%, 90%, 100%, 110%, 120%, 130%, 140%, 150%, 160%, 170%, 180%, 190%, 200%, 300%, 400%, 500%, or more compared to the activity of the starting or unmodified FVIIa polypeptide.

### 1. Increased resistance to inhibitors

Like most biological systems, the coagulation cascade is regulated by positive and negative mechanisms. These mechanisms often act by inhibiting or enhancing the activity of one or more factors involved in the cascade, either directly or indirectly. Hence, of interest are therapeutic coagulation factors that are resistant to the inhibitory mechanisms that negatively regulate their activity. In particular, of interest are modified FVII polypeptides that are resistant to the inhibitory molecules that normally inhibit FVII activity. The primary regulator of the TF/FVIIa complex is tissue factor pathway inhibitor (TFPI). Also inhibitory to FVIIa activity, albeit to a lesser extent, is antithrombin III (AT-III).

**a. TFPI**

Tissue factor pathway inhibitor is a single-chain polypeptide encoded by 9 exons located on chromosome 2q31-q32.1. TFPI (also referred to as TFPI-1) is a Kunitz-type inhibitor that is synthesized primarily by endothelial cells and contains a negatively charged amino terminal region, three tandem Kunitz-type inhibitor domains, and.a highly basic carboxyl-terminal tail (Wun et al., J. Biol. Chem. 263:6001 (1988). Through alternative mRNA splicing, two different forms of TFPI are generated: TFPIa and β. TFPIα is a secreted protein with a molecular weight of approximately 46 kDa. The precursor polypeptide is 304 amino acids in length (SEQ ID NO:101), and is processed by cleavage of the 28 amino acid signal peptide, resulting in secretion of a 276 amino acid mature glycoprotein (SEQ ID NO:102). The mature protein contains 18 cysteine residues and forms 9 disulphide bridges when correctly folded. The primary sequence also contains three Asn-X-Ser/Thr N-linked glycosylation consensus sites, the asparagine residues located at positions 145, 195 and 256. The Kunitz-1 and -2 domains are responsible for binding and inhibition of the TF/FVIIa complex and FXa, respectively (see *e.g.*, Figure 4). The Kunitz-3 domain, which lacks proteinase inhibitory activity, and the C-terminus of TFPIα have been shown to be involved in its cell-surface localization (Piro et al. (2004) Circulation 110:3567-3572).

TFPIβ is an alternatively spliced form of TFPI in which the Kunitz-3 domain and the C-terminal region of TFPIα is replaced with an unrelated C-terminal region that directs the attachment of a glycosylphosphatidylinositol (GPI) anchor. The precursor polypeptide (SEQ ID NO:103) is processed to a mature protein that is 223 amino acids (SEQ ID NO:104). Based on protein mass, TFPIβ (28 kDa) is considerably smaller than TFPIα (36 kDa). Both migrate with the same apparent molecular mass (46 kDa) on sodium dodecylsulfate-polyacrylamide gel electrophoresis (SDS-PAGE), suggesting a difference in post-translational modifications. TFPIα and β are bound at the cell surface in a GPI-dependent manner. TFPIβ contains a GPI-anchor at its C-terminus, whereas TFPIα is apparently bound to a not yet identified GPI-linked protein(s). Although TFPIβ represents only 20% of total surface-TFPI, it accounts for most of the anti-TF/FVIIa activity, suggesting a potential alternative role for cell-surface TFPIα, such as binding and clearance of FXa (Piro et al. (2005) J Thromb Haemost 3:2677-2683).

TFPI inhibits the coagulation cascade in at least two ways; by binding to the active site of FXa, and by inactivating the TF/FVIIa complex (see *e.g.,* Figure 4). The first Kunitz-domain is binds FVIIa, while the second domain binds FXa (Girard et al. (1989) Nature 338:518-520). Free TFPI binds FVIIa very slowly in comparison with its binding of FXa. Once bound to FXa however, the TFPI/FXa complex displays strong affinity for the TF/FVIIa complex. Their interaction results in the formation of a heterotetrameric complex at the endothelial cell surface in which FVIIa is catalytically inactive (Figure 4). This quaternary structure therefore prevents TF/FVIIa-initiation of the coagulation cascade.

At least one other homolog of TFPI exists in humans, TFPI-2, also known as placental protein 5 (PP5) and matrix-associated serine protease inhibitor (MSPI). TFPI-2 is a 32 kDa matrix-associated Kunitz-type serine proteinase inhibitor which exhibits inhibitory activity toward a broad spectrum of proteinases, including trypsin, plasmin, chymotrypsin, cathepsin G, plasma kallikrein, and the factor VIIa-tissue factor complex. The 213 amino acid mature TFPI-2 protein (SEQ ID NO:105) contains three Kunitz-type domains that exhibit 43%, 35% and 53% primary sequence identity with TFPI Kunitz-type domains 1, 2, and 3, respectively. The first Kunitz-type domain of human TFPI-2 contains all the structural elements for the inhibition of the serine proteases, although kinetic and molecular studies indicate that TFPI-2 is a more effective inhibitor of plasmin than several other serine proteinases, including TF/FVIIa (Chand et al. (2004) J Biol Chem 279:17500-17507). It is thought to play an important role in the regulation of extracellular matrix digestion and remodeling. In this context, reduced synthesis of TFPI-2 has been related to numerous pathophysiological processes such as inflammation, angiogenesis, atherosclerosis, retinal degeneration and tumor growth/metastasis (Chand et al. (2005) Thromb Haemost 94:1122-30). Thus, while homologous to TFPI, TFPI-2 is not thought to be as important a factor in the coagulation pathway. The sequence alignment of the Kunitz-1 domain sequence of TFPI-1 and TFPI-2 is set forth in Figure 6b.

Immunodepletion of TFPI in a rabbit model enhanced coagulation (Warn-Cramer et al. (1993) Arterioscl Thromb 13:1551-1557, Ragni et al. (2000) Circulation 102:113-117), indicating that disruption of the formation of TF/FVIIa-TFPI/FXa quaternary complex can inhibit the negative regulation of TF/FVIIa initiation of coagulation. Additionally, disruption of the interaction of FVIIa with TFPI can decrease the clearance of FVIIa. Decreased clearance can be manifested as increased half-life. Also, FVIIa bound to cell surface TF can be internalized and degraded without depleting the TF on the cell surface. This form of internalization and degradation is significantly increased in the presence of TFPI/FXa, which forms a quaternary complex with the cell-surface TF/FVIIa. The internalization and degradation that follows binding is mediated through a low density lipoprotein receptor-related protein (LRP)-dependent coated pit pathway (Iakhiaev et al., (1999) J. Biol. Chem 274:36995-37003). TFPI also can help mediate FVIIa clearance through other mechanisms, such as hepatic or renal clearance mechanisms.

**Modifications to effect increased resistance to TFPI**

Provided herein are modified FVII polypeptides exhibiting increased resistance to TFPI. Such resistance to TFPI can be achieved, for example, by mutation of one or more residues in FVII involved in the interaction and binding with TFPI to reduce or prevent such binding, thereby making the modified FVII polypeptides resistant to the naturally inhibitory effects of TFP1 with respect to coagulation initiation. When evaluated in an appropriate *in vitro* assay, or *in vivo,* such as following administration to a subject as a pro-coagulant therapeutic, the modified TFPI-resistant FVII polypeptides can display increased coagulant activity as compared with unmodified FVII polypeptides. Among FVIIa mutants that exhibit increased TFPI resistance, are mutants that have increased half-life. For example, the mutant described in Example 9 exhibits increased half-life.

Provided herein are modified FVII polypeptides having one or more mutations in FVII/TFPI contact residues or residues in close proximity to the interaction interface. Such contact residues include the aspartic acid residue at position 196 (D196, $Asp^{196}$), the lysine residue at position 197 (K197, $Lys^{197}$), the lysine residue at position 199 (K199, $Lys^{199}$), the threonine residue at position 239 (T239, $Thr^{239}$), the arginine residue at position 290 (R290, $Arg^{290}$), and the lysine residue at position 341 (K341, $Lys^{341}$), with numbering relative to the amino acid positions of a mature FVII polypeptide set forth in SEQ ID NO:3. When identified using chymotrypsin numbering, these residues correspond to D60, K60a, K60c, T99, R147 and K192 (Figure 7). A residue that is in close proximity to the FVII/TFPI interface, and which can be modified to cause steric hindrance, is the glycine residue at position 237 (G237, $Gly^{237}$) by mature FVII numbering, which corresponds to G97 ($Gly^{97}$) by chymotrypsin numbering.

The identified residues can be modified such as by amino acid replacement, deletion or substitution. Alternatively, amino acid insertions can be used to alter the conformation of a targeted amino acid residue or the protein structure in the vicinity of a targeted amino acid residue. For example, the identified residues can be replaced or substituted with another amino acid in order to disrupt the interaction between FVII and TFPI and inhibit efficient binding of the two proteins, thereby generating a TFPI-resistant modified FVII polypeptide. For example, substitution of an amino acid that sterically hinders interaction of FVII and TFPI is contemplated (i.e. substitution of $Gly^{237}$ ($Gly^{97}$) with another larger amino acid). In other embodiments, the TFPI-resistant modified FVII polypeptides can be generated by replacing a contact residue with an alternative amino acid such that charge-reversal or charge-neutralization is achieved at the identified position. These types of mutations can significantly affect the electrostatic contributions of the substituted FVII amino acid and disrupt the normal interaction of the contact residue with corresponding TFPI contact residue(s). For example, a negatively charged aspartic acid residue in FVII, which might normally interact favorably with a positively charged arginine residue in TFPI, can be replaced with a positively charged lysine to not only eliminate the original favorable electrostatic interaction but also create a repulsive force between the residues and interfere with efficient binding of the proteins. This mutation, therefore, interferes with efficient binding of the proteins. Hence, a negatively charged amino acid (i.e. an acidic amino acid) such as aspartic acid (Asp, D) or glutamic acid (Glu, E) can be substituted with a positively charged amino acid (ie. a basic amino acid) such as lysine (Lys, K), arginine (Arg, R), or histidine (His, H). Conversely, a basic amino acid such as lysine (Lys, K), arginine (Arg, R), or histidine (His, H) can be substituted with an acidic amino acid such as aspartic acid (Asp, D) or glutamic acid (Glu, E). Also, any contact residue can be substituted with a neutral amino acid such as alanine (Ala, A), tyrosine (Tyr, Y) methionine (Met, M), or leucine (Leu, L), isoleucine (Ile, I), valine (Val, V), phenylalanine (Phe, F), proline (Pro, P) or glycine (Gly, G), serine (Ser, S), threonine (Thr, T), asparagine (Asn, N), glutamine (Gln, Q), tryptophan (Trp, W) and cysteine (Cys, C). Exemplary of neutral amino acids are hydrophobic amino acids, which include isoleucine (Ile, I), phenylalanine (Phe, F), valine (Val, V), leucine (Leu, L), tryptophan (Trp, W), methionine (Met, M), alanine (Ala, A), glycine (Gly, G), cysteine (Cys, C) and tyrosine (Tyr, Y).

In some embodiments, the glycine at position 237 by mature FVII numbering is replaced such that the new amino acid causes steric hindrance and inhibits the interaction between FVII and TFPI. Glycine is the smallest and simplest of the amino acids, containing only a single hydrogen atom in its side chain. Due to its small size, glycine can fit into small spaces and can adopt particular conformations that other amino acids can not. Substitution of the glycine at position 237 with an amino acid with a larger side chain could cause steric hindrance with other amino acids in the FVII protein, thereby altering the conformation of the TFPI binding interface, and/or cause steric hindrance with amino acids in the TFPI protein. Both situations could result in impaired interaction and binding of FVII and TFPI, thereby increasing the resistance of the modified FVII polypeptide to the inhibitory effects of TFPI. Any of the 19 natural amino acids contain larger side chains than glycine, and could be used to modify the FVII polypeptide. Exemplary of these are tryptophan (Trp, W), isoleucine (Ile, I), valine (Val, V) and threonine (Thr, T).

In other examples, amino acid insertions are incorporated into the FVII polypeptide near the identified residues to interfere with the interaction between TFPI and FVII. One or more amino acid residues can be inserted at one or more positions in the FVII polypeptide. For example, a tyrosine residue can be inserted between D196 and K197 (corresponding to D60 and K60a, respectively, by chymotrypsin numbering). This modification is D196K197insY (i.e. D196 and K197 are the positions in between which a tyrosine has been inserted (insY)). In another example, two amino acid residues are inserted at a position identified as being important for interaction with TFPI. For example, an alanine and a serine can be inserted between G237 and T238 (corresponding to G97 and T98, respectively, by chymotrypsin numbering), resulting in the modification G237T238insAS. Such insertion mutations could result in impaired interaction and binding of FVII and TFPI, thereby increasing the resistance of the modified FVII polypeptide to the inhibitory effects of TFPI.

Table 5 provides non-limiting examples of exemplary amino acid replacements at the identified residues, corresponding to amino acid positions of a mature FVII polypeptide as set forth in SEQ ID NO:3. As noted, such FVII polypeptides are designed to increase resistance to TFPI by inhibiting FVII/TFPI binding, and therefore have increased coagulant activity. In reference to such mutations, the first amino acid (one-letter abbreviation) corresponds to the amino acid that is replaced, the number corresponds to the position in the mature FVII polypeptide sequence with reference to SEQ ID NO: 3, and the second amino acid (one-letter abbreviation) corresponds to the amino acid selected that replaces the first amino acid at that position. The amino acid positions for mutation also are referred to by the chymotrypsin numbering scheme. In Table 5 below, the sequence identifier (SEQ ID NO) is identified in which exemplary amino acid sequences of the modified FVII polypeptide are set forth, and also any polypeptide identification numbers.

**Table 5.**

| Modification - mature FVII numbering | Modification -chymotrypsin numbering | Polypeptide ID number | SEQ ID NO |
|---|---|---|---|
| D 196K | D60K | CB554 | 18 |
| D196R | D60R | CB555 | 19 |
| D196A | D60A | CB556 | 20 |
| D196Y | D60Y | CB601 | 125 |
| D196F | D60F | CB600 | 126 |
| D196W | D60W | CB602 | 127 |
| D196L | D60L | CB603 | 128 |
| D196I | D60I | CB604 | 129 |
| K197Y | K60aY | CB561 | 21 |
| K197A | K60aA | CB559 | 22 |
| K197E | K60aE | CB558 | 23 |
| K197D | K60aD | CB557 | 24 |
| K197L | K60aL | CB560 | 25 |
| K197M | K60aM | CB599 | 26 |
| K197I | K60aI | CB595 | 130 |
| K197V | K60aV | CB596 | 131 |
| K197F | K60aF | CB597 | 132 |
| K197W | K60aW | CB598 | 133 |
| K199A | K60cA | CB564 | 27 |
| K199D | K60cD | CB562 | 28 |
| K 199E | K60cE | CB563 | 29 |
| G237W | G97W | CB605 | 134 |
| G237T | G97T | CB606 | 135 |
| G237I | G97I | CB607 | 136 |
| G237V | G97V | CB608 | 137 |
| T239A | T99A | CB565 | 30 |
| R290A | R1A7A | CB568 | 31 |
| R290E | R147E | CB567 | 32 |
| R290D | R147D | CB566 | 33 |
| R290N | R147N | CB697 | 206 |

(continued)

| Modification - mature FVII numbering | Modification -chymotrypsin numbering | Polypeptide ID number | SEQ ID NO |
|---|---|---|---|
| R290Q | R147Q | CB698 | 207 |
| R290K | R147K | CB699 | 208 |
| R290M | R147M | CB700 | 209 |
| R290V | R147V | CB701 | 210 |
| K341E | K192E | | 34 |
| K341 R | K192R | CB569 | 35 |
| K341Q | K192Q | CB609 | 138 |
| K341N | K192N | CB733 | 211 |
| K341M | K192M | CB734 | 212 |
| K341 D | K192D | CB735 | 213 |
| G237T238insA | G97T98insA | CB853 | 214 |
| G237T238insS | G97T98insS | CB854 | 215 |
| G237T238insV | G97T98insV | CB855 | 216 |
| G237T238insAS | G97T98insAS | CB856 | 217 |
| G237T238insSA | G97T98insSA | CB857 | 218 |
| D196K197insK | D60K60ainsK | CB858 | 219 |
| D196K197insR | D60K60ainsR | CB859 | 220 |
| D196K197insY | D60K60ainsY | CB860 | 221 |
| D196K197insW | D60K60ainsW | CB861 | 222 |
| D196K197insA | D60K60ainsA | CB862 | 223 |
| D196K197insM | D60K60ainsM | CB863 | 224 |
| K197I198insE | K60aI60binsE | CB864 | 225 |
| K197I198insY | K60aI60binsY | CB865 | 226 |
| K197I198insA | K60aI60binsA | CB866 | 227 |
| K197I198insS | K60aI60binsS | CB867 | 228 |

In a further embodiment, combination mutants can be generated. Included among such combination mutants are those having two or more mutations of the residues D196, K197, K199, G237, T239, R290 or K341 based on numbering of a mature FVII set forth in SEQ ID NO:3 (corresponding to D60, K60a, K60c, G97, T99, R147 and K 192, respectively, based on chymotrypsin numbering). For example, a modified FVII polypeptide can possess amino acid substitutions at 2, 3, 4, 5, 6 or 7 of the identified positions. As noted above, residues are replaced such that favorable interactions between FVIIa and TFPI are eliminated, and unfavorable interactions are introduced, or steric hindrance is introduced, at one more positions. Hence, a modified polypeptide can display 1, 2, 3,4, 5,6 or 7 mutations that provide one or more of steric hindrance, charge-reversal, charge-neutralization, or other unfavourable interaction, or a combination thereof. For example, two or more mutations can be made whereby negatively charged amino acids such as aspartic acid (Asp, D) or glutamic acid (Glu, E) can be substituted with a positively charged amino acid such as lysine (Lys, K), arginine (Arg, R), or histidine (His, H), or vice versa, or neutral substitutions can be made by replacement of any amino acid with, for example, alanine (Ala, A), tyrosine (Tyr, Y) methionine (Met, M), or leucine (Leu, L), isoleucine (Ile, I), valine (Val, V), phenylalanine (Phe, F), proline (Pro, P) or glycine (Gly, G), serine (Ser, S), threonine (Thr, T), asparagine (Asn, N), glutamine (Gln, Q), tryptophan (Trp, W) and cysteine (Cys, C) or any combination thereof. Exemplary of neutral amino acids are hydrophobic amino acids, which include isoleucine (Ile, I), phenylalanine (Phe, F), valine (Val, V), leucine (Leu, L), tryptophan (Trp, W), methionine (Met, M), alanine (Ala, A), glycine (Gly, G), cysteine (Cys, C) and tyrosine (Tyr, Y). In another example, two or more mutations can be made where at least one is a charge reversal or charge-neutralization and another is a substitution that replaces glycine with amino acids that contain a larger side chain, such as tryptophan (Trp, W), isoleucine (Ile, I), valine (Val, V) and threonine (Thr, T). In other examples, an insertion mutation can be included in the modified FVII polypeptide with other insertion mutations, or with one or more amino acid substitutions, such as amino acid substitutions that introduce unfavourable interactions or steric hindrance between FVII and TFPI. Any permutation of combinations can be included in a modified FVII polypeptide using the modifications set forth in Table 5, above. For example, FVII polypeptides provided herein can contain D196K and one or more additional replacement modifications at amino acid position K197, K199, G237, T239, R290 and/or K341, and/or an insertion after D196, K197 and/or G237. One of skill in art can readily generate all possible combinations using the modifications set forth in Table 5.

For example, FVII variants include those that include as one of their mutations an amino acid substitution at position 197, based on numbering of a mature FVII set forth in SEQ ID NO:3. Any amino acid residue can be substituted for the lysine residue that occupies position 197 in an unmodified FVII polypeptide. In some embodiments, the lysine is replaced by a tyrosine (Y), an alanine (A), a glutamic acid (E), an aspartic acid (D), a leucine (L), a methionine (M), an isoleucine (I), a valine (V), a phenylalanine (F), or a tryptophan (W) residue. For example, exemplary of a modified FVII polypeptide is one that contains the substitution K197L or K197E. In another example, a FVII polypeptide containing a modification at position 197 (such as a replacement, deletion or addition) also can include another modification at another position. Generally such other modification is at a position that is contemplated to modulate the interaction of FVII with TFPI, such as by removing a favorable interaction, creating an unfavorable interaction, introducing steric hindrance, or a combination thereof. Included among such additional modifications are modifications at amino acid positions corresponding to D196, K199, G237, T239, R290 or K341, such as described above. Other additional modifications known in the art, such as described below, also are contemplated. Hence, in addition to modification at position 197, a FVII polypeptide can contain 1, 2, 3, 4, 5, 6 or more amino acid modifications. For example, exemplary of FVII variants having at least a modification at position 197 include those containing 2 substitutions, such as D196F and K197E, those containing 3 substitutions, such as at D196R, K197M, and K199E, those containing 4 substitutions, such as D196R, K197E, K199E and R290E, those containing 5 substitutions, such as K197L, K199D, G237T, R290D and K341Q, those containing 6 substitutions, such as D196F, K197L, K199E, G237V, T239A and R290D, and those containing 7 substitutions, such as D196Y, K197L, K199A, G237T, T239A, R290D and K341R Exemplary FVII combination mutants that include as one of their mutations an amino acid substitution at position 197, based on numbering of a mature FVII set forth in SEQ ID NO:3, are included in Table 6.

Exemplary FVII polypeptides that contain two or more amino acid replacements at one or more amino acid residues of D196, K197, K199, G237, T239, R290 or K341, corresponding to amino acid positions of a mature FVII polypeptide as set forth in SEQ ID NO:3, are those provided in Table 6. In the table, amino acid positions for mutation also are referred to by the chymotrypsin numbering scheme. Such modifications are designed to increase resistance to TFPI by inhibiting FVII binding to TFPI, and therefore increase coagulation activity.

**Table 6.**

| Modification - mature FVII numbering | Modification - chymotrypsin numbering | Polypeptide ID No. | SEQ ID NO |
|---|---|---|---|
| D196R/R290E | D60R/R147E | CB579 | 36 |
| D196K/R290E | D60K/R147E | CB580 | 37 |
| D196R/R290D | D60R/R147D | CB581 | 38 |
| D196R/K197E/K199E | D60R/K60aE/K60cE | CB586 | 39 |
| D196K/K197E/K199E | D60K/K60aE/K60cE | CB587 | 40 |
| D196R/K197E/K199E/ R290E | D60R/K60aE/K60cE/ R147E | CB588 | 41 |
| D196R/ K197M/ K199E | D60R/K60aM/K60cE | CB589 | 42 |
| D196R/K197M/K199E/ R290E | D60R/K60aM/K60cE/ R147E | CB590 | 43 |
| D196K/K197L | D60K/K60aL | CB610 | 139 |
| D196F/K197L | D60F/K60aL | CB612 | 140 |
| D196L/K197L | D60L/K60aL | CB611 | 141 |
| D196M/K197L | D60M/K60aL | CB613 | 142 |

(continued)

| Modification - mature FVII numbering | Modification - chymotrypsin numbering | Polypeptide ID No. | SEQ ID NO |
|---|---|---|---|
| D196W/K197L | D60W/K60aL | CB614 | 143 |
| D196F/K197E | D60F/K60aE | CB615 | 144 |
| D196W/K197E | D60W/K60aE | CB616 | 145 |
| D196V/K197E | D60V/K60aE | CB617 | 146 |
| K197E/K341Q | K60aE/K192Q | CB637 | 229 |
| K197L/K341Q | K60aL/K192Q | CB638 | 230 |
| G237V/K341Q | G97V/K192Q | CB670 | 231 |
| K197E/G237V/K341Q | K60aE/G97V/K192Q | CB671 | 235 |
| K197E/K199E | K60aE/K60cE | CB688 | 232 |
| K197E/G237V | K60aE/G97V | CB689 | 233 |
| K199E/K341Q | K60cE/K192Q | CB694 | 234 |
| K197E/K199E/K341Q | K60aE/K60cE/K192Q | CB691 | 250 |

The FVII polypeptides containing one or more amino acid substitutions at the above-identified residues also can contain additional mutations, such as those described in International Patent Publication No. WO2004/083361, Neuenschwander et al., (1995) Biochemistry 34:8701-8707, Chang et al., (1999) Biochemistry 38:10940-10948, and Iakhiaev et al., (2001) Thromb. Haemost. 85:458-463. Thus, in one embodiment, the FVII polypeptides provided herein can contain one or more amino acid substitutions at one or more amino acid residues of Q176, D196, K197, K199, G237, T239, R290, E296, K341 and Q366, corresponding to amino acid positions of a mature FVII polypeptide as set forth in SEQ ID NO:3. For example, the one or more additional mutations can include, but are not limited to, any one or more of Q176A, D196N, G237L, E296A, K341N, K341Q, K341E, Q366A, Q366E and Q366G.

**b. Antithrombin III (AT-III)**

Antithrombin III (also known as antithrombin or AT-III) is an important anticoagulant serpin (serine protease inhibitor). AT-III is synthesized as a precursor protein containing 464 amino acid residues (SEQ ID NO:122). In the course of secretion a 32 residue signal peptide is cleaved to generate a 432 amino acid mature human antithrombin (SEQ ID NO:123). The 58 kDa AT-III glycoprotein circulates in the blood and functions as a serine protease inhibitor (serpin) to inhibit a large number of serine proteases of the coagulation system. The principal targets of AT-III are thrombin and factor Xa, although AT-III also has been shown to inhibit the activities of FIXa, FXIa, FXIIa and, to a lesser extent, FVIIa. The action of AT-III is greatly enhanced by glycosaminoglycans, such as the naturally occurring heparan sulphate or the various tissue-derived heparins that are widely used as anticoagulants in clinical practice. AT-III binds in a highly specific manner to a unique pentasaccharide sequence in heparin that induces a conformational change in the reactive center loop. In such a conformation, the reactive center loop of AT-III can more efficiently interact with the reactive site of the serine protease, and effect inhibition.

AT-III is not normally inhibitory to free plasma FVIIa, even in the presence of heparin, likely due to the zymogen-like conformation of FVIIa which prevents efficient interaction with AT-III. The inhibitory effects of AT-III do increase, however, once FVIIa complexes with TF. Binding of AT-III to theTF/FVIIa complex can release FVIIa from TF and maintains it in an inactive complex with AT-III. The increased affinity of AT-III for TF-bound FVIIa compared with FVIIa alone presumably reflects the maturation of the active site of FVIIa when it is complexed with TF, therefore making it amenable to AT-III binding (Rao et al. (1993) Blood 81:2600-2607). Thus, the impact of AT-III on FVIIa is proportional to the intrinsic activity of the FVIIa molecule itself. While FVIIa retains its zymogen-like conformation, AT-III has little effect. If, however, FVIIa changes conformation to a more active form, such as by binding TF, or by specific *in vitro* modifications, AT-III inhibition increases significantly. FVIIa polypeptides that are modified to have increased intrinsic activity often display simultaneous increases in susceptibility to AT-III inhibition. For example, modification of one or more amino acids in the activation pocket of FVIIa, such as by amino acid replacements corresponding to K337A, L305V, M298Q, V158D and E296V substitutions (relative to the mature FVII sequence set forth in SEQ ID NO:3), results in increased sensitivity to of the FVIIa polypeptide to AT-III thereby inhibiting FVIIa activity by up to 90% (Persson et al.

(2001) PNAS 98:13583-13588). In another example, induction of a more zymogen-like conformation by modification of amino acids involved in an α-helix of FVIIa, while increasing the activity of the modified FVIIa protein, also increases its susceptibility to AT-III (Persson et al. (2004) Biochem J 379:497-503).

**Modifications to effect increased resistance to AT-III**

Modifications can be made to a FVII polypeptide that increase its resistance to inhibition by AT-III. Generally, such modified FVII polypeptides retain at least one activity of a FVII polypeptide. Typically, such modifications include one or more amino acid substitutions at any position of the FVII polypeptide that are directly involved in the interaction of FVIIa (or the TF/FVIIa complex) with AT-III or in other residues that affect the position or conformation of residues that are directly involved in interactions between FVIIa (or the TF/FVIIa complex) and AT-III. Modified FVII polypeptides that have increased resistance for AT-III can exhibit a reduction in the extent of inhibition under specified conditions or in the second order rate constant for inhibition by AT-III by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the extent of inhibition or the second order rate constant for inhibition of unmodified or wild-type FVII polypeptide either *in vivo, ex vivo*, or *in vitro.* The modified FVII polypeptides are therefore resistant to the naturally inhibitory effects of AT-III with respect to coagulation initiation. When evaluated in an appropriate *in vitro* or *ex vivo* assay, or *in vivo,* such as following administration to a subject as a pro-coagulant therapeutic, the modified AT-III-resistant FVII polypeptides display increased coagulant activity as compared with unmodified FVII polypeptides.

As described herein below, one of skill in the art can empirically or rationally design modified FVII polypeptides that display increased resistance to AT-III. Such modified FVII polypeptides can be tested in assays known to one of skill in the art to determine if such modified FVII polypeptides display increased resistance to AT-III. For example, the second order rate constant of inhibition by AT-III can be measured for such modified FVIIa polypeptides. Generally, a modified FVII polypeptide that has increased resistance to AT-III will exhibit decreased binding under specified conditions (e.g, following injection of a fixed amount of protein into a patient) and/or decreased inhibition by AT-III. Typically, such assays are performed on a two-chain form of FVII, such as the activated form of FVII (FVIIa). Further, assays to determine effects of AT-III are generally performed in the presence of heparin and the presence of tissue factor, although such assays also can be performed in the absence of one or both cofactors.

Modified FVII polypeptides that have increased resistance for AT-III can exhibit a reduction in the extent of inhibition under specified conditions or in the second order rate constant for inhibition by AT-III by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the extent of inhibition or the second order rate constant for inhibition of unmodified or wild-type FVII polypeptide either *in vivo* or *in vitro.* Such resulting modified FVII polypeptides that have increased resistance for AT-III can exihibit a reduction in binding and/or affinity for AT-III by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the binding and/or affinity of unmodified or wild-type FVII polypeptide either *in vivo* or *in vitro.* Increased resistance to AT-III by such modified FVII polypeptides also can be manifested as increased coagulation activity in the presence of AT-III. The coagulation activity of the AT-III-modified FVII polypeptides can be increased by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the coagulation activity of unmodified or wild-type FVII polypeptide either in *vivo, ex vivo* or *in vitro.*

**2. Binding to activated platelets**

Modifications also can be made to a FVII polypeptide that increase its coagulation activity during TF-dependent and/or TF-independent initiation of coagulation. This proposed mechanism of TF-independent coagulation initiation involves direct activation of FX and FIX by FVIIa (that is not in a complex with TF), which is effected on the surface of an activated platelet. FVIIa binds activated platelets through an interaction between residues in the Gla domain of the FVIIa polypeptide, and phosphatidylserines and other negatively-charged phospholipids expressed on the platelet surface. This interaction is relatively weak, and likely does not play a significant role the initial burst of thrombin production normally observed during coagulation, which is very likely a result of TF-dependent activity. As discussed above, the TF-independent initiation of coagulation and the relatively weak interaction of FVIIa with the platelet surface could account for the requirement for administration of high doses of rFVIIa to achieve efficacy in the clinic. For example, only about 1% of the 10nM FVII in plasma is present as TF/FVIIa, but the therapeutic dose of rFVIIa is 90 μg/kg or about 50 nM in plasma, far above this level. Thus, high doses of rFVIIa compensate for the low-affinity binding of rFVIIa to activated platelets, resulting in sufficient coagulant activity of the therapeutic during treatment. Thus, of therapeutic interest are modified FVII polypeptides that can be administered at lower dosages due to increased binding and/or affinity for activated platelets, sufficient to effect the initiation of coagulation. Modified FVII polypeptides provided herein are designed to exhibit increased binding and/or affinity for activated platelets through modification of the Gla domain.

Interaction of residues in the γ-carboxylated Gla domain of vitamin K-dependent plasma proteins, such as FVII, FIX, FX, prothrombin, protein C and protein S, and negatively charged phospholipids on the membrane surface is important for hemostasis. The Gla domains of vitamin K-dependent plasma proteins typically contain approximately 45 amino acids, of which 9 to 12 glutamic acid residues are post-translationally modified by vitamin K-dependent carboxylation to form γ-carboxyglutamate (Gla). The amino acids that form the Gla domain are positioned immediately after those that form the signal peptide and propeptide of the proteins, and are therefore situated at the N-terminus following processing and cleavage of the precursor polypeptides to the mature proteins. For example, the amino acids that form the Gla domain in FVII are at positions 39-83 of the precursor polypeptide set forth in SEQ ID NO:1, positions 61-105 of the precursor polypeptide set forth in SEQ ID NO: 2, and positions 1 to 45 of the mature polypeptide set forth in SEQ ID NO:3. Of these, the 10 glutamic acid residues at positions E6, E7, E14, E19, E20, E25, E26, E29 and E35 of the mature FVII polypeptide set forth in SEQ ID NO: 3 are modified by carboxylation to generate γ-carboxyglutamate (Gla) residues.

Because glutamic acid is only a weak $Ca^{2+}$ chelator and γ-carboxyglutamate is a much stronger one, this modification step by the vitamin K carboxylase significantly increases the $Ca^{2+}$ binding affinity of the Gla domain of the protein. Calcium also binds the protease domain of FVIIa, where it facilitates conformational changes required for optimal activity. Through binding to sites in both the Gla and the protease domains of FVIIa, calcium enhances binding to TF arid phospholipids as well as the catalytic efficiency for activation of FX. The γ-carboxylated Gla domain binds seven $Ca^{2+}$ ions with variable affinity, which induces the conformational change that enables the Gla domain to interact with the C-terminal domain of TF. $Ca^{2+}$ binding also promotes interaction of FVIIa with negatively charged phospholipids on the platelet membrane, the bulk of which are phosphatidylserines. Phosphatidylserine (PS) is a key component in the negatively charged membrane that supports blood coagulation. In most cells, PS is present in the inner leaflet of the cell membrane, and therefore not exposed to plasma. Specific cellular processes, such as activation of platelets, results in translocation of PS to the outer, plasma-oriented surface, presenting a binding site for the Gla domain of vitamin K-dependent proteins (Hemker et al. (1983) Blood Cells 9:303-317).

In addition to its involvement in the interactions of FVIIa with TF and activated platelets, the Gla domain of FVIIa also is implicated in the binding and activation of FX. This can be through an indirect mechanism, in which the FVIIa Gla domain helps to align $Lys^{165}$ and $Lys^{166}$ of TF with the Gla domain of FX, an important step in activation of FX to FXa. Alternatively, the Gla domain of FVIIa interacts directly with the Gla domain of FX to correctly align the enzyme and substrate prior to activation (Neuenschwander et al. (1994) J Biol Chem 269:8007-8013, Huang et al. (1996) J Biol Chem 271:21752-21757). The arginine at position 36 of FVIIa has been shown to be important for effective docking of the FX Gla-domain in the absence of phospholipid, demonstrating that the Gla-domain of FVIIa participates in protein-protein interactions with FX (Ruf et al. (1999) Biochemistry 38:1957-1966).

Although the Gla domains of the different vitamin K-dependent plasma proteins display significant homology, they bind negatively charged phospholipid membranes with very different affinity, with dissociation constants ($K_d$'s) varying by more than 1000-fold. Human FVII displays one of the lowest affinities for phospholipid membranes among these proteins (McDonald et al. (1997) Biochemistry 36:5120-5127). The precise interactions responsible for binding of the Gla domain to PS are not fully understood, although it seems likely that individual amino acid residues within this domain contribute to the different affinities observed. Earlier studies indicated that there was a correlation between amino acids at positions 10, 32 and 33 (relative to the mature FVII protein) and overall membrane affinity (McDonald et al. (1997) Biochemistry 36:5120-5127). Subsequent mutation analysis also has revealed that specific residues contribute to binding affinity. For example, replacement of the histidine at position 10 of the mature form of protein C with a proline resulted in a 3-fold decrease in membrane affinity (Shen et al. (1997) Biochemistry 36:16025-16031). Conversely, mutation of specific residues in the Gla domain of the vitamin K-dependent plasma proteins can produce proteins with higher membrane affinity, and enhanced function. The effects of mutation at certain positions within the Gla domain, however, are not necessarily conserved among the different proteins. For example, mutation of bovine protein C by substitution of Q32E and N33D mediated a large increase in membrane binding, while the corresponding mutations in human protein C showed minimal changes in binding. Analogous mutation of human FVII (K32E) resulting in a 13-fold increase in binding affinity compared with the wild-type FVII protein (Harvey et al. (2003) J Biol Chem 278:8363-8369).

### a. Modification by introduction of a heterologous Gla domain

Due to its relatively low binding affinity for activated platelets, the Gla domain of FVII is a target for modification, with the aim of enhancing the interaction between the modified FVII and the phospholipid membrane, thereby increasing coagulation activity. Modification can be effected by substitution of specific amino acids that are involved in this interaction (*see, e.g.*, Shah et al. PNAS 95: 4429-4234, Harvey et al. (2003) J Biol Chem 278:8363-8369). Alternatively, modification can be effected by substitution of the entire Gla domain with the Gla domain of another vitamin K-dependent protein *i.e.* Gla domain swap. This type of modification results in a chimeric protein, such as that which resulted when the Gla domain of protein C was replaced with the Gla domain of FVII (Geng et al. (1997) Thromb Haemost 77:926-933).

Typically, such modification includes introduction, such as by addition or substitution, of a heterologous Gla domain, or a sufficient portion thereof to effect phospholipids binding into a region of the FVII polypeptide to generate a chimeric modified FVII polypeptide. Generally, such a chimeric FVII polypeptide retains at least one activity of FVII. The binding and/or affinity of Gla-modified FVII polypeptides for activated platelets can be increased by at least about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the binding and/or affinity of unmodified or wild-type FVII polypeptide either *in vivo* or *in vitro.* The binding and/or affinity for activated platelets by modified FVII polypeptides also can be manifested as increased coagulation activity. The coagulation activity of the Gla-modified FVII polypeptides can be increased by at least or about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the coagulation activity of unmodified or wild-type FVII polypeptide either *in vivo* or *in vitro.*

A Gla domain, or sufficient portion thereof, contained within any polypeptide can be used as a source of a heterologous Gla domain for introduction or replacement of a region of a FVII polypeptide. Typically, such a heterologous Gla domain exhibits binding affinity for phospholipids, for example, phospholipids present on the surface of an activated platelet. Generally, the choice of a heterologous Gla domain is one which exhibits higher affinity for phospholipids as compared to the affinity of the Gla domain of FVII. The exact Gla domain, or sufficient portion thereof, used as a heterologous domain for modification of a FVII polypeptide can be rationally or empirically determined. Exemplary of other Gla-containing polypeptides include, but are not limited to, FIX, FX, prothrombin, protein C, protein S, osteocalcin, matrix Gla protein, Growth-arrest-specific protein 6 (Gas6), and protein Z. The Gla domains of these exemplary proteins are set forth in any of SEQ ID NOS: 110-118, 120 and 121. For example, 2, 3, 4, 5, 6, 7, 8, 9, 10, 20, 30, 40 or more contiguous amino acids, or the entire Gla domain, of a heterologous Gla domain can be introduced into a FVII polypeptide. In addition, introduction of the Gla domain into a FVII polypeptide also can include additional amino acids not part of the Gla domain of the heterologous polypeptide so long as the additional amino acids do not significantly weaken the phospholipid binding ability of the introduced Gla domain.

In some examples, the introduction is by addition of the Gla domain to the FVII polypeptide such that the heterologous Gla domain is inserted into the endogenous Gla domain or into another region or domain of the FVII polypeptide so long as the modified FVII polypeptide retains at least one activity of FVII. In such examples, the native Gla domain of the FVII polypeptide is retained in the polypeptide, although in some instances the amino acid sequence that make up the native Gla domain is interrupted. In other examples, the heterologous Gla domain, or a sufficient portion thereof, is inserted adjacent to, either on the N- or C- terminus, of the native Gla domain such that the native Gla domain is not interrupted. In an additional example, the heterologous Gla domain, or a sufficient portion thereof, is inserted into another domain of the FVII polypeptide.

Also provided herein are modified Gla-domain FVII polypeptides where all or a contiguous portion of the endogenous Gla domain of FVII is removed and is replaced with a heterologous Gla domain, or a sufficient portion thereof to effect phospholipid binding, so long as the modified FVII polypeptide retains at least one activity of FVII. Such modification also is referred to as a Gla domain swap. For example, all or a portion of the Gla domain of FVII, such as is set forth in SEQ ID NO:119, corresponding to amino acids 1-45 of the sequence of amino acids set forth in SEQ ID NO: 3, can be removed from a FVII polypeptide and replaced with a heterologous Gla domain, or a sufficient portion thereof. The heterologous portion includes any Gla domain known to one in the art that binds to phospholipids, including, but not limited to, a Gla domain having the sequence of amino acids set forth in any of SEQ ID NOS: 110-118, 120 and 121. In some cases, a sufficient portion of a Gla domain, such as a sufficient portion of any of SEQ ID NOS: 110-118, 120 and 121 to effect phospholipids binding, can replace the endogenous Gla domain, or a portion of the endogenous Gla domain, of FVII. For example, the Gla domain of FVII can be swapped with the Gla domain of Protein C set forth in SEQ ID NO:113. The resulting modified FVII polypeptide contains a Gla domain from protein C followed by its own EGF-1, EGF-2 and serine protease domain.

In some examples, the heterologous Gla domain contains mutations that further effect increased binding to activated platelets, due to increased phospholipids binding. For example, if the protein C Gla domain is introduced to generate a modified FVII polypeptide, the protein C Gla domain can contain amino acid mutations that confer increased phospholipid binding.

In other examples, the heterologous Gla domain can contain further mutations that confer FVII-like functions to the Gla domain. For example, as noted above, R36 of the FVII Gla domain set forth in SEQ ID NO:119 can be involved in interactions with FX. Hence, the heterologous Gla domain can contain further modifications, such as any required to maintain an arginine at position 36 of the mature FVII polypeptide, as set forth in SEQ ID NO:3, or any other modifications required to maintain FX-activation properties of the modified FVIIa polypeptide (Ruf et al. (1999) Biochem 38:1957-1966). Thus, in some examples, a corresponding mutation to R36 can be made in the heterologous Gla domain. The corresponding position can be determined by one of skill in the art, such as by alignment of amino acid sequences.

As described herein below, one of skill in the art can empirically or rationally design modified FVII polypeptides containing a heterologous Gla domain such that the resulting FVII polypeptide retains at least one FVII activity. Typically, such modified FVII polypeptides retain their ability to bind and activate FX. In some instances, such modified FVII polypeptides also retain their ability to bind and activate FIX. The resulting Gla- modified FVII polypeptides include those that retain their ability to bind to TF. The Gla-swapped modified FVII polypeptides also include those having increased intrinsic activity in the absence of TF. Hence, the resulting Gla-modified FVII polypeptides include those that do not bind TF or that bind TF poorly compared with wild type FVIIa. Typically, such modified FVII polypeptides display increased phospholipid binding. In particular, such modified FVII polypeptides exhibit increased binding to phosphatidylserine. The increased binding to phospholipids can be assayed on isolated phospholipids or on the surface of activated platelets. Such assays are typically performed on a two-chain form of FVII, such as the activated form of FVII (FVIIa).

Provided herein are modified FVII polypeptides that contain a heterologous Gla domain. Exemplary of such modified FVII polypeptides are those in which the endogenous Gla domain is replaced with all or a portion of the Gla domain of any one of FIX (SEQ ID NO:110), FX (SEQ ID NO:111), thrombin (SEQ ID NO:112), Protein C (SEQ ID NO:113) or Protein S (SEQ ID NO:114). Such modified FVII polypeptides can exhibit increased binding to activated platelets, resulting in increased coagulant activity. Table 7 sets forth exemplary modifications that can be made to a FVII polypeptide to replace the endogenous Gla domain with that of FIX, FX, protein C, protein S or thrombin. The table provides the name of the modification (e.g. Gla Swap FX) and the details of the modification, including the amino acid poisitions at which the exogenous Gla domain is inserted in the FVII polypeptide, and the sequence of the exogenous Gla domain. The sequence identifier (SEQ ID NO) also is provided in which exemplary amino acid sequences of the modified FVII polypeptide are set forth, and also any polypeptide identification numbers. For example, the "Gla swap FIX" modification (A1Y44delinsYNSGKLEEFVQGNLERECMEEKCSFEEAREVFENTERTTEFWK QY) involves deletion of the endogenous FVII Gla domain by deleting amino acid residues A1 to Y44 (residues corresponding to a mature FVII polypeptide set forth in SEQ ID NO:3) and insertion of 45 amino acid residues that correspond to amino acid residues Y1 to Y45 of the FIX Gla domain set forth in SEQ ID NO: 110. Similarly, the Gla Swap FX modification involves deletion of amino acid residues A1 to Y44 (residues corresponding to a mature FVII polypeptide set forth in SEQ ID NO:3) and insertion of 44 amino acid residues that correspond to A1 to Y44 of the FX Gla domain set forth in SEQ ID NO:111. The Gla Swap Thrombin modification involves deletion of amino acid residues A1 to Y44 (residues corresponding to a mature FVII polypeptide set forth in SEQ ID NO:3) and insertion of 44 amino acid residues that correspond to amino acid residues Y1 to Y44 of the Thrombin Gla domain set forth in SEQ ID NO:112. The Gla Swap Protein C modification involves deletion of amino acid residues A1 to Y44 (residues corresponding to a mature FVII polypeptide set forth in SEQ ID NO:3) and insertion of 44 amino acid residues that correspond to amino acid residues A1 to H44 of the Protein C Gla domain set forth in SEQ ID NO:113. The Gla Swap Protein S modification involves deletion of amino acid residues A1 to Y44 (residues corresponding to a mature FVII polypeptide set forth in SEQ ID NO:3) and insertion of 44 amino acid residues that correspond to amino acid residues Y1 to Y44 of the Protein S Gla domain set forth in SEQ ID NO:114.

Table 7.

| Modification name | Modification - mature FVII Numbering | Modification - chymotrypsin numbering | Poly peptide ID No. | SEQ ID NO |
|---|---|---|---|---|
| Gla swap FIX | A1Y44delinsYNSGKLEEFVQ GNLERECMEEKCSFEEARE VFENTERTTEFWKQY | A[1]Y[44]delinsYNSGKLEEF VQGNLERECMEEKCSFEEA REVFENTERTTEFWKQY | CB728 | 236 |
| Gla swap FX | A1Y44delinsANSFLEEMKKG HLERECMEETCSYEEAREV FEDSDKTNEFWNKY | A[1]Y[44]delinsANSFLEEMK KGHLERECMEETCSYEEAR EVFEDSDKTNEFWNKY | CB729 | 237 |
| Gla Swap Prot C | A1Y44delinsANSFLEELRHSS LERECIEEICDFEEAKEIFQN VDDTLAFWSKH | A[1]Y[44]delinsANSFLEELR HSSLERECIEEICDFEEAKEI FQNVDDTLAFWSKH | CB730 | 238 |
| Gla Swap Prot S | A1Y44delinsANSLLEETKQG NLERECIEELCNKEEAREVF ENDPETDYFYPKY | A[1]Y[44]delinsANSLLEETK QGNLERECIEELCNKEEARE VFENDPETDYFYPKY | CB731 | 239 |
| Gla swap Thrombin | A1Y44delinsANTFLEEVRKG NLERECVEETCSYEEAFEAL ESSTATDVFWAKY | A[1]Y[44]delinsANTFLEEVR KGNLERECVEETCSYEEAF EALESSTATDVFWAKY | CB732 | 240 |

Modified FVII polypeptides containing a heterologous Gla domain can exhibit increased coagulant activity at lower dosages as compared to a wild-type FVII molecule, such as NovoSeven®, due to increased binding and/or affinity for activated platelets. The coagulation activity of the Gla-modified FVII polypeptides can be increased by at least or about 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the coagulation activity of unmodified or wild-type FVII polypeptide either *in vivo, ex vivo* or *in vitro*.

### 3. Combinations and Additional Modifications

In addition to modification of FVII polypeptides to have increased TFPI-resistance, increased resistance to AT-III, increased TF-dependent and/or TF-independent catalytic activity, improved pharmacokinetic properties, such as increased half-life, and/or increased binding and/or affinity for phospholipid membranes, modified FVII polypeptides provided herein also include those that exhibit more than one of the above-noted properties. For example, a FVII polypeptide can be modified such that the resulting polypeptide displays increased binding and/or affinity for phospholipids and also displays increased resistance to TFPI. Accordingly, a FVII polypeptide can be modified by introduction of a heterologous Gla domain from another vitamin K-dependent plasma protein and by substitution of any one or more of the amino acids at positions 196, 197, 199, 237, 239, 290 or 341 relative to the mature FVII polypeptide set forth in SEQ ID NO:3.

Further, any modified FVII polypeptide provided herein also can contain one or more other modifications described in the art. Typically, such additional modifications are those that themselves result in an increased coagulant activity of the modified polypeptide and/or and increased stability of the polypeptide. Accordingly, the resulting modified FVII polypeptides exhibit an increased coagulant activity. The additional modifications can include, for example, any amino acid substitution, deletion or insertion known in the art, typically any that increases the coagulant activity and/or stability of the FVII polypeptide. Any modified FVII polypeptide provided herein can contain 1, 2, 3, 4, 5, 6, 7, 8, 9, 10, 11, 12, 13, 14, 15, 16, 17, 18, 19, 20 or more additional amino acid modifications, so long as the resulting modified FVII polypeptide retains a FVII activity of the wild-type or unmodified polypeptide.

In one example, the additional modification can be made to the FVII polypeptide sequence such that its interaction with other factors, molecules and proteins is altered. For example, the amino acid residues that are involved in the interaction with tissue factor (TF) can be replaced such that the affinity of the modified FVII polypeptide for TF is increased. Other modifications include, but are not limited to, modification of amino acids that are involved in interactions with factor X, factor IX, TFPI and AT-III.

Additional modifications also can be made to a modified FVII polypeptide provided herein that alter the conformation or folding of the polypeptide. These include, for example, the replacement of one or more amino acids with a cysteine such that a new disulphide bond is formed, or modifications that stabilize an $\alpha$-helix conformation, thereby imparting increased activity to the modified FVII polypeptide.

Additional modifications also can be made to the FVII polypeptide to effect post-translational modifications. For example, the polypeptide can be modified to include additional glycosylation sites such that the resulting modified FVII polypeptide has increased glycosylation compared to an unmodified FVII polypeptide. Modifications also can be made to introduce amino acid residues that can be subsequently linked to a chemical moiety, such as one that acts to increase stability of the modified FVII polypeptide. A FVII polypeptide can also be altered by modifying potential cleavage site(s) for endogeneous proteases, thereby decreasing degradation of the FVIIa variant and increasing the stability of the modified FVII polypeptide.

Additionally, amino acids substitutions, deletions or insertions can be made in the endogenous or heterologous Gla domain such that the modified FVII polypeptide displays increased binding and/or affinity for phospholipid membranes. In other examples, the modified FVII polypeptides provided herein can display deletions in the endogenous Gla domain, or substitutions in the positions that are normally gamma-carboxylated (US20070037746).

The following sections describe non-limiting examples of exemplary modifications described in the art to effect increased stability and/or coagulant activity of a FVII polypeptide. As discussed above, such modifications also can be additionally included in any modified FVII polypeptide provided herein. The amino acid positions referenced below correspond to the mature FVII polypeptide as set forth in SEQ ID NO:3. Corresponding mutations can be made in other FVII polypeptides, such as allelic, species or splices variants of the mature FVII polypeptide set forth in SEQ ID NO:3.

#### a. Modifications that increase intrinsic activity

In one example, additional modifications can be made to a modified factor VII polypeptide provided herein that result in increased catalytic activity toward factor X and/or Factor IX. For example, modifications can be made to the amino acids that are involved in the interaction with its cofactor, TF, such that the resulting modified FVII polypeptide has increased affinity for TF, and thereby displays increased activity toward FX and/or FIX. Modifications also can be made to the activation pocket of the FVII polypeptide, such that the intrinsic activity of the modified FVII polypeptide toward FX and/or FIX is increased compared to the activity of the unmodified polypeptide. Another modification strategy that results in increased activity involves modification of the FVII polypeptide such that the folding and conformation of the protein is altered to a more active form or an equilibrium between highly active and inactive (or less active) conformations is shifted in favor of the highly active conformation(s). A more active polypeptide also can be achieved by modification of the amino acids involved in the β-strands of the FVII polypeptide. For example, amino acid substitutions can be made that introduce new cysteine pairs that can form new disulphide bonds which can function to "lock" the modified FVII polypeptide into a more active form.

Examples of additional modifications that can be included in the modified FVII polypeptides provided herein to increase the intrinsic activity of the modified FVII polypeptide include, but are not limited to, those described in Persson et al. (2004) Biochem J. 379:497-503, Maun et al. (2005) Prot Sci 14:1171-1180, Persson et al. (2001) PNAS 98:13583-13588, Persson et al. (2002) Eur J Biochem 269:5950-5955, Soejima et al. (2001) J Biol Chem 276:17229-17235, Soejima et al. (2002) J Biol Chem 277:49027-49035, WO200183725, WO2002022776, WO2002038162, WO2003027147, WO200338162, WO2004029090, WO2004029091, WO2004108763 and WO2004111242. Non-limiting examples of exemplary amino acid modifications described in the art that can result in increased intrinsic activity of the modified FVII polypeptide include any one or more of S278C/V302C, L279C/N301C, V280C/V301C, S281C/V299C, S314E, L39E, L39Q, L39H, I42R, S43Q, S53N, K62E, K62R, K62D, K62N, K62Q, K62T, L65Q, L65S, F71D, F71Y, F71E, F71Q, F71N, P74S, P74A, A75E, A75D, E77A, E82Q, E82N, T83K, E116D, K157V, K157L, K157I, K157M, K157F, K157W, K157P, K157G, K157S, K157T, K157C, K157Y, K157N, K157E, K157R, K157H, K157D, K157Q, V158L, V158I, V158M, V158F, V158W, V158P, V158G, V158S, V158T, V158C, V158Y, V158N, V158E, V158R, V158K, V158H, V158D, V158Q, A274M, A274L, A274K, A274R, A274D, A274V, A274I, A274F, A274W, A274P, A274G, A274T, A274C, A274Y, A274N, A274E, A274H, A274S, A274Q, F275H, E296V, E296L, E296I, E296M, E296F, E296W, E296P, E296G, E296S, E296T, E296C, E296Y, E296N, E296K, E296R, E296H, E296D, E296Q, M298Q, M298V, M298L, M298I, M298F, M298W, M298P, M298G, M298S, M298T, M298C, M298Y, M298N, M298K, M298R, M298H, M298E, M298D, R304Y, R304F, R304L, R304M, L305V, L305Y, L305I, L305F, L305A, L305M, L305W, L305P, L305G, L305S, L305T, L305C, L305N, L305E, L305K, L305R, L305H, L305D, L305Q, M306D, M306N, D309S, D309T, S314A, S314V, S3141, S314M, S314F, S314W, S314P, S314G, S314L, S314T, S314C, S314Y, S314N, S314E, S314K, S314R, S314H, S314D, S314Q, D334G, D334E, D334A, D334V, D334I, D334M, D334F, D334W, D334P, D334L, D334T, D334C, D334Y, D334N, D334K, D334R, D334H, D334S, D334Q, S336G, S336E, S336A, S336V, S336I, S336M, S336F, S336W, S336P, S336L, S336T, S336C, S336Y, S336N, S336K, S336R, S336H, S336D, S336Q, K337L, K337V, K3371, K337M, K337F, K337W, K337P, K337G, K337S, K337T, K337C, K337Y, K337N, K337E, K337R, K337H, K337D, K337Q, F374P, F374A, F374V, F3741, F374L, F374M, F374W, F374G, F374S, F374T, F374C, F374Y, F374N, F374E, F374K, F374R, F374H, F374D, F374Q, and substitution of positions 300-322, 305-322, 300-312, or 305-312 with the corresponding amino acids from trypsin, thrombin or FX, and substitution of positions 310-329, 311-322 or 233-329 with the corresponding amino acids from trypsin.

### b. Modifications that increase resistance to proteases

Modified FVII polypeptides provided herein also can contain additional modifications that result in increased resistance of the polypeptide to proteases. For example, amino acid substitutions can be made that remove one or more potential proteolytic cleavage sites. The modified FVII polypeptides can thus be made more resistant to proteases, thereby increasing the stability and half-life of the modified polypeptide.

Examples of additional modifications that can be included in the modified FVII polypeptides provided herein to increase resistance to proteases include, but are not limited to, those described in United States Patent No. US5580560 or International Published Application Nos. WO1988010295 and WO2002038162. Non-limiting examples of exemplary modifications described in the art that can result in increased resistance of the modified FVII polypeptide to inhibitors and/or proteases include any one or more of; K32Q, K32E, K32G, K32H, K32T, K32A, K32S, K38T, K38D, K38L, K38G, K38A, K38S, K38N, K38H, I42N, I42S, I42A, I42Q, Y44N, Y44S, Y44A, Y44Q, F278S, F278A, F278N, F278Q, F278G, R290G, R290A, R290S, R290T, R290K, R304G, R304T, R304A, R304S, R304N, R315G, R315A, R315S, R315T, R315Q, Y332S, Y332A, Y332N, Y332Q, Y332G, k341E, K341Q, K341G, K341T, K341A and K341S.

### c. Modifications that increase affinity for phospholipids

The coagulant activity of FVII can be enhanced by increasing the binding and/or affinity of the polypeptide for phospholipids, such as those expressed on the surface of activated platelets. For example, additional amino acid substitutions can be made at particular positions in the endogenous Gla domain of a FVII polypeptide that result in a modified FVII polypeptide with increased ability to bind phosphatidylserine and other negatively charged phospholipids. Thus, such modifications also can be included in a modified FVII polypeptide provided herein, provided the such modified FVII polypeptides possess an endogenous Gla domain, *i.e.* have not already been subjected to modification resulting in substitution of the endogenous Gla domain with that of another vitamin K-dependent protein.

Examples of additional modifications to increase phospholipid binding and/or affinity and that can be made to a modified FVII polypeptide provided herein that contains an endogenous FVII Gla domain, include, but are not limited to, those described in Harvey et al. (2003) J Biol Chem 278:8363-8369, US20030100506, US20040220106, US20060240526, US6017882, US6693075, US6762286, WO200393465 and WO2004111242. Exemplary of such modifications include any one or more of; an insertion of a tyrosine at position 4, or modification of any one or more of P10Q, P10E, P10D, P10N, R28F, R28E, K32E, K32D, D33F, D33E, D33K A34E, A34D, A34I, A34L, A34M, A34V, A34F, A34W, A34Y, R36D, R36E, K38E and K38D.

### d. Modifications that alter glycosylation

Alteration of the glycosylation levels of a protein has been described in the art as a means to reduce immunogenicity, increase stability, reduce the frequency of administration and/or reduce adverse side effects such as inflammation. Normally, this is effected by increasing the glycosylation levels. The glycosylation site(s) provides a site for attachment for a carbohydrate moiety on the polypeptide, such that when the polypeptide is produced in a eukaryotic cell capable of glycosylation, it is glycosylated.

There are four naturally occurring glycosylation sites in FVII; two N-glycosylation sites at N145 and N322, and two O-glycosylation sites at S52 and S60, corresponding to amino acid positions in the mature FVII polypeptide set forth in SEQ ID NO:3. In one embodiment, additional modifications can be made to a modified FVII polypeptide provided herein such that glycosylation at the above sites is disrupted. This can result in a modified FVII polypeptide with increased coagulant activity (*see, e.g.*, WO2005123916). Non-limiting examples of exemplary modifications described in the art that can result in decreased glycosylation and increased activity of the modified FVII polypeptide as compared to an unmodified FVII polypeptide include, but are not limited to; N145Y, N145G, N145F, N145M, N145S, N145I, N145L, N145T, N145V, N145P, N145K, N145H, N145Q, N145E, N145R, N145W, N145D, N145C, N322Y, N322G, N322F, N322M, N322S, N322I, N322L, N322T, N322V, N322P, N322K, N322H, N322Q, N322E, N322R, N322W and N322C.

In another embodiment, further modifications can be made to the amino acid sequence of the modified FVII polypeptides provided herein such that additional glycosylation sites are introduced, thus increasing the level of glycosylation of the modified FVII polypeptide as compared to an unmodified FVII polypeptide. The glycosylation site can be an N-linked or O-linked glycosylation site. Examples of modifications that can be made to a FVII polypeptide that introduce one or more new glycosylation sites include, but are not limited to, those that are described in US6806063 and WO200393465. Non-limiting examples of exemplary modifications described in the art that can result in increased glycosylation of the modified FVII polypeptide as compared to an unmodified FVII polypeptide include, but are not limited to; F4S, F4T, P10N, Q21N, W41N, S43N, A51N, G58N, L65N, G59S, G59T, E82S, E82T, N95S, N95T, G97S, G97T, Y101N, D104N, T106N, K109N, G117N, G124N, S126N, T128N, A175S, A175T, G179N, I186S, I186T, V188N, R202S, R202T, I205S, I205T, D212N, E220N, I230N, P231N, P236N, G237N, V253N, E265N, T267N, E270N, R277N, L280N, G291N, P303S, P303ST, L305N, Q312N, G318N, G331N, D334N, K337N, G342N, H348N, R353N, Y357N, I361N, V376N, R379N, M391N, K32N/A34S, K32N/A34T, F31N/D33S, F31N/D33T, I30N/K32S, 130N/K32T, A34N/R36S, A34N/R36T, K38N/F40S, K38N/F40T, T37N/L39S, T37N/L39T, R36N/K38S, R36N/K38T, L39N/W41S, L39N/W41T, F40N/I42S, F40N/142T, I42N/ Y44S, I42N/ Y44T, Y44N/ D46S, Y44N/ D46T, D46N/D48S, D46N/D48T, G47N/Q49S, G47N/Q49T, K143N/ N145S, K143N/ N145T, E142N/R144S, E142N/R144T, L141N/K143S, L141N/K143T, I140N/E142S/, I140N/E142T, R144N/A146S, R144N/A146T, A146N/K148S, A146N/K148T, S147N/P149S/, S147N/P149T, R290N/A292S, R290N/A292T, D289N/G291S, D289N/G291T, L288N/R290S, L288N/R290T, L287N/D289S, L287N/D289, A292N/A294S, A292N/A294T, T293N/L295S, T293N/L295T, R315N/V317S, R315N/V317T, S314N/K316S, S314N/K316T, Q313N/R315S, Q313N/R315T, K316N/G318S, K316N/G318T, V317N/D319S, V317N/D319T,K341N/ D343S, K341N/ D343T, S339N/K341S, S339N/K341T, D343N/G345S, D343N/G345T, R392N/E394S, R392N/E394T, L390N/ R392S, L390N/ R392T, K389N/M391S, K389N/M391T, S393N/P395S, S393N/P395T, E394N/R396S, E394N/R396T, P395N/P397S, P395N/P397T, R396N/G398S, R396N/G398T, P397N/V399S, P397N/V399T, G398N/L400S, G398N/L400T, V399N/L401S, V399N/L401T, L400N/R402S, L400N/R402T, L401N/A403S, L401N/A403T, R402N/P404S, R402N/P404T, A403N/F405S, A403N/F405T, P404N/P406S and P404N/P406T.

### e. Modifications to facilitate chemical group linkage

Additional modifications of a modified FVII polypeptide provided herein also can be made to facilitate subsequent linkage of a chemical group. One or more amino acid substitutions or insertions can be made such that a chemical group can be linked to a modified FVII polypeptide via the substituted amino acid. For example, a cysteine can be introduced to a modified FVII polypeptide, to which a polyethylene glycol (PEG) moiety can be linked to confer increased stability and serum half-life. Other attachment residues include lysine, aspartic acid and glutamic acid residues. In some embodiments, amino acids residues are replaced to reduce the number of potential linkage positions. For example, the number of lysines can be reduced. Examples of modifications that can be made to the amino acid sequence of a FVII polypeptide which can facilitate subsequent linkage with a chemical group include, but are not limited to, those that are described in US20030096338, US20060019336, US6806063, WO200158935 and WO2002077218. Non-limiting examples of exemplary modifications of a FVII polypeptides that can facilitate subsequent linkage with a chemical group include, but are not limited to; Q250C, R396C, P406C, I42K, Y44K, L288K, D289K, R290K, G291K, A292K, T293K, Q313K, S314K, R315K, V317K, L390K, M391K, R392K, S393K, E394K, P395K, R396K, P397K, G398K, V399K, L400K, L401K, R402K, A403K, P404K, F405K, I30C, K32C, D33C, A34C, T37C, K38C, W41C, Y44C, S45C, D46C, L141C, E142C, K143C, R144C, L288C, D289C, R290C, G291C, A292C, S314C, R315C, K316C, V317C, L390C, M391C, R392C, S393C, E394C, P395C, R396C, P397C, G398C, V399C, L401C, R402C, A403C, P404C, I30D, K32D, A34D, T37D, K38D, W41D, Y44D, S45D, D46C, L141D, E142D, K143D, R144D, L288D, R290D, G291D, A292D, Q313D, S314D, R315D, K316D, V317D, L390D, M391D, R392D, S393D, P395D, R396D, P397D, G398D, V399D, L401D, R402D, A403D, P404D, I30E, K32E, A34E, T37E, K38E, W41E, Y44E, S45E, D46C, L141E, E142E, K143E, R144E, L288E, R290E, G291E, A292E, Q313E, S314E, R315E, K316E, V317E, L390E, M391E, R392E, S393E, P395E, R396E, P397E, G398E, V399E, L401E, R402E, A403E, P404E, K18R, K32R, K38R, K62R, K85R, K109R, K137R, K143R, K148R, K157R, K161R, K197R, K199R, K316R, K337R, K341R, K389R, K18Q, K32Q, K38Q, K62Q, K85Q, K109Q, K137Q, K143Q, K148Q, K157Q, K161Q, K197Q, K199Q, K316Q, K337Q, K341Q, K389Q, K18N, K32N, K38N, K62N, K85N, K109N, K137N, K143N, K148N, K157N, K161N, K197N, K199N, K316N, K337N, K341N, K389N, K18H, K32H, K38H, K62H, K85H, K109H, K137H, K143H, K148H, K157H, K161H, K197H, K199H, K316H, K337H, K341H and K389H.

**f. Exemplary combination modifications**

Provided herein are modified FVII polypeptides that have two or more modifications designed to affect one or more properties or activities of an unmodified FVII polypeptide. In some examples, the two or more modifications alter two or more properties or activities of the FVII polypeptide. The modifications can be made to the FVII polypeptides such that one or more of resistance to TFPI, resistance to AT-III, intrinsic activity, amidolytic activity, catalytic activity (in the presence or absence of TF), phospholipid binding and/or affinity, glycosylation, resistance to proteases, half-life and interaction with and/or activation of other factors or molecules, such as FX and FIX, is altered. Typically, the two or more modifications are combined such that the resulting modified FVII polypeptide has increased coagulant activity, increased duration of coagulant activity, and/or an enhanced therapeutic index compared to an unmodified FVII polypeptide. The modified FVIIa polypeptide may exhibit a faster onset of coagulant activity either *in vitro, ex vivo,* or *in vivo.* The modifications can include amino acid substitution, insertion or deletion. The increased coagulant activity, increased duration of coagulant activity, increased onset of coagulant activity, and/or an enhanced therapeutic index of the modified FVII polypeptide containing two or more modifications can be increased by at least or about 1%, 2%, 3%, 4%, 5%, 6%, 7%, 8%, 9%, 10%, 20%, 30%, 40%, 50%, 60%, 70%, 80%, 90%, 100%, 110%, 120%, 130%, 140%, 150%, 160%, 170%, 180%, 190%, 200%, 300%, 400%, 500%, or more compared to the activity of the starting or unmodified FVIIa polypeptide.

Provided herein are modified FVII polypeptides that contain two or more modifications that are introduced into an unmodified FVII polypeptide to alter two or more activities or properties. The modified FVII polypeptides can contain 2, 3, 4, 5, 6 or more modifications. Further, each modification can involve one or more amino acid residues. For example, a modified FVII polypeptide can contain two modifications each of which is a single amino acid substitution. In another example, a modified FVII polypeptide can contain two modifications, one of which is a single amino acid substitution and the other of which involves deletion of more than one amino acid residue and then insertion of more than one amino acid residue. For example, a modified FVII polypeptide provided herein can contain the amino acid substitution M298Q (residues corresponding to a mature FVII polypeptide set forth in SEQ ID NO:3) to increase intrinsic activity and a Gla Swap FIX modification, which involves deletion of the endogenous FVII Gla domain by deleting amino acid residues A1 to Y44 (residues corresponding to a mature FVII polypeptide set forth in SEQ ID NO:3) and insertion of 45 amino acid residues that correspond to amino acid residues Y1 to Y45 of the FIX Gla domain set forth in SEQ ID NO:110.

Modified FVII polypeptides provided herein can have two or more modifications selected solely from those set forth in Tables 5 to 8. In other examples, the modified FVII polypeptide contains two or more modifications where one or more modifications are selected from those set forth in Tables 5 to 8 and one or more modifications are additional modifications that are not set forth in Tables 5 to 8, such as, for example, modifications described in the art. In some examples, the one or more additional modifications can be selected from those set forth in Section D.3.a-e, above. For example, a modified FVII polypeptide can contain a modification at one or more of amino acid residues D196, K197, K199, G237, T239, R290 or K341 based on numbering of a mature FVII set forth in SEQ ID NO:3 (corresponding to D60, K60a, K60c, G97, T99, R147 and K192, respectively, based on chymotrypsin numbering), which can increase resistance to TFPI, and a modification at one or more amino acid residues that affects intrinsic activity, such as, for example, V158 and M298, (V21 and M156, respectively, based on chymotrypsin numbering). For example, a modified FVII polypeptide can contain two amino acid substitutions that increase resistance to TFPI, such as K197E and G237V, and one amino acid substitution that increases intrinsic activity, such as M298Q, resulting in a FVII polypeptide with increased coagulant activity.

Non-limiting exemplary combination modifications are provided in Table 9. These exemplary combination modifications include two or more modifications that are designed to alter two or more activities or properties of a FVII polypeptide, including, but not limited to, resistance to TFPI, resistance to AT-III, intrinsic activity, amidolytic activity, catalytic activity (in the presence and/or absence of TF), phospholipid binding and/or affinity, glycosylation, resistance to proteases, half-life and interaction with and/or activation of other factors or molecules, such as FX and FIX. Modified FVII polypeptides containing such combination modifications can have increased coagulant activity, increased duration of coagulant activity, increased onset of coagulant activity and/or an enhanced therapeutic index. The modifications set forth in Table 8 below use the same nomenclature and numbering systems as described in Tables 5 to 7, above. For example, the "Gla Swap FIX" modification involves deletion of the endogenous FVII Gla domain by deleting amino acid residues A1 to Y44 (residues corresponding to a mature FVII polypeptide set forth in SEQ ID NO:3) and insertion of 45 amino acid residues that correspond to amino acid residues Y1 to Y45 of the FIX Gla domain set forth in SEQ ID NO:110, as described above. Amino acid positions correspond to amino acid positions of a mature FVII polypeptide as set forth in SEQ ID NO:3 and also are referred to by the chymotrypsin numbering scheme. In Table 9 below, the sequence identifier (SEQ ID NO) is identified in which exemplary amino acid sequences of the modified FVII polypeptide are set forth, and also any polypeptide identification numbers.

**Table 8**.

| Modification - mature FVII numbering | Modification - chymotrypsin numbering | Polypeptide ID No. | SEQ ID NO |
|---|---|---|---|
| V158D/G237V/E296V/M298Q | V21D/G97V/E154V/M156Q | CB669 | 241 |
| K197E/G237V/M298Q | K60aE/G97V/M156Q | CB690 | 242 |
| K197E/G237V/M298Q/K341Q | K60aE/G97V/M156Q/K192Q | CB692 | 243 |
| K197E/K199E/G237V/M298Q/ K341Q | K60aE/K60cE/G97V/M156Q/ K192Q | CB693 | 244 |
| G237V/M298Q | G97V/M156Q | CB695 | 245 |
| G237V/M298Q/K341Q | G97V/M156Q/K192Q | CB696 | 246 |
| M298Q/Gla Swap FIX | M156Q/Gla Swap FIX | CB850 | 247 |
| K197E/M298Q | K60aE/M156Q | CB902 | 248 |
| M298Q/K341D | | CB945 | 249 |

### E. Design and Methods for Modifying FVII

Provided herein are modified FVII polypeptides. The FVII polypeptides are modified such that they can exhibit alterations in one or more activities or properties compared to an unmodified FVII polypeptide. Activities and properties that can be altered as a result of modification include, but are not limited to, coagulation or coagulant activity; pro-coaguant activity; proteolytic or catalytic activity such as activity that effects factor X (FX) activation or Factor IX (FIX) activation; antigenicity, such as that assessed by the ability to bind to or compete with a polypeptide for binding to an anti-FVII antibody; ability to bind tissue factor, factor X or factor IX; ability to bind to phospholipids; half-life; three-dimensional structure; pI; and/or conformation. Among the modified FVII polypeptides provided herein are those that have increased coagulant activity. Among these are those whose increase in coagulant activity results from or involves increased resistance of the modified FVII to TFPI, increased resistance to AT-III, improved pharmacokinetic properties, such as increased half-life, increased catalytic activity, and/or increased binding to activated platelets. Exemplary methods to identify such modified FVII polypeptides and the modified polypeptides are provided herein. For example, a modified FVII polypeptides that exhibits increased resistance to TFPI, increased resistance to AT-III and/or increased binding to activated platelets can be generated rationally or empirically by: (a) rationally targeting sites that are contemplated to be involved in such properties or, (b) empirically testing modified FVII polypeptides in functional assays for resistance to TFPI, resistance to AT-III, improved pharmacokinetic properties, such as increased half-life, and/or increased binding to phospholipids or activated platelets. In many cases, a combination of rational and empirical design approaches can be used to generate modified FVII polypeptides

Once a domain, region or amino acid is identified for modification using a rational or empirical approach, any method known in the art to effect mutation of any one or more amino acids in a target protein can be employed. Methods include standard site-directed mutagenesis (using *e.g.*, a kit, such as kit such as QuikChange available from Stratagene) of encoding nucleic acid molecules, or by solid phase polypeptide synthesis methods. In addition, modified chimeric proteins provided herein (i.e. Gla domain swap) can be generated by routine recombinant DNA techniques. For example, chimeric polypeptides can be generated using restriction enzymes and cloning methodologies for routine subcloning of the desired chimeric polypeptide components. Once identified and generated, modified FVII polypeptides can then be assessed for activity, such as catalytic activity or coagulant activity, using any one or more of the various assays known in the art or described herein below.

**1. Rational**

In some examples, modified FVII polypeptides provided herein are designed by rational modification to increase coagulant activity. In such a method, the domains, regions or amino acids responsible for activity of a FVII polypeptide are known or can be rationally determined. For example, various databases in the public domain provide sequence and domain information related to wild-type FVII (see, *e.g.*, the ExPASy Proteomics server ca.expasy.org). Other information in the public domain can be accessed to determine the amino acids, regions and/or domains in a FVII polypeptide that are involved in a specific interaction or activity. Such sources include, for example, scientific journals, sequence and function databases, or patent databases. In some instances, direct evidence can be used to determine the influence of one or more amino acids on a given interaction or activity. In other examples, this is extrapolated indirectly from, for example, evidence or information regarding related proteins that display similar activities and/or interactions. In such examples, those amino acids that are responsible for the activity or interaction in the related protein can be used to determine which are the corresponding amino acids in the FVII polypeptide by alignment of the related polypeptide with the FVII polypeptide, based on sequence and/or structural similarities. Thus, the amino acids in the FVII polypeptide that are likely to be involved in the given activity or interaction can be determined. In addition, homology modeling can be used to predict residues that play important roles in the formation and/or stabilization of protein/protein interactions, and this information can be used to design variants with improved properties.

Once the domains, regions and/or amino acids responsible for activity of a FVII polypeptide are determined, they can be modified such that the modifications are predicted to result in increased coagulant activity of the polypeptide. This can be effected in several ways depending upon the activity or interaction being targeted. In some examples, one or more amino acid substitutions, insertions or deletions can be made that increase the affinity of the modified FVII for other proteins or molecules.

In one example, modifications can be made to any domain, region or amino acid(s) such that the affinity or interaction of the modified FVII for other proteins is altered, i.e. increased or decreased depending on the target interaction to be modified. To effect such a modification, the domains, regions and/or amino acids involved or contemplated to be involved in such interaction are known or can be rationally determined. For example, a modified FVII can be rationally designed to have a decreased interaction with an inhibitory protein, such as TFPI or AT-III, so as to increase the coagulant activity of the modified FVII polypeptide. A method of rationally designing a FVII polypeptide to have a decreased interaction with TFPI is set forth in Example 1. Similar approaches can be performed to increase or decrease the interaction of FVII with any other polypeptide for which FVII is known to interact or bind.

For example, FVII was modified to display increased resistance to TFPI. TFPI, in a complex with FXa, binds to the TF/FVIIa complex to form a quaternary complex in which the catalytic activity of FVIIa is inhibited. The first Kunitz domain of TFPI-1 (TFPI-1 K1) is the domain that is responsible for the interaction with, and inhibition of, FVIIa. Identification of the FVII amino acid residues that are involved in this interaction can facilitate the design of FVII polypeptides that are resistant to TFPI. Various approaches can be used to determine which of the amino acid residues in FVII are involved in this interaction, including, but not limited to, mutagenesis, scanning approaches, and rational design such as computer modeling. The crystal structure of FVIIa in complex with TFPI can be used as a basis for determining contact residues. Alternatively, and as described in Example 1, where a crystal structure is unavailable, computer modeling can be generated through a series of alignments and extrapolations from known structures and sequences of related proteins and their interactions with each other.

The results of the computer modeling can be used to identify residues that are directly involved in contact with a desired target protein, such as for example, TFPI, and/or those residues that are indirectly involved in the interaction, for example, due to close proximity to contact residues. For example, using the computer modeling results as set forth in Example 1, an alignment of the TFPI-1 and TFPI-2 first Kunitz domains was made to determine which are the corresponding "contact" residues in TFPI-1 (Figure 5b). Replacement amino acids can be identified in order to alter the interaction of the FVII polypeptide with the target protein. The replacement amino acids can be up to all 19 other naturally occurring amino acids, as well as "unnatural" amino acids. Alternatively, such replacement amino acids can be identified by *in silico* mutagenesis based on rational assumptions of the interaction. Example 1 sets forth an *in silico* approach for the identification of replacement amino acids whereby analysis of the computer model revealed residues involved in electrostatic complementarity between Factor VII and TFPI-1 K1. For example, the negatively-charged D60 of FVII (by chymotrypsin numbering) appears to be involved in an electrostatic interaction with the positively-charged R20 of TFPI-1. This residue is therefore a candidate for mutagenesis such the electrostatic complementarity with R20 of TFPI is abolished, thereby generating a modified FVII polypeptide that has increased resistance to TFPI. This can be effected by charge-reversal or charge neutralization substitutions. For example, an amino acid substitution can be made to replace the negatively-charged aspartic acid with a positively-charged lysine. Such a charge-reversal substitution establishes a repulsive charge-charge interaction at this site, and interferes with the binding and interaction of TFPI with FVIIa. In another example, the negatively-charged D60 of FVII can be replaced with a basic arginine to negate the positive electrostatic complementarity with R20 of TFPI and introduce an unfavorable interaction. Such rationally-designed modifications can be made to one or more of the FVII residues that are determined to be involved in direct or indirect contact and interaction with residues of TFPI.

In another example, rational modifications of a FVII polypeptide can be made based on known functions of domains or regions of the polypeptide. For example, the Gla domain of FVII is known to be responsible for the binding of FVIIa to phospholipids, such as those displayed on activated platelets, albeit very weakly. Other vitamin K-dependent proteins, such as for example, FIX, FX, prothrombin, protein C and protein S, also possess Gla domains that effect binding of the protein to negatively-charged phospholipids, in many cases with a much greater affinity than the Gla domain of FVII. Introduction of a heterologous Gla domain into a FVII polypeptide is contemplated to increase the affinity of FVII for phospholipids. For example, FX (and FXa) display a relatively high affinity for phospholipids, compared with that of FVII (and FVIIa). Therefore, in one example, the Gla domain of FX, or a sufficient portion of the Gla domain of FX to effect phospholipid binding, can be introduced into a FVII polypeptide to generate a chimeric modified FVII polypeptide.

If necessary, a combination of rational and empirical methods can be used to design a FVII polypeptide. For example, the binding of Gla-domain containing polypeptides can be tested and screened for to identify which polypeptides exhibit the highest binding and/or affinity to phospholipids, such as for example, on platelet surfaces. Alternatively or additionally, a series of FVII polypeptides containing introduction of various heterologous Gla domains, or sufficient portions thereof, can be tested to determine which chimeric polypeptide exhibits the highest affinity and/or binding to phospholipids.

### 2. Empirical (i.e. screening)

Modified FVII polypeptides also can be generated empirically and then tested or screened to identify those having the particular activity or property contemplated. For example, modified FVII polypeptides can be generated by mutating any one or more amino acid residues of a FVII polypeptide using any method commonly known in the art (see also published U.S. Appln. No. 2004/0146938). Such modified FVII polypeptides can be tested in functional assays of coagulation to determine if they are "Hits" for increasing coagulant activity. The Hits can be further tested to determine if they display increased resistance to inhibitors such as TFPI or AT-III and/or display increased binding to phospholipids or improved pharmacokinetic properties, such as increased half-life, using any of the assays described herein or known to one of skill in the art.

Examples of methods to mutate FVII include methods that result in random mutagenesis across the entire sequence or methods that result in focused mutagenesis of a select region or domain of the FVII sequence. In one example, the number of mutations made to the polypeptide is 1, 2, 3, 4, 5, 6, 7, 8, 9, 10, 11, 12, 13, 14, 15, 16, 17,18,19,20,30,40 or more.

#### a. Random Mutagenesis

Any of a variety of general approaches for directed protein evolution based on mutagenesis can be employed. Any of these, alone or in combination can be used to modify a polypeptide such as FVII to achieve a desired property. Such methods include random mutagenesis, where the amino acids in the starting protein sequence are replaced by all (or a group) of the 20 natural amino acids (as well as unnatural amino acids) either in single or multiple replacements at different amino acid positions on the same molecule, at the same time. Another method, restricted random mutagenesis, introduces either all or some of the 20 natural amino acids (as well as unnatural amino acids) or DNA-biased residues. The bias is based on the sequence of the DNA and not on that of the protein in a stochastic or semi-stochastic manner, respectively, within restricted or predefined regions of the protein known in advance to be involved in the biological activity being "evolved." Exemplary methods for modifying a protease are described in US 2004146938. Additionally, any method known in the art can be used to modify or alter a protease polypeptide sequence, such as a FVII protease polypeptide.

Random mutagenesis methods include, for example, use of *E. coli* XL1red, UV irradiation, chemical modification such as by deamination, alkylation, or base analog mutagens, or PCR methods such as DNA shuffling, cassette mutagenesis, site-directed random mutagenesis, or error prone PCR (see *e.g.* U.S. Application No.: 2006-0115874). Such examples include, but are not limited to, chemical modification by hydroxylamine (Ruan, H., et al. (1997) Gene 188:35-39), the use of dNTP analogs (Zaccolo, M., et al. (1996) J. Mol. Biol. 255:589-603), or the use of commercially available random mutagenesis kits such as, for example, GeneMorph PCR-based random mutagenesis kits (Stratagene) or Diversify random mutagenesis kits (Clontech). The Diversify random mutagenesis kit allows the selection of a desired mutation rate for a given DNA sequence (from 2 to 8 mutations/1000 base pairs) by varying the amounts of manganese ($Mn^{2+}$) or dGTP in the reaction mixture. Raising manganese levels initially increases the mutation rate, with a further mutation rate increase provided by increased concentration of dGTP. Even higher rates of mutation can be achieved by performing additional rounds of PCR.

### b. Focused Mutagenesis

Focused mutation can be achieved by making one or more mutation in a predetermined region of a gene sequence, for example, in regions of the protease domain that mediate catalytic activity, regions of the Gla domain that bind to phospholipids, regions of the FVII polypeptide that bind inhibitors or molecules such as TFPI, AT-III or $Zn^{2+}$, such as those provided herein, or regions of the protein that interact with factors or receptos involved in clearance of FVIIa, such as those provided herein. In one example, any one or more amino acids of a polypeptide are mutated using any standard single or multiple site-directed mutagenesis kit such as for example QuikChange (Stratagene). In another example, any one or more amino acids of a protease are mutated by saturation mutagenesis (Zheng et al. (2004) Nucl. Acids. Res., 32:115). In one example, a saturation mutagenesis technique is used in which the residue(s) of a region or domain are mutated to each of the 20 possible natural amino acids (as well as unnatural amino acids) (see for example the Kunkle method, Current Protocols in Molecular Biology, John Wiley and Sons, Inc., Media Pa.). In such a technique, a degenerate mutagenic oligonucleotide primer can be synthesized which contains randomization of nucleotides at the desired codon(s) encoding the selected amino acid(s). Exemplary randomization schemes include NNS- or NNK-randomization, where N represents any nucleotide, S represents guanine or cytosine and K represents guanine or thymine. The degenerate mutagenic primer is annealed to the single stranded DNA template and DNA polymerase is added to synthesize the complementary strand of the template. After ligation, the double stranded DNA template is transformed into *E. coli* for amplification.

In an additional example, focused mutagenesis can be restricted to amino acids that are identified as hot spots in the initial rounds of screening. For example, following selection of modified FVII polypeptides from randomly mutagenized combinatorial libraries, a disproportionate number of mutations can be observed at specific positons or regions. These are designated "hot spots", and can be observed through more than one round of mutagenesis and screening. Functional assays can then be perfomed on the modified FVII polypeptides to determine whether the muations at the hot spots correlate with the one or more desired property or activity. If correlation between the hot spots and the desired activity or property is verified, focused mutagenesis can be then used to specifically target these hot spots for further mutagenesis. This strategy allows for a more diverse and deep mutagenesis at particular specified positions, as opposed to the more shallow mutagenesis that occurs following random mutagenesis of a polypeptide sequence. For example, saturation mutagenesis can be used to mutate "hot spots" such as by using oligos containing NNt/g or NNt/c at these positions.

### c. Screening

The modified polypeptides can be tested in screening assays individually, or can be tested as collections such as in libraries. In one example, the FVII variant polypeptides are randomly generated by mutagenesis, and cloned individually. Activity assessment is then individually performed on each individual protein mutant molecule, following protein expression and measurement of the appropriate activity. In some examples, the individual clones can be assayed in an addressable array, such that they are physically separated from each other so that the identity of each individual polypeptide is known based on its location in the array. For example, if each one is the single product of an independent mutagenesis reaction, the specific mutation can be easily determined without the need for sequencing. Alternatively, sequencing can be performed on the resulting modified polypeptides to determine those mutations that confer an activity.

In another example, the modified FVII polypeptides can be screened as collections or in a library. For example, a library of FVII polypeptides can be displayed on a genetic package for screening, including, but not limited to any replicable vector, such as a phage, virus, or bacterium, that can display a polypeptide moiety. The plurality of displayed polypeptides is displayed by a genetic package in such a way as to allow the polypeptide to bind and/or interact with a target polypeptide. Exemplary genetic packages include, but are not limited to, bacteriophages (see, *e.g*., Clackson et al. (1991) Making Antibody Fragments Using Phage Display Libraries, Nature, 352:624-628; Glaser et al. (1992) Antibody Engineering by Condon-Based Mutagenesis in a Filamentous Phage Vector System, J. Immunol., 149:3903 3913; Hoogenboom et al. (1991) Multi-Subunit Proteins on the Surface of Filamentous Phage: Methodologies for Displaying Antibody (Fate) Heavy and 30 Light Chains, Nucleic Acids Res., 19:4133-41370), baculoviruses (see, *e.g.,* Boublik et al. (1995) Eukaryotic Virus Display: Engineering the Major Surface Glycoproteins of the Autographa California Nuclear Polyhedrosis Virus (ACNPV) for the Presentation of Foreign Proteins on the Virus Surface, Bio/Technology, 13:1079-1084), bacteria and other suitable vectors for displaying a protein, such as a phage-displayed protease. For example bacteriophages of interest include, but are not limited to, T4 phage, M 13 phage and HI phage. Genetic packages are optionally amplified such as in a bacterial host.

Phage display is known to those of skill in the art and is described, for example, in Ladner et al., U.S. Pat. No. 5,223,409; Rodi et al. (2002) Curr. Opin. Chem. Biol. 6:92-96; Smith (1985) Science 228:1315-1317; WO 92/18619; WO 91/17271; WO 92/20791; WO 92/15679; WO 93/01288; WO 92/01047; WO 92/09690; WO 90/02809; de Haard et al. (1999) J. Biol. Chem 274:18218-30; Hoogenboom et al. (1998) Immunotechnology 4:1-20; Hoogenboom et al. (2000) Immunol Today 2:371-8; Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Rebar et al. (1996) Methods Enzymol. 267:129-49; Hoogenboom et al. (1991) Nuc Acid Res 19:4133-4137; and Barbas et al. (1991) PNAS 88:7978-7982. Nucleic acids suitable for phage display, *e.g.*, phage vectors, are known in the art (see, *e.g.*, Andris-Widhopf et al. (2000) J Immunol Methods, 28: 159-81, Armstrong *et al.* (1996) Academic Press, Kay *et al.,* Ed. pp.35-53; Corey et al. (1993) Gene 128(1):129-34; Cwirla et al. (1990) Proc Natl Acad Sci USA 87(16):6378-82; Fowlkes et al. (1992) Biotechniques 13(3):422-8; Hoogenboom et al. (1991) Nuc Acid Res 19(15):4133-7; McCafferty et al. (1990) Nature 348(6301):552-4; McConnell et al. (1994) Gene 151(1-2):115-8; Scott and Smith (1990) Science 249(4967):386-90).

Libraries of variant FVII polypeptides for screening also can be expressed on the surfaces of cells, for example, prokaryotic or eukaryotic cells. Exemplary cells for cell surface expression include, but are not limited to, bacteria, yeast, insect cells, avian cells, plant cells, and mammalian cells (Chen and Georgiou (2002) Biotechnol Bioeng 79: 496-503). In one example, the bacterial cells for expression are *Escherichia coli.*

Variant polypeptides can be expressed as a fusion protein with a protein that is expressed on the surface of the cell, such as a membrane protein or cell surface-associated protein. For example, a variant protease can be expressed in *E. coli* as a fusion protein with an *E. coli* outer membrane protein (*e.g.* OmpA), a genetically engineered hybrid molecule of the major *E. coli* lipoprotein (Lpp) and the outer membrane protein OmpA or a cell surface-associated protein (*e.g.* pili and flagellar subunits). Generally, when bacterial outer membrane proteins are used for display of heterologous peptides or proteins, it is achieved through genetic insertion into permissive sites of the carrier proteins. Expression of a heterologous peptide or protein is dependent on the structural properties of the inserted protein domain, since the peptide or protein is more constrained when inserted into a permissive site as compared to fusion at the N- or C-terminus of a protein. Modifications to the fusion protein can be done to improve the expression of the fusion protein, such as the insertion of flexible peptide linker or spacer sequences or modification of the bacterial protein (*e.g.* by mutation, insertion, or deletion, in the amino acid sequence). Enzymes, such as β-lacatamase and the Cex exoglucanase of *Cellulomonas fimi,* have been successfully expressed as Lpp-OmpA fusion proteins on the surface of *E. coli* (Francisco J.A. and Georgiou G. Ann N YAcad Sci. 745:372-382 (1994) and Georgiou G. et al. Protein Eng. 9:239-247 (1996)). Other peptides of 15-514 amino acids have been displayed in the second, third, and fourth outer loops on the surface of OmpA (Samuelson et al. J. Biotechnol. 96: 129-154 (2002)). Thus, outer membrane proteins can carry and display heterologous gene products on the outer surface of bacteria.

It is also possible to use other display formats to screen libraries of variant polypeptides. Exemplary other display formats include nucleic acid-protein fusions, ribozyme display (see e.g. Hanes and Pluckthun (1997) Proc. Natl. Acad. Sci. U.S.A. 13:4937-4942), bead display (Lam, K. S. et al. Nature (1991) 354, 82-84;, K. S. et al. (1991) Nature, 354, 82-84; Houghten, R. A. et al. (1991) Nature, 354, 84-86; Furka, A. et al. (1991) Int. J. Peptide Protein Res. 37, 487-493; Lam, K. S., et al. (1997) Chem. Rev., 97, 411-448; U.S. Published Patent Application 2004-0235054) and protein arrays (see *e.g.* Cahill (2001) J. Immunol. Meth. 250:81-91, WO 01/40803, WO 99/51773, and US2002-0192673-A1)

In specific other cases, it can be advantageous to instead attach the variant polypeptides or phage libraries or cells expressing variant polypeptides to a solid support. For example, in some examples, cells expressing variant FVII polypeptides can be naturally adsorbed to a bead, such that a population of beads contains a single cell per bead (Freeman et al. Biotechnol. Bioeng. (2004) 86:196-200). Following immobilization to a glass support, microcolonies can be grown and screened with a chromogenic or fluorogenic substrate. In another example, variant FVII polypeptides or phage libraries or cells expressing variant proteases can be arrayed into titer plates and immobilized.

To identify those modified FVII polypeptides that exhibit increase coagulant activity, modified FVII polypeptides are screened individually or in a library and tested in functional assays to identify those that display increased resistance to inhibitors such as TFPI and AT-III, increased binding to activated platelets of phospholipids, increased half-life and/or display increased catalytic activity. Such assays are described herein or are known to those of skill in the art. For example, modified FVII polypeptides are tested for proteolytic activity. FVII polypeptides, alone or in the presence of TF, are incubated with varying concentrations of chromogenic substrate, such as the peptidyl substrate Spectrozyme FVIIa ($CH_3SO_2$-D-CHA-But-Arg-pNA.AcOH). Cleavage of the substrate is monitored by absorbance and the rate of substrate hydrolysis determined by linear regression using software readily available. In a further example, resistance of modified FVII polypeptides to TFPI or AT-III is assessed by incubation of the inhibitor with FVII polypeptides that have, in some instances, been preincubated with TF. The activity of FVII is then measured using any one or more of the activity or coagulation assays known in the art, and inhibition by TFPI or AT-III is assessed by comparing the activity of FVII polypeptides incubated with the inhibitor, with the activity of FVII polypeptides that were not incubated with the inhibitor.

The specific mutation of the candidate polypeptide can be determined using routine recombinant DNA techniques, such as sequencing In one embodiment, combinations of "Hits" can be made to further increase the properties and/or coagulant activities of the modified FVII polypeptide.

### 3. Selecting FVII variants

FVII polypeptide variants designed and identified by any one or more of the approaches described above, can be selected to identify those candidate FVII polypeptides that exhibit the desired properties or activities. The selection of variant FVII polypeptides is based on 1) first, testing the variant polypeptide for the specific activity or property being modified (i.e. resistance to TFPI, resistance to AT-III, $Zn^{2+}$ binding, catyltic activity, half-life, binding and/or affinity for phospholipids; and 2) second, testing the variant polypeptide for retention of a FVII activity required for hemostatsis and coagulation. Included among FVII activites that are required for coagulation include, for example, enzymatic, proteolytic or catalytic activity such as to effect factor X (FX) activation or factor IX (FIX) activation. Other FVII activites that can be assessed include, but are not limited to, antigenicity, the ability to bind tissue factor, factor X or factor IX, and the ability to bind to phospholipids. Thus, a modified FVII polypeptide, such as any provided herein or identified in the methods provided here, must retain some level, either increased or decreased, of a FVII activity required for coagulant activity. Standard assays known in the art or described herein below can be performed *in vitro* or *in vivo* in order to perform each of the above two assessments. For example, the proteolytic or catalytic activity of FVII can be assessed using various methods, including measuring the cleavage of a synthetic substrate, or measuring the activation of factor X (*see*, *e.g.* Examples 4, 5 and 11 below), and the resistance to TFPI or AT-III can be assessed by assaying for inhibition by TFPI or AT-III, respectively (see, *e.g*., Examples 7, 12 and 16). In addition, *in vivo* assays for procoagulant activity, such as is described, for example, in Examples 8 and 14 also can be employed.

The overall effect of any candidate FVII polypeptide variant is to exhibit a procoagulant activity. For example, a variant polypeptide can be increased in its resistance to TFPI, resistance to AT-III, half-life and/or binding and/or affinity for phospholipids, while also exhibiting an increase in catalytic activity. Such a FVII polypeptide would be selected as a candidate FVII variant for increasing coagulant activity.

In some cases, however, a FVII polypeptide modified to have improved coagulation activity due to any one or more of increased resistance to TFPI, increased resistance to AT-III, increased half-life or increased binding and/or affininty for phospholipids, may also exhibit a decrease in the catalytic activity or other activity required for coagulation as a result of the particular modification. These effects could result from, for example, conformational changes in the modified FVII polypeptides that interfere with binding to another molecule, conformational changes that result in an altered active site, or amino acid substitutions that directly involve one or more amino acid residues that are responsible for interaction with another molecule.

Thus, in another example, a variant polypeptide that is increased in its resistance to TFPI, resistance to AT-III, increased half-life and/or affinity for phospholipids may exhibit a concomitant decrease in its catalytic activity. The level of decrease in a FVII activity, such as a catalytic activity, that would be acceptable to ensure improved coagulant activity is dependent on the concomitant increase in the property that is being modified for improvement (i.e. increased resistance to TFPI), and can be empirically determined. Therefore, the results of such assessments set forth above can be balanced to identify those variant polypeptides that exhibit improved properties, while at the same time retaining at least a sufficient FVII activity, i.e. catalytic activity, to effect coagulation. Typically, the greater the increase in the property contemplated for modification (i.e. the greater the increase in resistance to TFPI), the greater the acceptable reduction in proteolytic or catalytic activity.

For example, a FVII polypeptide that exhibits a 100-fold increase in resistance to TFPI or AT-III can exhibit a decrease in proteolytic or catalytic activity that is decreased at or about 1.5-fold, 2, 3, 4, 5, 6, 7, 8, 9, 10, 20, 30, 40, 50, 60, 70, 80, 90-fold or more compared to the activity of an unmodified FVII polypeptide, and still be a viable candidate for increasing coagulant activity. Conversely, if resistance to TFPI or AT-III is increased by 10-fold, the level of decrease in catalytic activity to sustain an overall procoagulant activity would typically be a decrease of at or about 1.5-fold, 2, 3, 4, 5, 6, 7, 8, 9-fold or more as compared to the catalytic activity of an unmodified polypeptide. One of skill in the art can assess concomitant changes in TFPI resistance, AT-III resistance, activated platelet binding, half-life and proteolytic or catalytic activity, or any other activity or property, to determine whether the modified FVII polypeptide would be useful as a procoagulant therapeutic, such as, for example, to treat bleeding episodes in hemophilia patients.

## F. Production of FVII polypeptides

FVII polypeptides, including modified FVII polypeptides, or domains thereof of FVII or other vitamin-K polypeptide, can be obtained by methods well known in the art for protein purification and recombinant protein expression. Any method known to those of skill in the art for identification of nucleic acids that encode desired genes can be used. Any method available in the art can be used to obtain a full length (*i.e.*, encompassing the entire coding region) cDNA or genomic DNA clone encoding a FVII polypeptide or other vitamin-K polypeptide, such as from a cell or tissue source, such as for example from liver. Modified FVII polypeptides can be engineered as described herein, such as by site-directed mutagenesis.

FVII can be cloned or isolated using any available methods known in the art for cloning and isolating nucleic acid molecules. Such methods include PCR amplification of nucleic acids and screening of libraries, including nucleic acid hybridization screening, antibody-based screening and activity-based screening.

Methods for amplification of nucleic acids can be used to isolate nucleic acid molecules encoding a FVII polypeptide, including for example, polymerase chain reaction (PCR) methods. A nucleic acid containing material can be used as a starting material from which a FVII-encoding nucleic acid molecule can be isolated. For example, DNA and mRNA preparations, cell extracts, tissue extracts (e.g. from liver), fluid samples (*e.g.* blood, serum, saliva), samples from healthy and/or diseased subjects can be used in amplification methods. Nucleic acid libraries also can be used as a source of starting material. Primers can be designed to amplify a FVII-encoding molecule. For example, primers can be designed based on expressed sequences from which a FVII is generated. Primers can be designed based on back-translation of a FVII amino acid sequence. Nucleic acid molecules generated by amplification can be sequenced and confirmed to encode a FVII polypeptide.

Additional nucleotide sequences can be joined to a FVII-encoding nucleic acid molecule, including linker sequences containing restriction endonuclease sites for the purpose of cloning the synthetic gene into a vector, for example, a protein expression vector or a vector designed for the amplification of the core protein coding DNA sequences. Furthermore, additional nucleotide sequences specifying functional DNA elements can be operatively linked to a FVII-encoding nucleic acid molecule. Examples of such sequences include, but are not limited to, promoter sequences designed to facilitate intracellular protein expression, and secretion sequences designed to facilitate protein secretion. Additional nucleotide sequences such as sequences specifying protein binding regions also can be linked to FVII-encoding nucleic acid molecules. Such regions include, but are not limited to, sequences to facilitate uptake of FVII into specific target cells, or otherwise enhance the pharmacokinetics of the synthetic gene.

The identified and isolated nucleic acids can then be inserted into an appropriate cloning vector. A large number of vector-host systems known in the art can be used. Possible vectors include, but are not limited so, plasmids or modified viruses, but the vector system must be compatible with the host cell used. Such vectors include, but are not limited to, bacteriophages such as lambda derivatives, or plasmids such as pBR322 or pUC plasmid derivatives or the Bluescript vector (Stratagene, La Jolla, CA). The insertion into a cloning vector can, for example, be accomplished by ligating the DNA fragment into a cloning vector which has complementary cohesive termini. Insertion can be effected using TOPO cloning vectors (Invitrogen, Carlsbad, CA). If the complementary restriction sites used to fragment the DNA are not present in the cloning vector, the ends of the DNA molecules can be enzymatically modified. Alternatively, any site desired can be produced by ligating nucleotide sequences (linkers) onto the DNA termini; these ligated linkers can contain specific chemically synthesized oligonucleotides encoding restriction endonuclease recognition sequences. In an alternative method, the cleaved vector and FVII protein gene can be modified by homopolymeric tailing. Recombinant molecules can be introduced into host cells via, for example, transformation, transfection, infection, electroporation and sonoporation, so that many copies of the gene sequence are generated.

In specific examples, transformation of host cells with recombinant DNA molecules that incorporate the isolated FVII protein gene, cDNA, or synthesized DNA sequence enables generation of multiple copies of the gene. Thus, the gene can be obtained in large quantities by growing transformants, isolating the recombinant DNA molecules from the transformants and, when necessary, retrieving the inserted gene from the isolated recombinant DNA.

### 1. Vectors and Cells

For recombinant expression of one or more of the FVII proteins, the nucleic acid containing all or a portion of the nucleotide sequence encoding the FVII protein can be inserted into an appropriate expression vector, *i.e.*, a vector that contains the necessary elements for the transcription and translation of the inserted protein coding sequence. Exemplary of such a vector is any mammalian expression vector such as, for example, pCMV. The necessary transcriptional and translational signals also can be supplied by the native promoter for a FVII genes, and/or their flanking regions.

Also provided are vectors that contain nucleic acid encoding the FVII or modified FVII. Cells containing the vectors also are provided. The cells include eukaryotic and prokaryotic cells, and the vectors are any suitable for use therein.

Prokaryotic and eukaryotic cells, including endothelial cells, containing the vectors are provided. Such cells include bacterial cells, yeast cells, fungal cells, Archea, plant cells, insect cells and animal cells. The cells are used to produce a FVII polypeptide or modified FVII polypeptide thereof by growing the above-described cells under conditions whereby the encoded FVII protein is expressed by the cell, and recovering the expressed FVII protein. For purposes herein, the FVII can be secreted into the medium.

In one embodiment, vectors containing a sequence of nucleotides that encodes a polypeptide that has FVII activity and contains all or a portion of the FVII polypeptide, or multiple copies thereof, are provided. The vectors can be selected for expression of the FVII polypeptide or modified FVII polypeptide thereof in the cell or such that the FVII protein is expressed as a secreted protein. When the FVII is expressed the nucleic acid is linked to nucleic acid encoding a secretion signal, such as the *Saccharomyces cerevisiae* $\alpha$-mating factor signal sequence or a portion thereof, or the native signal sequence.

A variety of host-vector systems can be used to express the protein coding sequence. These include but are not limited to mammalian cell systems infected with virus (*e.g*. vaccinia virus, adenovirus and other viruses); insect cell systems infected with virus (*e.g*. baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system used, any one of a number of suitable transcription and translation elements can be used.

Any methods known to those of skill in the art for the insertion of DNA fragments into a vector can be used to construct expression vectors containing a chimeric gene containing appropriate transcriptional/translational control signals and protein coding sequences. These methods can include *in vitro* recombinant DNA and synthetic techniques and *in vivo* recombinants (genetic recombination). Expression of nucleic acid sequences encoding a FVII polypeptide or modified FVII polypeptide, or domains, derivatives, fragments or homologs thereof, can be regulated by a second nucleic acid sequence so that the genes or fragments thereof are expressed in a host transformed with the recombinant DNA molecule(s). For example, expression of the proteins can be controlled by any promoter/enhancer known in the art. In one example, the promoter is not native to the genes for a FVII protein. Promoters which can be used include but are not limited to the SV40 early promoter (Bemoist and Chambon, Nature 290:304-310 (1981)), the promoter contained in the 3' long terminal repeat of Rous sarcoma virus (Yamamoto et al. Cell 22:787-797 (1980)), the herpes thymidine kinase promoter (Wagner et al., Proc. Natl. Acad. Sci. USA 78:1441-1445 (1981)), the regulatory sequences of the metallothionein gene (Brinster et al., Nature 296:39-42 (1982)); prokaryotic expression vectors such as the $\beta$-lactamase promoter (Jay et al., (1981) Proc. Natl. Acad. Sci. USA 78:5543) or the *tac* promoter (DeBoer et al., Proc. Natl. Acad. Sci. USA 80:21-25 (1983)); see also "Useful Proteins from Recombinant Bacteria": in Scientific American 242:79-94 (1980)); plant expression vectors containing the nopaline synthetase promoter (Herrar-Estrella et al., Nature 303:209-213 (1984)) or the cauliflower mosaic virus 35S RNA promoter (Garder et al., Nucleic Acids Res. 9:2871 (1981)), and the promoter of the photosynthetic enzyme ribulose bisphosphate carboxylase (Herrera-Estrella et al., Nature 310:115-120 (1984)); promoter elements from yeast and other fungi such as the Gal4 promoter, the alcohol dehydrogenase promoter, the phosphoglycerol kinase promoter, the alkaline phosphatase promoter, and the following animal transcriptional control regions that exhibit tissue specificity and have been used in transgenic animals: elastase I gene control region which is active in pancreatic acinar cells (Swift et al., Cell 38:639-646 (1984); Ornitz et al., Cold Spring Harbor Symp. Quant. Biol. 50:399-409 (1986); MacDonald, Hepatology 7:425-515 (1987)); insulin gene control region which is active in pancreatic beta cells (Hanahan et al., Nature 315:115-122 (1985)), immunoglobulin gene control region which is active in lymphoid cells (Grosschedl et al., Cell 38:647-658 (1984); Adams et al., Nature 318:533-538 (1985); Alexander et al., Mol. Cell Biol. 7:1436-1444 (1987)), mouse mammary tumor virus control region which is active in testicular, breast, lymphoid and mast cells (Leder et al., Cell 45:485-495 (1986)), albumin gene control region which is active in liver (Pinckert et al., Genes and Devel. 1:268-276 (1987)), alpha-fetoprotein gene control region which is active in liver (Krumlauf et al., Mol. Cell. Biol. 5:1639-1648 (1985); Hammer et al., Science 235:53-58 1987)), alpha-1 antitrypsin gene control region which is active in liver (Kelsey et al., Genes and Devel. 1:161-171 (1987)), beta globin gene control region which is active in myeloid cells (Mogram et al., Nature 315:338-340 (1985); Kollias et al., Cell 46:89-94 (1986)), myelin basic protein gene control region which is active in oligodendrocyte cells of the brain (Readhead et al., Cell 48:703-712 (1987)), myosin light chain-2 gene control region which is active in skeletal muscle (Sani, Nature 314:283-286 (1985)), and gonadotrophic releasing hormone gene control region which is active in gonadotrophs of the hypothalamus (Mason et al., Science 234:1372-1378 (1986)).

In a specific embodiment, a vector is used that contains a promoter operably linked to nucleic acids encoding a FVII polypeptide or modified FVII polypeptide, or a domain, fragment, derivative or homolog, thereof, one or more origins of replication, and optionally, one or more selectable markers (*e.g.,* an antibiotic resistance gene). Vectors and systems for expression of FVII polypeptides include the well known *Pichia* vectors (available, for example, from Invitrogen, San Diego, CA), particularly those designed for secretion of the encoded proteins. Exemplary plasmid vectors for expression in mammalian cells include, for example, pCMV. Exemplary plasmid vectors for transformation of *E.coli* cells, include, for example, the pQE expression vectors (available from Qiagen, Valencia, CA; see also literature published by Qiagen describing the system). pQE vectors have a phage T5 promoter (recognized by *E. coli* RNA polymerase) and a double lac operator repression module to provide tightly regulated, high-level expression of recombinant proteins in *E. coli*, a synthetic ribosomal binding site (RBS II) for efficient translation, a 6XHis tag coding sequence, $t_0$ and T1 transcriptional terminators, ColE1 origin of replication, and a beta-lactamase gene for conferring ampicillin resistance. The pQE vectors enable placement of a 6xHis tag at either the N- or C-terminus of the recombinant protein. Such plasmids include pQE 32, pQE 30, and pQE 31 which provide multiple cloning sites for all three reading frames and provide for the expression of N-terminally 6xHis-tagged proteins. Other exemplary plasmid vectors for transformation of *E. coli* cells, include, for example, the pET expression vectors (see, U.S. patent 4,952,496; available from NOVAGEN, Madison, WI; see, also literature published by Novagen describing the system). Such plasmids include pET 11a, which contains the T71ac promoter, T7 terminator, the inducible *E. coli* lac operator, and the lac repressor gene; pET 12a-c, which contains the T7 promoter, T7 terminator, and the *E. coli* ompT secretion signal; and pET 15b and pET19b (NOVAGEN, Madison, WI), which contain a His-Tag™ leader sequence for use in purification with a His column and a thrombin cleavage site that permits cleavage following purification over the column, the T7-lac promoter region and the T7 terminator.

## 2. Expression systems

FVII polypeptides (modified and unmodified) can be produced by any methods known in the art for protein production including *in vitro* and *in vivo* methods such as, for example, the introduction of nucleic acid molecules encoding FVII into a host cell, host animal and expression from nucleic acid molecules encoding FVII *in vitro.* FVII and modified FVII polypeptides can be expressed in any organism suitable to produce the required amounts and forms of a FVII polypeptide needed for administration and treatment. Expression hosts include prokaryotic and eukaryotic organisms such as *E. coli,* yeast, plants, insect cells, mammalian cells, including human cell lines and transgenic animals. Expression hosts can differ in their protein production levels as well as the types of post-translational modifications that are present on the expressed proteins. The choice of expression host can be made based on these and other factors, such as regulatory and safety considerations, production costs and the need and methods for purification.

Expression in eukaryotic hosts can include expression in yeasts such as *Saccharomyces cerevisiae* and *Pichia pastoria,* insect cells such as *Drosophila* cells and *lepidopteran* cells, plants and plant cells such as tobacco, corn, rice, algae, and lemna. Eukaryotic cells for expression also include mammalian cells lines such as Chinese hamster ovary (CHO) cells or baby hamster kidney (BHK) cells. Eukaryotic expression hosts also include production in transgenic animals, for example, including production in serum, milk and eggs. Transgenic animals for the production of wild-type FVII polypeptides are known in the art (U.S. Patent Publication Nos. 20020166130 and 20040133930) and can be adapted for production of modified FVII polypeptides provided herein.

Many expression vectors are available and known to those of skill in the art for the expression of FVII. The choice of expression vector is influenced by the choice of host expression system. Such selection is well within the level of skill of the skilled artisan. In general, expression vectors can include transcriptional promoters and optionally enhancers, translational signals, and transcriptional and translational termination signals. Expression vectors that are used for stable transformation typically have a selectable marker which allows selection and maintenance of the transformed cells. In some cases, an origin of replication can be used to amplify the copy number of the vectors in the cells.

FVII or modified FVII polypeptides also can be utilized or expressed as protein fusions. For example, a fusion can be generated to add additional functionality to a polypeptide. Examples of fusion proteins include, but are not limited to, fusions of a signal sequence, a tag such as for localization, *e.g.* a $his_6$ tag or a myc tag, or a tag for purification, for example, a GST fusion, and a sequence for directing protein secretion and/or membrane association.

In one embodiment, the FVII polypeptide or modified FVII polypeptides can be expressed in an active form, whereby activation is achieved by autoactivation of the polypeptide following secretion. In another example, the protease is expressed in an inactive, zymogen form.

Methods of production of FVII polypeptides can include coexpression of one or more additional heterologous polypeptides that can aid in the generation of the FVII polypeptides. For example, such polypeptides can contribute to the post-translation processing of the FVII polypeptides. Exemplary polypeptides include, but are not limited to, peptidases that help cleave FVII precursor sequences, such as the propeptide sequence, and enzymes that participate in the modification of the FVII polypeptide, such as by glycosylation, hydroxylation, carboxylation, or phosphorylation, for example. An exemplary peptidase that can be coexpressed with FVII is PACE/furin (or PACE-SOL), which aids in the cleavage of the FVII propeptide sequence. An exemplary protein that aids in the carboxylation of the FVII polypeptide is the warfarin-sensitive enzyme vitamin K 2,3-epoxide reductase (VKOR), which produces reduced vitamin K for utilization as a cofactor by the vitamin K-dependent γ-carboxylase (Wajih et al., J. Biol. Chem. 280(36)31603-31607). A subunit of this enzyme, VKORC1, can be coexpressed with the modified FVII polypeptide to increase the γ-carboxylation The one or more additional polypeptides can be expressed from the same expression vector as the FVII polypeptide or from a different vector.

### a. Prokaryotic expression

Prokaryotes, especially *E. coli*, provide a system for producing large amounts of FVII *(see,* for example, Platis et al. (2003) Protein Exp. Purif. 31(2): 222-30; and Khalizzadeh et al. (2004) J. Ind. Microbiol. Biotechnol. 31 (2): 63-69). Transformation of *E. coli* is a simple and rapid technique well known to those of skill in the art. Expression vectors for *E. coli* can contain inducible promoters that are useful for inducing high levels of protein expression and for expressing proteins that exhibit some toxicity to the host cells. Examples of inducible promoters include the lac promoter, the trp promoter, the hybrid tac promoter, the T7 and SP6 RNA promoters and the temperature regulated $\lambda P_L$ promoter.

FVII can be expressed in the cytoplasmic environment of *E. coli.* The cytoplasm is a reducing environment and for some molecules, this can result in the formation of insoluble inclusion bodies. Reducing agents such as dithiothreitol and β-mercaptoethanol and denaturants (*e.g.*, such as guanidine-HCl and urea) can be used to resolubilize the proteins. An alternative approach is the expression of FVII in the periplasmic space of bacteria which provides an oxidizing environment and chaperonin-like and disulfide isomerases leading to the production of soluble protein. Typically, a leader sequence is fused to the protein to be expressed which directs the protein to the periplasm. The leader is then removed by signal peptidases inside the periplasm. Examples of periplasmic-targeting leader sequences include the pelB leader from the pectate lyase gene and the leader derived from the alkaline phosphatase gene. In some cases, periplasmic expression allows leakage of the expressed protein into the culture medium. The secretion of proteins allows quick and simple purification from the culture supernatant. Proteins that are not secreted can be obtained from the periplasm by osmotic lysis. Similar to cytoplasmic expression, in some cases proteins can become insoluble and denaturants and reducing agents can be used to facilitate solubilization and refolding. Temperature of induction and growth also can influence expression levels and solubility. Typically, temperatures between 25°C and 37°C are used. Mutations also can be used to increase solubility of expressed proteins. Typically, bacteria produce aglycosylated proteins. Thus, if proteins require glycosylation for function, glycosylation can be added *in vitro* after purification from host cells.

### b. Yeast

Yeasts such as *Saccharomyces cerevisiae, Schizosaccharomyces pombe, Yarrowia lipolytica, Kluyveromyces lactis,* and *Pichia pastoris* are useful expression hosts for FVII (see for example, Skoko et al. (2003) Biotechnol. Appl. Biochem. 38(Pt3):257-65). Yeast can be transformed with episomal replicating vectors or by stable chromosomal integration by homologous recombination. Typically, inducible promoters are used to regulate gene expression. Examples of such promoters include GAL1, GAL7, and GAL5 and metallothionein promoters such as CUP1. Expression vectors often include a selectable marker such as LEU2, TRP1, HIS3, and URA3 for selection and maintenance of the transformed DNA. Proteins expressed in yeast are often soluble and co-expression with chaperonins, such as Bip and protein disulfide isomerase, can improve expression levels and solubility. Additionally, proteins expressed in yeast can be directed for secretion using secretion signal peptide fusions such as the yeast mating type alpha-factor secretion signal from *Saccharomyces cerevisiae* and fusions with yeast cell surface proteins such as the Aga2p mating adhesion receptor or the *Arxula adeninivorans* glucoamylase. A protease cleavage site (e.g., the Kex-2 protease) can be engineered to remove the fused sequences from the polypeptides as they exit the secretion pathway. Yeast also is capable of glycosylation at Asn-X-Ser/Thr motifs.

### c. Insects and insect cells

Insects and insect cells, particularly using a baculovirus expression system, are useful for expressing polypeptides such as FVII or modified forms thereof (*see,* for example, Muneta et al. (2003) J. Vet. Med. Sci. 65(2):219-23). Insect cells and insect larvae, including expression in the haemolymph, express high levels of protein and are capable of most of the post-translational modifications used by higher eukaryotes. Baculoviruses have a restrictive host range which improves the safety and reduces regulatory concerns of eukaryotic expression. Typically, expression vectors use a promoter such as the polyhedrin promoter of baculovirus for high level expression. Commonly used baculovirus systems include baculoviruses such as *Autographa californica* nuclear polyhedrosis virus (AcNPV), and the *Bombyx mori* nuclear polyhedrosis virus (BmNPV) and an insect cell line such as Sf9 derived from *Spodoptera frugiperda, Pseudaletia unipuncta* (A7S) and *Danaus plexippus* (DpN1). For high level expression, the nucleotide sequence of the molecule to be expressed is fused immediately downstream of the polyhedrin initiation codon of the virus. Mammalian secretion signals are accurately processed in insect cells and can be used to secrete the expressed protein into the culture medium. In addition, the cell lines *Pseudaletia unipuncta* (A7S) and *Danaus plexippus* (DpN1) produce proteins with glycosylation patterns similar to mammalian cell systems.

An alternative expression system in insect cells is the use of stably transformed cells. Cell lines such as the Schnieder 2 (S2) and Kc cells (*Drosophila melanogaster*) and C7 cells (*Aedes albopictus*) can be used for expression. The Drosophila metallothionein promoter can be used to induce high levels of expression in the presence of heavy metal induction with cadmium or copper. Expression vectors are typically maintained by the use of selectable markers such as neomycin and hygromycin.

**d. Mammalian cells**

Mammalian expression systems can be used to express FVII polypeptides. Expression constructs can be transferred to mammalian cells by viral infection such as adenovirus or by direct DNA transfer such as liposomes, calcium phosphate, DEAE-dextran and by physical means such as electroporation and microinjection. Expression vectors for mammalian cells typically include an mRNA cap site, a TATA box, a translational initiation sequence (Kozak consensus sequence) and polyadenylation elements. Such vectors often include transcriptional promoter-enhancers for high level expression, for example the SV40 promoter-enhancer, the human cytomegalovirus (CMV) promoter, and the long terminal repeat of Rous sarcoma virus (RSV). These promoter-enhancers are active in many cell types. Tissue and cell-type promoters and enhancer regions also can be used for expression. Exemplary promoter/enhancer regions include, but are not limited to, those from genes such as elastase I, insulin, immunoglobulin, mouse mammary tumor virus, albumin, alpha-fetoprotein, alpha 1-antitrypsin, beta-globin, myelin basic protein, myosin light chain-2, and gonadotropic releasing hormone gene control. Selectable markers can be used to select for and maintain cells with the expression construct. Examples of selectable marker genes include, but are not limited to, hygromycin B phosphotransferase, adenosine deaminase, xanthine-guanine phosphoribosyl transferase, aminoglycoside phosphotransferase, dihydrofolate reductase and thymidine kinase. Fusion with cell surface signaling molecules such as TCR-$\zeta$ and $Fc_{\varepsilon}RI$-$\gamma$ can direct expression of the proteins in an active state on the cell surface.

Many cell lines are available for mammalian expression including mouse, rat human, monkey, and chicken and hamster cells. Exemplary cell lines include, but are not limited to, BHK (i.e. BHK-21 cells), 293-F, CHO, Balb/3T3, HeLa, MT2, mouse NS0 (non-secreting) and other myeloma cell lines, hybridoma and heterohybridoma cell lines, lymphocytes, fibroblasts, Sp2/0, COS, NIH3T3, HEK293, 293S, 293T, 2B8, and HKB cells. Cell lines also are available adapted to serum-free media which facilitates purification of secreted proteins from the cell culture media. One such example is the serum free EBNA-1 cell line (Pham et al., (2003) Biotechnol. Bioeng. 84:332-42). Expression of recombinant FVII polypeptides exhibiting similar structure and post-translational modifications as plasma-derived FVII are known in the art (see, *e.g.,* Jurlander et al. (2003) Semin Throm Hemost). Methods of optimizing vitamin K-dependent protein expression are known. For example, supplementation of vitamin K in culture medium or co-expression of vitamin K-dependent $\gamma$-carboxylases (Wajih et al., J. Biol. Chem. 280(36)31603-31607) can aid in post-translational modification of vitamin K-dependent proteins, such as FVII polypeptides.

**e. Plants**

Transgenic plant cells and plants can be used for the expression of FVII. Expression constructs are typically transferred to plants using direct DNA transfer such as microprojectile bombardment and PEG-mediated transfer into protoplasts, and with agrobacterium-mediated transformation. Expression vectors can include promoter and enhancer sequences, transcriptional termination elements, and translational control elements. Expression vectors and transformation techniques are usually divided between dicot hosts, such as Arabidopsis and tobacco, and monocot hosts, such as corn and rice. Examples of plant promoters used for expression include the cauliflower mosaic virus promoter, the nopaline synthase promoter, the ribose bisphosphate carboxylase promoter and the ubiquitin and UBQ3 promoters. Selectable markers such as hygromycin, phosphomannose isomerase and neomycin phosphotransferase are often used to facilitate selection and maintenance of transformed cells. Transformed plant cells can be maintained in culture as cells, aggregates (callus tissue) or regenerated into whole plants. Because plants have different glycosylation patterns than mammalian cells, this can influence the choice to produce FVII in these hosts. Transgenic plant cells also can include algae engineered to produce proteins *(see,* for example, Mayfield et al. (2003) PNAS 100:438-442). Because plants have different glycosylation patterns than mammalian cells, this can influence the choice to produce FVII in these hosts.

**2. Purification**

Methods for purification of FVII polypeptides from host cells depend on the chosen host cells and expression systems. For secreted molecules, proteins are generally purified from the culture media after removing the cells. For intracellular expression, cells can be lysed and the proteins purified from the extract. When transgenic organisms such as transgenic plants and animals are used for expression, tissues or organs can be used as starting material to make a lysed cell extract. Additionally, transgenic animal production can include the production of polypeptides in milk or eggs, which can be collected, and if necessary further the proteins can be extracted and further purified using standard methods in the art.

FVII can be purified using standard protein purification techniques known in the art including but not limited to, SDS-PAGE, size fraction and size exclusion chromatography, ammonium sulfate precipitation, chelate chromatography and ionic exchange chromatography. For example, FVII polypeptides can be purified by anion exchange chromatography. Exemplary of a method to purify FVII polypeptides is by using an ion exchange column that permits binding of any polypeptide that has a functional Gla domain, followed by elution in the presence of calcium (See *e.g.,* Example 3). Affinity purification techniques also can be used to improve the efficiency and purity of the preparations. For example, antibodies, receptors and other molecules that bind FVII can be used in affinity purification. In another example, purification also can be enhanced using a soluble TF (sTF) affinity column (Maun et al. (2005) Prot Sci 14:1171-1180). Expression constructs also can be engineered to add an affinity tag such as a myc epitope, GST fusion or $His_6$ and affinity purified with myc antibody, glutathione resin, and Ni-resin, respectively, to a protein. Purity can be assessed by any method known in the art including gel electrophoresis and staining and spectrophotometric techniques.

The FVII protease can be expressed and purified to be in an inactive form (zymogen form) or alternatively the expressed protease can be purified into an active form, such as by autocatalysis. For example, FVII polypeptides that have been activated via proteolytic cleavage of the $Arg^{152}$-$Ile^{153}$ can be prepared *in vitro* (i.e. FVIIa; two-chain form). The FVII polypeptides can be first prepared by any of the methods of production described herein, including, but not limited to, production in mammalian cells followed by purification. Cleavage of the FVII polypeptides into the active protease form, FVIIa, can be accomplished by several means. For example, autoactivation during incubation with phospholipid vesicles in the presence of calcium can be achieved in 45 minutes (Nelsestuen et al. (2001) J Biol Chem 276:39825-31). FVII polypeptides also can be activated to completion by incubation with factor Xa, factor XIIa or TF in the presence calcium, with or without phospholipids (see *e.g.,* Example 3 and Broze et al. (1980) J Biol Chem 255:1242-1247, Higashi et al. (1996) J Biol Chem 271:26569-26574, Harvey et al. J Biol Chem 278:8363-8369).

### 3. Fusion Proteins

Fusion proteins containing a modified FVII polypeptide and one or more other polypeptides also are provided. Pharmaceutical compositions containing such fusion proteins formulated for administration by a suitable route are provided. Fusion proteins are formed by linking in any order the modified FVII polypeptide and an agent, such as an antibody or fragment thereof, growth factor, receptor, ligand, and other such agent for the purposes of facilitating the purification of a FVII polypeptide, altering the pharmacodynamic properties of a FVII polypeptide by directing, for example, by directing the polypeptide to a targeted cell or tissue, and/or increasing the expression or secretion of the FVII polypeptide. Typically any FVII fusion protein retains at least about 30%, 40%, 50%, 60%, 70%, 80%, 90%, or 95% coagulant activity compared with a non-fusion FVII polypeptide, including 96%, 97%, 98%, 99% or greater coagulant activity compared with a non-fusion polypeptide.

Linkage of a FVII polypeptide with another polypeptide can be effected directly or indirectly via a linker. In one example, linkage can be by chemical linkage, such as via heterobifunctional agents or thiol linkages or other such linkages. Fusion also can be effected by recombinant means. Fusion of a FVII polypeptide to another polypeptide can be to the N- or C- terminus of the FVII polypeptide. Non-limiting examples of polypeptides that can be used in fusion proteins with a FVII polypeptide provided herein include, for example, a GST (glutathione S-transferase) polypeptide, Fc domain from immunoglobulin G, or a heterologous signal sequence. The fusion proteins can contain additional components, such as *E. coli* maltose binding protein (MBP) that aid in uptake of the protein by cells *(see,* International PCT application No. WO 01/32711).

A fusion protein can be produced by standard recombinant techniques. For example, DNA fragments coding for the different polypeptide sequences can be ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another example, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, *e.g.*, Ausubel et al. (eds.) CURRENT PROTOCOLS IN MOLECULAR BIOLOGY, John Wiley & Sons, 1992). Moreover, many expression vectors are commercially available that already encode a fusion moiety (*e.g.*, a GST polypeptide). A FVII-encoding nucleic acid can be cloned into such an expression vector such that the fusion moiety is linked in-frame to the protease protein.

### 4. Polypeptide modification

Modified FVII polypeptides can be prepared as naked polypeptide chains or as a complex. For some applications, it can be desirable to prepare modified FVII in a "naked" form without post-translational or other chemical modifications. Naked polypeptide chains can be prepared in suitable hosts that do not post-translationally modify FVIL Such polypeptides also can be prepared in in *vitro* systems and using chemical polypeptide synthesis. For other applications, particular modifications can be desired including pegylation, albumination, glycosylation, carboxylation, hydroxylation, phosphorylation, or other known modifications. Modifications can be made *in vitro* or, for example, by producing the modified FVII in a suitable host that produces such modifications.

### 5. Nucleotide sequences

Nucleic acid molecules encoding FVII or modified FVII polypeptides are provided herein. Nucleic acid molecules include allelic variants or splice variants of any encoded FVII polypeptide. Exemplary of nucleic acid molecules provided herein are any that encode a modified FVII polypeptide provided herein, such as any encoding a polypeptide set forth in any of SEQ ID NOS: 18-43, 125-150 or 206-250. In one embodiment, nucleic acid molecules provided herein have at least 50, 60, 65, 70, 75, 80, 85, 90, 91, 92, 93, 94, 95, or 99% sequence identity or hybridize under conditions of medium or high stringency along at least 70% of the full-length of any nucleic acid encoding a FVII polypeptide provided herein. In another embodiment, a nucleic acid molecule can include those with degenerate codon sequences encoding any of the FVII polypeptides provided herein.

## G. Assessing modified FVII polypeptide activities

The activities and properties of FVII polypeptides can be assessed *in vitro* and/or *in vivo.* Assays for such assessment are known to those of skill in the art and are known to correlate tested activities and results to therapeutic and *in vivo* activities. In one example, FVII variants can be assessed in comparison to unmodified and/or wild-type FVII. In another example, the activity of modified FVII polypeptides can be assessed following exposure *in vitro* or *in vivo* to TFPI or AT-III and compared with that of modified FVII polypeptides that have not been exposed to TFPI or AT-III. *In vitro* assays include any laboratory assay known to one of skill in the art, such as for example, cell-based assays including coagulation assays, binding assays, protein assays, and molecular biology assays. *In vivo* assays include FVII assays in animal models as well as administration to humans. In some cases, activity of FVII *in vivo* can be determined by assessing blood, serum, or other bodily fluid for assay determinants. FVII variants also can be tested *in vivo* to assess an activity or property, such as therapeutic effect.

Typically, assays described herein are with respect to the two-chain activated form of FVII, i.e. FVIIa. Such assays also can be performed with the single chain form, such as to provide a negative control since such form typically does not contain proteolytic or catalytic activity required for the coagulant activity of FVII. In addition, such assays also can be performed in the presence of cofactors, such as TF, which in some instances augments the activity of FVII.

### *1. In vitro* assays

Exemplary *in vitro assays* include assays to assess polypeptide modification and activity. Modifications can be assessed using *in vitro* assays that assess γ-carboxylation and other post-translational modifications, protein assays and conformational assays known in the art. Assays for activity include, but are not limited to, measurement of FVII interaction with other coagulation factors, such as TF, factor X and factor IX, proteolytic assays to determine the proteolytic activity of FVII polypeptides, assays to determine the binding and/or affinity of FVII polypeptides for phosphatidylserines and other phospholipids, and cell based assays to determine the effect of FVII polypeptides on coagulation.

Concentrations of modified FVII polypeptides can be assessed by methods well-known in the art, including but not limited to, enzyme-linked immunosorbant assays (ELISA), SDS-PAGE; Bradford, Lowry, BCA methods; UV absorbance, and other quantifiable protein labeling methods, such as, but not limited to, immunological, radioactive and fluorescent methods and related methods.

Assessment of cleavage products of proteolysis reactions, including cleavage of FVII polypeptides or products produced by FVII protease activity, can be performed using methods including, but not limited to, chromogenic substrate cleavage, HPLC, SDS-PAGE analysis,ELISA, Western blotting, immunohistochemistry, immunoprecipitation, NH2-terminal sequencing, and protein labeling.

Structural properties of modified FVII polypeptides can also be assessed. For example, X-ray crystallography, nuclear magnetic resonance (NMR), and cryoelectron microscopy (cryo-EM) of modified FVII polypeptides can be performed to assess three-dimensional structure of the FVII polypeptides and/or other properties of FVII polypeptides, such as $Ca^{2+}$ or cofactor binding.

Additionally, the presence and extent of FVII degradation can be measured by standard techniques such as sodium dodecyl sulfate polyacrylamide gel electrophoresis (SDS-PAGE), and Western blotting of electrophoresed FVII-containing samples. FVII polypeptides that have been exposed to proteases can also be subjected to N-terminal sequencing to determine location or changes in cleavage sites of the modified FVII polypeptides.

**a. Post-translational modification**

FVII polypeptides also can be assessed for the presence of post-translational modifications. Such assays are known in the art and include assays to measure glycosylation, hydroxylation, and carboxylation. In an exemplary assay for glycosylation, carbohydrate analysis can be performed, for example, with SDS page analysis of FVII polypeptides exposed to hydrazinolysis or endoglycosidase treatment. Hydrazinolysis releases N- and O-linked glycans from glycoproteins by incubation with anhydrous hydrazine, while endoglycosidase release involves PNGase F, which releases most N-glycans from glycoproteins. Hydrazinolysis or endoglycosidase treatment of FVII polypeptides generates a reducing terminus that can be tagged with a fluorophore or chromophore label. Labeled FVII polypeptides can be analyzed by fluorophore-assisted carbohydrate electrophoresis (FACE). The fluorescent tag for glycans also can be used for monosaccharide analysis, profiling or fingerprinting of complex glycosylation patterns by HPLC. Exemplary HPLC methods include hydrophilic interaction chromatography, electronic interaction, ionexchange, hydrophobic interaction, and size-exclusion chromatography. Exemplary glycan probes include, but are not limited to, 3-(acetylamino)-6-aminoacridine (AA-Ac) and 2-aminobenzoic acid (2-AA). Carbohydrate moieties can also be detected through use of specific antibodies that recognize the glycosylated FVII polypeptide. An exemplary assay to measure β-hydroxylation comprises reverse phase HPLC analysis of FVII polypeptides that have been subjected to alkaline hydrolysis (Przysiecki et al. (1987) PNAS 84:7856-7860). Carboxylation and γ-carboxylation of FVII polypeptides can be assessed using mass spectrometry with matrix-assisted laser desorption ionization time-of-flight (MALDI-TOF) analysis, as described in the art (*se, e.g.* Harvey et al. J Biol Chem 278:8363-8369, Maum et al. Prot Sci 14:1171-1180). The interaction of a FVII polypeptide containing the propeptide (pro-FVII) with the carboxylase responsible for post-translational γ-carboxylate modification also can be assessed. The dissociation constant ($K_d$) following incubation of carboxylase with flourescin-labeled pro-FVII polypeptides can be measured by determining the amount of bound carboxylase by anisotropy (Lin et al. (2004) J Biol Chem 279:6560-6566).

**b. Proteolytic activity**

Modified FVII polypeptides can be tested for proteolytic activity. The proteolytic activity of FVII can be measured using chromogenic substrates such as Chromozym t-PA ($MeSO_2$-D-Phe-Gly-Arg-pNA), S-2288 (H-D-Ile- Pro- Arg-pNA), S-2266 (H-D-Val-Leu-Arg-pNA), S-2765 (Z-D-Arg-Gly-Arg-pNA), Spectrozyme FXa and Spectrozyme FVIIa (CH3SO2-D-CHA-But-Arg-pNA). FVII polyeptides, alone or in the presence of TF, are incubated with varying concentrations of chromogenic substrate. Cleavage of the substrate can be monitored by absorbance and the rate of substrate hydrolysis determined by linear regression using software readily available.

The activation of coagulation factor substrates, such as FX, by FVII polypeptides also can be assessed. FVII polypeptides, with or without preincubation with TF, can be incubated with purified FX (available commercially). The amount of active FXa produced as a consequence of incubation with FVII polypeptides is measured as activity of FXa for a chromogenic substrate, such as S-2222 or Spectrafluor FXa (CH3SO2-D-CHA-Gly-Arg-AMC.AcOH), which is monitored via absorbance changes (Harvey et al. J Biol Chem 278:8363-8369, *see also* Example 5 below). A source of phospholipid also can be included in the incubation of FVII and FX (Nelsestuen et al. (2001) J Biol Chem 276:39825-31).

**c. Coagulation activity**

FVII polypeptides can be tested for coagulation activity by using assays well known in the art. For example, some of the assays include, but are not limited to, a two stage clotting assay (Leibman et al., (1985) PNAS 82:3879-3883); the prothrombin time assay (PT, which can measure TF-dependent activity of FVIIa in the extrinsic pathway); assays which are modifications of the PT test; the activated partial thromboplastin time (aPTT, which can measure TF-independent activity of FVIIa); activated clotting time (ACT); recalcified activated clotting time; the Lee-White Clotting time; or thromboelastography (TEG) (Pusateri et al. (2005) Critical Care 9:S15-S24). For example, coagulation activity of a modified FVII polypeptide can be determined by a PT-based assay where FVII is diluted in FVII-deficient plasma, and mixed with prothrombin time reagent (recombinant TF with phospholipids and calcium), such as that available as Innovin™ from Dade Behring. Clot formation is detected optically and time to clot is determined and compared against FVII-deficient plasma alone.

**d. Binding to and/or inhibition by other proteins**

Inhibition assays can be used to measure resistance of modified FVII polypeptides to FVII inhibitors, such as, for example, TFPI and AT-III, or molecules such as $Zn^{2+}$. Assessment of inhibition to other inhibitors also can be tested and include, but are not limited to, other serine protease inhibitors, and FVII-specific antibodies. Inhibition can be assessed by incubation of, for example, TFPI, AT-III or $Zn^{2+}$ with FVII polypeptides that have been preincubated with TF. The activity of FVII can then be measured using any one or more of the activity or coagulation assays described above, and inhibition by TFPI, AT-III or $Zn^{2+}$ can be assessed by comparing the activity of FVII polypeptides incubated with the inhibitor, with the activity of FVII polypeptides that were not incubated with the inhibitor.

FVII polypeptides can be tested for binding to other coagulation factors and inhibitors. For example, FVII direct and indirect interactions with cofactors, such as TF, substrates, such as FX and FIX, and inhibitors, such as TFPI, antithrombin III and heparin can be assessed using any binding assay known in the art, including, but not limited to, immunoprecipitation, column purification, non-reducing SDS-PAGE, BIAcore® assays, surface plasmon resonance (SPR), fluorescence resonance energy transfer (FRET), fluorescence polarization (FP), isothermal titration calorimetry (ITC), circular dichroism (CD), protein fragment complementation assays (PCA), Nuclear Magnetic Resonance (NMR) spectroscopy, light scattering, sedimentation equilibrium, small-zone gel filtration chromatography, gel retardation, Far-western blotting, fluorescence polarization, hydroxyl-radical protein footprinting, phage display, and various two-hybrid systems. In one example, $Zn^{2+}$ binding is assessed using equilibrium analysis (Petersen et al., (2000) Protein Science 9:859-866)

**e. Phospholipid affinity**

Modified FVII polypeptide binding and/or affinity for phosphotidlyserine (PS) and other phospholipids can be determined using assays well known in the art. Highly pure phospholipids (for example, known concentrations of bovine PS and egg phosphatidylcholine (PC), which are commercially available, such as from Sigma, in organic solvent can be used to prepare small unilamellar phospholipid vesicles. FVII polypeptide binding to these PS/PC vesicles can be determined by relative light scattering at 90° to the incident light. The intensity of the light scatter with PC/PS alone and with PC/PS/FVII is measured to determine the dissociation constant (Harvey et al. J Biol Chem 278:8363-8369). Surface plasma resonance, such as on a BIAcore biosensor instrument, also can be used to measure the affinity of FVII polypeptides for phospholipid membranes (Sun et al. Blood 101:2277-2284).

**2. Non-human animal models**

Non-human animal models can be used to assess activity, efficacy and safety of modified FVII polypeptides. For example, non-human animals can be used as models for a disease or condition. Non-human animals can be injected with disease and/or phenotype-inducing substances prior to administration of FVII variants, such as any FVII variant set forth in any of SEQ ID NOS: 18-43, 125-150 or 206-250, to monitor the effects on disease progression. Genetic models also are useful. Animals, such as mice, can be generated which mimic a disease or condition by the overexpression, underexpression or knock-out of one or more genes, such as, for example, factor VIII knock-out mice that display hemophilia A (Bi et al. (1995) Nat Gen 10:119-121). Such animals can be generated by transgenic animal production techniques well-known in the art or using naturally-occurring or induced mutant strains. Examples of useful non-human animal models of diseases associated with FVII include, but are not limited to, models of bleeding disorders, in particular hemophilia, or thrombotic disease. Non-human animal models for injury also can be used to assess an activity, such as the coagulation activity, of FVII polypeptides. These non-human animal models can be used to monitor activity of FVII variants compared to a wild type FVII polypeptide.

Animal models also can be used to monitor stability, half-life, and clearance of modified FVII polypeptides. Such assays are useful for comparing modified FVII polypeptides and for calculating doses and dose regimens for further non-human animal and human trials. For example, a modified FVII polypeptide, such as any FVII variant provided herein including, for example, any set forth in any of SEQ ID NOS: 18-43, 125-150 or 206-250, can be injected into the tail vein of mice. Blood samples are then taken at time-points after injection (such as minutes, hours and days afterwards) and then the level of the modified FVII polypeptides in bodily samples including, but not limited to, serum or plasma can be monitored at specific time-points for example by ELISA or radioimmunoassay. Blood samples from various time points following injection of the FVII polypeptides also be tested for coagulation activity using various methods methods, such as is described in Examples 9 and 14. These types of pharmacokinetic studies can provide information regarding half-life, clearance and stability of the FVII polypeptides, which can assist in determining suitable dosages for administration as a procoagulant.

Modified FVII polypeptides, such as any set forth in any of SEQ ID NOS: 18-. 43, 125-150 or 206-250, can be tested for therapeutic effectiveness using animal models for hemophilia. In one non-limiting example, an animal model such as a mouse can be used. Mouse models of hemophilia are available in the art and can be employed to test modified FVII polypeptides. For example, a mouse model of hemophilia A that is produced by injection with anti-FVIII antibodies can be used to assess the coagulant activity of FVII polypeptides (*see e.g.* Examples 8 and 14, and Tranholm et al. Blood (2003)102:3615-3620). A mouse model of hemophilia B also can be used to test FVII polypeptides (Margaritis et al. (2004) J Clin Invest 113:1025-1031). Non-mouse models of bleeding disorders also exist. FVII polypeptide activity can be assessed in rats with warfarin-induced bleeding or melagatran-induced bleeding (Diness et al. (1992) Thromb Res 67:233-241, Elg et al. (2001) Thromb Res 101:145-157), and rabbits with heparin-induced bleeding (Chan et al. (2003) J Thromb Haemost 1:760-765). Inbred hemophilia A, hemophilia B and von Willebrand disease dogs that display severe bleeding also can be used in non-human animal studies with FVII polypeptides (Brinkhous et al. (1989) PNAS 86:1382-1386). The activity of FVII polypeptides also can be assessed in a rabbit model of bleeding in which thrombocytopenia is induced by a combination of gamma-irradiation and the use of platelet antibodies (Tranholm et al. (2003) Thromb Res 109:217-223).

In addition to animals with generalized bleeding disorders, injury and trauma models also can be used to evaluate the activity of FVII polypeptides, and their safety and efficacy as a coagulant therapeutic. Non-limiting examples of such models include a rabbit coronary stenosis model (Fatorutto et al. (2004) Can J Anaesth 51:672-679), a grade V liver injury model in pigs (Lynn et al. (2002) J Trauma 52:703-707), a grade V liver injury model in pigs (Martinowitz et al. (2001) J Trauma 50:721-729) and a pig aortotomy model (Sondeem et al. (2004) Shock 22:163-168).

### 3. Clinical Assays

Many assays are available to assess activity of FVII for clinical use. Such assays can include assessment of coagulation, protein stability and half-life *in vivo,* and phenotypic assays. Phenotypic assays and assays to assess the therapeutic effect of FVII treatment include assessment of blood levels of FVII (*e.g.* measurement of serum FVII prior to administration and time-points following administrations including, after the first administration, immediately after last administration, and time-points in between, correcting for the body mass index (BMI)), assessment of blood coagulation *in vitro* using the methods described above following treatment with FVII (*e.g.* PT assay), and phenotypic response to FVII treatment including amelioration of symptoms over time compared to subjects treated with an unmodified and/or wild type FVII or placebo. Patients treated with FVII polypeptides can be monitored for blood loss, transfusion requirement, and hemoglobin. Patients can be monitored regularly over a period of time for routine or repeated administrations, or following administration in response to acute events, such as hemorrhage, trauma, or surgical procedures.

## H. Formulation and Administration

Compositions for use in treatment of bleeding disorders are provided herein. Such compositions contain a therapeutically effective amount of a factor VII polypeptide as described herein. Effective concentrations of FVII polypeptides or pharmaceutically acceptable derivatives thereof are mixed with a suitable pharmaceutical carrier or vehicle for systemic, topical or local administration. Compounds are included in an amount effective for treating the selected disorder. The concentration of active compound in the composition will depend on absorption, inactivation, excretion rates of the active compound, the dosage schedule, and amount administered as well as other factors known to those of skill in the art.

Pharmaceutical carriers or vehicles suitable for administration of the compounds provided herein include any such carriers known to those skilled in the art to be suitable for the particular mode of administration. Pharmaceutical compositions that include a therapeutically effective amount of a FVII polypeptide described herein also can be provided as a lyophilized powder that is reconstituted, such as with sterile water, immediately prior to administration.

### 1. Formulations

Pharmaceutical compositions containing a modified FVII can be formulated in any conventional manner by mixing a selected amount of the polypeptide with one or more physiologically acceptable carriers or excipients. Selection of the carrier or excipient is within the skill of the administering profession and can depend upon a number of parameters. These include, for example, the mode of administration (i.e., systemic, oral, nasal, pulmonary, local, topical, or any other mode) and disorder treated. The pharmaceutical compositions provided herein can be formulated for single dosage (direct) administration or for dilution or other modification. The concentrations of the compounds in the formulations are effective for delivery of an amount, upon administration, that is effective for the intended treatment. Typically, the compositions are formulated for single dosage administration. To formulate a composition, the weight fraction of a compound or mixture thereof is dissolved, suspended, dispersed, or otherwise mixed in a selected vehicle at an effective concentration such that the treated condition is relieved or ameliorated.

The modified FVII polypeptides provided herein can be formulated for administration to a subject as a two-chain FVIIa protein. The modified FVII polypeptides can be activated by any method known in the art prior to formulation. For example, FVII can undergo autoactivation during purification by ion exchange chromatography (Jurlander et al. (2001) Semin Thromb Hemost 27:373-384). The modified FVII polypeptides also can be activated by incubation with FXa immobilized on beads (Kemball-Cook et al. (1998) J Biol Chem 273:8516-8521), or any other methods known in the art (see also Example 3 below). The inclusion of calcium in these processes ensures full activation and correct folding of the modified FVIIa protein. The modified FVII polypeptides provided herein also can be formulated for administration as a single chain protein. The single-chain FVII polypeptides can be purified in such a way as to prevent cleavage (see, *e.g.,* US6677440). The modified FVII polypeptides provided herein can be formulated such that the single-chain and two-chain forms are contained in the pharmaceutical composition, in any ratio by appropriate selection of the medium to eliminate or control autoactivation.

The compound can be suspended in micronized or other suitable form or can be derivatized to produce a more soluble active product. The form of the resulting mixture depends upon a number of factors, including the intended mode of administration and the solubility of the compound in the selected carrier or vehicle. The resulting mixtures are solutions, suspensions, emulsions and other such mixtures, and can be formulated as an non-aqueous or aqueous mixture, creams, gels, ointments, emulsions, solutions, elixirs, lotions, suspensions, tinctures, pastes, foams, aerosols, irrigations, sprays, suppositories, bandages, or any other formulation suitable for systemic, topical or local administration. For local internal administration, such as, intramuscular, parenteral or intra-articular administration, the polypeptides can be formulated as a solution suspension in an aqueous-based medium, such as isotonically buffered saline or are combined with a biocompatible support or bioadhesive intended for internal administration. The effective concentration is sufficient for ameliorating the targeted condition and can be empirically determined. To formulate a composition, the weight fraction of compound is dissolved, suspended, dispersed, or otherwise mixed in a selected vehicle at an effective concentration such that the targeted condition is relieved or ameliorated.

Generally, pharmaceutically acceptable compositions are prepared in view of approvals for a regulatory agency or other prepared in accordance with generally recognized pharmacopeia for use in animals and in humans. Pharmaceutical compositions can include carriers such as a diluent, adjuvant, excipient, or vehicle with which an isoform is administered. Such pharmaceutical carriers can be sterile liquids, such as water and oils, including those of petroleum, animal, vegetable or synthetic origin, such as peanut oil, soybean oil, mineral oil, and sesame oil. Water is a typical carrier when the pharmaceutical composition is administered intravenously. Saline solutions and aqueous dextrose and glycerol solutions also can be employed as liquid carriers, particularly for injectable solutions. Compositions can contain along with an active ingredient: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polvinylpyrrolidine, celluloses and derivatives thereof, povidone, crospovidones and other such binders known to those of skill in the art. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, and ethanol. A composition, if desired, also can contain minor amounts of wetting or emulsifying agents, or pH buffering agents, for example, acetate, sodium citrate, cyclodextrine derivatives, sorbitan monolaurate, triethanolamine sodium acetate, triethanolamine oleate, and other such agents. These compositions can take the form of solutions, suspensions, emulsion, tablets, pills, capsules, powders, and sustained release formulations. Capsules and cartridges of *e.g.,* gelatin for use in an inhaler or insufflator can be formulated containing a powder mix of a therapeutic compound and a suitable powder base such as lactose or starch. A composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, and other such agents. Preparations for oral administration also can be suitably formulated with protease inhibitors, such as a Bowman-Birk inhibitor, a conjugated Bowman-Birk inhibitor, aprotinin and camostat. Examples of suitable pharmaceutical carriers are described in "Remington's Pharmaceutical Sciences" by E. W. Martin. Such compositions will contain a therapeutically effective amount of the compound, generally in purified form, together with a suitable amount of carrier so as to provide the form for proper administration to a subject or patient.

The formulation should suit the mode of administration. For example, the modified FVII can be formulated for parenteral administration by injection (e.g., by bolus injection or continuous infusion). The injectable compositions can take such forms as suspensions, solutions or emulsions in oily or aqueous vehicles. The sterile injectable preparation also can be a sterile injectable solution or suspension in a nontoxic parenterally-acceptable diluent or solvent, for example, as a solution in 1-4, butanediol. Sterile, fixed oils are conventionally employed as a solvent or suspending medium. For this purpose any bland fixed oil can be employed, including, but not limited to, synthetic mono- or diglycerides, fatty acids (including oleic acid), naturally occurring vegetable oils like sesame oil, coconut oil, peanut oil, cottonseed oil, and other oils, or synthetic fatty vehicles like ethyl oleate. Buffers, preservatives, antioxidants, and the suitable ingredients, can be incorporated as required, or, alternatively, can comprise the formulation.

The polypeptides can be formulated as the sole pharmaceutically active ingredient in the composition or can be combined with other active ingredients. The polypeptides can be targeted for delivery, such as by conjugation to a targeting agent, such as an antibody. Liposomal suspensions, including tissue-targeted liposomes, also can be suitable as pharmaceutically acceptable carriers. These can be prepared according to methods known to those skilled in the art. For example, liposome formulations can be prepared as described in U.S. Patent No. 4,522,811. Liposomal delivery also can include slow release formulations, including pharmaceutical matrices such as collagen gels and liposomes modified with fibronectin (*see,* for example, Weiner et al. (1985) J Pharm Sci. 74(9): 922-5). The compositions provided herein further can contain one or more adjuvants that facilitate delivery, such as, but are not limited to, inert carriers, or colloidal dispersion systems. Representative and non-limiting examples of such inert carriers can be selected from water, isopropyl alcohol, gaseous fluorocarbons, ethyl alcohol, polyvinyl pyrrolidone, propylene glycol, a gel-producing material, stearyl alcohol, stearic acid, spermaceti, sorbitan monooleate, methylcellulose, as well as suitable combinations of two or more thereof. The active compound is included in the pharmaceutically acceptable carrier in an amount sufficient to exert a therapeutically useful effect in the absence of undesirable side effects on the subject treated. The therapeutically effective concentration can be determined empirically by testing the compounds in known *in vitro* and *in vivo* systems, such as the assays provided herein.

### a. Dosages

The precise amount or dose of the therapeutic agent administered depends on the particular FVII polypeptide, the route of administration, and other considerations, such as the severity of the disease and the weight and general state of the subject. Local administration of the therapeutic agent will typically require a smaller dosage than any mode of systemic administration, although the local concentration of the therapeutic agent can, in some cases, be higher following local administration than can be achieved with safety upon systemic administration. If necessary, a particular dosage and duration and treatment protocol can be empirically determined or extrapolated. For example, exemplary doses of recombinant and native FVII polypeptides can be used as a starting point to determine appropriate dosages. For example, a recombinant FVII (rFVIIa) polypeptide that has been activated to rFVIIa, Novoseven®, has been administered to patients with hemophilia A or hemophilia B, who are experiencing a bleeding episode, at a dosage of 90 μg/kg by bolus infusion over 2 to 5 minutes, achieving an effective circulating level of at least 2 μg/ml. The dose is repeated every 2 hours until hemostasis is achieved. The modified FVII polypeptides provided herein can be effective at reduced dosage amounts and/or frequencies compared to such a recombinant FVII. For example, at the modified FVII polypeptides provided herein can be administered at a dosage of 80 μg/kg, 70 μg/kg, 60 μg/kg, 50 μg/kg, 40 μg/kg, 30 μg/kg, 20 μg/kg, 15 μg/kg or less. In some examples, the dosages can be higher, such as 100 μg/kg, 110 μg/kg, 120 μg/kg, or higher. The duration of treatment and the interval between injections will vary with the severity of the bleed and the response of the patient to the treatment, and can be adjusted accordingly. Factors such as the level of activity and half-life of the modified FVII in comparison to the unmodified FVII can be taken into account when making dosage determinations. Particular dosages and regimens can be empirically determined.

In another example, a recombinant FVII (rFVIIa) polypeptide that has been activated to rFVIIa, Novoseven®, has been administered to patients with congenital FVII deficiency who are experiencing a bleeding episode, at a dosage of 15-30 μg/kg by bolus infusion over 2 to 5 minutes. The dose is repeated every 4-6 hours until hemostasis is achieved. The modified FVII polypeptides provided herein can be effective at reduced dosage amounts and/or frequencies compared to such a recombinant FVIL For example, the modified FVII polypeptides provided herein can be administered at a dosage of 20 μg/kg, 15 μg/kg, 10 μg/kg, 5 μg/kg, 3 μg/kg or less. In some examples, the dosages can be higher, such as 35 μg/kg, 40 μg/kg, 45 μg/kg, or higher. The duration of treatment and the interval between injections will vary with the severity of the bleed and the response of the patient to the treatment, and can be adjusted accordingly. Factors such as the level of activity and half-life of the modified FVII in comparison to the unmodified FVII can be used in making dosage determinations. For example, a modified FVII polypeptide that exhibits a longer half-life than an unmodidief FVII polypeptide can be administered at lower doses and/or less frequently than the unmodified FVII polypeptide. Similarly, the dosages required for thereapeutic effect using a modified FVII polypeptide that displays increased coagulant activity compared with an unmodified FVII polypeptide can be reduced in frequency and amount. Particular dosages and regimens can be empirically determined by one of skill in the art.

### b. Dosage forms

Pharmaceutical therapeutically active compounds and derivatives thereof are typically formulated and administered in unit dosage forms or multiple dosage forms. Formulations can be provided for administration to humans and animals in dosage forms that include, but are not limited to, tablets, capsules, pills, powders, granules, sterile parenteral solutions or suspensions, oral solutions or suspensions, and oil water emulsions containing suitable quantities of the compounds or pharmaceutically acceptable derivatives thereof. Each unit dose contains a predetermined quantity of therapeutically active compound sufficient to produce the desired therapeutic effect, in association with the required pharmaceutical carrier, vehicle or diluent. Examples of unit dose forms include ampoules and syringes and individually packaged tablets or capsules. In some examples, the unit dose is provided as a lyophilized powder that is reconstituted prior to administration. For example, a FVII polypeptide can be provided as lyophilized powder that is reconstituted with a suitable solution to generate a single dose solution for injection. In some examples, the lyophilized powder can contain the FVII polypeptide and additional components, such as salts, such that reconstitution with sterile distilled water results in a FVII polypeptide in a buffered or saline solution. Unit dose forms can be administered in fractions or multiples thereof. A multiple dose form is a plurality of identical unit dosage forms packaged in a single container to be administered in segregated unit dose form. Examples of multiple dose forms include vials, bottles of tablets or capsules or bottles of pints or gallons. Hence, multiple dose form is a multiple of unit doses that are not segregated in packaging.

### 2. Administration of modified FVII polypeptides

The FVII polypeptides provided herein (*i.e*. active compounds) can be administered *in vitro, ex vivo,* or *in vivo* by contacting a mixture, such as a body fluid or other tissue sample, with a FVII polypeptide. For example, when administering a compound *ex vivo,* a body fluid or tissue sample from a subject can be contacted with the FVII polypeptides that are coated on a tube or filter, such as for example, a tube or filter in a bypass machine. When administering *in vivo,* the active compounds can be administered by any appropriate route, for example, orally, nasally, pulmonary, parenterally, intravenously, intradermally, subcutaneously, intraarticularly, intracisternally, intraocularly, intraventricularly, intrathecally, intramuscularly, intraperitoneally, intratracheally or topically, as well as by any combination of any two or more thereof, in liquid, semi-liquid or solid form and are formulated in a manner suitable for each route of administration. The modified FVII polypeptides can be administered once or more than once, such as twice, three times, four times, or any number of times that are required to achieve a therapeutic effect. Multiple administrations can be effected via any route or combination of routes, and can be administered hourly, every 2 hours, every three hours, every four hours or more.

The most suitable route for administration will vary depending upon the disease state to be treated, for example the location of the bleeding disorder. Generally, the FVII polypeptides will be administered by intravenous bolus injection, with an administration (infusing) time of approximately 2-5 minutes. In other examples, desirable blood levels of FVII can be maintained by a continuous infusion of the active agent as ascertained by plasma levels. It should be noted that the attending physician would know how to and when to terminate, interrupt or adjust therapy to lower dosage due to toxicity, or bone marrow, liver or kidney dysfunctions. Conversely, the attending physician would also know how to and when to adjust treatment to higher levels if the clinical response is not adequate (precluding toxic side effects). In other examples, the location of the bleeding disorder might indicate that the FVII formulation is administered via alternative routes. For example, local administration, including administration into the brain (*e.g.,* intraventricularly) might be performed when the patient is experiencing bleeding in this region. Similarly, for treatment of bleeding in the joints, local administration by injection of the therapeutic agent into the joint (i.e., intraarticularly, intravenous or subcutaneous means) can be employed. In other examples, topical administration of the therapeutic agent to the skin, for example formulated as a cream, gel, or ointment, or administration to the lungs by inhalation or intratracheally, might be appropriate when the bleeding is localized to these areas.

The instances where the modified FVII polypeptides are be formulated as a depot preparation, the long-acting formulations can be administered by implantation (for example, subcutaneously or intramuscularly) or by intramuscular injection. Thus, for example, the therapeutic compounds can be formulated with suitable polymeric or hydrophobic materials (for example as an emulsion in an acceptable oil) or ion exchange resins, or as sparingly soluble derivatives, for example, as a sparingly soluble salt.

The compositions, if desired, can be presented in a package, in a kit or dispenser device, that can contain one or more unit dosage forms containing the active ingredient. The package, for example, contains metal or plastic foil, such as a blister pack. The pack or dispenser device can be accompanied by instructions for administration. The compositions containing the active agents can be packaged as articles of manufacture containing packaging material, an agent provided herein, and a label that indicates the disorder for which the agent is provided.

### 3. Administration of nucleic acids encoding modified FVII polypeptides (gene therapy)

Also provided are compositions of nucleic acid molecules encoding the modified FVII polypeptides and expression vectors encoding them that are suitable for gene therapy. Rather than deliver the protein, nucleic acid can be administered *in vivo,* such as systemically or by other route, or *ex vivo,* such as by removal of cells, including lymphocytes, introduction of the nucleic therein, and reintroduction into the host or a compatible recipient.

Modified FVII polypeptides can be delivered to cells and tissues by expression of nucleic acid molecules. Modified FVII polypeptides can be administered as nucleic acid molecules encoding modified FVII polypeptides, including *ex vivo* techniques and direct *in vivo* expression. Nucleic acids can be delivered to cells and tissues by any method known to those of skill in the art. The isolated nucleic acid sequences can be incorporated into vectors for further manipulation. As used herein, vector (or plasmid) refers to discrete elements that are used to introduce heterologous DNA into cells for either expression or replication thereof. Selection and use of such vehicles are well within the skill of the artisan.

Methods for administering modified FVII polypeptides by expression of encoding nucleic acid molecules include administration of recombinant vectors. The vector can be designed to remain episomal, such as by inclusion of an origin of replication or can be designed to integrate into a chromosome in the cell. Modified FVII polypeptides also can be used in *ex vivo* gene expression therapy using non-viral vectors. For example, cells can be engineered to express a modified FVII polypeptide, such as by integrating a modified FVII polypeptide encoding-nucleic acid into a genomic location, either operatively linked to regulatory sequences or such that it is placed operatively linked to regulatory sequences in a genomic location. Such cells then can be administered locally or systemically to a subject, such as a patient in need of treatment.

Viral vectors, include, for example adenoviruses, adeno-associated viruses (AAV), poxviruses, herpes viruses, retroviruses and others designed for gene therapy can be employed. The vectors can remain episomal or can integrate into chromosomes of the treated subject. A modified FVII polypeptide can be expressed by a virus, which is administered to a subject in need of treatment. Viral vectors suitable for gene therapy include adenovirus, adeno-associated virus (AAV), retroviruses, lentiviruses, vaccinia viruses and others noted above. For example, adenovirus expression technology is well-known in the art and adenovirus production and administration methods also are well known. Adenovirus serotypes are available, for example, from the American Type Culture Collection (ATCC, Rockville, MD). Adenovirus can be used *ex vivo,* for example, cells are isolated from a patient in need of treatment, and transduced with a modified FVII polypeptide-expressing adenovirus vector. After a suitable culturing period, the transduced cells are administered to a subject, locally and/or systemically. Alternatively, modified FVII polypeptide-expressing adenovirus particles are isolated and formulated in a pharmaceutically-acceptable carrier for delivery of a therapeutically effective amount to prevent, treat or ameliorate a disease or condition of a subject. Typically, adenovirus particles are delivered at a dose ranging from 1 particle to $10^{14}$ particles per kilogram subject weight, generally between $10^6$ or $10^8$ particles to $10^{12}$ particles per kilogram subject weight. In some situations it is desirable to provide a nucleic acid source with an agent that targets cells, such as an antibody specific for a cell surface membrane protein or a target cell, or a ligand for a receptor on a target cell. FVII also can be targeted for delivery into specific cell types. For example, adenoviral vectors encoding FVII polypeptides can be used for stable expression in nondividing cells, such as liver cells (Margaritis et al. (2004) J Clin Invest 113:1025-1031). In another example, viral or nonviral vectors encoding FVII polypeptides can be transduced into isolated cells for subsequent delivery. Additional cell types for expression and delivery of FVII might include, but are not limited to, fibroblasts and endothelial cells.

The nucleic acid molecules can be introduced into artificial chromosomes and other non-viral vectors. Artificial chromosomes, such as ACES (see, Lindenbaum et al. (2004) Nucleic Acids Res. 32(21):e172) can be engineered to encode and express the isoform. Briefly, mammalian artificial chromosomes (MACs) provide a means to introduce large payloads of genetic information into the cell in an autonomously replicating, non-integrating format. Unique among MACs, the mammalian satellite DNA-based Artificial Chromosome Expression (ACE) can be reproducibly generated *de novo* in cell lines of different species and readily purified from the host cells' chromosomes. Purified mammalian ACEs can then be re-introduced into a variety of recipient cell lines where they have been stably maintained for extended periods in the absence of selective pressure using an ACE System. Using this approach, specific loading of one or two gene targets has been achieved in LMTK(-) and CHO cells.

Another method for introducing nucleic acids encoding the modified FVII polypeptides is a two-step gene replacement technique in yeast, starting with a complete adenovirus genome (Ad2; Ketner et al. (1994) PNAS 91: 6186-6190) cloned in a Yeast Artificial Chromosome (YAC) and a plasmid containing adenovirus sequences to target a specific region in the YAC clone, an expression cassette for the gene of interest and a positive and negative selectable marker. YACs are of particular interest because they permit incorporation of larger genes. This approach can be used for construction of adenovirus-based vectors bearing nucleic acids encoding any of the described modified FVII polypeptides for gene transfer to mammalian cells or whole animals.

The nucleic acids can be encapsulated in a vehicle, such as a liposome, or introduced into a cells, such as a bacterial cell, particularly an attenuated bacterium or introduced into a viral vector. For example, when liposomes are employed, proteins that bind to a cell surface membrane protein associated with endocytosis can be used for targeting and/or to facilitate uptake, e.g. capsid proteins or fragments thereof tropic for a particular cell type, antibodies for proteins which undergo internalization in cycling, and proteins that target intracellular localization and enhance intracellular half-life.

For *ex vivo* and *in vivo* methods, nucleic acid molecules encoding the modified FVII polypeptide is introduced into cells that are from a suitable donor or the subject to be treated. Cells into which a nucleic acid can be introduced for purposes of therapy include, for example, any desired, available cell type appropriate for the disease or condition to be treated, including but not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, *e.g.,* such as stem cells obtained from bone marrow, umbilical cord blood, peripheral blood, fetal liver, and other sources thereof.

For *ex vivo* treatment, cells from a donor compatible with the subject to be treated or the subject to be treated cells are removed, the nucleic acid is introduced into these isolated cells and the modified cells are administered to the subject. Treatment includes direct administration, such as, for example, encapsulated within porous membranes, which are implanted into the patient (see, *e.g.*, U.S. Patent Nos. 4,892,538 and 5,283,187. Techniques suitable for the transfer of nucleic acid into mammalian cells *in vitro* include the use of liposomes and cationic lipids (*e.g.,* DOTMA, DOPE and DC-Chol) electroporation, microinjection, cell fusion, DEAE-dextran, and calcium phosphate precipitation methods. Methods of DNA delivery can be used to express modified FVII polypeptides *in vivo.* Such methods include liposome delivery of nucleic acids and naked DNA delivery, including local and systemic delivery such as using electroporation, ultrasound and calcium-phosphate delivery. Other techniques include microinjection, cell fusion, chromosome-mediated gene transfer, microcell-mediated gene transfer and spheroplast fusion.

*In vivo* expression of a modified FVII polypeptide can be linked to expression of additional molecules. For example, expression of a modified FVII polypeptide can be linked with expression of a cytotoxic product such as in an engineered virus or expressed in a cytotoxic virus. Such viruses can be targeted to a particular cell type that is a target for a therapeutic effect. The expressed modified FVII polypeptide can be used to enhance the cytotoxicity of the virus.

*In vivo* expression of a modified FVII polypeptide can include operatively linking a modified FVII polypeptide encoding nucleic acid molecule to specific regulatory sequences such as a cell-specific or tissue-specific promoter. Modified FVII polypeptides also can be expressed from vectors that specifically infect and/or replicate in target cell types and/or tissues. Inducible promoters can be use to selectively regulate modified FVU polypeptide expression. An exemplary regulatable expression system is the doxycycline-inducible gene expression system, which has been used to regulate recombinant FVII expression (Srour et al.(2003) Thromb Haemost. 90(3): 398-405).

Nucleic acid molecules, as naked nucleic acids or in vectors, artificial chromosomes, liposomes and other vehicles can be administered to the subject by systemic administration, topical, local and other routes of administration. When systemic and in *vivo,* the nucleic acid molecule or vehicle containing the nucleic acid molecule can be targeted to a cell.

Administration also can be direct, such as by administration of a vector or cells that typically targets a cell or tissue. For example, tumor cells and proliferating can be targeted cells for *in vivo* expression of modified FVII polypeptides. Cells used for *in vivo* expression of an modified FVII polypeptide also include cells autologous to the patient. Such cells can be removed from a patient, nucleic acids for expression of an modified FVII polypeptide introduced, and then administered to a patient such as by injection or engraftment.

## I. Therapeutic Uses

The modified FVII polypeptides provided herein can be used for treatment of any condition for which recombinant FVII is employed. Typically, such treatments include those where increased coagulation, such as increased hemostatic responses, are desired. Modified FVII polypeptides have therapeutic activity alone or in combination with other agents. The modified polypeptides provided herein are designed to retain therapeutic activity but exhibit modified properties, particularly increased resistance to TFPI, increased resistance to AT-III, increased catalytic activity, increased half-life and/or increased binding and/or affinity for activated platelets. Such modified properties, for example, can improve the therapeutic effectiveness of the polypeptides due to increased coagulant activity of the modified FVII polypeptides. This section provides exemplary uses of and administration methods. These described therapies are exemplary and do not limit the applications of modified FVII polypeptides.

The modified FVII polypeptides provided herein can be used in various therapeutic as well as diagnostic methods in which FVII is employed. Such methods include, but are not limited to, methods of treatment of physiological and medical conditions described and listed below. Modified FVII polypeptides provided herein can exhibit improvement of *in vivo* activities and therapeutic effects compared to wild-type FVII, including lower dosage to achieve the same effect, and other improvements in administration and treatment such as fewer and/or less frequent administrations, decreased side effects and increased therapeutic effects. Although it is understood that the modified FVII polypeptides can be administered as a FVII zymogen (*i.e.* single chain form), typically the modified FVII polypeptides provided herein are administered in activated two-chain form following, for example, autoactivation or activation by other coagulation factors, such as during purification.

In particular, modified FVII polypeptides are intended for use in therapeutic methods in which FVII has been used for treatment. Such methods include, but are not limited to, methods of treatment of diseases and disorders, such as, but not limited to, blood coagulation disorders, hematologic disorders, hemorrhagic disorders, hemophilias, such as hemophilia A, hemophilia B and factor VII deficiency, and acquired blood disorders, such as acquired factor VII deficiency caused by liver disease. Modified FVII polypeptides also can be used in the treatment of additional bleeding diseases and disorders, such as, but not limited to, thrombocytopenia (*e.g.*, such as due to chemotherapeutic regimes), Von Willebrand's disease, hereditary platelet disorders (*e.g.,* storage pool disease such as Chediak-Higashi and Hermansky-Pudlak syndromes, thromboxane A2 dysfunction, Glanzmann's thrombasthenia, and Bernard-Soulier syndrome), hemolytic-uremic syndrome, Hereditary Hemorhhagic Telangiectasia, also known as Rendu-Osler-Weber syndrome, allergic purpura (Henoch Schonlein purpura) and disseminated intravascular coagulation.

In some embodiments, the bleedings to be treated by FVII polypeptides occur in organs such as the brain, inner ear region, eyes, liver, lung, tumor tissue, gastrointestinal tract. In other embodiments, the bleeding is diffuse, such as in haemorrhagic gastritis and profuse uterine bleeding. Patients with bleeding disorders, such as for example, hemophilia A and B, often are at risk of bleeding complications during surgery or trauma. Such bleeding can be manifested as acute haemarthroses (bleedings in joints), chronic hemophilic arthropathy, haematomas, (*e.g.,* muscular, retroperitoneal, sublingual and retropharyngeal), haematuria (bleeding from the renal tract), central nervous system bleedings, gastrointestinal bleedings (*e.g.,* UGI bleeds) and cerebral hemorrhage, which also can be treated with modified FVII polypeptides. Additionally, any bleeding associated with surgery (*e.g.*, hepatectomy), or dental extraction can be treated with modified FVII polypeptides. In one embodiment, the modified FVII polypeptides can be used to treat bleeding episodes due to trauma, or surgery, or lowered count or activity of platelets, in a subject. Exemplary methods for patients undergoing surgery include treatments to prevent hemorrhage and treatments before, during, or after surgeries such as, but not limited to, heart surgery, angioplasty, lung surgery, abdominal surgery, spinal surgery, brain surgery, vascular surgery, dental surgery, or organ transplant surgery, including transplantation of bone marrow, heart, lung, pancreas, or liver.

Treatment of diseases and conditions with modified FVII polypeptides can be effected by any suitable route of administration using suitable formulations as described herein including, but not limited to, injection, pulmonary, oral and transdermal administration. Treatment typically is effected by intravenous bolus administration.

If necessary, a particular dosage and duration and treatment protocol can be empirically determined or extrapolated. For example, exemplary doses of recombinant and native FVII polypeptides can be used as a starting point to determine appropriate dosages. For example, a recombinant FVII (rFVIIa) polypeptide that has been activated to rFVIIa, Novoseven®, has been administered to patients with hemophilia A or hemophilia B, who are experiencing a bleeding episode, at a dosage of 90 μg/kg by bolus infusion over 2 to 5 minutes, achieving an effective circulating level of at least 2 μg/ml, with a mean half-life of 2.7 hours. The dose is repeated every 2 hours until hemostasis is achieved. Modified FVII polypeptides that are have an increased coagulant activity, due to increased resistance to TFPI, increased resistance to AT-III, increased catalytic activity, increased half-life and/or increased binding and/or affinity for activated platelets, can be effective at reduced dosage amounts and/or frequencies compared to such a recombinant FVII. Dosages for wild-type or unmodified FVII polypeptides can be used as guidance for determining dosages for modified FVII polypeptides. Factors such as the level of activity and half-life of the modified FVII in comparison to the unmodified FVII can be used in making such determinations. Particular dosages and regimens can be empirically determined.

Dosage levels and regimens can be determined based upon known dosages and regimens, and, if necessary can be extrapolated based upon the changes in properties of the modified polypeptides and/or can be determined empirically based on a variety of factors. Such factors include body weight of the individual, general health, age, the activity of the specific compound employed, sex, diet, time of administration, rate of excretion, drug combination, the severity and course of the disease, and the patient's disposition to the disease and the judgment of the treating physician. The active ingredient, the polypeptide, typically is combined with a pharmaceutically effective carrier. The amount of active ingredient that can be combined with the carrier materials to produce a single dosage form or multi-dosage form can vary depending upon the host treated and the particular mode of administration.

The effect of the FVII polypeptides on the clotting time of blood can be monitored using any of the clotting tests known in the art including, but not limited to, whole blood prothrombin time (PT), the activated partial thromboplastin time (aPTT), the activated clotting time (ACT), the recalcified activated clotting time, or the Lee-White Clotting time.

Upon improvement of a patient's condition, a maintenance dose of a compound or compositions can be administered, if necessary; and the dosage, the dosage form, or frequency of administration, or a combination thereof can be modified. In some cases, a subject can require intermittent treatment on a long-term basis upon any recurrence of disease symptoms or based upon scheduled dosages. In other cases, additional administrations can be required in response to acute events such as hemorrhage, trauma, or surgical procedures.

The following are some exemplary conditions for which FVII (administered as FVIIa) has been used as a treatment agent alone or in combination with other agents.

### 1. Congenital bleeding disorders

#### a. Hemophilia

Congenital hemophilia is a recessive blood disorder in which there are decreased levels of coagulation factors in the plasma, leading to disruption of the coagulation cascade and increased blot clotting time. Hemophilia A, which accounts for approximately 85% of all cases of hemophilia, results from mutations(s) in the factor VIII gene on the X chromosome, leading to a deficiency or dysfunction of the FVIII protein. Hemophilia B is caused by a deficiency or dysfunction of the coagulation factor, FIX, generally resulting from point mutations or deletions in the FIX gene on X chrmosome. The worldwide incidence of hemophilia A is approximately 1 case per 5000 male individuals, and 1 case per 25000 males for hemophilia B. Hemophilia A and B are further classified as mild, moderate, or severe. A plasma level with 5%-25% of normally functioning factor VIII or IX is classified as mild, 1%-5% is moderate, and less that 1% is severe. Hemophilia C, often referred to as FXI deficiency, is a relatively mild and rare disease, affecting about 1 in 100000 people in an autosomal recessive manner.

Hemophilia A and B manifests clinically in many ways. Minor cuts and abrasions will not result in excessive bleeding, but traumas and surgeries will. The patient also will have numerous joint and muscle bleeds and easy bruising. Hemarthrosis or bleeding into the joints is one of the major complications in hemophilia, and can occur spontaneously or in response to trauma. The hinge joints, such as the knee, elbow and ankle, are affected most frequently. The hip and shoulder are affected much less frequently as the ball and socket joint have more musculature surrounding them, thus protecting them more from injury. The bleeding can cause severe acute pain, restrict movement, and lead to secondary complications including synovial hypertrophy. Furthermore, the recurring bleeding in the joints can cause chronic synovitis, which can cause joint damage, destroying synovium, cartilage, and bone. Life-threatening hemorrhages, such as intracranial hemorrhage and bleeding in the central nervous system, also afflicts hemophilic subjects. Intracranial bleeding occurs in approximately 10% of patients with sever hemophilia, resulting in a 30% mortality rate. In contrast, Hemophilia C is more mild. Spontaneous bleeds are rarely seen, and bleeding into joints, soft tissues and muscles also is uncommon. Bleeding is generally treated with transfusion of fresh frozen plasma (FFP), FXI replacement therapy, or, for topical treatment, such treatment of external wounds or dental extractions, fibrin glue.

The most common treatment for hemophilia A or B is replacement therapy, in which the patient is administered FVIII or FIX. The formulations are available commercially as plasma-derived or recombinant products, with recombinant proteins now being the treatment of choice in previously untreated patients. While these therapies can be very successful, complications arise if the patient develops inhibitors to the newly administered factor VIII or factor IX. Inhibitors are IgG antibodies, mostly of the IgG4 subclass, that react with FVIII or FIX and interfere with pro-coagulant function. Inhibitors affect about 1 in 5 patients with severe hemophilia A. Most subjects develop these inhibitors soon after administration of the first infusions of factor VIII, which is often in early childhood, although subjects develop them later in life. Inhibitors also affect about 1 in 15 people with mild or moderate hemophilia A. These inhibitors usually develop during adulthood and not only destroy administered exogenous FVIII, but also destroy endogenous FVIII. As a result, mild and moderate hemophiliacs become severe. Clinically, hemophilia A patients with inhibitors are classified into high and low responders according to the strength of the anamnestic response they experience when they are re-exposed to FVIII. Inhibitors affect about 1 in 100 patients with hemophilia B. In most cases, the inhibitors develop after the first infusions of therapeutic factor IX and can be accompanied by allergic reactions.

The modified FVII polypeptides presented herein can be used to treat patients with hemophilia, particularly hemophilia patients with inhibitors. A recombinant FVIIa product (NovoSeven, Novo Nordisk) has been approved and licensed for the treatment of bleeding episodes in hemophilia A or B patients with inhibitors to FVIII or FIX and for the prevention of bleeding in surgical interventions or invasive procedures in hemophilia A or B patients with inhibitors to FVIII or FIX. Treatment with rFVIIa enhances thrombin generation while bypassing the requirement for FVIIIa and/or FIXa. Coagulation is initiated at the site of injury by the interaction of rFVIIa with TF, resulting in initial FX activation, thrombin generation, and activation of platelets. Complete coagulation by rFVIIa is can be effected by the TF-dependent and TF-independent mechanisms, where some of the thrombin generated can result from the direct activation of FX on activated platelets by rFVIIa alone, which itself binds activated platelets through low affinity interactions with the phospholipid membranes.

The modified FVII polypeptides provided herein can be used in therapies for hemophilia, including the treatment of bleeding episodes and the prevention of bleeding in surgical interventions or invasive procedures. The modified FVII polypeptides herein provide increased resistance to the TF/FVIIa complex inhibitor, TFPI, increased resistance to AT-III, increased catalytic activity, increased half-life and/or increased binding and/or affinity for activated platelets. The FVII polypeptides can therefore display higher coagulant activity in a TF-dependent manner (such as through increased resistance to TFPI, and/or a TF-independent manner (such as through increased binding and/or affinity for activated platelets). Thus, the modified FVII polypeptides can be used to deliver more active therapies for hemophilia. Examples of therapeutic improvements using modified FVII polypeptides include for example, but are not limited to, lower dosages, fewer and/or less frequent administrations, decreased side effects, and increased therapeutic effects.

The modified FVII polypeptides typically are administered as activated FVII (FVIIa) polypeptides. Modified FVII polypeptides can be tested for therapeutic effectiveness, for example, by using animal models. For example antibody-induced hemophilic mice, or any other known disease model for hemophilia, can be treated with modified FVII polypeptides. Progression of disease symptoms and phenotypes is monitored to assess the effects of the modified FVII polypeptides. Modified FVII polypeptides also can be administered to subjects such as in clinical trials to assess *in vivo* effectiveness in comparison to placebo controls and/or controls using unmodified FVII.

### b. FVII deficiency

Factor VII deficiency is an autosomal recessive bleeding disorder that affects approximately 1 in 500000 people. FVII deficiency can be clinically mild, moderate or severe, with mild to moderate deficiency characterized by increased bleeding after surgery and trauma. Patients with severe FVII deficiency (less than 1% FVII activity) experience similar symptoms to hemophilia. For example, FVII-deficient subjects are prone to joint bleeds, spontaneous nosebleeds, gastrointestinal bleeding, urinary tract bleeding. Intracerebral hemorrhaging and muscle bleeds have also been reported, while women can experience severe menorrhagia (heavy menstrual bleeding). Treatment can be effected by replacement therapy. A recombinant FVIIa product (NovoSeven®, Novo Nordisk) has been approved and licensed for the treatment of bleeding episodes in patients with congenital FVII deficiency and for the prevention of bleeding in surgical interventions or invasive procedures in patients with congenital FVII deficiency. Hence, the modified FVII polypeptides herein can be similarly used. The modified FVII polypeptides provided herein can be used in the treatment of bleeding episodes and the prevention of bleeding in surgical interventions or invasive procedures in FVII-deficient patients. For example, a neonatal patient presenting with severe FVII deficiency with intracranial hemorrhaging can be administered modified FVII polypeptides by intravenous bolus to effect coagulation and maintain hemostasis. Generally the modified FVII polypeptides are administered as activated FVII (FVIIa) polypeptides.

**c. Others**

Other bleeding disorders can be treated with the FVII polypeptides provided herein to promote coagulation. Congenital deficiencies of factors V and X also present with increased blood clotting times and can potentially be treated with administration of therapeutic doses of FVII. For example, a patient with factor X deficiency can be administered rFVIIa to control bleeding associated with splenectomy (Boggio et al. (2001) Br J Haematol 112:1074-1075). Spontaneous and surgery associated bleeding episodes associated with von Willebrand disease (vWD) also can be treated using the modified FVII polypeptides provided herein. VWD is a bleeding disorder caused by a defect or deficiency of the blood clotting protein, von Willebrand Factor (vWF), and is estimated to occur in 1% to 2% of the population. Subjects with vWD bruise easily, have recurrent nosebleeds, bleed after tooth extraction, tonsillectomy or other surgery, and women patients can have increased menstrual bleeding. Modified FVII polypeptides can be used to ameliorate spontaneous and surgery-associated bleeding in vWD patients (von Depka et al. (2006) Blood Coagul Fibrin 17:311-316). Other platelet-related bleeding disorders, such as for example, Glanzmann's thrombasthenia and Hermansky-Pudlak syndrome also are associated with reduced endogenous clotting activity. Excess spontaneous or surgery-associated bleeding in patients with platelet related bleeding disorders also can be controlled by therapeutic doses of the modified FVII polypeptides. For example, a patient with Glanzmann's thrombasthenia undergoing surgery can be treated before, during and/or after surgery with the modified FVII polypeptides to prevent major blood loss (van Buuren et al. (2002) Dig Dis Sci 47:2134-2136). Generally, the modified FVII polypeptides are administered as activated FVII (FVIIa) polypeptides.

**2. Acquired bleeding disorders**

**a. Chemotherapy-acquired thrombocytopenia**

Bleeding disorders also can be acquired, rather than congenital. For example, chemotherapy treatment, such as for leukemia and other cancers, can result in thrombocytopenia. This is likely due to a loss of platelet production in the bone marrow of patients receiving chemotherapy, and typically occurs 6-10 days after medication. Treatment of the acquired thrombocytopenia is usually by platelet, red blood cell or plasma transfusion, which serves to prevent any abnormal spontaneous bleeding that can result from platelet deficiency. Bleeding in patients with chemotherapy-induced thrombocytopenia, or any other acquired or congenital thrombocytopenia, also can be controlled by administration of therapeutic amounts of the modified FVII polypeptides provided herein. For example, a thrombocytopenic patient with uncontrolled bleeding, such as in the gastrointestinal tract, can be administered an intravenous bolus injection of a therapeutic amount of FVII polypeptide to stop hemorrhaging (Qerotziafas et al. (2002) Am J Hematol 69:219-222). Generally, the modified FVII polypeptides are administered as activated FVII (FVIIa) polypeptides.

**b. Other coagulopathies**

Other acquired coagulopathies can be treated using the modified FVII polypeptides presented herein. Coagulopathy can result from conditions including, but not limited to, fulminant hepatic failure (FHF; such as caused by hepatoxic drugs, toxins, metabolic diseases, infectious diseases and ischemia), other liver disease, including cirrhosis and disease associated with Wilson's disease, vitamin K deficiency (such as caused by antibiotic treatment or diet), hemolytic uremic syndrome, thrombotic thrombocytopenia (TTC) and disseminated intravascular coagulopathy (DIC). Conventional treatment is generally by transfusion with plasma, red blood cells (RBC), or platelets, but can be unsuccessful. In one example, the modified FVII polypeptides can be administered to a patient with FHF undergoing invasive procedures to prevent bleeding. Conventional treatment with fresh frozen plasma (FFP) often is unsuccessful and can require large quantities of plasma, producing volume overload and anasarca (a generalized infiltration of edema fluid into subcutaneous connective tissue). Treatment with therapeutic amounts of modified FVII polypeptides by intravenous bolus during, before and/or after invasive surgery, such as for example, liver biopsy or liver transplantation, can prevent bleeding and establish hemostasis in FHF patients. The patient can be monitored by PT of the blood to determine the efficacy of treatment (Shami et al. (2003) Liver Transpl 9:138-143). In another example, FVII can be administered to a patient with severe bleeding associated with coagulopathy, such as for example, severe post-cesarean intra-abdominal bleeding associated with liver dysfunction and DIC, that did not respond to conventional transfusions infusions (Moscardo et al. (2001) Br J Haematol 113:174-176). Further, the modified FVII polypeptides can be used to treat coagulopathy in neonatal and pediatric patients. In a particular example, the neonatal and pediatric patients do not respond to conventional treatment, such as RBC and platelet infusion. For example, neonates with severe pulmonary hemorrhaging associated with increased PTs who do not respond to RBC and platelet transfusion can be administered modified FVII polypeptides to decrease PT and establish hemostasis (Olomu et al. (2002) J Perinatol 22:672-674). The modified FVII polypeptides provided herein exhibit enhanced coagulation activity compared with unmodified FVII polypeptides, and can therefore be administered, for example, at lower doses, less frequently, and with fewer adverse reactions. Generally the modified FVII polypeptides are administered as activated FVII (FVIIa) polypeptides.

**c. Transplant-acquired bleeding**

Severe bleeding following bone marrow transplant (BMT) and stem cell transplant (SCT) is a relatively common and life-threatening complication associated with these procedures, due to the reduction of platelets. For example, diffuse alveolar hemorrhage (DAH) is a pulmonary complication of BMT with an estimated incidence of 1-21% in the transplant population, and a mortality rate of 60-100%. Conventional treatment of such bleeding episodes includes corticosteroid treatment and transfusion with plasma, platelets and/or RBC, although these are largely unsuccessful with an overall mortality rate of approximately 50% (Hicks et al. (2002) Bone Marrow Transpl 30:975-978). Administration of FVII by intravenous bolus, with or without concurrent treatment with corticosterioids and/or platelet infusion, can be performed to treat DAH and establish hemostasis (Hicks et al. (2002) Bone Marrow Transpl 30:975-978). The modified FVII polypeptides provided herein exhibit enhanced coagulation activity compared with unmodified FVII polypeptides, and might therefore be administered, for example, at lower doses, less frequently, over a shorter treatment duration, and with fewer adverse reactions for the same biological activity and efficacy. Generally the modified FVII polypeptides are administered as activated FVII (FVIIa) polypeptides.

**d. Anticoagulant therapy-induced bleeding**

Patients undergoing anticoagulant therapies for the treatment of conditions, such as thromboembolism, can exhibit bleeding episodes upon acute administration of anticoagulants, such as warfarin, heparin and fondaparinux, or develop hemorrhagic disorders as a result long term usage of such therapies. Treatments for bleeding episodes typically include administration of procoagulants, such as vitamin K, plasma, exogenous FIX, and protamines to neutralize heparin. Administration of exogenous FVII also can be performed to neutralize the effect of the anti-coagulants, increase PT, aPTT, and/or other markers of coagulation and establish hemostasis (Deveras et al. (2002) Ann Inten Med 137:884-888). The modified FVII polypeptides provided herein can be used in treatments to control bleeding episodes in patients with acquired bleeding disorders due to anticoagulant treatments. Generally the modified FVII polypeptides are administered as activated FVII (FVIIa) polypeptides.

**e. Aquired hemophilia**

Factor VIII inhibitors can develop spontaneously in otherwise healthy individuals, resulting in a condition known as "aquired hemophilia". Acquired hemophilia is a rare condition, with a yearly incidence of 0.2-1.0 per million population. The autoantibodies are mainly IgG4 antibodies, which, when bound to FVIII, inhibit FVIII activity by interfering with thrombin cleavage, von Willebrand factor interaction and/or phospholipid binding. This results in life-threatening hemorrhage in approximately 87% of affected patients. Common sites of bleeding are skin, mucosa, muscles and retroperitoneum, in contrast to patients with hereditary hemophilia who bleed predominantly in joints and muscles. Aquired hemophilia can be treated with an activated prothrombin complex concentrate or recombinant activated factor VII (NovoSeven®, Novo Nordisk) to control bleeding episodes. The modified FVII polypeptides provided herein exhibit enhanced coagulation activity compared with unmodified FVII polypeptides, and can therefore be administered, for example, at lower doses, less frequently, over a shorter treatment duration, and with fewer adverse reactions for the same biological activity and efficacy. Generally the modified FVII polypeptides are administered as activated FVII (FVIIa) polypeptides.

**3. Trauma and surgical bleeding**

FVII polypeptides can be used as therapy to treat bleeding associated with perioperative and traumatic blood loss in subjects with normal coagulation systems. For example, FVII polypeptides can be administered to a patient to promote coagulation and reduce blood loss associated with surgery and, further, reduce the requirement for blood transfusion. In one example, FVII polypeptides can be administered to subjects undergoing retropubic prostatectomy. Retropubic prostatectomy is often associated with major blood loss and a subsequent need for transfusion. Subjects undergoing such or similar surgery can be given an intravenous bolus of a therapeutic amount of FVII in the early operative phase to reduce perioperative blood loss by enhancing coagulation at the site of surgery. Reduction in blood loss results in elimination of the need for blood transfusion in these patients (Friederich et al. (2003) Lancet 361:201-205). FVII polypeptides can be administered to patients with normal coagulation undergoing other types of surgery to effect rapid hemostasis and prevent blood loss. Non-limiting examples of surgical procedures in which FVII, typically administered in the activated form (*i.e.* FVIIa), can be used a therapy to reduce perioperative bleeding include, but are not limited to, cardiac valve surgery (Al Douri et al. (2000) Blood Coag Fibrinol 11:S121-S127), aortic valve replacement (Kastrup et al. (2002) Ann Thorac Surg 74:910-912), resection of recurrent hemangiopericytoma (Gerlach et al. (2002) J Neurosurg 96:946-948), cancer surgery (Sajdak et al. (2002) Eur J Gynaecol Oncol 23:325-326), and surgery on duodenal ulcers (Vlot et al. (2000) Am J Med 108:421-423). Treatment with FVII can promote hemostasis at the site of surgery and reduce or prevent blood loss, thereby reducing or abolishing the need for transfusion. The modified FVII polypeptides provided herein are designed to exhibit enhanced coagulation activity compared with unmodified FVII polypeptides, and might therefore be administered, for example, at lower doses, less frequently, and with fewer adverse reactions. Generally the modified FVII polypeptides are administered as activated FVII (FVIIa) polypeptides.

Factor VII polypeptides also can be used to promote coagulation and prevent blood loss in subjects with traumatic injury. Trauma is defined as an injury to living tissue by an extrinsic agent, and is the fourth leading cause of death in the United States. Trauma is classified as either blunt trauma (resulting in internal compression, organ damage and internal hemorrhage) or penetrative trauma (a consequence of an agent penetrating the body and destroying tissue, vessel and organs, resulting in external hemorrhaging). Trauma can be caused by several events including, but not limited to, vehicle accidents (causing blunt and/or penetrative trauma), gun shot wounds (causing penetrative trauma), stabbing wounds (causing penetrative trauma), machinery accidents (causing penetrative and/or blunt trauma), and falls from significant heights (causing penetrative and/or blunt trauma). Uncontrolled hemorrhage as a result of trauma is responsible for most of the associated mortality. Diffuse coagulopathy is a relatively common complication associated with trauma patients, occurring in as many as 25-36% of subjects. Coagulopathy can develop early after injury, resulting from a variety of factors such as dilution and consumption of coagulation factors and platelets, fibrinolysis, acidosis, and hypothermia. Conventional management involves replacement therapy by transfusion with fresh frozen plasma (FFP) platelets, RBC and/or cryoprecipitate, correcting acidosis, and treating hypothermia. These steps often are insufficient to stop the bleeding and prevent death. Treatment by administration of therapeutic amounts of FVII can promote coagulation and reduce blood loss in trauma patients. For example, a patient with a gun shot injury presenting with massive blood, in addition to surgical intervention, be administered FVII to control coagulopathic bleeding (Kenet et al. (1999) Lancet 354:1879). Coagulant therapy with FVII can effectively reduce blood loss and hemorrhage in patients with blunt and penetrating trauma (Rizoli *et al.* (2006) Crit Care 10:R178). The modified FVII polypeptides provided herein are designed to exhibit enhanced coagulation activity compared with unmodified FVII polypeptides, and might therefore be administered, for example, at lower doses, less frequently, and with fewer adverse reactions. Generally the modified FVII polypeptides are administered as activated FVII (FVIIa) polypeptides.

### J. Combination Therapies

Any of the modified FVII polypeptides described herein can be administered in combination with, prior to, intermittently with, or subsequent to, other therapeutic agents or procedures including, but not limited to, other biologics, small molecule compounds and surgery. For any disease or condition, including all those exemplified above, for which FVII (including FVIIa and rFVIIa) is indicated or has been used and for which other agents and treatments are available, FVII can be used in combination therewith. Hence, the modified FVII polypeptides provided herein similarly can be used. Depending on the disease or condition to be treated, exemplary combinations include, but are not limited to, combination with other plasma purified or recombinant coagulation factors, procoagulants, such as vitamin K, vitamin K derivative and protein C inhibitors, plasma, platelets, red blood cells and corticosteroids.

### K. Articles of manufacture and kits

Pharmaceutical compounds of modified FVII polypeptides or nucleic acids encoding modified FVII polypeptides, or a derivative or a biologically active portion thereof can be packaged as articles of manufacture containing packaging material, a pharmaceutical composition which is effective for treating a hemostatic disease or disorder, and a label that indicates that modified FVII polypeptide or nucleic acid molecule is to be used for treating hemostatic disease or disorder.

The articles of manufacture provided herein contain packaging materials. Packaging materials for use in packaging pharmaceutical products are well known to those of skill in the art. See, for example, U.S. Patent Nos. 5,323,907, 5,052,558 and 5,033,352. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated as are a variety of treatments for any hemostatic disease or disorder.

Modified FVII polypeptides and nucleic acid molecules also can be provided as kits. Kits can include a pharmaceutical composition described herein and an item for administration. For example a modified FVII can be supplied with a device for administration, such as a syringe, an inhaler, a dosage cup, a dropper, or an applicator. The kit can, optionally, include instructions for application including dosages, dosing regimens and instructions for modes of administration. Kits also can include a pharmaceutical composition described herein and an item for diagnosis. For example, such kits can include an item for measuring the concentration, amount or activity of FVII or a FVII regulated system of a subject.

The following examples are included for illustrative purposes only and are not intended to limit the scope of the invention.

## L. Examples

### Example 1

### In silico generation of factor VII variants with increased resistance to TFPI

#### A. Modeling of the interaction between factor VII and TFPI

Computer modeling of the interaction between factor VII and its natural inhibitor, the first Kunitz domain (K1) on Tissue Factor Pathway Inhibitor (TFP1)-1 (TFPI-1 K1) was performed to determine the contact amino acid residues at the interface of the interaction site. Publicly available information from the protein data bank (rcsb.org/pdb/) was used to create the homology model. Neither the crystal structure for TFPI-1 K1 alone, nor of the quaternary complex between TF/FVIIa/TFPI-1/FXa, have yet been solved. Instead, computer modeling was performed using the 2.1 Å crystal structure of the ternary complex between TF, FVII and the 5L15 variant of bovine pancreatic trypsin inhibitor ($BPTI^{5L15}$; PDB code 1FAK_1) as a starting model for the process, followed by information about the crystal structure of the trypsin/TFPI-2 complex (Figure 6). $BPTI^{5L15}$ is a Kunitz domain-type serine protease, and displays homology to TFPI-1 and TFPI-2. The first Kunitz domain (K1) of $BPTI^{5L15}$ (SEQ ID NO:106) displays 45% primary sequence identity in the first Kunitz domain (K1) (Figure 5). The coordinates for the crystal structure of TFPI-2 K1 were extracted from the program database (pdb) file 1TFX from the protein data bank, which depicts the crystal structure of the trypsin/TFPI-2 complex. The TFPI-2 K1 coordinates were aligned onto the $BPTI^{5L15}$ three-dimensional coordinates of the crystal structure of TF/FVIIA/$BPTI^{5L15}$ (pdb file 1FAK1) using a rigid body c-$\alpha$ backbone alignment program in the PyMol software suite (pymol.sourceforge.net/). Analysis of the fit of TFPI-2 K1 into the model by measurement of the overlap resulted in a root-mean-square deviation (RMSD) of less than 1 Å. This indicated a precise alignment between the two structures, and the generation of a reliable model of a FVII and TFPI-2 K1 complex.

The side chains on TFPI-2 K1 that were shown by the model to be in close contact with the surface of FVII were identified and mutagenized *in silico* to correspond to the side chains present in TFPI-1 K1. The results of this analysis revealed an electrostatic complementarity between Factor VII and TFPI-1 K1 in several residues directly adjacent to the active site (*i.e.* 2$^{nd}$ sphere residues). The residues in FVII involved in the interaction with TFPI-1 K1 based on the above homology analysis are D60, K60a, K60c,T99, R147 and K192 by chymotrypsin numbering, which correspond to D196, K197, K199, T239, R290 and K341, respectively, by mature FVII numbering (Figure 7). Examination of the modeled interaction indicated that the glycine residue at position 97 by chymotrypsin numbering (G97), which corresponds to G237 by mature FVII numbering, is in close proximity to the contact residues. Modification at this position could result in steric hindrance, which would disrupt interaction of FVII with TFPI.

#### B. In silico mutation of factor VII to provide increased resistance to TFPI-1 K1

Amino acid residues positioned at or near the interface of the FVII/TFPI-1 K1 interaction were identified as described above. Variants of FVII with amino acid changes were designed that either a) replaced complementary electrostatic contacts between FVII and TFPI-1 with repulsive charge-charge contacts and/or b) negate positive electrostatic contacts by replacement of the charged residues on FVII with a neutral residue and/or c) cause steric hindrance by replacement of a residue containing a small side chain with residues containing larger side-chains. Exemplary variants are listed in Table 9.

**Table 9. Exemplary Factor VII Variants**

| Variant ature FVII numbering) | Variant (Chymotrypsin numbering) | ID | Variant polypeptide SEQ ID NO |
|---|---|---|---|
| D196K | D60K | CB554 | 18 |
| D196R | D60R | CB555 | 19 |
| D196A | D60A | CB556 | 20 |
| D196Y | D60Y | CB601 | 125 |
| D196F | D60F | CB600 | 126 |
| D196W | D60W | CB602 | 127 |
| D196L | D60L | CB603 | 128 |
| D196I | D60I | CB604 | 129 |
| K197Y | K60aY | CB561 | 21 |
| K197A | K60aA | CB559 | 22 |
| K197E | K60aE | CB558 | 23 |
| K197D | K60aD | CB557 | 24 |
| K197L | K60aL | CB560 | 25 |
| K197M | K60aM | CB599 | 26 |
| K197I | K60aI | CB595 | 130 |
| K197V | K60aV | CB596 | 131 |
| K197F | K60aF | CB597 | 132 |
| K197W | K60aW | CB598 | 133 |
| K199A | K60cA | CB564 | 27 |
| K199D | K60cD | CB562 | 28 |
| K199E | K60cE | CB563 | 29 |
| G237W | G97W | CB605 | 134 |
| G237T | G97T | CB606 | 135 |
| G237I | G97I | CB607 | 136 |
| G237V | G97V | CB608 | 137 |
| T239A | T99A | CB565 | 30 |
| R290A | R147A | CB568 | 31 |
| R290E | R147E | CB567 | 32 |
| R290D | R147D | CB566 | 33 |
| K341E | K192E | | 34 |
| K341R | K192R | CB569 | 35 |
| K341Q | K192Q | CB609 | 138 |
| D196R/R290E | D60R/R147E | CB579 | 36 |
| D196K/R290E | D60K/R147E | CB580 | 37 |
| D196R/R290D | D60R/R147D | CB581 | 38 |
| D196R/K197E/K199E | D60R/K60aE/K60cE | CB586 | 39 |
| D196K/K197E/K199E | D60K/K60aE/K60cE | CB587 | 40 |

(continued)

| Variant ature FVII numbering) | Variant (Chymotrypsin numbering) | ID | Variant polypeptide SEQ ID NO |
|---|---|---|---|
| D196R/K197E/ K199E/R290E | D60R/K60aE/K60cE/R147E | CB588 | 41 |
| D196R/K197M/ K199E | D60R/K60aM/K60cE | CB589 | 42 |
| D196R/K197M/ K199E/R290E | D60R/K60aM/K60cE/R147E | CB590 | 43 |
| D196K/K197L | D60K/K60aL | CB610 | 139 |
| D196F/K197L | D60F/K60aL | CB612 | 140 |
| D196L/K197L | D60L/K60aL | CB611 | 141 |
| D196M/K197L | D60M/K60aL | CB613 | 142 |
| D196W/K197L | D60W/K60aL | CB614 | 143 |
| D196F/K197E | D60F/K60aE | CB615 | 144 |
| D196W/K197E | D60W/K60aE | CB616 | 145 |
| D196V/K197E | D60V/K60aE | CB617 | 146 |

**Example 2**

**Cloning and expression of FVII**

**A. Cloning of FVII**

The nucleotides encoding the 466 amino acid human FVII isoform precursor polypeptide (P08709; set forth in SEQ ID NO:1) were cloned into the mammalian expression vector, pCMV Script (Stratagene; SEQ ID NO: 151), which contains a cytomegalovirus (CMV) promoter. Briefly, the CBO-125 (SEQ ID NO:152) and CBO-126 (SEQ ID NO:153) oligonucleotides were used as forward and reverse primers, respectively, to amplify the FVII sequence by PCR using human FVII cDNA (Invitrogen) as the template. The CBO-125 primer contained a BamHI restriction site (in bold), a Kozak sequence (double underlined), followed by 18 nucleotides with homology to the 5' end of the FVII cDNA sequence (underlined), including the ATG start codon. The CBO-126 primer contained an EcoRI restriction site (in bold), a stop codon (double underlined) and 21 nucleotides with homology to the 3' end of the FVII cDNA sequence (underlined).

CBO-125 forward primer
5' gcatcatgacgtgacggatccgccaccatggtctcccaggccctc 3'

CBO-126 reverse primer
5' gatcgtacgatacgtgaattcctagggaaatggggctcgcaggag 3'

Standard PCR reaction and thermocycling conditions were used in conjunction with the KoD HiFi PCR kit (EMD Biosciences), as recommended by the manufacturer. The PCR product was digested with BamH I and EcoR I restriction enzymes and ligated into the BamH I and EcoR I restriction sites of pCMV Script vector using standard molecular techniques. The vector was then transformed into *Escherechia coli.* Selected colonies were grown and bacterial cells harvested for purification of the plasmid using routine molecular biology techniques.

**B. Generation of FVII Variants**

FVII variants were generated using the QuikChange II XL Site-Directed Mutagenesis kit (Stratagene) according to the manufacturers instructions, with specifically designed oligonucleotides which served as primers that incorporated a particular mutation into newly synthesized DNA. The QuikChange method involves linear amplification of template DNA by the PfuUltra high-fidelity DNA polymerase. Complementary primers that include the desired mutation were extended during cycling using purified, double-stranded supercoiled pCMV Script vector that contained the cloned FVII cDNA sequence as a template. Extension of the primers resulted in incorporation of the mutation of interest into the newly synthesized strands, and resulted in a mutated plasmid with staggered nicks. Following amplification, the nucleic acid was treated with Dpn I, which digests the dam-methylated parental strands of the *E. coli*-derived pCMV Script vector. This resulted in "selection" of the newly-synthesized mutated plasmids, which were not methylated. The vector DNA containing the desired mutation(s) were transformed into XL10-Gold ultracompetent *E. coli* cells, where bacterial ligase repaired the nicks and allowed normal replication to occur.

FVII variants with single amino acid substitutions were made by targeting positions D196, K197, K199, G237, T239, R290 and K341, by mature FVII numbering (corresponding to D60, K60a, K60c, G97, T99, R147 and K192 by chymotrypsin numbering). FVII variants with multiple mutations at positions D196, K197 and K199, or mutations at positions D196 and K197; also were generated. The nucleotide sequence of one of the oligonucleotides from each complementary primer pair used to generate the FVII variants is provided in Table 10. The corresponding wild-type sequence also is shown for comparison. The 3 base pair sequence that encodes the substituted amino acid are shown in bold type. For example, to generate a FVII variant containing the substitution D196K (D60K by chymotrypsin numbering; CB554, SEQ ID NO:18), the CBO-157 D60K oligonucleotide, and an oligonucleotide that is complementary to CBO-157 D60K, were used with the QuickChange II XL Site-Directed Mutagenesis kit to replace a 3 base pair "gac" wild-type sequence with a 3 base pair "aag" sequence. The mutated vector encoding a FVII variant polypeptide containing a D196K substitution, was then transformed into XL10-Gold ultracompetent *E. coli* cells.

**Table 10. Oligonucleotides used to generate FVII variants**

| Position targeted | Primer name | Primer sequence | SEQ ID NO |
|---|---|---|---|
| D196 (D60) | D60 wild-type | gcggcccactgtttcgacaaaatcaagaactgg | 155 |
| | CBO-157 D60K | gcggcccactgtttcaagaaaatcaagaactgg | 156 |
| | CBO-158 D60R | gcggcccactgtttcaggaaaatcaagaactgg | 157 |
| | CBO-159 D60A | gcggcccactgtttcgcgaaaatcaagaactgg | 158 |
| | CBOLH-59 D60F | gcggcccactgtttctttaaaatcaagaactgg | 186 |
| | CBOLH-60 D60Y | gcggcccactgtttcataaaaatcaagaactgg | 187 |
| | CBOLH-61 D60W | gcggcccactgtttctggaaaatcaagaactgg | 188 |
| | CBOLH-62 D60L | gcggcccactgtttcgagaaaatcaagaactgg | 189 |
| | CBOLH-63 D601 | gcggcccactgtttcatcaaaatcaagaactgg | 190 |
| K197 (K60a) | K60a wild-type | gcccactgtttcgacaaaatcaagaactggagg | 159 |
| | CBO-160 K60aD: | gcccactgtttcgacgacatcaagaactggagg | 160 |
| | CBO-161 K60aE | gcccactgtttcgacgagatcaagaactggagg | 161 |
| | CBO-162 K60aA | gcccactgtttcgacgcgatcaagaactggagg | 162 |
| | CBO-163 K60aL | gcccactgtttcgacctcatcaagaactggagg | 163 |
| | CBO-164 K60aY | gcccactgtttcgactatatcaagaactggagg | 164 |
| | CBOLH-54 K60al | gcccactgtttcgacatcatcaagaactggagg | 181 |
| | CBOLH-55 K60aV | gcccactgtttcgacgtcatcaagaactggagg | 182 |
| | CBOLH-56 K60aF | gcccactgtttcgacttcatcaagaactggagg | 183 |
| | CBOLH-57 K60aW | gcccactgtttcgactggatcaagaactggagg | 184 |
| | CBOLH-58 K60aM | gcccactgtttcgacatgatcaagaactggagg | 185 |
| K199 (K60c) | K60c wild-type | tgtttcgacaaaatcaagaactggaggaacctg | 165 |
| | CBO-165 K60cD | tgtttcgacaaaatcgacaactggaggaacctg | 166 |
| | CBO-166 K60cE | tgtttcgacaaaatcgagaactggaggaacctg | 167 |
| | CBO-167 K60cA | tgtttcgacaaaatcgcgaactggaggaacctg | 168 |

(continued)

| Position targeted | Primer name | Primer sequence | SEQ ID NO |
|---|---|---|---|
| G237 (G97) | G97 wild-type | agcacgtacgtcccgggcaccaccaaccacgac | 191 |
| | CBOLH-64 G97W | agcacgtacgtcccgtggaccaccaaccacgac | 192 |
| | CBOLH-65 G97T | agcacgtacgtcccgacgaccaccaaccacgac | 193 |
| | CBOLH-66 G971 | agcacgtacgtcccgatcaccaccaaccacgac | 194 |
| | CBOLH-67 G97V | agcacgtacgtcccggtcaccaccaaccacgac | 195 |
| T239 (T99) | T99 wild-type | tacgtcccgggcaccaccaaccacgacatcgcg | 169 |
| | CBO-168 T99A | tacgtcccgggcaccgcgaaccacgacatcgcg | 170 |
| R290 (R147) | R147 wild-type | ggccagctgctggaccgtggcgccacggccctg | 171 |
| | CBO-169 R147D | ggccagctgctggacgacggcgccacggccctg | 172 |
| | CBO-170 R147E | ggccagctgctggacgagggcgccacggccctg | 173 |
| | CBO-171 R147A | ggccagctgctggacgcgggcgccacggccctg | 174 |
| K341 (K192) | K192 wild-type | agcaaggactcctgcaagggggacagtggaggc | 175 |
| | CBO-172 K192R | agcaaggactcctgccgcggggacagtggaggc | 176 |
| | CBOLH-68 K192Q | agcaaggactcctgccagggggacagtggaggc | 196 |
| D196/ K197/ K199 (D60/ K60a /K60c) | D60/ K60a/ K60c wild-type | gtggtctccgcggcccactgtttcgacaaaatcaagaactggaggaacctg atcgcggtg | 177 |
| | CBO-177 D60R/ K60aE / K60cE | gtggtctccgcggcccactgtttcagggagatcgaaaactggaggaacctg atcgcggtg | 178 |
| | CBO-178 D60K/ K60aE/ K60cE | gtggtctccgcggcccactgtttcaaggagatcgaaaactggaggaacctg atcgcggtg | 179 |
| | CBO-179 D60R / K60aM / K60cE | gtggtctccgcggcccactgtttcaggatgatcgaaaactggaggaacctg atcgcggtg | 180 |

(continued)

| Position targeted | Primer name | Primer sequence | SEQ ID NO |
|---|---|---|---|
| D196/ K197 (D60 /K60a) | D60 /K60a wild-type | gcggcccactgtttcgacaaaatcaagaactgg | 197 |
| | CBOLH-69 D60K/ K60aL | gcggcccactgtttcaagctcatcaagaactgg | 198 |
| | CBOLH-70 D60U K60aL | gcggcccactgtttcctcctcatcaagaactgg | 199 |
| | CBOLH-71 D60F/K60aL | gcggcccactgtttctttctcatcaagaactgg | 200 |
| | CBOLH-72 D60M/ K60aL | gcggcccactgtttcatgcteatcaagaactgg | 201 |
| | CBOLH-73 D60W/ K60aL | gcggcccactgtttctggctcatcaagaactgg | 202 |
| | CBOLH-74 D60F/ K60aE | gcggcccactgtttctttgagatcaagaactgg | 203 |
| | CBOLH-75 D60W/ K60aE | gcggcccactgtttctgggagatcaagaactgg | 204 |
| | CBOLH-76 D60V/ K60aE | gcggcccactgtttcgtcgagatcaagaactgg | 205 |

**C. Expression of FVII polypeptides**

For initial expression analysis by ELISA and Western Blot, FVII polypeptides were expressed in BHK-21 cells. For biochemical assays, such as those described below, the FVII polypeptides were expressed in FreestyleTM 293-F cells (Invitrogen).

The wild-type Factor VII polypeptide (CB553-02, SEQ ID NO:3) and variant FVII polypeptides were initially expressed in the baby hamster kidney cell line BHK-21 (ATCC CRL 1632). BHK-21 cells were cultured in Eagle's minimal essential medium (EMEM, Invitrogen) with 10% fetal calf serum (FCS) in 100mm culture dishes at 37°C and 5% $CO_2$. After growth to approximately 90% confluence, the cells were transfected with 24 μg of FVII plasmid DNA using the Lipofectamine 2000 kit (Invitrogen) as instructed by the manufacturer. The media was replaced 6 hours after transfection with EMEM without serum containing 1 μg/ml vitamin K1 (Sigma) and the cells were incubated for a further 72 hours. Expression of FVII in the cell culture media was assayed by ELISA or Western Blot.

For subsequent analyses using biochemical assays, the wild-type Factor VII polypeptide (CB553-02, SEQ ID NO:3) and variant FVII polypeptides were expressed in Freestyle™ 293-F cells (Invitrogen). Cells were cultured in Freestyle™ 293 media (Invitrogen) at 37°C and 8% $CO_2$ in Erlenmeyer flasks with vented caps. The cells were transfected using the manufacturer's suggested protocol. Briefly, after growth to $1\times10^6$ cells/ml, the cells were centrifuged and the media was exchanged. The cells were then transfected with 240 μg of FVII plasmid DNA for every 240 ml of cells using 293fectin (Invitrogen). In addition, 50 μl of a 1 mg/ml stock of Vitamin $K_1$ (Sigma) in ethanol was added for every 240 ml of cells. The cells were grown for 5 days then the culture supernatant was harvested. Expression of FVII in the cell culture media was assayed by ELISA.

**1. ELISA**

An immunoassay was used to quantify the amount of human FVII and FVIIa in a sample. Polyclonal antibodies to human FVII were used to capture and detect the protease in the solution. The immunoassay can be used to determine protein concentration of conditioned medium or a purified stock or to determine the concentration of FVII in another sample, for example, a human or mouse plasma sample. The baseline concentration of FVII in human blood is approximately 50 nM and the enzymatically active form, FVIIa, is approximately 1 nM.

To determine the amount of human FVII or FVIIa protein in samples a sandwich ELISA was performed. Ninety-six well flat bottom Maxisorp immuno plates (Nunc) were coated with 100 μl/well of 5 ng/μl avidin (NeutrAvidin, Pierce Biotech.). The plates were covered and incubated with shaking for 1 hour at room temperature (RT) followed by washing four times in PBS with 0.01 % Tween-20 (PBST). The plates were blocked for a minimum of 1 hour at RT with shaking by incubation with 1% bovine serum albumin (BSA) (w/v) in PBS added to each well at 200 μl/well. The blocked plates were then stored at 4°C until use (up to 2 weeks).

Before use, the plates were washed four times in PBST to remove the BSA, and 100 μl/well of a 1 ng/μl solution of biotinylated anti-Factor VII antibody (R&D Systems) was added to each well and the plate was incubated at room temperature for 45 minutes with shaking to allow complexation with the coated avidin. Excess unbound antibody was removed by washing the plate with PBST (four times).

Serial two-fold dilutions of a FVII standard (American Diagnostica; diluted in PBST), ranging from 50 ng/μl to 0.8 ng/μl, were added to the plate at 100 μl/well. A well containing PBST without any FVII also was included as a buffer only control. To assay purified samples (before and after activation, see Example 3) of FVII or FVIIa, the sample was first diluted 1:25 in PBST, and then serial 2-fold dilutions were made so that 25-fold, 50-fold, 100-fold and 200-fold dilutions were tested. The diluted samples were added to the wells in duplicate at 100 μl/well. To assay plasma samples containing FVII or FVIIa, the plasma sample was diluted 1:100 and 1:400 in PBST and added to the wells in duplicate at 100 μl/well. A plasma sample without FVII or FVIIa also was included to determine background levels. The plates were then incubated for 30 minutes at RT with shaking to allow for any FVII or FVIIa in the sample to complex with the anti-FVII antibody.

After incubation with sample, the plates were washed 4 times with PBST. A secondary antibody, Equine anti-human FVII (American Diagnostica), was diluted 1:5000 in PBST and added to each well at a volume of 100 μl. The plates were incubated for 30 minutes at room temperature with shaking to allow the added antibody to bind to the FVII or FVII complexes on the plate. To remove excess secondary antibody, the plates were washed with PBST (4 times). To detect the bound secondary antibody, 100 μl of goat anti-equine HRP conjugate at a 1:5000 dilution in PBST was added to each well. After incubation for 30 minutes at room temperature with shaking, the plates were washed four times with PBST and 100 μl/well of a solution containing a 1:1 mixture of TMB substrate and hydrogen peroxide solution (Pierce Biotech.) was added. The plates were shaken for approximately 1 minute at room temperature before addition of 100 μl/well of 2M $H_2SO_4$ to stop the reaction. The optical density at 450 nm was measured using a Molecular Device M5 Plate reader and the background value for the plate (measured with PBST alone) was subtracted from the measured value from each well. A standard curve was generated by plotting the concentration of the FVII standards versus the absorbance. A standard curve range of about 0.2 - 50 ng/ml was typically generated under the above ELISA conditions. The concentration of each sample was then determined using the standard curve and multiplying by the dilution factor, and an average and standard deviation was reported.

### 2. Western Blot

Expression of FVII in cell culture media also was assayed by Western blot. Aliquots containing the undiluted sample, or two serial 2-fold dilutions in PBS, of the cell culture medium from FVII-transfected BHK- 21 cells were labeled Conc. 1 (undiluted), Conc. 2 (2-fold dilution) and Conc. 3 (4-fold dilution). The samples were loaded on an SDS page gel next to 10, 25, and 50 nanograms of control plasma purified rFVII (American Diagnostica, CB553-01). FVII protein produced by BHK-21 cells was detected by Western blot using a primary polyclonal equine anti-FVII antibody (American Diagnostica; used at the manufacture's suggested concentration) and an HRP-conjugated anti-equine IgG secondary antibody (a 1:2000 dilution of 1mg/ml solution from Zymed Laboratories). Comparison of expression levels was made with the control plasma purified rFVII. The results show that concentrations ranging from about 20 ng to more than 50 ng of FVII was present in the cell culture aliquots.

## Example 3

### Purification and activation of FVII polypeptides

FVII polypeptides were purified using a Q Sepharose Fast Flow (XK16) column, to which FVII polypeptides with functional Gla domains will adsorb, followed by a calcium elution step. A volume of 240 ml of culture supernatant from the transfected FreestyleTM 293-F cells was diluted 2-fold with a solution containing 20 mM Tris pH 8.0 and 0.01% Tween 20, and then 1.5 ml of 500 mM EDTA pH 8.0 was added to the diluted sample. The samples were filtered before being loaded onto a Q Sepharose Fast Flow (XK16) column which had been pre-equilibrated first with Buffer B (20 mM Tris pH 8.0, 1 M NaCl, 0.01% Tween 20), then Buffer A (20 mM Tris pH 8.0,0.15 M NaCl, 0.01% Tween 20) at 8 ml/min. After being loaded, the column was washed with Buffer A until the absorbance of the flow-through at 280 nm reached a baseline. Buffer A was replaced with Buffer C (20 mM Tris pH 8.0, 0.15 M NaCl, 0.01% Tween 20, 5 mM $CaCl_2$) and a pump wash was performed to completely replace the buffer in the lines. Upon completion of the pump wash, Buffer C was applied to the column at 8 ml/min to elute the FVII polypeptides, which were collected in 5 ml fractions for 60 minutes. Following elution, the column was washed with Buffer B while still collecting fractions, until the pink pigment (from the culture media) was washed off the column. The column was then washed with Buffer A to prepare it for purification of FVII from the next sample.

The eluted fractions were further purified using a Mono Q 5/5 column (containing 1 ml resin), which was pre-equilibrated initially with Buffer B, and then with Buffer A, at 2 ml/min. The $5^{th}$ to $8^{th}$ 5ml fractions collected with buffer C above were pooled and diluted 2-fold with Buffer A, before 1.6 ml of 500 mM EDTA, pH 8.0 was added. Small aliquots (100μl) were optionally taken at this point for analysis, such as by ELISA. The combined sample was loaded onto the Mono Q column at 2 ml/min, then washed with Buffer A. To elute the bound FVII polypeptides, a gradient from 0% to 30% of Buffer B was run through the column over a period of 20 minutes, at 1 ml/min, and 0.5 ml fractions were collected. The column was then washed with Buffer B followed by Buffer A in preparation for purification of FVII from the next sample.

Purified FVII polypeptides were activated to FVIIa using biotinylated Factor Xa from the Restriction Protease Factor Xa Cleavage and Removal Kit (Roche). Typically, 7 fractions from the Mono Q purification were pooled in a 15 ml conical tube and 388 μl of 500 mM $CaCl_2$, 38.9 μl of 10% BSA in distilled water, and 3.2 μg of biotinylated Factor Xa were added. After incubation for 14-16 hrs at 37°C, 250 μl of Immobilized Avidin (Pierce) was added and the sample was mixed at 4°C for 30 minutes. The resulting solution was then filtered through an Econo-pak column (Bio-Rad), and the filtrate was mixed with another 250 μl of Immobilized Avidin for a further 30 minutes. The solution was filtered again and the filtrate was concentrated to approximately 300-500 μl using an Amicon Ultra-4 10kDa centrifugal filter (Millipore). The FVIIa concentration was then analyzed by ELISA (as described in Example 2.C.1) and the level of Factor VII activation was monitored by Western blot. Western blotting was performed essentially as described in Example 2.C.2, but instead using rabbit anti-human Factor VIIa antibody (Haematologic Technologies, Inc.) at 1:2000 for 1 hr as the primary antibody, followed by HRP-Goat Anti-Rabbit IgG (H+L) (Invitrogen) at 1:5000 for 30 minutes.

## Example 4

### Michaelis Menten kinetics constant determination of the amidolytic activity of FVIIa on a small molecule substrate.

The amidolytic activity of the FVII variants was assessed by measuring the Michaelis Menten kinetics constant of the FVIIa polypeptide one the peptidyl substrate Spectrozyme FVIIa ($CH_3SO_2$-D-CHA-But-Arg-pNA.AcOH). Lipidated human purified tissue factor (Innovin, Dade Behring, VWR Cat#68100-390) was included in the assay to provide for optimal activity of FVIIa. The TF-FVIIa complex cleaves Spectrozyme FVIIa as a highly specific chromogenic substrate releasing a paranitroaniline-chromophore (pNA), which can be monitored by measuring absorption at 405 nm. Enzyme activity is determined by monitoring the absorbance at 405 nm of the free pNA generated as a function of time.

The reactions were performed at three different enzyme concentrations. For the reaction, the FVIIa variants were first diluted to 40 nM in 1 × direct assay buffer (100 mM Tris pH 8.4, 100 mM NaCl, 5 mM $CaCl_2$, 0.01% BSA) in a 1.7 mL tube (low adhesion microfuge tubes from ISC Bioexpress). FVIIa was further diluted in the presence of TF (Innovin, Dade Behring) by diluting to 2 nM in a 12-well polypropylene reservoir (Axygen) as follows: 720 μl 5× direct buffer (500 mM Tris pH 8.4, 500 mM NaCl, 25 mM $CaCl_2$, 0.05% BSA), 180 μl 40 nM FVIIa, and 2700 μl 2×TF (6 nM stock solution reconstituted in 10 mL water). The diluted protease was incubated for 5 minutes at room temperature. The 2 nM stock of FVIIa was further diluted in 2-fold serial dilutions to give a 1 nM and 0.5 nM stock of protease, respectively, also in the presence of TF. The serial dilution reactions were as follows: first, 1800 μl of 2 nM stock of FVIIa/TF from above diluted into 360 μl 5 X direct buffer, 900 μl 2×TF, and 540 μl water. This diluted stock was diluted again 1:1 into 1800 μl 1×TF in direct buffer.

A dilution plate of the substrate Spectrozyme FVIIa (American Diagnostica) was made. The stock solution of Spectrozyme FVIIa was made by reconstitution of the 50 μmoles vial in distilled water to 10 mM and stored at 4°C. Eighty μl (10 mM Spectrozyme FVIIa) and 60 μl + 20 μl water (7.5 mM Spectrozyme FVIIa) of the 10 mM Spectrozyme FVIIa were added to wells in two adjacent columns of a 96-well polypropylene assay plate (Costar). The two wells were serially diluted 2-fold down each of the 8 wells of the respective column to make a series of 10× substrate concentrations ranging from 10 mM to 78 μM substrate down the wells of the first column and from 7.5 mM to 58.6 μM substrate down the wells of the second column.

Five μl of each Spectrozyme FVIIa substrate dilution was added to a 96-well clear half area assay plate (Costar). Forty five μl of each of the three FVIIa/TF dilutions were added to three groups of columns of the substrate series dilutions. During this step, care was taken to avoid introducing bubbles into the wells of the assay. If bubbles were introduced, they were removed by pricking with a clean needle before the beginning of each assy. The plates were mixed by shaking. Prior to initiation of the assay, the pathlength of the assay wells was measured using a Spectramax Gemini M5 plate reader spectrophotometer (Molecular Devices) by taking an endpoint reading and using the Pathcheck feature of the SoftMax Pro software (Molecular Devices). The increase in absorbance at 405 nm was measured every 30 seconds for one hour at 37°C.

The SoftMax Pro software was used to convert the absorbance rate (milliunits/sec) to concentration of pNA released (μM/sec) by using the pathlength and the extinction coefficient of the pNA leaving group at 405 nm, 9600 $M^{-1}cm^{-1}$. The conversion equation is as follows: Rate × (1/60 × 1000) × (1/9600 × Pathlength) × 100000. The results for each concentration of protease were graphed using Graph Pad Prism software with the substrate concentration on the X-axis and the determined μM/sec rates on the Y-axis. Using Graph Pad Prism 4 software Km and Ymax were determined by fitting the data to a Michaelis Menten equation as follows:

$$Y = ((k_{cat}K_m/1000000) \times X \times [E])/(1+(X + K_m)$$

where;

X is the substrate concentration (μM)
Y is the enzyme activity (μM/sec)
$k_{cat}K_m$) is the specificity constant ($M^{-1}sec^{-1}$)
$K_m$ is the Michaelis constant (μM)
E is the enzyme concentration (μM)

Initial values of E=1, Km = X at 0.5 × Y max and $k_{cat}K_m$ = 1000 were set.

The specific activity (munits/min/nM) of each of the FVIIa variants, wild-type FVIIa (CB553-02) was determined with the above-described assay using 420 μM Spectrozyme FVIIa (Table 11). The activity of the FVIIa variants typically were greater than that of wild-type FVIIa (measured at 2.7 munits/min/nM). The activity of the FVII variants were compared to variants described in the literature (CB591-CB594), and most FVII variants were found to have activity comparable to or greater than the activity of the variants that had been previously described in the literature.

**Table 11. Specific activity of FVIIa variants**

| ID | SEQ ID NO | Mutation (mature FVII numbering) | Mutation (chymotrypsin numbering) | Specific Activity (munits/min/nM) |
|---|---|---|---|---|
| CB553-02 | 3 | none | none | 2.7 |
| CB554 | 18 | D196K | D60K | 1.7 |
| CB555 | 19 | D196R | D60R | 1.9 |
| CB556 | 20 | D196A | D60A | 3.2 |
| CB557 | 24 | K197D | K60aD | 4.4 |
| CB558 | 23 | K197E | K60aE | 4.4 |
| CB559 | 22 | K197A | K60aA | 4.6 |
| CB560 | 25 | K197L | K60aL | 1.1 |
| CB561 | 21 | K197Y | K60aY | 5.2 |
| CB562 | 28 | K199D | K60cD | 5.5 |
| CB563 | 23 | K199E | K60cE | 5.2 |
| CB564 | 27 | K199A | K60cA | 3.8 |
| CB565 | 30 | T239A | T99A | 2.0 |
| CB566 | 33 | R290D | R147D | 5.5 |
| CB567 | 32 | R290E | R147E | 2.9 |
| CB568 | 31 | R290A | R147A | 3.9 |
| CB569 | 35 | K341R | K192R | 2.9 |
| CB579 | 36 | D196R/R290E | D60R/R147E | 2.9 |
| CB580 | 37 | D196K/R290E | D60K/R147E | 2.7 |
| CB581 | 38 | D196R/R290D | D60R/R147D | 3.1 |
| CB586 | 39 | D196R/K197E/ K199E | D60R/K60aE/ K60cE | 5.2 |
| CB587 | 40 | D196K/K197E/ K199E | D60K/K60aE/ K60cE | 4.8 |
| CB588 | 41 | D196R/K197E/ K199E/R290E | D60R/K60aE/ K60cE/R147E | 4.9 |
| CB589 | 42 | D196R/K197M/ K199E | D60R/K60aM/ K60cE | 5.4 |
| CB590 | 43 | D196R/K197M/ K199E/R290E | D60R/K60aM/ K60cE/R147E | 6.2 |
| CB591 | 147 | L305V/S314E/ L337A/F374Y | L163V/S170bE/ K188A/F225Y | 3.9 |
| CB592 | 148 | M298Q | M156Q | 3.6 |
| CB593 | 149 | V158D/E296V/ M298Q | V21D/E154V/ M 156Q | 3.9 |
| CB594 | 150 | V158D/E296V/ M298Q/K337A | V21D/E154V/ M156Q/K188A | 5.0 |

**Example 5**

**Determination of the catalytic activity of FVIIa for its substrate, Factor X**

The catalytic activity of the FVIIa variants for its substrate, Factor X (FX), was assessed indirectly in a fluorogenic assay by assaying for the activity of FXa, generated upon activation by FVIIa, on the synthetic substrate Spectrafluor FXa. A lipidated form of purified tissue factor (TF) was included in the assay to provide for optimal activity of FVIIa.

Enzyme activity of FXa for Spectrafluor FXa (CH3SO2-D-CHA-Gly-Arg-AMC.AcOH) was determined by measuring the increase in absorbance of the generated free fluorophore, AMC (7-amino-4-methylcoumarin), as a function of time.

Briefly, the FVIIa variants were initially diluted to 0.5 μM in 1 × direct assay buffer (100 mM Tris pH 8.4, 100 mM NaCl, 5 mM CaCl2, and 0.01% BSA), then further diluted to 0.1 nM in direct assay buffer. Lipidated full-length TF (Innovin; Dade Behring) was reconstituted in 20 mL water to make a 3 nM solution and diluted to 0.2 nM in 1 × direct assay buffer. Four hundred μl of 0.1 nM FVIIa was mixed with 400 μl 0.2 nM TF and incubated at room temperature for 5 minutes. The solution was diluted further by two, 2-fold dilutions into 1 × direct assay buffer containing 0.2 nM TF to obtain a total of three FVIIa dilutions of 0.05 nM, 0.025 nM, or 0.0125 nM FVIIa each containing 0.2 nM TF (FVIIa/TF solutions).

The substrate, Factor X (FX; American Diagnostica; 80 μg) was reconstituted in 135.6 μl distilled water to give a 10 μM stock and stored in aliquots at -80°C. The aliquots were not frozen and thawed more than once. The FX stock was diluted to 800 nM in direct assay buffer, then serially diluted 2-fold to obtain FX solutions ranging from 800 nM to 50 nM.

Spectrofluor Xa (American Diagnostica; 10 μmoles) was reconstituted in distilled water to 5 mM and stored at 4°C. To a 96-well black half area assay plate (Costar), 5 μl Spectrofluor Xa (American Diagnostica) was added to each well. Then, 25 μl of the FX solution was added to each well. To the last row of wells of the plate, a negative control in which no FX was added also was included in the assay. In duplicate, the three concentrations of the TF/FVIIa solutions were added at 20 μl to wells of respective columns of the plate so that each TF/FVIIa dilution was assayed against each FX dilution, with one set of columns containing no added TF/FVIIa (i.e. FX alone). The plates were mixed by shaking. The fluorescence was measured over time with a spectrafluorometer set to read every 30 seconds for 1 hour at 37°C (Ex: 380 nm, EM: 450 nm, Cut-off: 435 nm), and the time was reported in time squared units. Following the assay, a standard curve of AMC fluorescence in the same plate reader was generated to covert from fluorescence units to uM substrate released in the assay. A 1 mM AMC in DMSO (Invitrogen) was diluted to 0.02 mM in 1 × direct assay buffer. Six, two-fold serial dilutions of the AMC were made ranging from 20 nM to 0.625 nM in 1 × direct assay buffer. The fluorescence of the AMC was measured using the same assay conditions as described above and a graph of fluorescence versus concentration of AMC was plotted. The slope of the line was calculated, which served as the conversion factor for RFU to μM in subsequent calculations.

The kinetics constants for FVIIa activation of FX were calculated by performing linear regression analysis on the inverse of the substrate concentration versus the inverse of the velocity of substrate cleavage (in units of seconds$^2$), with $V_{max}$, FVIIa calculated as the inverse of the y-intercept, $K_m$, FVIIa as the slope at the y-intercept, and $V_{max/}$ $K_{m,}$ FVIIa as the inverse of the slope. The $k_{cat}$ value was then derived using the equation;

$$k_{cat}/K_{m,\ FVIIa} = V_{max}/K_{m,\ FVIIa} \times 1/(0.5 \times k2 \times [\text{FVIIa in } \mu M] \times (\text{RFU}/\mu M \text{ conversion factor}))$$

where; $k2 = ([S] \times k_{cat,\ FXa})/(K_{m,\ FXa} + [S])$, where $k_{cat,\ FXa}$ and $K_{m,\ FXa}$ are the constants for FXa cleavage of SpectrofluorXa determined experimentally using FXa standards as $k_{cat,\ FXa}$ = 117 sec$^{-1}$, and $K_{m,\ FXa}$ = 164 μM.

Using the above assay conditions, the kinetic constant k2 was determined to be 88.1 sec$^{-1}$.

The $K_m$ and $k_{cat}$ for each of the FVIIa variants was determined to assess the catalytic activity, $k_{cat}/K_m$ ($M^{-1}sec^{-1}$) of each for its substrate, FX (Table 12). The wild-type FVIIa protease (CB553-02) also was assessed and was found to exhibit an activity of $1.8 \times 10^7$ $M^{-1}sec^{-1}$. Factor VIIa activation of Factor X, as measured by Krishnaswamy, et al. (J. Biol. Chem. (1998) 273:8 4378-86) is 2.9 X $10^7$ $M^{-1}sec^{-1}$. Many of the variants displayed comparable activity to that of the unmodified protease. In some instances, FVIIa variants exhibited enhanced catalytic activity compared to the unmodified protease. For example, CB558, CB561 and CB563 exhibited catalytic activities of $5.2 \times 10^7$, $4.3 \times 10^7$ and $5.9 \times 10^7$ $M^{-1}sec^{-1}$.

**Table 12. Catalytic activity of FVIIa variants**

| ID | Mutation (mature FVII numbering) | Mutation (chymotrypsin numbering) | Catalytic activity kcat/Km (M-1sec-1) | $Log_{10}$ (kcat/ Km) | Km (mM) | kcat (sec-1) |
|---|---|---|---|---|---|---|
| CB553-02 | none | none | $1.8 \times 10^7$ | 7.3 | 0.039 | 0.71 |
| CB554 | D196K | D60K | $3.1 \times 10^7$ | 7.5 | 0.038 | 1.2 |
| CB555 | D196R | D60R | $2.6 \times 10^7$ | 7.4 | 0.036 | 0.95 |
| CB556 | D196A | D60A | $2.6 \times 10^7$ | 7.4 | 0.292 | 7.4 |

(continued)

| ID | Mutation (mature FVII numbering) | Mutation (chymotrypsin numbering) | Catalytic activity kcat/Km (M-1sec-1) | $Log_{10}$ (kcat/ Km) | Km (mM) | kcat (sec-1) |
|---|---|---|---|---|---|---|
| CB557 | K197D | K60aD | $2.5 \times 10^7$ | 7.4 | 0.046 | 1.2 |
| CB558 | K197E | K60aE | $5.2 \times 10^7$ | 7.7 | 0.020 | 1.1 |
| CB559 | K197A | K60aA | $3.6 \times 10^7$ | 7.6 | 0.026 | 0.94 |
| CB560 | K197L | K60aL | $9.3 \times 10^5$ | 6.0 | 0.641 | 0.60 |
| CB561 | K197Y | K60aY | $4.3 \times 10^7$ | 7.6 | 0.041 | 1.8 |
| CB562 | K199D | K60cD | $1.4 \times 10^7$ | 7.2 | 0.070 | 1.0 |
| CB563 | K199E | K60cE | $5.9 \times 10^7$ | 7.8 | 0.041 | 2.4 |
| CB564 | K199A | K60cA | $8.4 \times 10^6$ | 6.9 | 0.103 | 0.87 |
| CB565 | T239A | T99A | $6.1 \times 10^5$ | 5.8 | 0.128 | 0.078 |
| CB566 | R290D | R147D | $1.2 \times 10^6$ | 6.1 | 0.045 | 0.053 |
| CB567 | R290E | R147E | $6.8 \times 10^4$ | 4.8 | 1.9 | 0.014 |
| CB568 | R290A | R147A | $4.1 \times 10^6$ | 6.6 | 0.018 | 0.074 |
| CB569 | K341R | K192R | $2.7 \times 10^6$ | 6.4 | 0.092 | 0.25 |
| CB579 | D196R/R290E | D60R/R147E | $1.4 \times 10^6$ | 6.1 | 0.032 | 0.044 |
| CB580 | D196K/R290E | D60K/R147E | $1.9 \times 10^6$ | 6.3 | 0.025 | 0.047 |
| CB581 | D196R/R290D | D60R/R147D | $3.0 \times 10^6$ | 6.5 | 0.28 | 0.085 |
| CB586 | D196R/K197E/ K199E | D60R/K60aE/ K60cE | $7.8 \times 10^6$ | 6.9 | 0.033 | 0.26 |
| CB587 | D196K/K197E/ K199E | D60K/K60aE/ K60cE | $3.7 \times 10^6$ | 6.6 | 0.062 | 0.26 |
| CB588 | D196R/K197E/ K199E/R290E | D60R/K60aE/ K60cE/R147E | $5.3 \times 10^4$ | 4.7 | 0.888 | 0.047 |
| CB589 | D196R/K197M/ K199E | D60R/K60aM/ K60cE | $1.1 \times 10^7$ | 7.0 | 0.049 | 0.54 |
| CB590 | D196R/K197M/ K199E/R290E | D60R/K60aM/ K60cE/R147E | $4.6 \times 10^5$ †† | 5.7 | 0.030 | 0.014 |
| CB591 | L305V/S314E/ L337A/F374Y | L163V/S170bE/ K188A/F225Y | $6.3 \times 10^6$ | 6.8 | 0.235 | 1.5 |
| CB592 | M298Q | M156Q | $3.1 \times 10^7$ | 7.5 | 0.085 | 2.7 |
| CB593 | V158D/E296V/ M298Q | V21D/E154V/ M156Q | $4.9 \times 10^7$ | 7.7 | 0.063 | 3.1 |
| CB594 | V158D/E296V/ M298Q/K337A | V21D/E154V/ M156Q/K188A | $1.2 \times 10^8$ | 8.1 | 0.035 | 4.1 |
| †† Approximate value. | | | | | | |

**Example 6. Determination of the Concentration of Catalytically Viable Protease in a Solution**

The concentration of catalytically viable FVIIa in a stock solution was determined by titrating a complex of Factor VIIa and soluble Tissue Factor (sTF) with an irreversible peptide inhibitor of FVIIa, Phe-Phe-Arg-Chloromethylketone (FFR-CMK). The inhibitor binds to FVIIa but not to FVII. Extended incubation at a high concentration of FVIIa (50 nM) ensures complete titration of the protease. The residual activity of the FVIIa/TF complex after incubation with FFR-CMK was measured to determine the concentration of catalytically viable FVIIa in the original stock solution.

### A. 96-well assay plate format

A 96 well clear half area assay plate (Nunc) was pretreated by adding 150 μl/well of 1 × plate buffer (100 mM Tris pH 8.4, 100 mM NaCl, 0.01% BSA, 0.01 % Tween-20) to each well and incubating the plate at 37°C for a minimum of 1 hour. The buffer was removed completely by blotting on a paper towel and centrifuging the plate upside down to remove any remaining buffer, and the plate was air-dried for 1 hour and stored covered at room temperature (RT).

To prepare the FVIIa/sTF/FFR-CMK reaction mixture, a stock of FVIIa (American Diagnostica; diluted to 5 μM in 50% glycerol (v/v) and stored cold in aliquots at -20°C) or a FVIIa variant was first diluted to 500 nM in 1 × direct assay buffer (100 mM Tris pH 8.4, 100 mM NaCl, 5 mM $CaCl_2$, 0.01% BSA). The FVIIa/sTF mixture was then made by mixing 90 μl distilled water with 36 μl 5 × direct assay buffer, 18 μl 500 nM FVIIa, and 18 μl 5 μM sTF (Recombinant human Coagulation Factor III/ soluble tissue factor; R&D Systems; the stock solution used was 19.6 μM in 50% glycerol and was diluted to 5 μM in 1 × direct assay buffer and stored up to two weeks at 4°C). The components were then allowed to complex for 5 minutes at room temperature.

A stock solution of 10 mM FFR-CMK (Bachem) in DMSO (stored at -20°C) was diluted in water to 3.5 μM. Using one row of a polypropylene opaque storage plate (Costar #3363), serial two fold dilutions in water of the FFR-CMK were made across 11 wells of a 96-well opaque plate, with the last well of the row containing only water as a control. This is the 10× FFR-CMK inhibitor series solution. Into each well of a row of the pre-treated 96 well clear half area assay plate, 10.8 μl of the FVIIa/sTF mixture was added, followed by 1.2 μl of the 10× FFR-CMK inhibitor series. The solutions were mixed well and the plate was centrifuged at <3000 rpm for 5 minutes to remove drips in the wells. The plate was covered and incubated for 8 hours at 37°C.

To assay the residual activity of the FVIIa/TF complex, a mixture of the substrate Spectrozyme FVIIa (American Diagnostica, #217L; reconstituted stock of 50 μmole vial in 5 mL distilled water to 10 mM and stored at 4°C) and 5X direct buffer (500 mM Tris pH 8.4, 500 mM NaCl, 25 mM $CaCl_2$ and 0.05% BSA) was first prepared by mixing 360 μl 5 × direct assay buffer with 180 μl of a 10 mM solution of Spectrozyme FVIIa and 1080 μl of water. To each well of the assay plate, 108 μl of the prepared substrate solution was added. The wells were mixed and the plate was incubated at 37°C. The increase in absorbance at 405 nm was measured every 30 seconds for one hour at 37°C on a Spectramax Gemini M5 plate reader from Molecular Devices.

Using SoftMax Pro software (Molecular Devices), the absorbance rates were measured and the fractional activity of proteases incubated with an inhibitor was determined by dividing the measured rate by the rate of the uninhibited protease. The fractional activity was graphed against the concentration of FFR-CMK, and points that were >90% or < 10% of the uninhibited activity were discarded. A line was then drawn through the remaining points to determine the x-intercept, which represents the concentration of active protease in the solution. The values from multiple assays was measured and averaged and the standard deviation was determined.

### B. 384-well assay format

In order to increase the accuracy and throughput of the titration, the previous assay was modified for to a 384 well plate based format. Incubation was carried out for seven hours at a high protease concentration (250 nM) with a series of FFR-CMK concentrations spanning the range from 400 nM to 53 nM. The residual activity was measured by adding the FVIIa substrate (Mesyl-dFPR-ACC) and measuring the change in fluorescence signal over time.

Briefly, a 384 well black assay plate (Nunc) was pretreated as in Example 6. Then, a FVIIa/sTF solution was prepared by mixing 32.5 μl 1 μM FVIIa + 19.5 μl 5 μM sTF + 6.5 μl 5X AST buffer (100 mM Na Hepes, pH 7.5, 750 mM NaCl, 25 mM $CaCl_2$, 0.5% BSA, 0.5% PEG 8000) and 6.5 μl $dH_2O$. This reaction was incubated at room temperature for 5 minutes. One half of a 384 well plate accommodates 5 proteases measured in triplicate, and a single assay control sample. During the FVIIa/sTF incubation, the 20 mM FFR-CMK was diluted to 8 μM in 1 mM HCl, followed by 9 serial 1.25 fold dilutions across a 96 well plate. The remaining two wells of the row were left with 1 mM HCl alone. This row was then diluted 10 fold into 1X AST buffer and mixed to generate a series of FFR-CMK concentrations from 800 nM to 107 nM. To each row of the 384 well plate, 4 μl of the FFR-CMK dilution series was pipetted. Then, 4 μl of the FVIIa/sTF solution was mixed with the inhibitor series across eleven columns of the plate. The final column was filled with 4 μl 1X AST buffer to act as the plate blank. The plate was centrifuged, sealed, and incubated at room temperature for seven hours with shaking. The assay was initiated with 72 μl of 110 μM Mesyl-dFPR-ACC diluted in 1X AST buffer. The increase in fluorescence was monitored at Ex:380 nm and Em:460nm for 20 minutes with readings every 45 seconds at 37°C. The data was analyzed in the same manner as Example 6, with the following refinements. First, the residual activity was limited to those activities between 20% and 80% of the protease alone activity. Second, the y-intercept of the linear fit was constrained to fall between 0.9 and 1.1. Third, the x-intercept was constrained to those between 125 nM and 375 nM. The x-intercept for the three replicates of each protease were averaged and the value and standard deviation reported.

**Example 7**

**Determination of the $IC_{50}$ for TFPI Inhibition of FVIIa/TF**

The potency of the interaction between TFPI and the FVIIa/TF complex was assessed by measuring the level of inhibition of various concentrations of TFPI on the catalytic activity of a FVIIa/TF towards a substrate, Spectrazyme VIIa. The concentration of TFPI that was required for 50% inhibition ($IC_{50}$) was calculated for each FVII variant, and a FVIIa standard.

A 96 well clear half area assay plate (Nunc) was pretreated by adding 150 μl/well of 1 × plate buffer (100 mM Tris pH 8.4, 100 mM NaCl, 0.01% BSA, 0.01% Tween-20) to each well and incubating the plate at 37°C for a minimum of 1 hour. The buffer was removed completely by shaking and blotting the plate and centrifuging the plate upside down to remove the remaining buffer. The plate was air-dried for 1 hour, and stored at room temperature (RT).

In a 1.7 ml microfuge tube (low adhesion microfuge tube from ISC Bioexpress), a mixture of FVIIa/TF was prepared in a total volume of 450 μl by mixing 9 μl of 250 nM FVIIa (American Diagnostica, CB553-02 or a respective variant to be tested) was mixed with 337.5 μl of 2× TF (Innovin; Dade Behring; lyophilized product resuspended in 10 mL distilled water to generate 2X TF, which approximately equals 7 nM of lipidated TF), 90 μl 5× assay buffer (500 mM Tris pH 8.4, 500 mM NaCl, 25 mM $CaCl_2$, 0.05% BSA) and 13.5 μl of water, resulting in a solution containing 5 nM FVIIa and 5.2 nM TF. The mixture was incubated at room temperature for 5 minutes to allow the components to complex. To each well of 2 columns in the pretreated 96 well clear half area assay plate, 25 μl of the respective FVIIa/sTF mixture was added and the plate was covered to prevent evaporation.

Human Recombinant TFPI (R&D Systems) was initially dissolved in 33 μl 50% glycerol (v/v) to make a 10 μM stock for storage at -20°C. The TFPI stock was further diluted to 1.5 μM in a final 1 X direct buffer (100 mM Tris pH 8.4, 100 mM, NaCl, 5 mM $CaCl_2$, 0.01% BSA) in a polypropylene storage plate as follows: for each protease tested, 87.5 μl of a 1.5 μM solution of TFPI was made by mixing 13.1 μl 10 μM TFPI with 17.5 μl 5 x assay buffer and 56.9 μl distilled water. Serial 3-fold dilutions of the TFPI solution were made in 1 × assay buffer by mixing 27.5 μl TFPI into 55 μl 1 x assay buffer, such that solutions containing 750 nM, 250 nM, 83.3 nM, 27.8 nM, 9.26 nM, 3.1 nM, and 1.03 nM TFPI were generated. The final well of the series contained only 1X direct buffer as a control.

Twenty-five μl of each dilution of TFPI was added to 2 wells (i.e. in duplicate) of 2 columns of the 96 well clear half area assay plate containing the FVIIa/TF mixture, such that the protease mixture was assayed in duplicate with each TFPI dilution. A solution of 1 x assay buffer without TFPI also was added to 2 wells containing the FVIIa/TF mixture as a negative control. The plate was agitated briefly and then centrifuged at 3000 rpm for 5 minutes before incubation at 37°C for 1.5 hours.

A stock solution of Spectrazyme VIIa (American Diagnostica) was prepared by reconstituting 50 μmoles in 5 ml distilled water to 10 mM and storing at 4°C until use. Immediately prior to use, the solution was diluted to 600 μM in distilled water. Following incubation of the assay plate from above, 10 μl of the diluted Spectrazyme VIIa was added to each well of the assay plate. The reactions were mixed and the plate was incubated at 37°C. The increase in absorbance at 405 nm was measured every 30 seconds for one hour at 37°C, and the absorbance rate were calculated using SoftMax Pro software (Molecular Devices).

To determine the degree of inhibition by TFPI, the absorbance rates of protease reactions containing TFPI were first divided by the absorbance rate of reactions containing no TFPI (the control sample) to obtain the fractional activity, and the $log_{10}$ of each TFPI concentration was determined. Using GraphPad Prism Software, the $log_{10}$ [TFPI] was plotted against the fractional activity for each protease, and dose response curve was generated with a curve fit that assumed the top and bottom of the activity data are fixed at 1 and 0, respectively. The software was used to determine TFPI inhibition as both the log $IC_{50}$ ($pIC_{50}$) value, and the absolute $IC_{50}$ (TFPI inhibition in nM) for each protease, and its average and standard deviated was determined.

The level of inhibition of TFPI of each of the FVIIa variants in complex with sTF was determined and expressed as $IC_{50}$ or $pIC_{50}$ (Table 13). The degree to which TFPI inhibited the activity of unmodified FVIIa (CB553-02) in complex with sTF also was determined, and the $IC_{50}$ was found to be 88 nM. Nine of the FVIIa variants that were generated displayed a decreased potency of their interaction with TFPI, as reflected in increased $IC_{50}$ values.

**Table 13. Inhibition of FVIIa variants by TFPI**

| ID | Mutation (mature FVII numbering) | Mutation (chymotrypsin numbering) | $IC_{50}$ (nM) | $pIC_{50}$ |
|---|---|---|---|---|
| CB553-02 | none | none | 88 | 7.1 |
| CB554 | D196K | D60K | 45 | 7.4 |

(continued)

| ID | Mutation (mature FVII numbering) | Mutation (chymotrypsin numbering) | $IC_{50}$ (nM) | $pIC_{50}$ |
|---|---|---|---|---|
| CB555 | D196R | D60R | 37 | 7.4 |
| CB556 | D196A | D60A | 5 | 8.3 |
| CB557 | K197D | K60aD | 220 | 6.7 |
| CB558 | K197E | K60aE | 310 | 6.5 |
| CB559 | K197A | K60aA | 53 | 7.3 |
| CB560 | K197L | K60aL | 1100 | 5.9 |
| CB561 | K197Y | K60aY | 110 | 7.0 |
| CB562 | K199D | K60cD | 63 | 7.2 |
| CB563 | K199E | K60cE | 33 | 7.5 |
| CB564 | K199A | K60cA | 68 | 7.2 |
| CB565 | T239A | T99A | 320 | 6.5 |
| CB566 | R290D | R147D | 240 | 6.6 |
| CB567 | R290E | R147E | 440 | 6.4 |
| CB568 | R290A | R147A | 200 | 6.7 |
| CB569 | K341R | K192R | 300 | 6.5 |
| CB579 | D196R/R290E | D60R/R147E | 28 | 7.6 |
| CB580 | D196K/R290E | D60K/R147E | 52 | 7.3 |
| CB581 | D196R/R290D | D60R/R147D | 32 | 7.5 |
| CB586 | D196R/K197E/ K199E | D60R/K60aE/ K60cE | 13 | 7.9 |
| CB587 | D196K/K197E/ K199E | D60K/K60aE/ K60cE | 20 | 7.7 |
| CB588 | D196R/K197E/ K199E/R290E | D60R/K60aE/ K60cE/R147E | 28 | 7.6 |
| CB589 | D196R/K197M/ K199E | D60R/K60aM/ K60cE | 44 | 7.4 |
| CB590 | D196R/K197M/ K199E/R290E | D60R/K60aM/ K60cE/R147E | 66 | 7.2 |
| CB591 | L305V/S314E/ L337A/F374Y | L163V/S170bE/ K188A/F225Y | 43 | 7.4 |
| CB592 | M298Q | M156Q | 35 | 7.5 |
| CB593 | V158D/E296V/ M298Q | V21D/E154V/ M156Q | 41 | 7.4 |
| CB594 | V158D/E296V/ M298Q/K337A | V21D/E154V/ M156Q/K188A | 22 | 7.7 |

**Example 8**

***In* vivo assessment of wild-type FVIIa procoagulant activity**

A mouse model of hemophilia A was established to assess the procoagulant activity of FVIIa polypeptides. Hemophilia A was induced in CD-1 mice by administration of anti-FVIII antibodies, followed by surgical removal of the tips of the tails to initiate bleeding. The mice were then treated with FVIIa polypeptide and the time taken to stop bleeding, and the amount of blood lost during this time, was measured to determine the procoagulant activity of the FVIIa polypeptides.

Male CD-1 mice were anesthetized by intraperitoneal administration of both thiobarbital sodium at 100 mg/kg, and ketamine at 100 mg/kg. Lidocaine was administered by subcutaneous injection into the ventral neck to reduce sensitivity. The trachea and carotid artery were cannulated through a small skin incision in the neck to facilitate unrestricted breathing and the administration of anti-Factor VIII antibody, recombinant human Factor VIIa (rhFVIIa) and/or modified FVII polypeptides.

Cannulated mice were administered 3.76 mg sheep-anti-human-FVIII antibody (Affinity Biologicals, lot IG129R4, 612 mouse BU/ml) in 40 μL. This dose was determined by conducting an initial dose response experiment with the antibody, using 0.376, 0.94, 1.88 and 3.76 mg of anti-human-FVIII, and assessing blood loss and bleeding time. After 20 minutes, the tails of the mice were placed in 15mL tubes containing 39°C phosphate buffered saline (PBS) for a period of 10 minutes. At 30 minutes, the tails were briefly removed from the PBS solution and the last 5 mm of the tails were severed to initiate bleeding. The time at which bleeding began was noted. The tails were then returned to the tube containing 39°C PBS and allowed to bleed for 5 minutes (pre-bleed) to ensure that the mice had responded to the anti-FVIII antibody. Following the pre-bleed, the mice were administered FVIIa polypeptides or the vehicle in which the FVIIa proteins were prepared and delivered. FVIIa polypeptides were diluted in either PBS or a buffer composed of 52 mM sodium chloride, 10.2 mM calcium chloride dehydrate, 9.84 mM glycylglycine, 0.01% polysorbate 80 and 165 mM mannitol. The FVIIa preparations were administered at either 1, 3 or 10 mg/kg, in a volume equivalent to 3 mL/kg, *via* the carotid cannulae and the tails were placed in fresh tubes containing 39°C PBS. The bleeding was monitored for a period of 20 minutes and the times at which bleeding stopped were noted. The total bleeding time was calculated as the sum of the duration of bleeding during the pre-bleed, and the duration of bleeding following administration of FVIIa polypeptides, or PBS or buffer.

To determine the amount of blood lost during the bleeding episodes, the contents of the 15mL tubes were assayed for hemoglobin content. Triton X-100 was diluted 1 in 4 in sterile water and 100 μL was added to 1 mL of the samples to cause hemolysis. The absorbance of the samples was then measured at a wavelength of 546 nm. To calculate the amount of blood lost, the absorbance was read against a standard curve generated by measuring the absorbance at 546 nm of known volumes of murine blood, diluted in PBS and hemolysed as above with Triton X 100.

An experiment was conducted comparing rhFVIIa (CB533) generated as described above with the commercially available recombinant human FVIIa (NovoSeven®, Novo Nordisk) and blood loss was assessed following administration of a 3 mg/kg dose of each protein. The blood loss in the vehicle group (buffer, n = 15) was 671.9 ± 57.89 μl over the 20 minute period. This was reduced by the rhFVIIa produced by Catalyst Biosciences to 264.1 ± 56.59 μl and by NovoSeven® to 273.7 ± 53.93 μl (n = 14). This experiment demonstrated equivalency between the two proteins.

### Example 9

### Pharmakokinetic analysis of FVIIa polypeptides

Pharmacokinetic properties of FVIIa polypeptides were assessed by measuring the amount of human Factor VIIa in mouse plasma. Two assays were used to quantify FVIIa in plasma. An ELISA was used to quantify total FVIIa protein in mouse plasma and a FVIIa-dependant clotting assay (FVII:C) was used to quantify coagulant activity of the FVIIa polypeptides in plasma.

### A. Administration of FVIIa polypeptides to mice

Modified FVIIa polypeptides and the unmodified recombinant human FVIIa (rhFVIIa) protein (NovoSeven®, Novo Nordisk) were evaluated in pharmacokinetic studies. For each study, 18 male CD-1 mice were injected with an intravenous bolus dose (0.1-3.0 mg/kg, depending on the study) of rFVIIa. At 5, 15, 30, 60, 120, and 240 minutes post-injection, three mice from each injection protocol were euthanized using $CO_2$ asphyxiation and approximately 1.0 mL of blood was drawn into a 1.0 mL syringe via a percutaneous cardiac puncture. Each syringe was pre-loaded with sufficient sodium citrate to achieve a final concentration of 3.2% in 1mL of blood. The blood samples were then centrifuged at 9000 rpm for 8 min at 4°C. The plasma was removed to labeled individual 1.5 ml tubes (Eppendorf), snap frozen in liquid nitrogen and stored at -80°C. Additionally, one mouse per experiment was injected with vehicle alone (sham) and plasma from this mouse was used for background **FVIIa** activity determination.

### B. ELISA Assay

A commercially available kit, IMUBIND® Factor VII ELISA (American Diagnostica) was used to detect FVII protein in serum by ELISA. This kit employs a plate pre-coated with an anti-FVII/FVIIa antibody to capture the protein, and a biotinylated anti-FVII antibody for detection through a steptavidin labeled horseradish peroxidase. The kit was used according to the manufacturers' direction with the following exceptions: first, the standard curve has been narrowed to ensure a linear range over the entire concentration range and spans the concentrations of 0.88 ng/ml to 10 ng/ml; second, the purified FVIIa variant itself was used for the standard curve rather than the FVII standard provided with the kit because of differences in the antibody affinity. Experiments indicated that the complex of FVIIa with anti-thrombin III (ATIII), a potential plasma inhibitor of FVIIa, is detected at 75% of the level of the free protease, ensuring that the assay can detect the total FVIIa in the plasma sample in both the active and inactive forms.

Briefly, the plasma samples were thawed at room temperature and diluted 10 fold in sample buffer (PBS + 0.1% Triton X-100 + 1.0% BSA) then diluted serially 5.5 fold for four dilutions. The four diluted samples were diluted 2 fold onto the ELISA plate for final sample dilutions of 20, 110, 605 and 3327.5 fold. These four dilutions of mouse plasma covered a range of protease concentrations from 33,000 ng/ml to 20 ng/ml. Each sample was measured on two separate assays plates, and those measurements within the range of the standard curve were used to calculate the concentration of FVIIa variant in the plasma sample

**C. Clotting Assay**

A commercially available kit (STACLOT FVIIa-rTF, Diagnostica Stago, Parsippany, NJ) was used as the clotting assay. To determine the coagulant activity of the active FVIIa polypeptides, plasma samples were assayed using a FVIIa-dependant clotting assay (FVII:C). The assay was performed using reagents and instructions provided in a commercial kit and clotting time measured using an electromechanical clot detection instrument (STArt4, Diagnostica Stago, Parsippany, NJ). The kit was used according to the manufacturers' direction with the following exceptions: first, the purified FVIIa variant itself was used for the standard curve rather than the rhFVIIa standard provided with the kit; second, the following bulk commercial reagents were used for routine pharmacokinetic screening studies and gave comparable results to the kit reagents: soluble tissue factor (CalBioChem, La Jolla, Ca) and synthetic phospholipid blend (Avanti Polar Lipids, Alabaster, AL), TBSA buffer (Tris-NaCl, pH 7.5 with 1% BSA; DiaPharma, West Chester, Ohio), and 25 μM calcium chloride solution (Diagnostica Stago, Parsippany, NJ).

The clotting asaay was performed as follows. Frozen plasma samples were thawed at room temperature for approximately 45 min and then diluted 1:5000 in buffer. Fifty μl of the diluted plasma is combined with 50 μL Factor VII-deficient human plasma and 50 μL of relipidated tissue factor and pre incubated for 180 seconds. Following preincubation, 50 μL of calcium chloride solution (25 μM) was added to initiate clotting. Clotting time was determined using electromechanical clot detection. Each plasma sample was assayed in duplicate. The system was calibrated by constructing a standard curve using the clotting time of serial dilutions of buffer containing a known amount of the specific FVIIa variant being assayed. FVIIa concentrations in mouse plasma samples were calculated from the linear portion of the log FVIIa versus Log clotting time standard curve. The ability of plasma samples to induce clotting in Factor VII-deficient plasma was reported as ng FVIIa/mL of mouse plasma following background subtraction of endogenous wild type FVIIa in plasma from sham treated mice.

The half-life of each FVII protein was routinely determined by making a conventional fit of the natural log of the activity to a straight line, and measuring the time taken for the activity of FVIIa proteins to be reduced by half. For FVII proteins with multi exponential decay, half-life was determined from the terminal portion of the log plasma VS time profile. Additional pharmacokinetic parameters were calculated using commercially available software (WinNonLin v5.1, Pharsight Corporation, Mountain View, CA).

**D. Pharmacokinetic properties of CB728, CB735 and CB945**

Using the above protocol, the the pharmacokinetic properties of wild-type FVIIa and two FVIIa variants: CB728, CB735 and CB945 were assessed. The results are set forth in Table 14. CB735 and CB945 exhibited improved pharmacokinetic parameters compared to wild-type FVIIa.

**Table 14. Mouse Pharmacokinetic Parameters of FVIIa Variants**

| CB# | Mutation (mature FVII numbering) | IV Dose (mg/kg) | Plasma $AUC_{0-inf}$ (μg.min/mL)/Dose | Half-Life (min) | Cl (mL/min/kg) | Vd (mL/kg) | N |
|---|---|---|---|---|---|---|---|
| CB553 | WT | 0.1 | 606 | 35.1 | 2.02 | 98.8 | 5 |
| CB553 | WT | 1.0 | 798 | 50.2 | 1.55 | 118 | 2 |
| CB728 | Gla Swap FIX | 0.1 | 207 | 19.9 | 4.85 | 140 | 2 |
| CB735 | K341D | 0.1 | 2817 | 106 | 0.14 | 22.0 | 1 |
| CB945 | M156Q/K192D | 0.1 | 2430 | 78.1 | 0.41 | 46.3 | 1 |

**E. Pharmacokinetic properties of CB558**

The half-life of a modified FVIIa polypeptide that exhibited increased resistance to TFPI (CB-558; containing a K197E mutation) was measured and compared with that of an unmodified recombinant human FVIIa (rhFVIIa) protein (NovoSeven®, Novo Nordisk) in a pharmacokinetic study similar to that described above, with some modifications. Fifteen male CD-1 mice were injected with a 0.5mg/kg intravenous bolus dose of CB-558, and 15 mice were injected with a 0.5mg/kg intravenous bolus dose of rhFVIIa. At 5, 15, 30, 60 and 120 min post-injection, three mice from each injection protocol were euthanized using $CO_2$ asphyxiation and approximately 1.0 mL of blood was drawn into a 1.0 mL syringe via a percutaneous cardiac puncture. Each syringe was pre-loaded with sufficient sodium citrate to achieve a final concentration of 3.2% in 1mL of blood. The blood samples were then centrifuged at 9000 rpm for 8 min at 4°C. The plasma was removed to labeled individual 1.5 ml tubes (Eppendorf), snap frozen in liquid nitrogen and stored at -80°C.

To determine the coagulant activity and the half-life of the active FVIIa polypeptides, the plasma samples were assessed at Machaon Diagnostics, Inc. (Oakland, CA) using a FVIIa-dependant clotting assay (FVII:C) and an automated hemostasis instrument (AMAX 190plus, Trinity Biotech). Briefly, the frozen plasma samples were thawed in a 37°C water-bath for 5 minutes and then diluted 1:40 in Veronal Buffer. Six μl of the diluted plasma was added to 54 μl Veronal buffer in a cuvette and mixed with a metal bead. An equal amount (60 μl) of Factor VII-deficient human plasma (Precision Biologic) was then added to the cuvette, which was incubated at 37°C for 60 seconds with constant mixing.

To initiate the start of timing of the reaction, 120μL of thromboplastin (rabbit brain source, Thromboplastin-DS; Pacific Hemostasis) was added to the cuvette and constant mixing was maintained therein. The clotting reaction was monitored photo-optically at 405nm to detect formation of fibrin. The system was calibrated by analyzing the clotting time of serial dilutions of reference plasma with published FVII levels (Precision Biologic), and then analyzing the clotting time of normal control plasma (Precision Biologic) and abnormal control plasma (Precision Biologic).

The ability of plasma samples to induce clotting in Factor VII-deficient plasma was reported as a percentage of the clotting time observed in normal human plasma. The coagulant activity of plasma from both FVIIa (NovoSeven®)-treated and CB-558-treated mice decreased over time. The half-life of each FVII protein was determined by making a conventional fit of the natural log of the activity to a straight line, and measuring the time taken for the activity of FVIIa proteins to be reduced by half. The half-life of CB-558 was observed to be approximately twice that of FVIIa (NovoSeven®); 156 minutes compared with 67 minutes.

## Example 10

### Generation of additional FVII variants

A series of additional FVII mutants were generated to alter one or more properties and/or activities of FVII. In addition to modifications to increase TFPI resistance, modifications were designed to increase resistance to AT-III, increase catalytic activity, increase half-life, and/or increase affinity and/or binding to phospholipids, such as those on activated platelets. The modifications include amino acid replacement, insertion and deletion. FVII variants containing two or more modifications also were generated. Table 23 sets forth the FVII variants that were generated and expressed in Freestyle™ 293-F cells. Amino acid replacements were incorporated into FVII polypeptides using the methods described in Example 2.B, above, with an appropriate oligonucleotide.

Table 15 below sets forth the additional FVII variants that were generated. Gla swap FVII variants were generated in which amino acid residues A1 to Y44 (by mature FVII numbering) at the N-terminus of the wild-type FVII protein were replaced with amino acid residues corresponding to the Gla domain of FIX, FX, Protein C, Protein S or thrombin. The "Gla Swap FIX" FVII variant (i.e. a FVII polypeptide in which the endogenous Gla domain has been replaced with the Gla domain from FIX) contains amino acid residues A2 to Y45 of SEQ ID NO: 110 at the N-terminus; the "Gla Swap FX" FVII variant contains amino acid residues A1 to Y44 of SEQ ID NO: 11 at the N-terminus; the "Gla Swap Protein C" FVII variant contains amino acid residues A1 to H44 of SEQ ID NO: 113 at the N-terminus; the "Gla Swap Protein S" FVII variant contains amino acid residues A1 to Y44 of SEQ ID NO:114 at the N-terminus; and the "Gla Swap thrombin" FVII variant contains amino acid residues A1 to Y44 of SEQ ID NO: 115 at the N-terminus.

**Table 15. Exemplary Factor VII Variants**

| ID | Variant (mature FVII numbering) | Variant (Chymotrypsin numbering) | Variant polypeptide SEQ ID NO |
|---|---|---|---|
| CB697 | R290N | R147N | 206 |
| CB698 | R290Q | R147Q | 207 |
| CB699 | R290K | R147K | 208 |
| CB700 | R290M | R147M | 209 |
| CB701 | R290V | R147V | 210 |

(continued)

| ID | Variant (mature FVII numbering) | Variant (Chymotrypsin numbering) | Variant polypeptide SEQ ID NO |
|---|---|---|---|
| CB733 | K341N | K192N | 211 |
| CB734 | K341M | K192M | 212 |
| CB735 | K341D | K192D | 213 |
| CB853 | G237T238insA | G97T98insA | 214 |
| CB854 | G237T238insS | G97T98insS | 215 |
| CB855 | G237T238insV | G97T98insV | 216 |
| CB856 | G237T238insAS | G97T98insAS | 217 |
| CB857 | G237T238insSA | G97T98insSA | 218 |
| CB858 | D196K197insK | D60K60ainsK | 219 |
| CB859 | D196K197insR | D60K60ainsR | 220 |
| CB860 | D196K197insY | D60K60ainsY | 221 |
| CB861 | D196K197insW | D60K60ainsW | 222 |
| CB862 | D196K197insA | D60K60ainsA | 223 |
| CB863 | D196K197insM | D60K60ainsM | 224 |
| CB864 | K197I198insE | K60aI60binsE | 225 |
| CB865 | K197I198insY | K60aI60binsY | 226 |
| CB866 | K197I198insA | K60aI60binsA | 227 |
| CB867 | K197I198insS | K60aI60binsS | 228 |
| CB637 | K197E/K341Q | K60aE/K192Q | 229 |
| CB638 | K197L/K341Q | K60aL/K192Q | 230 |
| CB670 | G237V/K341Q | G97V/K192Q | 231 |
| CB688 | K197E/K199E | K60aE/K60cE | 232 |
| CB689 | K197E/G237V | K60aE/G97V | 233 |
| CB691 | K197E/K199E/K341Q | K60aE/K60cE/K192Q | 250 |
| CB694 | K199E/K341Q | K60cE/K192Q | 234 |
| CB671 | K197E/G237V/K341Q | K60aE/G97V/K192Q | 235 |
| CB669 | V158D/G237V/E296V/M298Q | V21D/G97V/E154V/M156Q | 241 |
| CB591 | L305V/S314E/ L337A/F374Y | L163V/S170bE/K188A/F225 Y | 147 |
| CB592 | M298Q | M156Q | 148 |
| CB593 | V158D/E296V/ M298Q | V21D/E154V/M156Q | 149 |
| CB594 | V158D/E296V/ M298Q/K337A | V21D/E154V/M156Q/K188 A | 150 |
| CB690 | K197E/G237V/M298Q | K60aE/G97V/M156Q | 242 |
| CB692 | K197E/G237V/M298Q/K341Q | K60aE/G97V/M156Q/K192Q | 243 |
| CB693 | K197E/K199E/G237V/M298Q /K341Q | K60aE/K60cE/G97V/M156Q /K192Q | 244 |
| CB695 | G237V/M298Q | G97V/M156Q | 245 |
| CB696 | G237V/M298Q/K341Q | G97V/M156Q/K192Q | 246 |
| CB902 | K197E/M298Q | K60aE/M156Q | 248 |

(continued)

| ID | Variant (mature FVII numbering) | Variant (Chymotrypsin numbering) | Variant polypeptide SEQ ID NO |
|---|---|---|---|
| CB945 | M298Q/K341D | M156Q/K192D | 249 |
| CB728 | Gla swap FIX | Gla swap FIX | 236 |
| CB729 | Gla swap FX | Gla swap FX | 237 |
| CB730 | Gla Swap Prot C | Gla Swap Prot C | 238 |
| CB731 | Gla Swap Prot S | Gla Swap Prot S | 239 |
| CB732 | Gla swap Thrombin | Gla swap Thrombin | 240 |
| CB850 | M298Q/Gla Swap FIX | M156Q/Gla Swap FIX | 247 |

**Example 11**

**Analysis of the catalytic activity of FVIIa variants for the substrate, Factor X**

The catalytic activity of the FVIIa variants for the substrate, Factor X (FX), was assessed indirectly in two types of chromogenic assays by assaying for the activity of FXa, generated upon activation by FVIIa, on the synthetic substrate Spectrafluor FXa. The two assays were performed either in the presence or the absence of lipidated tissue factor, to assess both TF-dependent and TF-independent activity. The FVII variants were expressed, purified and activated to FVIIa as described above in Examples 2 and 3. Although most FVII variants were expressed only in Freestyle™ 293-F cells, some also were expressed in BHK-21 cells.

***Lipidated tissue factor indirect assay***

The catalytic activity of the FVIIa variants in the presence of tissue factor was assessed using the assay described in Example 5, above, with minor modifications. One such modification was the use of a Factor X substrate protease that had been treated with ERG-CMK and FFR-CMK to reduce the background activity (Molecular Innovations). Two types of data analysis were performed using two separate assays; a linear range analysis assay and a hyperbolic range analysis assay. The linear range analysis assay used a range of Factor X concentrations between 0 and 150 nM to ensure accurate measurement of the kinetic constants in the linear range of the dose curve. In contrast, the hyperbolic range analysis assay used a range of Factor X concentrations between 0 and 1.44 μM to ensure accurate measurement of the kinetic constants with a saturating (hyperbolic) dose curve.

The lipidated tissue factor indirect assay with linear range data analysis was performed essentially as described in Example 5, above, with the following modifications. The FVIIa variant/TF solutions were prepared as 0.1 nM FVIIa/0.4 nM TF solutions and incubated for 30 minutes before being diluted two-fold in 0.4 nM TF down to a solution containing 1.5625 pM FVIIa/ 0.4 nM TF. Twenty-five μL of the FVIIa/TF solution was mixed with 25 μL of a substrate solution that contained 1.0 mM Spectrofluor FXa (American Diagnostica) and one of 300 nM, 200 mM, 133.3 nM, 88.9 nM, 59.3, 39.5 nM, 36.3 nM or 0 nM of Factor X (Molecular Innovations). Thus, the final concentrations for the assay were 0.8 pM FVIIa, 0.2 nM TF, 0.5 mM Spectrofluor FXa and 150 nM, 100 mM, 66.7 nM, 44.4 nM, 29.6 nM, 19.8 nM, 13.2 nM or 0 nM of Factor X (Molecular Innovations) in 50 μL/well. The AMC standard curve, which served as the conversion factor for RFU to μM in subsequent calculations, was expanded to include a dose range that covered from 0 μM to 100 μM AMC.

The lipidated tissue factor indirect assay with hyperbolic range data analysis was performed essentially as described in Example 5, above, with the following modifications. The FVIIa variant/TF solutions were prepared as 0.1 nM FVIIa/0.4 nM TF solutions and incubated for 30 minutes before being diluted two-fold in 0.4 nM TF down to 1.5625 pM (or 0.78 pM for proteases expected to have high activity) FVIIa/0.4 nM TF. Twenty-five μL of the FVIIa/TF solution was mixed with 25 μL of a substrate solution that contained 1.0 mM Spectrofluor FXa (American Diagnostica) and one of 1440 nM, 720 mM, 360 nM, 180 nM, 90 nM, 45 nM, 22.5 nM or 0 nM of Factor X (Molecular Innovations). Thus, the final concentrations for the assay were 0.8 (or 0.39) pM FVIIa, 0.2 nM TF, 0.5 mM Spectrofluor FXa and 7 nM, 720 mM, 360 nM, 180 nM, 90 nM, 45 nM, 22.5 nM, 11.25 nM or 0 nM of Factor X (Molecular Innovations) in 50 μL/well. The $k_{cat}$ and $K_m$ parameters are calculated using the Michaelis Menton hyperbolic equation of the form $(V_{max}/(1+(K_m x)))$. The AMC standard curve, which served as the conversion factor for RFU to μM in subsequent calculations, was expanded to include a dose range that covered from 0 μM to 100 μM AMC.

To determine the kinetic rate constants for the FVIIa or FVIIa variant activation of FX, raw data collected with the SoftMax Pro application (Molecular Devices) were exported as .XML files. Further data linear and non-linear analyses were performed with XLfit4, a software package for automated curve fitting and statistical analysis within the Microsoft Excel spreadsheet environment (IDBS Software).

For data collected using the linear range assay, the $k_{cat}/K_m$ ($M^{-1}$ $sec^{-1}$) kinetic constants are calculated directly from the slope of linear regression analyses of the FX concentration versus the velocity of the fluorogenic substrate cleavage (in $\mu M/sec^2$) where $k_{ca}/K_m$ = slope/[FVIIa] $\times 0.5 \times k_2$. The correction factor $k_2$ was determined to be 45 using the method described in Example 5 and kinetic constants for FXa cleavage of Spectrofluor FXa of $k_{cat,FXa}$ = 56 sec-1 and $K_{m,\ FXa}$ = 126 nM, determined experimentally with activated FX (FXa) that was previously active site titrated with AT-III/heparin. Excluding data points that resulted in $R^2$ values less than 0.98 ensured the linearity of the data sets used in the fitting routine.

Analyses of data collected using the hyperbolic range assay were calculated from non-linear regression analyses of the FX concentration versus the velocity of the fluorogenic substrate cleavage (in $\mu M/sec^2$). The individual $k_{cat}$ and $K_m$ parameters are calculated as fit parameters using the Michaelis Menton hyperbolic equation of the form $(Y_{max}/(1+(K_m/x)))$ where $k_{cat} = V_{max}$/[FVIIa] $\times 0.5 \times k_2$. The kinetic constant, $k_{cat}/K_M$ was calculated from the individual $k_{cat}$ and $K_m$ fitted parameters.

*Tissue factor-independent indirect assay*

The catalytic activity of the FVIIa variants in the presence of tissue factor was assessed in an indirect assay similar to that described above except that tissue factor was not included in the assay. Thus, the assay to assess TF-independent activity was performed essentially as described above, with the following modifications. The FVIIa variant solutions were diluted to 50 nM. Twenty-five $\mu$L of each FVIIa solution was mixed with 25 $\mu$L of a substrate solution that contained 1.0 mM Spectrofluor FXa (American Diagnostica) and one of 1050 nM, 700 mM, 466.7 nM, 311.1 nM, 207.4 nM, 138.3 nM, 92.2 nM or 0 nM of Factor X (Molecular Innovations). Thus, the final concentrations for the assay were 25 nM FVIIa, 0.5 mM Spectrofluor FXa and 525 mM, 350 nM, 233.3 nM, 155.6 nM, 103.7 nM, 69.1 nM, 46.1 nM or 0 nM of Factor X (Molecular Innovations) in 50 $\mu$L/well. Data analyses were performed as described for the linear range assay, above with no modifications.

Tables 16-18 provide the results of the assays that were performed to measure the catalytic activity of the FVIIa variants. Tables 15 and 16 provide the catalytic activity as measured in a TF-dependent Indirect Assay using FVIIa polyppetides expressed from 293-F cells and BHK-21 cells, respectively, and Table 17 provides the catalytic activity as measured in a TF-independent Indirect Assay using FVIIa polypetides expressed from 293-F cells and/or BHK-21 cells. The results are presented as the kinetic constant for catalytic activity, $k_{cat}/K_m$ ($M^{-1}sec^{-1}$), and also expressed as a percentage of the activity of the wild-type FVIIa, wherein the activity is catalytic activity, $k_{cat}/K_m$ ($M^{-1}sec^{-1}$) of each FVIIa variant for its substrate, FX. The use of the linear or hyperbolic range data analysis also is indicated for the values presented in the tables. Not all FVIIa variants were assayed in each assay. Some of the FVII variants assayed in Example 5, above, displayed slightly reduced or increased catalytic activity for FX in this set of assays compared to the assay described in Example 5. The difference in activities to those seen in Example 5 are likely are due to varied background activity of residual FXa in the FX substrate obtained from American Diagnostica. Several FVIIa variants exhibited increased catalytic activity compared to the wild-type FVIIa molecule. For example, CB760, which contains the Q366V mutation, has a catalytic activity of between 1.5 and 5 times that of wild-type FVIIa.

**Table 16. Catalytic activity of FVIIa variants: TF-Dependent Indirect Assay with FVIIa polypeptides from 293-F cells**

| ID | Mutation (mature FVII numbering) | Mutation (Chymotrypsin numbering) | $k_{cat}/K_M$ ($M^{-1}s^{-1}$) | $k_{cat}/K_M$ (% WT) | Assay Format |
|---|---|---|---|---|---|
| CB553 | WT | WT | $3.42 \times 10^7$ | 100 | linear |
| CB554 | D196K | D60K | $1.10 \times 10^7$ | 23 | hyperbolic |
| CB555 | D196R | D60R | $2.25 \times 10^7$ | 46 | hyperbolic |
| CB556 | D196A | D60A | $2.58 \times 10^7$ | 53 | hyperbolic |
| CB557 | K197D | K60aD | $3.05 \times 10^7$ | 63 | hyperbolic |
| CB558 | K197E | K60aE | $1.54 \times 10^7$ | 32 | hyperbolic |
| CB559 | K197A | K60aA | $3.67 \times 10^7$ | 75 | hyperbolic |
| CB560 | K197L | K60aL | $1.51 \times 10^7$ | 31 | hyperbolic |

(continued)

| ID | Mutation (mature FVII numbering) | Mutation (Chymotrypsin numbering) | $k_{cat}/K_M$ ($M^{-1}s^{-1}$) | $k_{cat}/K_M$ (% WT) | Assay Format |
|---|---|---|---|---|---|
| CB561 | K197Y | K60aY | $5.16 \times 10^7$ | 106 | hyperbolic |
| CB562 | K199D | K60cD | $4.62 \times 10^7$ | 95 | hyperbolic |
| CB563 | K199E | K60cE | $3.63 \times 10^7$ | 74 | hyperbolic |
| CB564 | K199A | K60cA | $5.56 \times 10^7$ | 114 | hyperbolic |
| CB565 | T239A | T99A | $1.66 \times 10^7$ | 34 | hyperbolic |
| CB566 | R290D | R147D | $5.80 x 10^6$ | 12 | hyperbolic |
| CB567 | R290E | R147E | $6.18 \times 10^6$ | 13 | hyperbolic |
| CB568 | R290A | R147A | $8.25 \times 10^6$ | 17 | hyperbolic |
| CB569 | K341R | K192R | $3.95 \times 10^7$ | 81 | hyperbolic |
| CB579 | D196R/R290E | D60R/R147E | $2.31 \times 10^6$ | 5 | hyperbolic |
| CB580 | D196K/R290E | D60K/R147E | $7.81 \times 10^4$ | 0 | hyperbolic |
| CB581 | D196R/R290D | D60R/R147D | $2.91 \times 10^6$ | 6 | hyperbolic |
| CB586 | D196R/K197E/K199E | K60cE/K60aE/D60R | $7.97 \times 10^6$ | 16 | hyperbolic |
| CB587 | D196K/K197E/K199E | K60cE/K60aE/D60K | $7.22 \times 10^6$ | 15 | hyperbolic |
| CB588 | D196R/K197E/ K199E/R290E | K60cE/K60aE/D60R/R147E | $3.66 \times 10^5$ | 1 | hyperbolic |
| CB589 | D196R/K197M/K199E | K60cE/K60aM/D60R | $1.81 \times 10^7$ | 37 | hyperbolic |
| CB590 | D196R/K197M/ K199E/R290E | K60cE/K60aM/D60R/R147E | $2.88 \times 10^5$ | 1 | hyperbolic |
| CB591 | L305V/S314E/L337A/F3 74Y | L163V/S170bE/K188A/F225 Y | $6.73 \times 10^7$ | 138 | hyperbolic |
| CB592 | M298Q | M156Q | $1.40 \times 10^8$ | 409 | linear |
| CB593 | V158D/E296V/M298Q | V21D/E154V/M156Q | $1.67 \times 10^8$ | 487 | linear |
| CB594 | V158D/E296V/M298Q/ K337A | V21D/E154V/M156Q/K188 A | $5.18 \times 10^8$ | 1061 | hyperbolic |
| CB595 | K197I | K60aI | $5.31 \times 10^7$ | 109 | hyperbolic |
| CB596 | K197V | K60aV | $5.15 \times 10^7$ | 106 | hyperbolic |
| CB597 | K197F | K60aF | $7.20 \times 10^7$ | 148 | hyperbolic |
| CB598 | K197W | K60aW | $6.25 \times 10^7$ | 128 | hyperbolic |
| CB599 | K197M | K60aM | $7.83 \times 10^7$ | 160 | hyperbolic |
| CB600 | D196F | D60F | $2.35 \times 10^7$ | 48 | hyperbolic |
| CB601 | D196Y | D60Y | $6.93 \times 10^7$ | 142 | hyperbolic |
| CB602 | D196W | D60W | $3.40 \times 10^7$ | 70 | hyperbolic |
| CB603 | D196L | D60L | $8.57 \times 10^7$ | 176 | hyperbolic |
| CB604 | D196I | D60I | $7.99 \times 10^7$ | 164 | hyperbolic |
| CB605 | G237W | G97W | $8.63 \times 10^7$ | 177 | hyperbolic |
| CB606 | G237T | G97T | $3.13 \times 10^7$ | 64 | hyperbolic |
| CB608 | G237V | G97V | $8.91 \times 10^7$ | 183 | hyperbolic |

(continued)

| ID | Mutation (mature FVII numbering) | Mutation (Chymotrypsin numbering) | $k_{cat}/K_M$ ($M^{-1}s^{-1}$) | $k_{cat}/K_M$ (% WT) | Assay Format |
|---|---|---|---|---|---|
| CB609 | K341Q | K192Q | $1.56 \times 10^7$ | 32 | hyperbolic |
| CB611 | K197L/D196L | K60aL/D60L | $4.39 \times 10^7$ | 90 | hyperbolic |
| CB612 | K197L/D196F | K60aL/D60F | $3.79 \times 10^6$ | 8 | hyperbolic |
| CB613 | K197L/D196M | K60aL/D60M | $1.86 \times 10^7$ | 38 | hyperbolic |
| CB614 | K197L/D196W | K60aL/D60W | $9.81 \times 10^6$ | 20 | hyperbolic |
| CB615 | K197E/D196F | K60aE/D60F | $1.87 \times 10^7$ | 38 | hyperbolic |
| CB616 | K197E/D196W | K60aE/D60W | $3.50 \times 10^7$ | 72 | hyperbolic |
| CB617 | K197E/D196V | K60aE/D60V | $6.50 \times 10^6$ | 13 | hyperbolic |
| CB637 | K197E/K341Q | K60aE/K192Q | $7.22 \times 10^6$ | 15 | hyperbolic |
| CB638 | K197L/K341Q | K60aL/K192Q | $6.66 \times 10^6$ | 14 | hyperbolic |
| CB669 | V158D/G237V/E296V/ M298Q | V21D/E154V/M156Q/G97V | $6.70 \times 10^7$ | 196 | linear |
| CB670 | G237V/K341Q | K192Q/G97V | $1.57 \times 10^7$ | 32 | hyperbolic |
| CB671 | K197V/G237V/K341Q | K60aE/K192Q/G97V | $7.05 \times 10^6$ | 21 | linear |
| CB688 | K197E/K199E | K60aE/K60cE | $9.85 \times 10^6$ | 20 | hyperbolic |
| CB689 | K197E/G237V | K60aE/G97V | $1.32 \times 10^7$ | 27 | hyperbolic |
| CB690 | K197E/G237V/M298Q | K60aE/G97V/M156Q | $3.19 \times 10^7$ | 93 | linear |
| CB691 | K197E/K199E/K341Q | K60aE/K60cE/K192Q | $3.89 \times 10^6$ | 8 | hyperbolic |
| CB692 | K197E/G237V/M298Q/ K341Q | K60aE/G97V/M156Q/K192Q | $6.42 \times 10^6$ | 13 | hyperbolic |
| CB693 | K197E/K199E/G237V/ M298Q/K341Q | K60aE/K60cE/G97V/M156Q /K192Q | $3.49 \times 10^6$ | 7 | hyperbolic |
| CB694 | K199E/K341Q | K60cE/K192Q | $7.64 \times 10^6$ | 16 | hyperbolic |
| CB695 | G237V/M298Q | G97V/M156Q | $4.30 \times 10^7$ | 125 | linear |
| CB696 | G237V/M298Q/K341Q | G97V/M156Q/K192Q | $4.25 \times 10^7$ | 124 | linear |
| CB697 | R290N | R 147N | $1.36 \times 10^7$ | 28 | hyperbolic |
| CB698 | R290Q | R147Q | $8.59 \times 10^6$ | 18 | hyperbolic |
| CB699 | R290K | R147K | $1.52 \times 10^7$ | 31 | hyperbolic |
| CB700 | R290M | R147M | $1.23 \times 10^7$ | 25 | hyperbolic |
| CB701 | R290V | R147V | $2.66 \times 10^6$ | 5 | hyperbolic |
| CB728 | Gla swap FIX | Gla swap FIX | $3.83 \times 10^7$ | 112 | linear |
| CB729 | Gla swap FX | Gla swap FX | $9.46 \times 10^6$ | 19 | hyperbolic |
| CB730 | Gla Swap Protein C | Gla Swap Prot C | $3.19 \times 10^6$ | 7 | hyperbolic |
| CB732 | Gla swap Thrombin | Gla swap Thrombin | $1.04 \times 10^7$ | 21 | hyperbolic |
| CB850 | M298Q/Gla Swap FIX | M156Q/Gla swap FIX | $7.51 \times 10^7$ | 219 | linear |
| CB733 | K341N | K192N | $2.35 \times 10^7$ | 69 | linear |
| CB734 | K341M | K192M | $8.35 \times 10^6$ | 17 | hyperbolic |
| CB735 | K341D | K192D | $1.44 \times 10^7$ | 42 | linear |

(continued)

| ID | Mutation (mature FVII numbering) | Mutation (Chymotrypsin numbering) | $k_{cat}/K_M$ ($M^{-1}s^{-1}$) | $k_{cat}/K_M$ (% WT) | Assay Format |
|---|---|---|---|---|---|
| CB854 | G237T238insS | G97T98insS | $1.32 \times 10^7$ | 38 | linear |
| CB855 | G237T238insV | G97T98insV | $1.23 \times 10^7$ | 36 | linear |
| CB856 | G237T238insAS | G97T98insAS | $1.12 \times 10^7$ | 33 | linear |
| CB858 | D196K197insK | D60K60ainsK | $3.03 \times 10^7$ | 88 | linear |
| CB859 | D196K197insR | D60K60ainsR | $4.81 \times 10^7$ | 140 | linear |
| CB860 | D196K197insY | D60K60ainsY | $3.93 \times 10^7$ | 115 | linear |
| CB866 | K197I198insA | K60aI60binsA | $4.11 \times 10^7$ | 120 | linear |
| CB902 | K197E/M298Q | K60aE/M156Q | $9.68 \times 10^7$ | 283 | linear |

**Table 17. Catalytic activity of FVIIa variants: TF-Dependent Indirect Assay with FVIIa polypeptides from BHK-21 cells**

| ID | Mutation (mature FVII numbering) | Mutation (Chymotrypsin numbering) | $k_{cat}/K_M$($M^{-1}s^{-1}$) | $k_{cat}/K_M$ (% WT) | Assay Format |
|---|---|---|---|---|---|
| CB553 | WT | WT | $5.42 \times 10^7$ | 100 | linear |
| CB592 | M298Q | M156Q | $9.34 \times 10^7$ | 172 | linear |
| CB593 | V158D/E296V/M298Q | V21D/E154V/M156Q | $2.04 \times 10^8$ | 376 | linear |
| CB728 | Gla swap FIX | Gla swap FIX | $8.70 \times 10^7$ | 160 | linear |
| CB735 | K341D | K192D | $1.02 \times 10^7$ | 19 | linear |
| CB853 | G237T238insA | G97T98insA | $1.60 \times 10^7$ | 30 | linear |
| CB854 | G237T238insS | G97T98insS | $1.67 \times 10^7$ | 31 | linear |
| CB855 | G237T238insV | G97T98insV | $1.66 \times 10^7$ | 31 | linear |
| CB856 | G237T238insAS | G97T98insAS | $1.68 \times 10^7$ | 31 | linear |
| CB857 | G237T238insSA | G97T98insSA | $1.83 \times 10^7$ | 34 | linear |
| CB858 | D196K197insK | D60K60ainsK | $3.55 \times 10^7$ | 65 | linear |
| CB859 | D196K197insR | D60K60ainsR | $7.24 \times 10^7$ | 133 | linear |
| CB862 | D196K197insA | D60K60ainsA | $4.31 \times 10^7$ | 79 | linear |
| CB863 | D196K197insM | D60K60ainsM | $3.61 \times 10^7$ | 67 | linear |
| CB864 | K197I198insE | K60aI60binsE | $2.80 \times 10^7$ | 52 | linear |
| CB865 | K197I198insY | K60aI60binsY | $4.27 \times 10^7$ | 79 | linear |
| CB867 | K197I198insS | K60aI60binsS | $6.35 \times 10^7$ | 117 | linear |
| CB945 | M298Q/K341D | M156Q/K192D | $1.04 \times 10^7$ | 19 | |

**Table 18. Catalytic activity of FVIIa variants: TF-Independent Indirect Assay**

| ID | Mutation (mature FVII numbering) | Mutation (Chymotrypsin numbering) | 293-F Cells | | BHK-21 Cells | |
|---|---|---|---|---|---|---|
| | | | $k_{cat}/K_M$ ($M^{-1}s^{-1}$) | $k_{cat}/K_M$ (% WT) | $k_{cat}/K_M$ ($M^{-1}s^{-1}$) | $k_{cat}/K_M$ (% WT) |
| CB553 | WT | WT | 22.60 | 100 | 1.58 | 100 |

(continued)

| ID | Mutation (mature FVII numbering) | Mutation (Chymotrypsin numbering) | 293-F Cells | | BHK-21 Cells | |
|---|---|---|---|---|---|---|
| | | | $k_{cat}/K_M$ ($M^{-1}s^{-1}$) | $k_{cat}/K_M$ (% WT) | $k_{cat}/K_M$ ($M^{-1}s^{-1}$) | $k_{cat}/K_M$ (% WT) |
| CB592 | M298Q | M156Q | 485.5 | 2029 | 310.25 | 1969 |
| CB593 | V158D/E296V/M29 8Q | V21D/E154V/M156Q | 5493.68 | 24313 | 3217.77 | 20423 |
| CB669 | V158D/G237V/E29 6V/M298Q | V21D/E154V/M156Q/ G97V | 2145.3 | 9494 | | |
| CB692 | K197E/G237V/M29 8Q/K341Q | K60aE/G97V/M156Q/ K192Q | 1 | 4 | | |
| CB695 | G237V/M298Q | G97V/M156Q | 24.35 | 108 | | |
| CB728 | Gla swap FIX | Gla swap FIX | 110 | 486 | 24 | 154 |
| CB850 | M298Q/Gla Swap FIX | M156Q/Gla swap FIX | 15.4 | 68 | | |
| CB902 | K197E/M298Q | K60aE/M156Q | 8.55 | 38 | | |

**Example 12**

**Determination of the Inhibition of FVIIa/TF or FVIIa by AT-III/heparin**

The potency of the interaction between the AT-III/heparin complex and FVIIa in the presence or absence of soluble tissue factor (sTF), i.e. TF-dependent or TF-independent, was assessed by measuring the level of inhibition of various concentrations of AT-III on the catalytic activity of FVIIa/sTF towards a substrate, Mesyl-FPR-ACC. The $K_{0.5}$ value was determined for each FVIIa variant tested, which corresponds to the molar concentration of AT-III that was required for 50% inhibition ($IC_{50}$) of FVIIa variant in a 30 minute assay at room temperature (~25°).

Two separate assays were prepared, one with TF and one without TF. A 2μM solution of AT-III/heparin (final 5 mM heparin) was prepared by mixing 26.4 μL of 151.7 μM AT-III (plasma purified human AT-III; Molecular Innovations) with 50 μL of 0.2 mM LMW heparin (CalBiochem), 400 μL or 5× assay buffer (100 mM Hepes, 750 mM NaCl, 25 mM $CaCl_2$, 0.05% BSA, 0.5% PEG 8000, pH 7.4) and 1.523 mL of reagent grade water. This solution was for use as the highest concentration in the TF-dependent assay. A solution containing 4 μM AT-III/heparin (final 5 mM heparin) was prepared for use in the TF-independent assay by mixing 52.8 μL of 151.7 μM AT-III (Molecular Innovations) with 50 μL of 0.2 mM LMW heparin (CalBiochem), 400 μL of 5 × assay buffer and 1.497 mL of reagent grade water. The AT-III/heparin solutions were incubated for 5-10 minutes at room temperature and then diluted twofold down in a 96 deep-well polypropylene plate with a final volume of 1 mL containing 5 μM heparin, resulting in dilutions of 2000, 1000, 500, 250, 125, 62.5, 31.25 and 0 nM, or 4000, 2000, 1000, 500, 250, 125, 62.5, and 0 nM. The FVIIa variants and wild-type FVIIa were diluted to 250 nM in 1 × assay buffer (20 mM Hepes, 150 mM NaCl, 5 mM $CaCl_2$, 0.01% BSA, 0.1% PEG 8000, pH 7.4). For the TF-dependent assay, 5 nM FVIIa/50 nM sTF complexes were formed by mixing 20 μL of FVIIa with 10 μL of 5 μM sTF (R&D Systems Human Coagulation Factor III: #2339-PA), 200 μL 5× assay buffer and 770 μL reagent grade water and incubating the solutions for 10-15 minutes at room temperature. For the TF-independent assay, 100 μL of FVIIa was mixed with 200 μL 5× assay buffer and 700 μL reagent grade water to produce 25 nM solutions of FVIIa. To start the assay, 25 μL of the FVIIa/TF or FVIIa alone solutions were separately mixed with 25 μL of each dilution of AT-III/heparin in wells of a 96-well black half area assay plate (Nunc). The final assay conditions for the TF-dependent assay were 2.5 nM FVIIa/25 nM sTF and AT-III/heparin concentrations ranging from 1000 nM to 0 nM. For the TF-independent assay, FVIIa concentrations were 12.5 nM FVIIa and AT-III/heparin concentrations ranged from 2000 nM to 0 nM. The plates were incubated for 30 minutes with shaking at room temperature (~25°C).

A stock solution of FVIIa substrate (Mesyl-FPR-ACC) was prepared by dissolving the substrate in DMSO to 20 mM then preparing a working solution of 0.5 mM in 1 × assay buffer. Following incubation of the assay plate from above, 50 μl of the FVIIa substrate was added to each well of the assay plate. The reactions were mixed and the residual activity of FVIIa was assessed by following the initial rates of substrate cleavage for 15 minutes in a fluorescence reader set to 30°C.

To determine the degree of inhibition by AT-III/heparin for FVIIa or FVIIa variants, raw data collected with the SoftMax Pro application (Molecular Devices) were exported as .XML files. Further non-linear data analyses were performed with XLfit4, a software package for automated curve fitting and statistical analysis within the Microsoft Excel spreadsheet environment (IDBS Software). The spreadsheet template was used to calculate the AT-III dilution series, ratio of AT-III to FVIIa, and the Vi/Vo ratios for each FVIIa replicate at each experimental AT-III concentration. Non-linear regression analyses of residual FVIIa activity (expressed as Vi/Vo) versus AT-III concentration was processed using XLfit4 and a hyperbolic inhibition equation of the form ((C+(Amp*(1-(X/($K_{0.5}$+X))))); where C = the offset (fixed at 0 to permit extrapolation of data sets that do not reach 100% inhibition during the course of the assay), Amp = the amplitude of the fit and $K_{0.5}$, which corresponds to the concentration of AT-III required for half-maximal inhibition under the assay conditions. For several FVIIa variants, AT-III inhibited less than 20-25% of the of the total protease activity at the highest tested concentration of AT-III, representing an upper limit of detection for the assay. Variants with less than 20-25% maximal inhibition were therefore assigned a lower limit $K_{0.5}$ value (5 μM for TF-dependent and 10 μM for TF-independent) and in most cases are expected to have AT-III resistances greater than the reported value.

Tables 19 and 20 provide the results of the assays that were performed using FVIIa variants expressed in Freestyle TM 293-F cells and/or BHK-21 cells, in the presence and absence of TF, respectively. The results are presented both as the fitted $K_{0.5}$ parameter and as a representation of the extent of AT-III resistance for each variant compared to the wild-type FVIIa expressed as a ratio of their fitted $K_{0.5}$ values ($K_{0.5}$ variant/ $K_{0.5}$ wild-type). Several FVIIa variants exhibited increased resistance to AT-III compared to wild-type FVIIa.

**Table 19. Inhibition of FVIIa variants by AT-III/heparin in the presence of TF**

| FVIIa | Mutation (mature FVII numbering) | Mutation (Chymotrypsin Numbering) | TF-Dependent ATIII Resistance Assay | | | |
|---|---|---|---|---|---|---|
| | | | 293-F Cells | | BHK-21 Cells | |
| | | | $K_{0.5}$ (nM) | $K_{0.5mut}/K_{0.5wt}$ | $K_{0.5}$ (nM) | $K_{0.5mut}/K_{0.5wt}$ |
| CB553 | WT | WT | 72.3 | 1.0 | 56.0 | 1.0 |
| CB593 | V158D/E296V/M298Q | V21D/E154V/M156Q | 75.1 | 1.0 | 79.0 | 1.4 |
| CB609 | K341Q | K192Q | 104.5 | 1.4 | | |
| CB733 | K341N | K192N | 41.2 | 0.6 | | |
| CB734 | K341M | K192M | 78.2 | 1.1 | | |
| CB735 | K341D | K192D | 1985.8 | 27.5 | | |
| CB853 | G237T238insA | G97T98insA | | | 169.5 | 3.0 |
| CB854 | G237T238insS | G97T98insS | | | 163.9 | 2.9 |
| CB855 | G237T238insV | G97T98insV | 189.6 | 2.6 | | |
| CB856 | G237T238insAS | G97T98insAS | | | 391.4 | 7.0 |
| CB857 | G237T238insSA | G97T98insSA | | | 266.9 | 4.8 |
| CB858 | D196K197insK | D60K60ainsK | | | 64.9 | 1.2 |
| CB859 | D196K197insR | D60K60ainsR | | | 34.0 | 0.6 |
| CB860 | D196K197insY | D60K60ainsY | 29.5 | 0.4 | | |
| CB862 | D196K197insA | D60K60ainsA | | | 60.7 | 1.1 |
| CB863 | D196K197insM | D60K60ainsM | | | 55.9 | 1.0 |
| CB864 | K197I198insE | K60a160binsE | | | 183.7 | 3.3 |
| CB865 | K197I198insY | K60a160binsY | | | 66.5 | 1.2 |
| CB867 | K197I198insS | K60a160binsS | | | 82.7 | 1.5 |
| CB728 | Gla swap FIX | Gla swap FIX | 68.8 | 1.0 | | |

**Table 20. Inhibition of FVIIa variants by AT-III/heparin in the absence of TF**

| FVIIa | Mutation (mature FVII numbering) | Mutation (Chymotrypsin Numbering) | TF-Independent ATIII Resistance Assay | | | |
|---|---|---|---|---|---|---|
| | | | 293-F Cells | | BHK-21 Cells | |
| | | | $K_{0.5}$ (nM) | $K_{0.5mut}/K_{0.5wt}$ | $K_{0.5}$ (nM) | $K_{0.5mut}/K_{0.5wt}$ |
| CB553 | WT | WT | 2265.8 | 1.0 | 2222.7 | 1.0 |
| CB593 | V158D/E296V/M298Q | V21D/E154V/M156Q | 389.7 | 0.2 | 415.6 | 0.2 |
| CB609 | K341Q | K192Q | 4622.6 | 2.0 | | |
| | | | | | | |
| CB733 | K341N | K192N | 1554.4 | 0.7 | | |
| CB734 | K341M | K192M | 5523.9 | 2.4 | | |
| CB735 | K341D | K192D | 10000.0 | >4.4 | | |
| CB853 | G237T238insA | G97T98insA | | | 10000.0 | >4.5 |
| CB854 | G237T238insS | G97T98insS | | | 10000.0 | >4.5 |
| CB855 | G237T238insV | G97T98insV | 10000.0 | >4.4 | | |
| CB856 | G237T238insAS | G97T98insAS | | | 10000.0 | >4.5 |
| CB857 | G237T238insSA | G97T98insSA | | | 10000.0 | >4.5 |
| CB858 | D196K197insK | D60K60ainsK | | | 9329.6 | 4.2 |
| CB859 | D196K197insR | D60K60ainsR | | | 4322.6 | 1.9 |
| CB860 | D196K197insY | D60K60ainsY | 2053.3 | 0.9 | | |
| CB862 | D196K197insA | D60K60ainsA | | | 7414.3 | 3.3 |
| CB863 | D196K197insM | D60K60ainsM | | | 7299.4 | 3.3 |
| CB864 | K197I198insE | K60aI60binsE | | | 10000.0 | >4.5 |
| CB865 | K197I198insY | K60aI60binsY | | | 8800.4 | 4.0 |
| CB867 | K197I198insS | K60aI60binsS | | | 10000.0 | >4.5 |
| CB728 | Gla swap FIX | Gla swap FIX | 1005.2 | 0.4 | | |

**Example 13**

**Determination of the resistance to TFPI of FVIIa variants**

The resistance of various FVIIa variants to TFPI was assessed using the assay described in Example 7, above. Table 21 provides the results of the assays. The results are expressed as the fold resistance of each FVIIa variant to TFPI compared to wild-type FVIIa.

**Table 21. Inhibition of FVIIa variants by TFPI**

| ID | Mutation (mature FVII numbering) | Mutation (chymotrypsin numbering) | TFPI fold resistance |
|---|---|---|---|
| CB553 | wt | wt | 1.0 |
| CB554 | D196K | D60K | 0.6 |
| CB555 | D196R | D60R | 0.5 |
| CB556 | D196A | D60A | <0.3 |
| CB557 | K197D | K60aD | 3.0 |

(continued)

| ID | Mutation (mature FVII numbering) | Mutation (chymotrypsin numbering) | TFPI fold resistance |
|---|---|---|---|
| CB558 | K197E | K60aE | 1.3 |
| CB559 | K197A | K60aA | 0.7 |
| CB560 | K197L | K60aL | 16.0 |
| CB561 | K197Y | K60aY | 1.5 |
| CB562 | K199D | K60cD | 0.9 |
| CB563 | K199E | K60cE | 1.3 |
| CB564 | K199A | K60cA | 0.9 |
| CB565 | T239A | T99A | 4.5 |
| CB566 | R290D | R147D | 3.3 |
| CB567 | R290E | R147E | 6.1 |
| CB568 | R290A | R147A | 2.8 |
| CB569 | K341R | K192R | 4.1 |
| CB579 | D196R/R290E | D60R/R147E | 0.4 |
| CB580 | D196K/R290E | D60K/R147E | 0.7 |
| CB581 | D196R/R290D | D60R/R147D | 0.4 |
| CB586 | D196R/K197E/K199E | D60R/K60aE/K60cE | <0.3 |
| CB587 | D196K/K197E/K199E | D60K/K60aE/K60cE | 0.3 |
| CB588 | D196R/K197E/ K199E/R290E | D60R/K60aE/K60cE/R147E | 0.4 |
| CB589 | D196R/K197M/K199E | D60R/K60aM/K60cE | 0.6 |
| CB590 | D196R/K197M/K199E/R290E | D60R/K60aM/K60cE/R147E | 0.9 |
| CB591 | L305V/S314E/L337A/F374Y | L163V/S170bE/K188A/F225Y | 0.6 |
| CB592 | M298Q | M156Q | 0.5 |
| CB593 | V158D/E296V/M298Q | V21D/E154V/M156Q | 0.8 |
| CB594 | V158D/E296V/M298Q/K337A | V21D/E154V/M156Q/K188A | 0.3 |
| CB595 | K197I | K60aI | 0.8 |
| CB596 | K197V | K60aV | 0.6 |
| CB597 | K197F | K60aF | <0.3 |
| CB598 | K197W | K60aW | <0.3 |
| CB599 | K197M | K60aM | 0.3 |
| CB600 | D196F | D60F | <0.3 |
| CB601 | D196Y | D60Y | <0.3 |
| CB602 | D196W | D60W | <0.3 |
| CB603 | D196L | D60L | <0.3 |
| CB604 | D196I | D60I | <0.3 |
| CB605 | G237W | G97W | <0.3 |
| CB606 | G237T | G97T | 0.3 |
| CB608 | G237V | G97V | 0.8 |
| CB609 | K341Q | K192Q | 12.2 |

(continued)

| ID | Mutation (mature FVII numbering) | Mutation (chymotrypsin numbering) | TFPI fold resistance |
|---|---|---|---|
| CB611 | K197L/D196L | K60aL/D60L | <0.3 |
| CB612 | K197L/D196F | K60aL/D60F | <0.3 |
| CB614 | K197L/D196W | K60aL/D60W | <0.3 |
| CB615 | K197F/D196F | K60aE/D60F | <0.3 |
| CB617 | K197E/D196V | K60aE/D60V | <0.3 |
| CB637 | K197E/K341Q | K60aE/K192Q | 80.7 |
| CB638 | K197L/K341Q | K60aL/K192Q | 39.0 |
| CB670 | G237V/K341Q | G97V1K192Q | 14.1 |
| CB671 | K197V/G237V/K341Q | K60aE/G97V/K192Q | 18.9 |
| CB688 | K197E/K199E | K60aE/K60cE | 2.1 |
| CB689 | K197F/G237V | K6DaE/G97V | 1.7 |
| CB691 | K197E/K199E/K341Q | K60aE/K60cE/K192Q | 34.3 |
| CB692 | K197E/G237V/M298Q/K341Q | K60aE/G97V/M156Q/K192Q | 15.2 |
| CB693 | K197E/K199E/G237V/M298Q/ K341Q | K60aE/K60cE/G97V/M156Q/ K192Q | 22.1 |
| CB694 | K199E/K341Q | K60cE/K192Q | 19.1 |
| CB696 | G237V/M298Q/K341Q | G97V/M156Q/K192Q | 3.2 |
| CB697 | R290N | R147N | 1.5 |
| CB698 | R290Q | R147Q | 1.2 |
| CB699 | R290K | R147K | 0.6 |
| CB700 | R290M | R147M | 1.0 |
| CB701 | R290V | R147V | 2.5 |
| CB733 | K341N | K192N | 10.7 |
| CB734 | K341M | K192M | 4.4 |
| CB735 | K341D | K192D | 148.0 |

## Example 14

### *In vivo* assessment of FVIIa polypeptide procoagulant activity

Mouse model of hemophilia A was established to assess the procoagulant activity of FVIIa polypeptides. Hemophilia A was induced in CD-1 mice by administration of anti-FVIII antibodies, followed by surgical removal of the tips of the tails to initiate bleeding (similar to that described above, in Example 8). Mice deficient in FVIII (FVIII$^{-/-}$ mice) also were used, but were not treated with anti-FVIII antibodies. The mice were then treated with FVIIa polypeptide and the amount of blood lost in 20 minutes was measured to determine the procoagulant activity of the FVIIa polypeptides.

### A. Analysis of FVIIa coagulant activity in CD-1 mice with induced hemophilia A

Initial experiments were carried out to determine the dose required and time and duration of effect of anti-human-FVIII antibodies when given by the intraperitoneal route to induce hemophilia in CD-1 mice. For the first lot of anti-FVIII (lot 1; Affinity Biologicals, lot IG129R4), this was based initially on the dose used for the cannulation experiments, described above in Example 8. The dose determined to cause a hemophilic state (uncontrolled bleeding over a 20 minute assay period) was 7.54 mg/mouse (80 μl of a 94.25 mg/ml stock solution). This lot had a neutralizing activity of 612 mouse BU/ml. For the second lot of anti-human FVIII (lot 2; Affinity Biologicals, lot IG1577R2, neutralizing activity of 474 mouse BU/ml) the dose used was 11.98 mg/mouse (120 μl of a 99.8 mg/ml stock solution) and was administered at 6 hours prior to tail cut.

To induce hemophilia, male CD-1 mice (25-35 g) were dosed intraperitoneally with lot 1 or lot 2 of anti-FVIII prior to the experiment. Male CD-1 and $FVIII^{-/-}$ mice were anesthetized by intraperitoneal administration of a ketamine/xylazine cocktail(100 mg/mL solution) and placed on a heated platform (39°C) to ensure there was no drop in body temperature. The procedure room was kept at a temperature of 82°F. Ten minutes prior to tail cut the tail was immersed in 10 mls of pre-warmed PBS (15ml centrifuge tube; 39°C). Eight to ten mice were injected with recombinant human FVIIa (Novoseven®, Novo Nordisk) or modified FVII polypeptides diluted in a buffer composed of 52 mM sodium chloride, 10.2 mM calcium chloride dehydrate, 9.84 mM glycylglycine, 0.01% polysorbate 80 and 165 mM mannitol via the tail vein in a single injection. Vehicle only also was injected into a group of mice as a control. If the injection was missed, the animal was excluded from the study. Injection with test agent or vehicle was made 5 minutes prior to tail cut. A tail cut was made using a razor blade 5mm from the end of the tail and blood was collected into PBS for a period of 20 minutes. At the end of the collection period, total blood loss was assessed. The collection tubes were mixed and a 1 ml aliquot of each sample was taken and assayed for hemoglobin content. Triton X-100 was diluted 1 in 4 in sterile water and 100 μL was added to the 1mL samples to cause hemolysis. The absorbance of the samples was then measured at a wavelength of 546 nm. To calculate the amount of blood lost, the absorbance was read against a standard curve generated by measuring the absorbance at 546 nm of known volumes of murine blood, diluted in PBS and hemolysed as above with Triton X 100.

A dose response study in which 0.3, 1 or 3 mg/kg of CB553 (wild-type FVIIa) was assessed also was performed. Mice that received the vehicle lost 1002.3 ± 60.71 μL in the 20 minute assay. This was reduced significantly in mice that were administered 3 mg/kg of of CB553, to 415.5 ± 90.85 μL ($p < 0.05$ using Kruskal-Wallis followed by Dunn's post test). Reducing the dose to 1 mg/kg resulted in blood loss of 679.57 ± 83.95 μL and a lower dose of 0.3 mg/kg resulted in blood loss of 852.42 ± 94.46 μL.

In a separate study, CB735 (K341D) and CB945 (M298Q/K341D) was assessed at a dose of 3 mg/kg. The vehicle only injection was used as a control. Groups of mice that received the vehicle only lost between 803 ± 92.18 μL and 813.1 ± 82.66 μL of blood in the 20 minute assay. This was similar to treatment with CB735 or CB945, which resulted in 746 ± 110.5μL and 870.9 ± 78.38 μL blood loss, respectively..

### B. Analysis of FVIIa coagulant activity in $FVIII^{-/-}$ mice

A mouse model of hemophilia A using mice deficient in FVIII ($FVIII^{-/-}$ mice) also was used to asses the coagulant activity of FVIIa polypeptides, suying the same protocols as described above except that the mice were not treated with anti-FVIII antibodies.

#### 1. Dose response study assessing wild-type FVIIa coagulant activity

Dose response studies to assess the coagulant activity of NovoSeven® and CB553 in $FVIII^{-/-}$ mcie at 0.3, 1, 3 and 6 mg/kg were performed. In the NovoSeven® experiment, the blood loss in the vehicle group was 912.79 ± 38.32μL, which was significantly reduced by NovoSeven® treatment at 6 and 3 mg/kg (to 361.74 ± 55.28 μL and 586.98 ± 60.56 μL; $p < 0.05$ using Kruskal-Wallis followed by Dunn's post test). Reducing the dose to 1 mg/kg resulted in blood loss of 674.84 ± 46.88 μL and at the lowest dose tested the value was 801.08 ± 41.39 μL. In the CB553 experiment, the vehicle control group produced blood loss of 904.08 ± 15.38μL. This was reduced significantly ($p < 0.05$ using Kruskal-Wallis followed by Dunn's post test) by CB553 at 6 mg/kg to 451.04 ± 74.17 μL. Reducing the dose to 3 mg/kg produced a blood loss value of 695.75 ± 60.50 μL while lowering the dose further to 1 and 0.3 mg/kg resulted in blood loss values near and at vehicle control levels (846.08 ± 34.17 μL and 936.43 ±31.39 μL, respectively).

## Example 15

### Determination of Factor VIIa binding to Soluble Tissue Factor

The ability of the FVIIa variants expressed from HEK 293 or BHK cells to bind soluble tissue factor (sTF) was assessed using Biacore surface plasmon resonance. The FVIIa variants are assessed through measurement of the binding profile at three protease concentrations in two duplicate experiments, using two different levels of sTF bound to a Biacore CM5 chip.

A new Series S CM5 sensor chip (GE Healthcare Cat #BR1006-68) was coupled with bovine serum albumin and soluble tissue factor using a Biacore T100 instrument. Coupling was effected using Biacore Coupling Buffer (30 mM Na Hepes pH 7.4, 135 mM NaCl, 1 mM EDTA, 0.01% Tween-20) with an Amine coupling kit (GE Healthcare Cat # BR-1000-50) and the protocol wizard in the Biacore T100 software. For the immobilization, all four cells of the chip were used. Cells 1 and 3 were be coupled with 500 response units (RU) bovine serum albumin reference protein diluted in Acetate buffer, pH 4.0 and cells 2 and 4 were coupled with 500 and 250 RU of sTF (R&D Systems) diluted in Acetate buffer, pH 4.5.

Each FVIIa variant, and the wild-type FVIIa protease, was tested at three concentrations and in duplicate. The proteases were diluted to 60 nM, 30 nM and 15 nM in 100 μL Biacore Assay buffer (200 mM Na Hepes, pH 7.4, 150. mM NaCl, 5 mM $CaCl_2$, 0.1% PEG 8000, 0.1% BSA, 0.01% Tween-20) in a 96 well assay plate. Assay Each sample was assayed in the Biacore T100 instrument using 120 seconds of contact time followed by 180 seconds of dissociation time at a 10 μL/min flow rate. A buffer blank also was assayed. The chip was regenerated with 50 mM EDTA, pH 7.0 for 60 seconds then 30 seconds. The assay to measure binding of wild-type FVIIa to sTF should yield three sets of curves that give a $K_d$ of approximately 8 nM.

Biacore T100 Evaluation software was used to analyze the data. Specifically, the Kinetics/Affnity 1:1 Binding analysis, which fits the data to the Langmuir isotherm, was utilized and the data was individually fit for two replicates of each variant at two response unit couplings. The four fit $K_d$ values were averaged and are presented in Table 22. FVIIa polypeptides containing the M156Q mutation tended to have lower $K_d$ results and thus bind more tightly to sTF.

**Table 22. Binding of FVIIa variants to soluble TF**

| ID | Mutation (mature FVII numbering) | Mutation (chymotrypsin numbering) | Affinity Kd (nM) | |
|---|---|---|---|---|
| | | | HEK 293 | BHK |
| CB553 | WT | WT | 7.9 | 9.0 |
| CB592 | M298Q | M156Q | 3.9 | |
| CB593 | V158D/E296V/M298Q | V21D/E154V/M156Q | 8.0 | |
| CB669 | Q158D/G237V/E296V/M298Q | V21D/E154V/M156Q/G97V | 14.9 | |
| CB690 | K197E/G237V/M298Q | K60aE/G97V/M156Q | 4.8 | |
| CB691 | K197E/K199E/K341Q | K60aE/K60cE/K192Q | 6.6 | |
| CB692 | K197E/G237V/M298Q/K341 Q | K60aE/G97V/M156Q/K192Q | 5.9 | |
| CB693 | K197E/K199E/G237V/M298 Q/K341Q | K60aE/K60cE/G97V/M156Q /K192Q | 6.4 | |
| CB695 | G237V/M298Q | G97V/M156Q | 3.8 | |
| CB696 | G237V/M298Q/K341Q | G97V/M156Q/K192Q | 2.5 | |
| CB946 | M298Q/K341D | M156Q/K192D | 7.9 | |

**Example 16**

**Surface plasmon resonance (SPR) screening of FVIIa variants for resistance to TFPI**

The relative resistance of various FVIIa variants to inhibition by human recombinant soluble TFPI was evaluated using a high-throughput surface plasmon resonance (SPR) assay with the Biacore T100 instrument. The relative resistance of FVIIa variants to inhibition by TFPI was assessed by measurement of the relative amount of FVIIa variant bound to soluble TFPI immobilized on a Biacore CM5 sensor chip compared to the amount of wild-type FVIIa bound subsequent to a standardized injection time and protease concentration.

For every experiment, soluble TFPI (R&D Systems) was immobilized to a new 4-flow cell Biacore CM5 Series S sensor chip (GE Healthcare) using the amine coupling protocol available within the Biacore T-100 control Software (GE Healthcare) and the reagents provided with the Amine Coupling Kit (GE Healthcare). All four available flow cells were utilized for immobilization of two different densities of TFPI and bovine serum albumin (BSA), which served as a blocking agent in the reference cells. BSA was diluted to 5 μg/mL in sodium acetate (pH 4.0) and immobilized in flow-cells 1 and 3 at 1000 and 2000 response units (RU), respectively. For TFPI immobilization, lyophilized soluble TFPI (10 μg) was resuspended in 100μ L of 1X Coupling Buffer (30 mM Hepes, 135 mM NaCl, 1 mM EDTA, 0.01% Tween-20, pH 7.4) to a concentration of 0.1 mg/mL. A total of 20 μL of 0.1 mg/mL TFPI as diluted to 10 μg/mL in sodium acetate pH 4.0 for immobilization to flow-cells 2 and 4 at 1000 and 2000 RU, respectively. Coupling buffer was used as the running buffer during the immobilization steps.

Each sample of FVIIa was prepared at a final concentration of 320 nM in 1X Running Buffer (20 mM Hepes, 150 mM NaCl, 5 mM CaCl2, 0.1% PEG 8000, 0.1% BSA, 0.01% Tween-20, pH 7.4) containing 620 nM sTF (Human Coagulation Factor III; R&D Systems). Generally, each **FVIIa** variant was diluted 10-fold into 1X Running Buffer before the final dilution of 320 nM. FVIIa/sTF complexes were prepared at a final volume of 120 μL in duplicate allowing for up to 48 unique FVIIa variants to be loaded into a 96-well storage plate and evaluated with duplicate injections in a single run. The FVIIa/sTF complex was incubated at RT for 10-15 min before initiation of the first sample injection.

A standardized binding analysis method was created within the Biacore Control Software (GE Healthcare) in which every FVIIa replicate is injected for 180 seconds of association time followed by a short 60 seconds of dissociation at a flow rate of 10 μL/min. Regeneration of the sensor chip followed the dissociation phase for 30 seconds with 10 mM glycine, 500 mM NaCl, pH 3.0 and then a 60 second stabilization period with 1X Running Buffer at the same 10 μL/min flow rate. Two assay reference points were recorded for each run and subsequent data analysis, one 5 seconds prior to the conclusion of the association phase (binding) and a second reported 5 seconds before the conclusion of the dissociation phase (dissociation). Before initiating a full assay, the sensor chip was tested with a single injection of 320 nM wild-type FVIIa/sTF for 180 seconds, which should give a response of approximately 400-450 RU and 750-850 RU for binding to flow-cells 2 (1000 RU) and 4 (200 RU), respectively.

Data analysis was performed first with the Biacore T100 Evaluation Software (GE Healthcare) to inspect the assay validation parameters, which include verifying that binding to the reference cell is minimal, baseline drift and the binding of control blank injections (running buffer). Data tables were generated within this application that indicated the amount of FVIIa variant bound (in RU) at both the binding report point and the dissociation report point. The data tables were subsequently exported for further analysis within the Microsoft Excel spreadsheet environment. The raw data points (RU bound) were corrected for control binding to the sensor chip and then a ratio of the amount of wild-type FVIIa bound (in RU) to the amount of FVIIa variant bound (in RU) was taken for each parameter and reported as Binding (wt/variant) and Dissociation (wt/variant), Resistance to TFPI inhibition is reflected as an increase in the ratio for one or both of the evaluated parameters. For instance, a Binding (wt/variant) or Dissociation (wt/variarit) value of 20 for a particular FVIIa variant indicates that that variant is 20-fold more resistant to TFPI inhibition than wild-type FVIIa. Several variants exhibited increased resistance to TFPI inhibition. For example, CB609, CB637, CB691, CB856 and CB857 are among the group that exhibited 20 to 60-fold resistance and variants containing the K341D by mature FVII numbering (corresponding to by chymotrypsin numbering) such as CB735, have ratios indicating significant resistance to TFPI (greater than 50 -150-fold) and a variants containing the K192D mutation 9CB945) has a ratio indicating significant resistance to TFPI (greater than 40 -150-fold). In some cases, the rate of dissociation was affected more than the rate of association. Some examples of variants that exhibited this profile are CB735, CB854, CB855, CB856 and CB857.

**Table 23. Resistance of FVIIa variants to inhibition by TFPI**

| ID | Mutation (mature FVII Numbering) | Mutation (Chymotrypsin Numbering) | TF-Dependent TFPI Resistance Assay | | | |
|---|---|---|---|---|---|---|
| | | | 293-F Cells | | BHK-21 Cells | |
| | | | Binding (wt/ variant) | Dissociation (wt/ variant) | Binding (wt/ variant) | Dissociation (wt/ variant) |
| CB553 | WT | WT | 1.0 | 1.0 | 1.0 | 1.0 |
| CB558 | K197A | K60aE | 1.4 | 1.3 | | |
| CB563 | K197E | K60cE | 1.4 | 1.4 | | |
| CB592 | M298Q | M156Q | 1.9 | 1.8 | | |
| CB593 | V158D/E296V/M298Q | V21D/E154V/M156Q | 0.8 | 0.8 | 1.0 | 1.0 |
| CB608 | G237V | G97V | 2.2 | 1.8 | | |
| CB609 | K341Q | K192Q | 13.0 | 19.7 | | |
| CB637 | K197E/K341Q | K60aE/K192Q | 67.2 | 171.1 | | |
| CB670 | G237V/K341Q | K192Q/G97V | 12.8 | 12.4 | | |
| CB671 | K197V/G237V/K341Q | K60aE/K192Q/G97V | 18.9 | 21.8 | | |
| CB688 | K197E/K199E | K60aE/K60cE | 2.2 | 2.1 | | |
| CB689 | K197E/G237V | K60aE/G97V | 1.8 | 1.6 | | |
| CB691 | K197E/K199E/K341Q | K60aE/K60cE/K192Q | 33.9 | 68.1 | | |
| CB692 | K197E/G237V/M298Q/K34 1Q | K60aE/G97V/M 156Q/K192Q | 15.9 | 21.5 | | |
| CB693 | K197E/K199E/G237V/M298 0/k341Q | K60aE/K60cF/G97V/M156Q /K192Q | 23.4 | 43.5 | | |
| CB694 | K199E/K341Q | K6OcE/K192Q | 18.8 | 30.7 | | |
| CB695 | G237V/M298Q | G97V/M156Q | 0.9 | 0.8 | | |
| CB696 | G237V/M298Q/K341Q | G97V/M156Q/K192Q | 4.5 | 6.0 | | |
| CB697 | R290N | R147N | 1.6 | 1.5 | | |
| CB698 | R290Q | R147Q | 1.3 | 1.2 | | |
| CB699 | R290K | R147K | 0.7 | 0.7 | | |
| CB700 | R290M | R147M | 1.1 | 1.1 | | |

(continued)

| ID | Mutation (mature FVII Numbering) | Mutation (Chymotrypsin Numbering) | TF-Dependent TFPI Resistance Assay | | | |
|---|---|---|---|---|---|---|
| | | | 293-F Cells | | BHK-21 Cells | |
| | | | Binding (wt/ variant) | Dissociation (wt/ variant) | Binding (wt/ variant) | Dissociation (wt/ variant) |
| CB701 | R290V | R147V | 2.6 | 2.7 | | |
| CB733 | K341N | K192N | 11.3 | 63.8 | | |
| CB134 | K341M | K192M | 4.9 | 5.4 | | |
| CB735 | K341D | K192D | 185.6 | 380.7 | | |
| CB853 | G237T238insA | G97T98insA | | | 6.5 | 18.2 |
| CB854 | G23TIZ38insS | G97T98insS | | | 5.2 | 13.0 |
| CB855 | G237T238insV | G97T98insV | | | 7.3 | 16.8 |
| CB856 | G237T238insAS | G97T98insAS | | | 22.8 | 154.5 |
| CB857 | G237T238insSA | G97T98insSA | | | 21.5 | 146.5 |
| CB858 | D196K197insK | D60K60ainsK | | | 1.0 | 0.8 |
| CB859 | D196K197insR | D60K60ainsR | | | 0.5 | 0.5 |
| CB862 | D196K197insA | D60K60ainsA | | | 0.5 | 0.4 |
| CB863 | 0196K197insM | D60K60ainsM | | | 0.4 | 0.4 |
| CB864 | K1971198insE | K60a160binsE | | | 1.3 | 1.1 |
| CB965 | K1971198insY | K60a160binsY | | | 1.1 | 1.0 |
| CB867 | K1971198insS | K60a160binsS | | | 0.7 | 0.6 |
| CB902 | K197E/M298Q | K60aE/M156Q | 2.6 | 2.3 | | |
| CB945 | M298Q/K341D | M156Q/K192D | 146.8 | 196.5 | | |

**Example 17**

**Determination of the Concentration of Catalytically Active Protease using the Active Site Titrant 4-methylumbelliferyl *p*'-guanidinobenzoate (MUGB)**

The concentration of catalytically active FVIIa in a stock solution was determined by titrating a complex FVIIa and soluble tissue factor (sTF) with 4-methylumbelliferyl *p*'-guanidinobenzoate (MUGB), a fluorogenic ester substrate developed as an active site for trypsin-like serine proteases. The assay was carried out essentially as described by Payne et al. (Biochemistry (1996) 35:7100-7106) with a few minor modifications. MUGB readily reacts with FVIIa, but not FVII or inactive protease, to form an effectively stable acyl-enzyme intermediate under conditions in which the concentration of MUGB is saturating and deacylation is especially slow and rate limiting for catalysis. Under these conditions, the FVIIa protease undergoes a single catalytic turnover to release the 4-methylumbelliferone fluorophore (4-MU). When the initial burst of fluorescence is calibrated to an external concentration standard curve of 4-MU fluorescence, the concentration of active sites may be calculated.

Assays were performed with a 2 mL reaction volume in a 1 cm x 1 cm quartz cuvette under continuous stirring. Each reaction contained 0.5 μM sTF (R&D Systems Human) in an assay buffer containing 50 mM Hepes, 100 mM NaCl, 5 mM $CaCl_2$ and 0.1% PEG 8000, PH 7.6. The 4-MU standard solution was freshly prepared at a stock concentration of 0.5 M in DMSO and the concentration confirmed by absorbance spectroscopy at 360 nm using an extinction coefficient of 19,000 M-1 cm-1 in 50 mM Tris buffer, pH 9.0. MUGB was prepared at a stock concentration of 0.04 M in DMSO based on the dry weight. Assays were initiated by adding 4 μL of 4 mM MUGB (8 μM final concentration) to a solution of 0.5 μM sTF (20.2 μL of 49.4 μM sTF) in 1 × assay buffer and first measuring the background hydrolysis of MUGB for ~150-200 seconds before the addition of FVIIa or FVIIa variant to a final concentration of ~100-200 nM based on the initial ELISA (Example 2C. 1) or the active site titration with FFR-CMK (Example 6). The release of 4-MU fluorescence in the burst phase of the reaction was followed for an additional 1000-1200 seconds. A standard curve of free 4-MU was prepared by titration of the absorbance-calibrated 4-MU into 1 × assay buffer containing 0.5 μM sTF in 20 nM steps in to a final concentration of 250 nM.

For data analysis, reaction traces were imported into the Graphpad Prism software package and the contribution of background hydrolysis was subtracted from the curve by extrapolation of the initial measured rate of spontaneous MUGB hydrolysis, which was typically less than 5% of the total fluorescence burst. The corrected curve was fit to a single exponential equation with a linear component (to account for the slow rate of deacylation) of the form $\Delta$Fluorescence = $Amp(1-e^{-kt})+Bt$, where Amp = the amplitude of the burst phase under the saturating assay conditions outline above, *k* is the observed first order rate constant for acyl-enzyme formation and B is a bulk rate constant associated with complete turnover of MUGB. The concentration of active FVIIa protease is calculated by comparison of the fit parameter for amplitude to the 4-MU standard curve. The values from multiple assays were measured, averaged and the standard deviation determined.

Since modifications will be apparent to those of skill in this art, it is intended that this invention be limited only by the scope of the appended claims.

## Claims

1. A modified factor VII (FVII) polypeptide, comprising:

a heterologous Gla domain, or a sufficient portion thereof to effect phospholipid binding, whereby the modified FVII polypeptide exhibits increased phospholipid affinity or binding compared with an unmodified FVII polypeptide that does not contain the heterologous Gla domain, wherein:

the sufficient portion contains 30 or more contiguous amino acids from the heterologous Gla domain; and the heterologous Gla domain is selected from among a Gla domain in Factor IX (FIX), Factor X (FX), prothrombin, protein C, protein S, osteocalcin, Growth-arrest-specific protein 6 (Gas6) and protein Z; and

one or more further amino acid modification(s) that increases resistance to antithrombin-III (AT-III), increases affinity for tissue factor (TF), increases intrinsic activity, increases TF-dependent catalytic or coagulant activity, increases coagulant activity, alters the conformation of the polypeptide to alter zymogenicity, increases catalytic or coagulant activity by shifting the equilibrium between highly active and less active FVIIa conformations in favor of the highly active conformations, increases resistance to proteases, decreases glycosylation, increases glycosylation, reduces immunogenicity, increases stability, and/or facilitates chemical group linkage.

**2.** The modified FVII polypeptide of claim 1, wherein the heterologous Gla domain has a sequence of amino acids set forth in any of SEQ ID NOS: 110-115, 117 and 118, or the sufficient portion thereof.

**3.** The modified FVII polypeptide of claim 1 or claim 2, wherein all of the native FVII Gla domain is removed and is replaced with the heterologous Gla domain.

**4.** The modified FVII polypeptide of any of claims 1-3, wherein the heterologous Gla domain is selected from among a Gla domain in Factor IX (FIX), Factor X (FX), prothrombin, protein C and protein S.

**5.** The modified FVII polypeptide of any of claims 1-4, wherein the further modification(s) increases resistance to tissue factor pathway inhibitor (TFPI) compared to an unmodified FVII polypeptide.

**6.** The modified FVII polypeptide of claim 5, wherein the further modification(s) is one or more amino acid modification(s) at positions selected from among D196, K197, K199, G237, T239, R290, and K341 in a FVII polypeptide having a sequence of amino acids set forth in SEQ ID NO:3 or in corresponding residues in a FVII polypeptide, wherein the further modification(s) confer increased TFPI resistance.

**7.** The modified FVII polypeptide of claim 6, wherein the one or more amino acid modification(s) are selected from among D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341R, K341N, K341M, K341D, K341Q, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA and K197I198insS.

**8.** The modified FVII polypeptide of claim 7, wherein the one or more amino acid modification(s) are selected from among D196R/R290E, D196K/R290E, D196R/R290D, D196R/ K197E/K199E, D196K/K197E/K199E, D196R/K197E/ K199E/R290E, D196R/ K197M/ K199E, and D196R/ K197M/K199E/R290E.

**9.** The modified FVII polypeptide of any of claims 1-8, comprising one or more further amino acid modification(s) at positions Q176, M298 or E296 in a FVII polypeptide having a sequence of amino acids set forth in SEQ ID NO:3 or in corresponding residues in a FVII polypeptide.

**10.** The modified FVII polypeptide of claim 9, wherein the amino acid modifications are selected from among Q176A, M298Q, E296V and E296A.

**11.** The modified FVII polypeptide of claim 9 or claim 10, comprising M298Q/ A1Y44delinsYNSGKLEEFVQGNLERECMEEKCSFEEAREVFENTERTTEFWKQY.

**12.** The modified FVII polypeptide of any of claims 1-11, comprising one or more further amino acid modification(s) selected from among S279CN302C, L280C/N301C, V281C/V302C, S282C/V299C, insertion of a tyrosine at position 4, F4S, F4T, P10Q, P10E, P10D, P10N, Q21N, R28F, R28E, I30C, 130D, I30E, K32D, K32Q, K32E, K32G, K32H, K32T, K32C, K32A, K32S, D33C, D33F, D33E, D33K, A34C, A34E, A34D, A34I, A34L, A34M, A34V, A34F, A34W, A34Y, R36D, R36E, T37C, T37D, T37E, K38C, K38E, K38T, K38D, K38L, K38G, K38A, K38S, K38N, K38H, L39E, L39Q, L39H, W41N, W41C, W41E, W41D, I42R, I42N, I42S, I42A, I42Q, I42N, I42S, I42A, I42Q, I42K, S43Q, S43N, Y44K, Y44C, Y44D, Y44E, S45C, S45D, S45E, D46C, A51N, S53N, G58N, G59S, G59T, K62E, K62R, K62D, K62N, K62Q, K62T, L65Q, L65S, L65N, F71D, F71Y, F71E, F71Q, F71N, P74S, P74A, A75E, A75D, E77A, E82Q, E82N, E82S, E82T, T83K, N95S, N95T, G97S, G97T, Y101N, D104N, T106N, K109N, E116D, G117N, G124N, S126N, T128N, L141C, L141D, L141E, E142D, E142C, K143C, K143D, K143E, R144E, R144C, R144D, N145Y, N145G, N145F, N145M, N145S, N145I, N145L, N145T, N145V, N145P, N145K, N145H, N145Q, N145E, N145R, N145W, N145D, N145C, K157V, K157L, K157I, K157M, K157F, K157W, K157P, K157G, K157S, K157T, K157C, K157Y, K157N, K157E, K157R, K157H, K157D, K157Q, V158L, V158I, V158M, V158F, V158W, V158P, V158G, V158S, V158T, V158C, V158Y, V158N, V158E, V158R, V158K, V158H, V158D, V158Q, A175S, A175T, G179N, I186S, I186T, V188N, R202S, R202T, I205S, I205T, D212N, E220N, I230N, P231N, P236N, G237N, Q250C, V253N, E265N, T267N, E270N, A274M, A274L, A274K, A274R, A274D, A274V, A274I, A274F, A274W, A274P, A274G, A274T, A274C, A274Y, A274N, A274E, A274H, A274S, A274Q, F275H, R277N, F278S, F278A, F278N, F278Q, F278G, L280N, L288K, L288C, L288D, D289C, D289K, L288E, R290C, R290G, R290A, R290S, R290T, R290K, R290D, R290E, G291E, G291D, G291C, G291N, G291K, A292C, A292K, A292D, A292E, T293K, E296V, E296L, E296I, E296M, E296F, E296W, E296P, E296G, E296S, E296T, E296C, E296Y, E296N, E296K, E296R, E296H, E296D, E296Q, M298Q, M298V, M298L, M298I, M298F, M298W, M298P, M298G, M298S, M298T, M298C, M298Y, M298N, M298K, M298R, M298H, M298E, M298D, P303S, P303T, R304Y, R304F, R304L, R304M, R304G, R304T, R304A, R304S, R304N, L305V, L305Y, L305I, L305F, L305A, L305M, L305W, L305P, L305G, L305S, L305T, L305C, L305N, L305E, L305K, L305R, L305H, L305D, L305Q, M306D, M306N, D309S, D309T, Q312N, Q313K, Q313D, Q313E, S314A, S314V, S314I, S314M, S314F, S314W, S314P, S314G, S314L, S314T, S314C, S314Y, S314N, S314E, S314K, S314R, S314H, S314D, S314Q, R315K, R315G, R315A, R315S, R315T, R315Q, R315C, R315D, R315E, K316D, K316C, K316E, V317C, V317K, V317D, V317E, G318N, N322Y, N322G, N322F, N322M, N322S, N322I, N322L, N322T, N322V, N322P, N322K, N322H, N322Q, N322E, N322R, N322W, N322C, G331N, Y332S, Y332A, Y332N, Y332Q, Y332G, D334G, D334E, D334A, D334V, D334I, D334M, D334F, D334W, D334P, D334L, D334T, D334C, D334Y, D334N, D334K, D334R, D334H, D334S, D334Q, S336G, S336E, S336A, S336V, S336I, S336M, S336F, S336W, S336P, S336L, S336T, S336C, S336Y, S336N, S336K, S336R, S336H, S336D, S336Q, K337L, K337V, K337I, K337M, K337F, K337W, K337P, K337G, K337S, K337T, K337C, K337Y, K337N, K337E, K337R, K337H, K337D, K337Q, K341E, K341Q, K341G, K341T, K341A, K341S, G342N, H348N, R353N, Y357N, I361N, F374P, F374A, F374V, F374I, F374L, F374M, F374W, F374G, F374S, F374T, F374C, F374Y, F374N, F374E, F374K, F374R, F374H, F374D, F374Q, V376N, R379N, L390C, L390K, L390D, L390E, M391D, M391C, M391K, M391N, M391E, R392C, R392D, R392E, S393D, S393C, S393K, S393E, E394K, P395K, E394C, P395D, P395C, P395E, R396K, R396C, R396D, R396E, P397D, P397K, P397C, P397E, G398K, G398C, G398D, G398E, V399C, V399D, V399K, V399E, L400K, L401K, L401C, L401D, L401E, R402D, R402C, R402K, R402E, A403K, A403C, A403D, A403E, P404E, P404D, P404C, P404K, F405K, P406C, K32N/A34S, K32N/A34T, F31N/D33S, F31N/D33T, I30N/K32S, I30N/K32T, A34N/R36S, A34N/R36T, K38N/F40S, K38N/F40T, T37N/L39S, T37N/L39T, R36N/K38S, R36N/K38T, L39N/W41S, L39N/W41T, F40N/I42S, F40N/I42T, I42N/ Y44S, I42N/ Y44T, Y44N/ D46S, Y44N/ D46T, D46N/D48S, D46N/D48T, G47N/Q49S, G47N/Q49T, K143N/ N145S, K143N/ N145T, E142N/R144S, E142N/R144T, L141N/K143S, L141N/K143T, I140N/E142S, I140N/E142T, R144N/A146S, R144N/A146T, A146N/K148S, A146N/K148T, S147N/P149S, S147N/P149T, R290N/A292S, R290N/A292T, D289N/G291S, D289N/G291T, L288N/R290S, L288N/R290T, L287N/D289S, A292N/A294S, A292N/A294T, T293N/L295S, T293N/L295T, R315N/V317S, R315N/V317T, S314N/ K316S, S314N/ K316T, Q313N/ R315S, Q313N/ R315T, K316N/G318S, K316N/G318T, V317N/D319S, V317N/D319T, K341N/ D343S, K341N/ D343T, S339N/K341S, S339N/K341T, D343N/G345S, D343N/G345T, R392N/E394S, R392N/E394T, L390N/ R392S, L390N/ R392T, K389N/M391S, K389N/M391T, S393N/P395S, S393N/P395T, E394N/R396S, E394N/R396T, P395N/P397S, P395N/P397T, R396N/G398S, R396N/G398T, P397NN399S, P397N/V399T, G398N/L400S, G398N/L400T, V399N/L401S, V399N/L401T, L400N/R402S, L400N/R402T, L401N/A403S, L401N/A403T, R402N/P404S, R402N/P404T, A403N/F405S, A403N/F405T, P404N/P406S and P404N/P406T.

**13.** The modified FVII polypeptide of any of claims 1-12, comprising substitution of positions 300-322, 305-322, 300-312, or 305-312 with the corresponding amino acids from trypsin, thrombin or FX, or substitution of positions 310-329, 311-322 or 233-329 with the corresponding amino acids from trypsin.

**14.** The modified FVII polypeptide of any of claims 1-13, wherein the unmodified FVII polypeptide has a sequence of amino acids set forth in SEQ ID NO:3.

**15.** The modified FVII polypeptide of claim 14 having a sequence of amino acids set forth in SEQ ID NO:247.

**16.** The modified FVII polypeptide of any of claims 1-15 that is an active or a mature polypeptide.

**17.** The modified FVII polypeptide of any of claims 1-16, wherein only the primary sequence is modified.

**18.** The modified FVII polypeptide of any of claims 1-17, further comprising a chemical modification or a post-translational modification.

**19.** The modified FVII polypeptide of claim 18, wherein the FVII polypeptide is glycosylated, carboxylated, hydroxylated, sulfated, phosphorylated, albuminated, or conjugated to a polyethylene glycol (PEG) moiety.

**20.** The modified FVII polypeptide of any of claims 1-19 that is a single-chain polypeptide or is a two chain polypeptide.

**21.** The modified FVII polypeptide of any of claims 1-20 that is active or activated.

**22.** The modified polypeptide of any of claims 1-21, wherein the coagulation activity is increased.

**23.** A nucleic acid molecule, comprising a sequence of nucleotides encoding a modified FVII polypeptide of any of claims 1-22.

**24.** A vector, comprising the nucleic acid molecule of claim 23.

**25.** The vector of claim 24, wherein the vector is a prokaryotic vector, viral vector, or a eukaryotic vector.

**26.** The vector of claim 24 or claim 25, wherein the vector is a mammalian vector.

**27.** The vector of claim 25, wherein the vector is a viral vector selected from among an adenovirus, an adeno-associated-virus, a retrovirus, a herpes virus, a lentivirus, a poxvirus, and a cytomegalovirus.

**28.** A cell, comprising the vector of any of claims 24-27.

**29.** The cell of claim 28 that is a eukaryotic cell.

**30.** The cell of claim 29, wherein the eukaryotic cell is a mammalian cell.

**31.** The cell of claim 30, wherein the mammalian cell is selected from among baby hamster kidney cells (BHK-21) or 293 cells or CHO cells.

**32.** The cell of claim 28 that is a yeast cell.

**33.** The cell of claim 32 that is a *Pichia sp.* cell.

**34.** The cell of any of claims 28-33, wherein the cell expresses the modified FVII polypeptide.

**35.** A modified FVII polypeptide that is produced by the cell of any of claims 28-34.

**36.** A pharmaceutical composition, comprising a therapeutically effective concentration or amount of a modified FVII polypeptide of any of claims 1-22 and 35, or a nucleic acid molecule of claim 23 or a vector of any of claims 24-27 or a cell of any of claims 28-34, in a pharmaceutically acceptable vehicle.

**37.** The pharmaceutical composition of claim 36 that is formulated for local, systemic, or topical administration.

**38.** The pharmaceutical composition of claim 36 or claim 37 that is formulated for oral, nasal, pulmonary buccal, transdermal, subcutaneous, intraduodenal, enteral, parenteral, intravenous, or intramuscular administration.

**39.** The pharmaceutical composition of any of claims 36-38 that is formulated for controlled-release.

**40.** A modified FVII polypeptide of any of claims 1-22 and 35 for use in treating a disease or condition that is treated by administration of FVII or a pro-coagulant.

**41.** Use of a pharmaceutical composition of any of claims 36-39 in the preparation of a medicament for treatment of a disease or condition that is treated by administration of FVII or a pro-coagulant.

**42.** A modified FVII polypeptide for use according to claim 40, or the use of claim 41, wherein the disease or condition is treated by administration of a zymogen or active form of FVII.

**43.** A modified FVII polypeptide for use according to claim 40 or claim 42, or the use of claim 41 or claim 42, wherein the disease or condition to be treated is selected from among blood coagulation disorders, hematologic disorders, hemorrhagic disorders, hemophilias, factor VII deficiency, bleeding disorders, surgical bleeding, and bleeding resulting from trauma or is due to a bleeding complication due to surgery or trauma.

**44.** A modified FVII polypeptide for use according to claim 43, or the use of claim 43, wherein the hemophilia is hemophilia A or hemophilia B or hemophilia C or is acquired.

## Patentansprüche

**1.** Modifiziertes Faktor VII (FVII)-Polypeptid, umfassend:

eine heterologe Gla-Domäne oder einen ausreichenden Teil davon, um Phospholipidbindung zu bewirken, wobei das modifizierte FVII-Polypeptid gesteigerte Phospholipidaffinität oder -bindung aufweist verglichen mit einem unmodifizierten FVII-Polypeptid, welches nicht die heterologe Gla-Domäne enthält, wobei:

der ausreichende Teil 30 oder mehr zusammenhängende Aminosäuren der heterologen Gla-Domäne enthält; und
die heterologe Gla-Domäne ausgewählt ist aus einer Gla-Domäne in Faktor IX (FIX), Faktor X (FX), Prothrombin, Protein C, Protein S, Osteocalcin, Growth-arrest-specific protein 6 (Gas6) und Protein Z; und

eine oder mehrere weitere Aminosäuremodifikation(en), die Resistenz gegen Antithrombin-III (AT-III) steigert, Affinität für Gewebefaktor (engl.: tissue factor, TF) steigert, intrinsische Aktivität steigert, TF-abhängige katalytische oder gerinnungsfördernde Aktivität steigert, gerinnungsfördernde Aktivität steigert, die Konformation des Polypeptids verändert, um Zymogenität zu verändern, katalytische oder gerinnungsfördernde Aktivität steigert durch Verschieben des Gleichgewichts zwischen hoch aktiven und weniger aktiven FVIIa-Konformationen zugunsten der hoch aktiven Konformationen, Resistenz gegen Proteasen steigert, Glykosylierung verringert, Glykosylierung steigert, Immunogenität reduziert, Stabilität steigert, und/oder Verbindung chemischer Gruppen erleichtert.

**2.** Modifiziertes FVII-Polypeptid nach Anspruch 1, wobei die heterologe Gla-Domäne eine in einer von SEQ ID NOS: 110-115, 117 und 118 dargelegte Sequenz von Aminosäuren oder den ausreichenden Teil davon aufweist.

**3.** Modifiziertes FVII-Polypeptid nach Anspruch 1 oder Anspruch 2, wobei die gesamte native FVII Gla-Domäne entfernt ist und mit der heterologen Gla-Domäne ersetzt ist.

**4.** Modifiziertes FVII-Polypeptid nach einem der Ansprüche 1-3, wobei die heterologe Gla-Domäne ausgewählt ist aus einer Gla-Domäne in Faktor IX (FIX), Faktor X (FX), Prothrombin, Protein C und Protein S.

**5.** Modifiziertes FVII-Polypeptid nach einem der Ansprüche 1-4, wobei die weitere(n) Modifikation(en) Resistenz gegen Gewebefaktor-Weg-Inhibitor (engl.: tissue factor pathway inhibitor, TFPI) steigert verglichen mit einem unmodifizierten FVII-Polypeptid.

**6.** Modifiziertes FVII-Polypeptid nach Anspruch 5, wobei die weitere(n) Modifikation(en) eine oder mehrere Aminosäuremodifikation(en) an Positionen, ausgewählt aus D196, K197, K199, G237, T239, R290 und K341 in einem FVII-Polypeptid mit einer in SEQ ID NO:3 dargelegten Sequenz von Aminosäuren, oder in entsprechenden Resten in einem FVII-Polypeptid ist, wobei die weitere(n) Modifikation(en) gesteigerte TFPI-Resistenz vermitteln.

**7.** Modifiziertes FVII-Polypeptid nach Anspruch 6, wobei die eine oder mehreren Aminosäuremodifikation(en) ausgewählt sind aus D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341 E, K341 R, K341 N, K341M, K341 D, K341 Q, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA und K197I198insS.

**8.** Modifiziertes FVII-Polypeptid nach Anspruch 7, wobei die eine oder mehreren Aminosäuremodifikation(en) ausgewählt sind aus D196R/R290E, D196K/R290E, D196R/R290D, D196R/ K197E/K199E, D196K/K197E/K199E, D196R/K197E/ K199E/R290E, D196R/ K197M/ K199E, und D196R/ K197M/K199E/R290E.

**9.** Modifiziertes FVII-Polypeptid nach einem der Ansprüche 1-8, umfassend eine oder mehrere weitere Aminosäuremodifikation(en) an Position Q176, M298 oder E296 in einem FVII-Polypeptid mit einer in SEQ ID NO:3 dargelegten Sequenz von Aminosäuren oder in entsprechenden Resten in einem FVII-Polypeptid.

**10.** Modifiziertes FVII-Polypeptid nach Anspruch 9, wobei die Aminosäuremodifikationen ausgewählt sind aus Q176A, M298Q, E296V und E296A.

**11.** Modifiziertes FVII-Polypeptid nach Anspruch 9 oder Anspruch 10, umfassend M298Q/A1Y44delinsYNSGKLEEFVQGNLERECMEEKCSFEEAREVFEN TERTTEFWKQY.

**12.** Modifiziertes FVII-Polypeptid nach einem der Ansprüche 1-11, umfassend eine oder mehrere weitere Aminosäuremodifikation(en), ausgewählt aus S279C/V302C, L280C/N301C, V281C/V302C, S282C/V299C, Insertion eines Tyrosins an Position 4, F4S, F4T, P10Q, P10E, P10D, P10N, Q21N, R28F, R28E, I30C, 130D, I30E, K32D, K32Q, K32E, K32G, K32H, K32T, K32C, K32A, K32S, D33C, D33F, D33E, D33K, A34C, A34E, A34D, A34I, A34L, A34M, A34V, A34F, A34W, A34Y, R36D, R36E, T37C, T37D, T37E, K38C, K38E, K38T, K38D, K38L, K38G, K38A, K38S, K38N, K38H, L39E, L39Q, L39H, W41 N, W41C, W41 E, W41 D, I42R, I42N, I42S, I42A, I42Q, I42N, I42S, I42A, I42Q, I42K, S43Q, S43N, Y44K, Y44C, Y44D, Y44E, S45C, S45D, S45E, D46C, A51 N, S53N, G58N, G59S, G59T, K62E, K62R, K62D, K62N, K62Q, K62T, L65Q, L65S, L65N, F71 D, F71 Y, F71 E, F71Q, F71N, P74S, P74A, A75E, A75D, E77A, E82Q, E82N, E82S, E82T T83K, N95S, N95T, G97S, G97T, Y101N, D104N, T106N, K109N, E116D, G117N, G124N, S126N, T128N, L141C, L141D, L141E, E142D, E142C, K143C, K143D, K143E, R144E, R144C, R144D, N145Y, N145G, N145F, N145M, N145S, N145I, N145L, N145T, N145V, N145P, N145K, N145H, N145Q, N145E, N145R, N145W, N145D, N145C, K157V, K157L, K157I, K157M, K157F, K157W, K157P, K157G, K157S, K157T, K157C, K157Y, K157N, K157E, K157R, K157H, K157D, K157Q, V158L, V158I, V158M, V158F, V158W, V158P, V158G, V158S, V158T, V158C, V158Y, V158N, V158E, V158R, V158K, V158H, V158D, V158Q, A175S, A175T, G179N, I186S, I186T, V188N, R202S, R202T, I205S, I205T, D212N, E220N, I230N, P231 N, P236N, G237N, Q250C, V253N, E265N, T267N, E270N, A274M, A274L, A274K, A274R, A274D, A274V, A274I, A274F, A274W, A274P, A274G, A274T, A274C, A274Y, A274N, A274E, A274H, A274S, A274Q, F275H, R277N, F278S, F278A, F278N, F278Q, F278G, L280N, L288K, L288C, L288D, D289C, D289K, L288E, R290C, R290G, R290A, R290S, R290T, R290K, R290D, R290E, G291 E, G291 D, G291 C, G291 N, G291K, A292C, A292K, A292D, A292E, T293K, E296V, E296L, E296I, E296M, E296F, E296W, E296P, E296G, E296S, E296T, E296C, E296Y, E296N, E296K, E296R, E296H, E296D, E296Q, M298Q, M298V, M298L, M298I, M298F, M298W, M298P, M298G, M298S, M298T, M298C, M298Y, M298N, M298K, M298R, M298H, M298E, M298D, P303S, P303T, R304Y, R304F, R304L, R304M, R304G, R304T, R304A, R304S, R304N, L305V, L305Y, L305I, L305F, L305A, L305M, L305W, L305P, L305G, L305S, L305T, L305C, L305N, L305E, L305K, L305R, L305H, L305D, L305Q, M306D, M306N, D309S, D309T, Q312N, Q313K, Q313D, Q313E, S314A, S314V, S314I, S314M, S314F, S314W, S314P, S314G, S314L, S314T, S314C, S314Y, S314N, S314E, S314K, S314R, S314H, S314D, S314Q, R315K, R315G, R315A, R315S, R315T, R315Q, R315C, R315D, R315E, K316D, K316C, K316E, V317C, V317K, V317D, V317E, G318N, N322Y, N322G, N322F, N322M, N322S, N322I, N322L, N322T, N322V, N322P, N322K, N322H, N322Q, N322E, N322R, N322W, N322C, G331 N, Y332S, Y332A, Y332N, Y332Q, Y332G, D334G, D334E, D334A, D334V, D334I, D334M, D334F, D334W, D334P, D334L, D334T, D334C, D334Y, D334N, D334K, D334R, D334H, D334S, D334Q, S336G, S336E, S336A, S336V, S336I, S336M, S336F, S336W, S336P, S336L, S336T, S336C, S336Y, S336N, S336K, S336R, S336H, S336D, S336Q, K337L, K337V, K337I, K337M, K337F, K337W, K337P, K337G, K337S, K337T, K337C, K337Y, K337N, K337E, K337R, K337H, K337D, K337Q, K341E, K341Q, K341G, K341T, K341A, K341S, G342N, H348N, R353N, Y357N, I361N, F374P, F374A, F374V, F374I, F374L, F374M, F374W, F374G, F374S, F374T, F374C, F374Y, F374N, F374E, F374K, F374R, F374H, F374D, F374Q, V376N, R379N, L390C, L390K, L390D, L390E, M391 D, M391 C, M391 K, M391 N, M391 E, R392C, R392D, R392E, S393D, S393C, S393K, S393E, E394K, P395K, E394C, P395D, P395C, P395E, R396K, R396C, R396D, R396E, P397D, P397K, P397C, P397E, G398K, G398C, G398D, G398E, V399C, V399D, V399K, V399E, L400K, L401K, L401C, L401 D, L401 E, R402D, R402C, R402K, R402E, A403K, A403C, A403D, A403E, P404E, P404D, P404C, P404K, F405K, P406C, K32N/A34S, K32N/A34T, F31 N/D33S, F31 N/D33T, I30N/K32S, I30N/K32T, A34N/R36S, A34N/R36T, K38N/F40S, K38N/F40T, T37N/L39S, T37N/L39T, R36N/K38S, R36N/K38T, L39N/W41 S, L39N/W41 T, F40N/I42S, F40N/I42T, I42N/ Y44S, I42N/ Y44T, Y44N/ D46S, Y44N/ D46T, D46N/D48S, D46N/D48T, G47N/Q49S, G47N/Q49T, K143N/ N145S, K143N/N145T, E142N/R144S, E142N/R144T, L141N/K143S, L141N/K143T, I140N/E142S, I140N/E142T, R144N/A146S, R144N/A146T, A146N/K148S, A146N/K148T, S147N/P149S, S147N/P149T, R290N/A292S, R290N/A292T, D289N/G291S, D289N/G291T, L288N/R290S, L288N/R290T, L287N/D289S, A292N/A294S, A292N/A294T, T293N/L295S, T293N/L295T, R315N/V317S, R315N/V317T, S314N/ K316S, S314N/ K316T, Q313N/ R315S, Q313N/ R315T, K316N/G318S, K316N/G318T, V317N/D319S, V317N/D319T, K341N/ D343S, K341N/ D343T, S339N/K341 S, S339N/K341T, D343N/G345S, D343N/G345T, R392N/E394S, R392N/E394T, L390N/ R392S, L390N/ R392T, K389N/M391S, K389N/M391T, S393N/P395S, S393N/P395T, E394N/R396S, E394N/R396T, P395N/P397S, P395N/P397T, R396N/G398S, R396N/G398T, P397N/V399S, P397N/V399T, G398N/L400S, G398N/L400T, V399N/L401 S, V399N/L401 T, L400N/R402S, L400N/R402T, L401N/A403S, L401N/A403T, R402N/P404S, R402N/P404T, A403N/F405S, A403N/F405T, P404N/P406S und P404N/P406T.

**13.** Modifiziertes FVII-Polypeptid nach einem der Ansprüche 1-12, umfassend Substitution von Positionen 300-322, 305-322, 300-312 oder 305-312 mit den entsprechenden Aminosäuren von Trypsin, Thrombin oder FX, oder Substitution von Positionen 310-329, 311-322 oder 233-329 mit den entsprechenden Aminosäuren von Trypsin.

**14.** Modifiziertes FVII-Polypeptid nach einem der Ansprüche 1-13, wobei das unmodifizierte FVII-Polypeptid eine in SEQ ID NO:3 dargelegte Sequenz von Aminosäuren aufweist.

**15.** Modifiziertes FVII-Polypeptid nach Anspruch 14, welches eine in SEQ ID NO: 247 dargelegte Sequenz von Aminosäuren aufweist.

**16.** Modifiziertes FVII-Polypeptid nach einem der Ansprüche 1-15, welches ein aktives oder ein reifes Polypeptid ist.

**17.** Modifiziertes FVII-Polypeptid nach einem der Ansprüche 1-16, wobei nur die Primärsequenz modifiziert ist.

**18.** Modifiziertes FVII-Polypeptid nach einem der Ansprüche 1-17, weiterhin umfassend eine chemische Modifikation oder eine post-translationale Modifikation.

**19.** Modifiziertes FVII-Polypeptid nach Anspruch 18, wobei das FVII-Polypeptid glykosyliert, carboxyliert, hydroxyliert, sulfatiert, phosphoryliert, albuminiert oder an eine Polyethylenglykol (PEG)-Gruppe konjugiert ist.

**20.** Modifiziertes FVII-Polypeptid nach einem der Ansprüche 1-19, welches ein einzelkettiges Polypeptid ist oder ein zweikettiges Polypeptid ist.

**21.** Modifiziertes FVII-Polypeptid nach einem der Ansprüche 1-20, welches aktiv oder aktiviert ist.

**22.** Modifiziertes Polypeptid nach einem der Ansprüche 1-21, wobei die Gerinnungsaktivität gesteigert ist.

**23.** Nukleinsäuremolekül, umfassend eine Sequenz von Nukleotiden, welche für ein modifiziertes FVII-Polypeptid nach einem der Ansprüche 1-22 kodiert.

**24.** Vektor, umfassend das Nukleinsäuremolekül nach Anspruch 23.

**25.** Vektor nach Anspruch 24, wobei der Vektor ein prokaryotischer Vektor, viraler Vektor oder ein eukaryotischer Vektor ist.

**26.** Vektor nach Anspruch 24 oder Anspruch 25, wobei der Vektor ein Säugetiervektor ist.

**27.** Vektor nach Anspruch 25, wobei der Vektor ein viraler Vektor ist, ausgewählt aus einem Adenovirus, einem adenoassoziierten Virus, einem Retrovirus, einem Herpesvirus, einem Lentivirus, einem Pockenvirus und einem Cytomegalovirus.

**28.** Zelle, umfassend den Vektor nach einem der Ansprüche 24-27.

**29.** Zelle nach Anspruch 28, welche eine eukaryotische Zelle ist.

**30.** Zelle nach Anspruch 29, wobei die eukaryotische Zelle eine Säugetierzelle ist.

**31.** Zelle nach Anspruch 30, wobei die Säugetierzelle ausgewählt ist aus Babyhamster-Nierenzellen (BHK-21) oder 293-Zellen oder CHO-Zellen.

**32.** Zelle nach Anspruch 28, welche eine Hefezelle ist.

**33.** Zelle nach Anspruch 32, welche eine *Pichia sp*.-Zelle ist.

**34.** Zelle nach einem der Ansprüche 28-33, wobei die Zelle das modifizierte FVII-Polypeptid exprimiert.

**35.** Modifiziertes FVII-Polypeptid, welches von der Zelle nach einem der Ansprüche 28-34 produziert wird.

**36.** Pharmazeutische Zusammensetzung, umfassend eine therapeutisch wirksame Konzentration oder Menge eines modifizierten FVII-Polypeptids nach einem der Ansprüche 1-22 und 35 oder eines Nukleinsäuremoleküls nach Anspruch 23 oder eines Vektors nach einem der Ansprüche 24-27 oder einer Zelle nach einem der Ansprüche 28-34, in einem pharmazeutisch annehmbaren Trägerstoff.

**37.** Pharmazeutische Zusammensetzung nach Anspruch 36, welche für lokale, systemische oder topische Verabreichung formuliert ist.

**38.** Pharmazeutische Zusammensetzung nach Anspruch 36 oder Anspruch 37, welche für orale, nasale, pulmonale buccale, transdermale, subkutane, intraduodenale, enterale, parenterale, intravenöse oder intramuskuläre Verabreichung formuliert ist.

**39.** Pharmazeutische Zusammensetzung nach einem der Ansprüche 36-38, welche für kontrollierte Freisetzung formuliert ist.

**40.** Modifiziertes FVII-Polypeptid nach einem der Ansprüche 1-22 und 35 zur Verwendung beim Behandeln einer Erkrankung oder eines Zustands, welche/welcher durch Verabreichung von FVII oder eines Pro-Gerinnungsmittels behandelt wird.

**41.** Verwendung einer pharmazeutischen Zusammensetzung nach einem der Ansprüche 36-39 in der Herstellung eines Medikaments zur Behandlung einer Erkrankung oder eines Zustands, welche/welcher durch Verabreichung von FVII oder eines Pro-Gerinnungsmittels behandelt wird.

**42.** Modifiziertes FVII-Polypeptid zur Verwendung nach Anspruch 40 oder Verwendung nach Anspruch 41, wobei die Erkrankung oder der Zustand durch Verabreichung eines Zymogens oder einer aktiven Form von FVII behandelt wird.

**43.** Modifiziertes FVII-Polypeptid zur Verwendung nach Anspruch 40 oder 42 oder Verwendung nach Anspruch 41 oder Anspruch 42, wobei die zu behandelnde Erkrankung oder Zustand ausgewählt ist aus Blutgerinnungsstörungen, hämatologischen Störungen, hämorrhagischen Störungen, Hämophilien, Faktor VII-Defizienz, Blutungsstörungen, operationsbedingter Blutung und von Trauma resultierender Blutung, oder Folge einer Blutungskomplikation in Folge von Operation oder Trauma ist.

**44.** Modifiziertes FVII-Polypeptid zur Verwendung nach Anspruch 43 oder Verwendung nach Anspruch 43, wobei die Hämophilie Hämophilie A oder Hämophilie B oder Hämophilie C oder erworbene ist.

## Revendications

**1.** Polypeptide modifié du facteur VII (FVII), comprenant :

un domaine Gla hétérologue, ou une partie suffisante de celui-ci pour effectuer une liaison aux phospholipides, moyennant quoi le polypeptide modifié du FVII présente une affinité accrue pour les phospholipides ou une liaison aux phospholipides accrue par rapport à un polypeptide non modifié du facteur FVII qui ne contient pas le domaine Gla hétérologue, dans lequel :

la partie suffisante contient 30 acides aminés contigus ou plus provenant du domaine Gla hétérologue ; et le domaine Gla hétérologue est choisi parmi un domaine Gla présent dans le Facteur IX (FIX), le Facteur X (FX), la prothrombine, la protéine C, la protéine S, l'ostéocalcine, la protéine 6 spécifique de l'arrêt de la croissance (Gas6) et la protéine Z ; et
une ou plusieurs modifications supplémentaires d'acides aminés qui augmentent la résistance à l'antithrombine III (AT-III), augmentent l'affinité pour le facteur tissulaire (TF), augmentent l'activité intrinsèque, augmentent l'activité catalytique ou coagulante dépendante du facteur TF, augmentent l'activité coagulante, modifient la conformation du polypeptide pour modifier la zymogénicité, augmentent l'activité catalytique ou coagulante en déplaçant l'équilibre entre les conformations de FVIIa fortement actives et moins actives en faveur des conformations fortement actives, augmentent la résistance aux protéases, diminuent la glycosylation, augmentent la glycosylation, réduisent l'immunogénicité, augmentent la stabilité, et/ou facilitent la liaison à des groupements chimiques.

**2.** Polypeptide modifié du facteur FVII de la revendication 1, dans lequel le domaine Gla hétérologue a une séquence d'acides aminés établie dans l'une des séquences SEQ ID N° : 110 à 115, 117 et 118, ou la partie suffisante de celui-ci.

**3.** Polypeptide modifié du facteur FVII de la revendication 1 ou de la revendication 2, dans lequel tout le domaine Gla natif du facteur FVII est enlevé et est remplacé par le domaine Gla hétérologue.

**4.** Polypeptide modifié du facteur FVII de l'une des revendications 1 à 3, dans lequel le domaine Gla hétérologue est choisi parmi un domaine Gla présent dans le Facteur IX (FIX), le Facteur X (FX), la prothrombine, la protéine C et la protéine S.

**5.** Polypeptide modifié du facteur FVII de l'une des revendications 1 à 4, dans lequel la/les modification(s) supplémentaire(s) augmente/augmentent la résistance à l'inhibiteur de la voie du facteur tissulaire (TFPI) par rapport à un polypeptide non modifié du facteur FVII.

**6.** Polypeptide modifié du facteur FVII de la revendication 5, dans lequel la/les modification(s) supplémentaire(s) correspond/correspondent à une ou plusieurs modifications d'acides aminés à des positions choisies parmi D196, K197, K199, G237, T239, R290, et K341 dans un polypeptide du facteur FVII ayant une séquence d'acides aminés établie dans la séquence SEQ ID N° : 3 ou au niveau de résidus correspondants dans un polypeptide du facteur FVII, dans lequel la/les modification(s) supplémentaire(s) confère/confèrent une résistance accrue à l'inhibiteur TFPI.

**7.** Polypeptide modifié du facteur FVII de la revendication 6, dans lequel la ou les plusieurs modifications d'acides aminés sont choisies parmi les modifications D196K, D196R, D196A, D196Y, D196F, D196M, D196W, D196L, D196I, K197Y, K197A, K197E, K197D, K197L, K197M, K197I, K197V, K197F, K197W, K199A, K199D, K199E, G237W, G237T, G237I, G237V, T239A, R290A, R290E, R290D, R290N, R290Q, R290K, K341E, K341R , K341N, K341M, K341D, K341Q, G237T238insA, G237T238insS, G237T238insV, G237T238insAS, G237T238insSA, D196K197insK, D196K197insR, D196K197insY, D196K197insW, D196K197insA, D196K197insM, K197I198insE, K197I198insY, K197I198insA et K197I198insS.

**8.** Polypeptide modifié du facteur FVII de la revendication 7, dans lequel les une ou plusieurs modifications d'acides aminés sont choisies parmi les modifications D196R/R290E, D196K/R290E, D196R/R290D, D196R/K197E/K199E, D196K/K197E/K199E, D196R/K197E/K199E/R290E, D196R/K197M/K199E, et D196R/K197M/K199E/R290E.

**9.** Polypeptide modifié du facteur FVII de l'une des revendications 1 à 8, comprenant une ou plusieurs modifications supplémentaires d'acides aminés à la position Q176, M298 ou E296 dans un polypeptide du facteur FVII ayant une séquence d'acides aminés établie dans la séquence SEQ ID N° : 3 ou au niveau de résidus correspondants dans un polypeptide du facteur FVII.

**10.** Polypeptide modifié du facteur FVII de la revendication 9, dans lequel les modifications d'acides aminés sont choisies parmi Q176A, M298Q, E296V et E296A.

**11.** Polypeptide modifié du facteur FVII de la revendication 9 ou de la revendication 10, comprenant une modification M298Q/A1Y44delinsYNSGKLEEFVQGNLERECMEEKCSFEEAREVFENTERTTEFWKQY.

**12.** Polypeptide modifié du facteur FVII de l'une des revendications 1 à 11, comprenant une ou plusieurs modifications supplémentaires d'acides aminés choisies parmi les modifications S279C/V302C, L280C/N301C, V281C/V302C, S282C/V299C, l'insertion d'une tyrosine à la position 4, F4S, F4T, P10Q, P10E, P10D, P10N, Q21N, R28F, R28E, I30C, I30D, I30E, K32D, K32Q, K32E, K32G, K32H, K32T, K32C, K32A, K32S, D33C, D33F, D33E, D33K, A34C, A34E, A34D, A34I, A34L, A34M, A34V, A34F, A34W, A34Y, R36D, R36E, T37C, T37D, T37E, K38C, K38E, K38T, K38D, K38L, K38G, K38A, K38S, K38N, K38H, L39E, L39Q, L39H, W41N, W41C, W41E, W41D, I42R, I42N, I42S, I42A, I42Q, I42N, I42S, I42A, I42Q, I42K, S43Q, S43N, Y44K, Y44C, Y44D, Y44E, S45C, S45D, S45E, D46C, A51N, S53N, G58N, G59S, G59T, K62E, K62R, K62D, K62N, K62Q, K62T, L65Q, L65S, L65N, F71D, F71Y, F71E, F71Q, F71N, P74S, P74A, A75E, A75D, E77A, E82Q, E82N, E82S, E82T, T83K, N95S, N95T, G97S, G97T, Y101N, D104N, T106N, K109N, E116D, G117N, G124N, S126N, T128N, L141C, L141D, L141E, E142D, E142C, K143C, K143D, K143E, R144E, R144C, R144D, N145Y, N145G, N145F, N145M, N145S, N145I, N145L, N145T, N145V, N145P, N145K, N145H, N145Q, N145E, N145R, N145W, N145D, N145C, K157V, K157L, K157I, K157M, K157F, K157W, K157P, K157G, K157S, K157T, K157C, K157Y, K157N, K157E, K157R, K157H, K157D, K157Q, V158L, V158I, V158M, V158F, V158W, V158P, V158G, V158S, V158T, V158C, V158Y, V158N, V158E, V158R, V158K, V158H, V158D, V158Q, A175S, A175T, G179N, I186S, I186T, V188N, R202S, R202T, I205S, I205T, D212N, E220N, I230N, P231N, P236N, G237N, Q250C, V253N, E265N, T267N, E270N, A274M, A274L, A274K, A274R, A274D, A274V, A274I, A274F, A274W, A274P, A274G, A274T, A274C, A274Y, A274N, A274E, A274H, A274S, A274Q, F275H, R277N, F278S, F278A, F278N, F278Q, F278G, L280N, L288K, L288C, L288D, D289C, D289K, L288E, R290C, R290G, R290A, R290S, R290T, R290K, R290D, R290E, G291E, G291D, G291C, G291N, G291K, A292C, A292K, A292D, A292E, T293K, E296V, E296L, E296I, E296M, E296F, E296W, E296P, E296G, E296S, E296T, E296C, E296Y, E296N, E296K, E296R, E296H, E296D, E296Q, M298Q, M298V, M298L, M298I, M298F, M298W, M298P, M298G, M298S, M298T, M298C, M298Y, M298N, M298K, M298R, M298H, M298E, M298D, P303S, P303T, R304Y, R304F, R304L, R304M, R304G, R304T, R304A, R304S, R304N, L305V, L305Y, L305I, L305F, L305A, L305M, L305W, L305P, L305G, L305S, L305T, L305C, L305N, L305E, L305K, L305R, L305H, L305D, L305Q, M306D, M306N, D309S, D309T, Q312N, Q313K, Q313D, Q313E, S314A, S314V, S314I, S314M, S314F, S314W, S314P, S314G, S314L, S314T, S314C, S314Y, S314N, S314E, S314K, S314R, S314H, S314D, S314Q, R315K, R315G, R315A, R315S, R315T, R315Q, R315C, R315D, R315E, K316D, K316C, K316E, V317C, V317K, V317D, V317E, G318N, N322Y, N322G, N322F, N322M, N322S, N322I, N322L, N322T, N322V, N322P, N322K, N322H, N322Q, N322E, N322R, N322W, N322C, G331N, Y332S, Y332A, Y332N, Y332Q, Y332G, D334G, D334E, D334A, D334V, D334I, D334M, D334F, D334W, D334P, D334L, D334T, D334C, D334Y, D334N, D334K, D334R, D334H, D334S, D334Q, S336G, S336E, S336A, S336V, S336I, S336M, S336F, S336W, S336P, S336L, S336T, S336C, S336Y, S336N, S336K, S336R, S336H, S336D, S336Q, K337L, K337V, K337I, K337M, K337F, K337W, K337P, K337G, K337S, K337T, K337C, K337Y, K337N, K337E, K337R, K337H, K337D, K337Q, K341E, K341Q, K341G, K341T, K341A, K341S, G342N, H348N, R353N, Y357N, I361N, F374P, F374A, F374V, F374I, F374L, F374M, F374W, F374G, F374S, F374T, F374C, F374Y, F374N, F374E, F374K, F374R, F374H, F374D, F374Q, V376N, R379N, L390C, L390K, L390D, L390E, M391D, M391C, M391K, M391N, M391E, R392C, R392D, R392E, S393D, S393C, S393K, S393E, E394K, P395K, E394C, P395D, P395C, P395E, R396K, R396C, R396D, R396E, P397D, P397K, P397C, P397E, G398K, G398C, G398D, G398E, V399C, V399D, V399K, V399E, L400K, L401K, L401C, L401D, L401E, R402D, R402C, R402K, R402E, A403K, A403C, A403D, A403E, P404E, P404D, P404C, P404K, F405K, P406C, K32N/A34S, K32N/A34T, F31N/D33S, F31N/D33T, I30N/K32S, I30N/K32T, A34N/R36S, A34N/R36T, K38N/F40S, K38N/F40T, T37N/L39S, T37N/L39T, R36N/K38S, R36N/K38T, L39N/W41S, L39N/W41T, F40N/I42S, F40N/I42T, I42N/Y44S, I42N/Y44T, Y44N/D46S, Y44N/D46T, D46N/D48S, D46N/D48T, G47N/Q49S, G47N/Q49T, K143N/N145S, K143N/N145T, E142N/R144S, E142N/R144T, L141N/K143S, L141N/K143T, I140N/E142S, I140N/E142T, R144N/A146S, R144N/A146T, A146N/K148S, A146N/K148T, S147N/P149S, S147N/P149T, R290N/A292S, R290N/A292T, D289N/G291S, D289N/G291T, L288N/R290S, L288N/R290T, L287N/D289S, A292N/A294S, A292N/A294T, T293N/L295S, T293N/L295T, R315N/V317S, R315N/V317T, S314N/K316S, S314N/K316T, Q313N/R315S, Q313N/R315T, K316N/G318S, K316N/G318T, V317N/D319S, V317N/D319T, K341N/D343S, K341N/D343T, S339N/K341S, S339N/K341T, D343N/G345S, D343N/G345T, R392N/E394S, R392N/E394T, L390N/R392S, L390N/R392T, K389N/M391S, K389N/M391T, S393N/P395S, S393N/P395T, E394N/R396S, E394N/R396T, P395N/P397S, P395N/P397T, R396N/G398S, R396N/G398T, P397N/V399S, P397N/V399T, G398N/L400S, G398N/L400T, V399N/L401S, V399N/L401T, L400N/R402S, L400N/R402T, L401N/A403S, L401N/A403T, R402N/P404S, R402N/P404T, A403N/F405S, A403N/F405T, P404N/P406S et P404N/P406T.

**13.** Polypeptide modifié du facteur FVII de l'une des revendications 1 à 12, comprenant la substitution des positions 300 à 322, 305 à 322, 300 à 312, ou 305 à 312 par les acides aminés correspondants de la trypsine, de la thrombine ou du facteur FX, ou la substitution des positions 310 à 329, 311 à 322 ou 233 à 329 par les acides aminés correspondants de la trypsine.

**14.** Polypeptide modifié du facteur FVII de l'une des revendications 1 à 13, dans lequel le polypeptide non modifié du facteur FVII a une séquence d'acides aminés établie dans la séquence SEQ ID N° : 3.

**15.** Polypeptide modifié du facteur FVII de la revendication 14, ayant une séquence d'acides aminés établie dans la séquence SEQ ID N° : 247.

**16.** Polypeptide modifié du facteur FVII de l'une des revendications 1 à 15, qui est un polypeptide actif ou mature.

**17.** Polypeptide modifié du facteur FVII de l'une des revendications 1 à 16, dans lequel seule la séquence primaire est modifiée.

**18.** Polypeptide modifié du facteur FVII de l'une des revendications 1 à 17, comprenant en outre une modification chimique ou une modification post-traductionnelle.

**19.** Polypeptide modifié du facteur FVII de la revendication 18, dans lequel le polypeptide du facteur FVII est glycosylé, carboxylé, hydroxylé, sulfaté, phosphorylé, contenant de l'albumine, ou conjugué à un groupe fonctionnel de polyéthylèneglycol (PEG).

**20.** Polypeptide modifié du facteur FVII de l'une des revendications 1 à 19, qui est un polypeptide à chaîne unique ou est un polypeptide à deux chaînes.

**21.** Polypeptide modifié du facteur FVII de l'une des revendications 1 à 20 qui est actif ou activé.

**22.** Polypeptide modifié de l'une des revendications 1 à 21, dans lequel l'activité de coagulation est accrue.

**23.** Molécule d'acides nucléiques, comprenant une séquence de nucléotides codant pour un polypeptide modifié du facteur FVII de l'une des revendications 1 à 22.

**24.** Vecteur, comprenant la molécule d'acides nucléiques de la revendication 23.

**25.** Vecteur de la revendication 24, dans lequel le vecteur est un vecteur procaryote, un vecteur viral, ou un vecteur eucaryote.

**26.** Vecteur de la revendication 24 ou de la revendication 25, dans lequel le vecteur est un vecteur de mammifère.

**27.** Vecteur de la revendication 25, dans lequel le vecteur est un vecteur viral choisi parmi un adénovirus, un virus adéno-associé, un rétrovirus, un virus de l'herpès, un lentivirus, un poxvirus, et un cytomégalovirus.

**28.** Cellule, comprenant le vecteur de l'une des revendications 24 à 27.

**29.** Cellule de la revendication 28, qui est une cellule eucaryote.

**30.** Cellule de la revendication 29, dans laquelle la cellule eucaryote est une cellule de mammifère.

**31.** Cellule de la revendication 30, dans laquelle la cellule de mammifère est choisie parmi des cellules rénales de bébé hamster (BHK-21) ou des cellules 293 ou des cellules CHO.

**32.** Cellule de la revendication 28, qui est une cellule de levure.

**33.** Cellule de la revendication 32, qui est une cellule de *Pichia sp.*

**34.** Cellule de l'une des revendications 28 à 33, dans laquelle la cellule exprime le polypeptide modifié du facteur FVII.

**35.** Polypeptide modifié du facteur FVII qui est produit par la cellule de l'une des revendications 28 à 34.

**36.** Composition pharmaceutique, comprenant une concentration ou quantité thérapeutiquement efficace d'un polypeptide modifié du facteur FVII de l'une des revendications 1 à 22 et 35, ou d'une molécule d'acides nucléiques de la revendication 23 ou d'un vecteur de l'une des revendications 24 à 27 ou d'une cellule de l'une des revendications 28 à 34, dans un véhicule pharmaceutiquement acceptable.

**37.** Composition pharmaceutique de la revendication 36, qui est formulée pour une administration locale, systémique ou topique.

**38.** Composition pharmaceutique de la revendication 36 ou de la revendication 37, qui est formulée pour une administration par voie orale, nasale, pulmonaire, buccale, transdermique, sous-cutanée, intra-duodénale, entérale, parentérale, intraveineuse ou intramusculaire.

**39.** Composition pharmaceutique de l'une des revendications 36 à 38, qui est formulée pour une libération contrôlée.

**40.** Polypeptide modifié du facteur FVII de l'une des revendications 1 à 22 et 35, pour une utilisation dans le traitement d'une maladie ou d'une affection qui est traitée par l'administration de facteur FVII ou d'un pro-coagulant.

**41.** Utilisation d'une composition pharmaceutique de l'une des revendications 36 à 39, dans la préparation d'un médicament destiné au traitement d'une maladie ou d'une affection qui est traitée par l'administration du facteur FVII ou d'un pro-coagulant.

**42.** Polypeptide modifié du facteur FVII pour une utilisation selon la revendication 40, ou l'utilisation de la revendication 41, dans lequel la maladie ou l'affection est traitée par l'administration d'un zymogène ou d'une forme active du facteur FVII.

**43.** Polypeptide modifié du facteur FVII pour une utilisation selon la revendication 40 ou la revendication 42, ou l'utilisation de la revendication 41 ou de la revendication 42, dans lequel la maladie ou l'affection à traiter est choisie parmi des troubles de la coagulation sanguine, des troubles hématologiques, des troubles hémorragiques, des hémophilies, une déficience en facteur VII, des troubles hémostatiques, un saignement chirurgical et un saignement résultant d'un traumatisme ou est dû à une complication hémorragique due à une intervention chirurgicale ou à un traumatisme.

**44.** Polypeptide modifié du facteur FVII pour une utilisation selon la revendication 43, ou l'utilisation de la revendication 43, dans lequel l'hémophilie est l'hémophilie A ou l'hémophilie B ou l'hémophilie C ou est acquise.

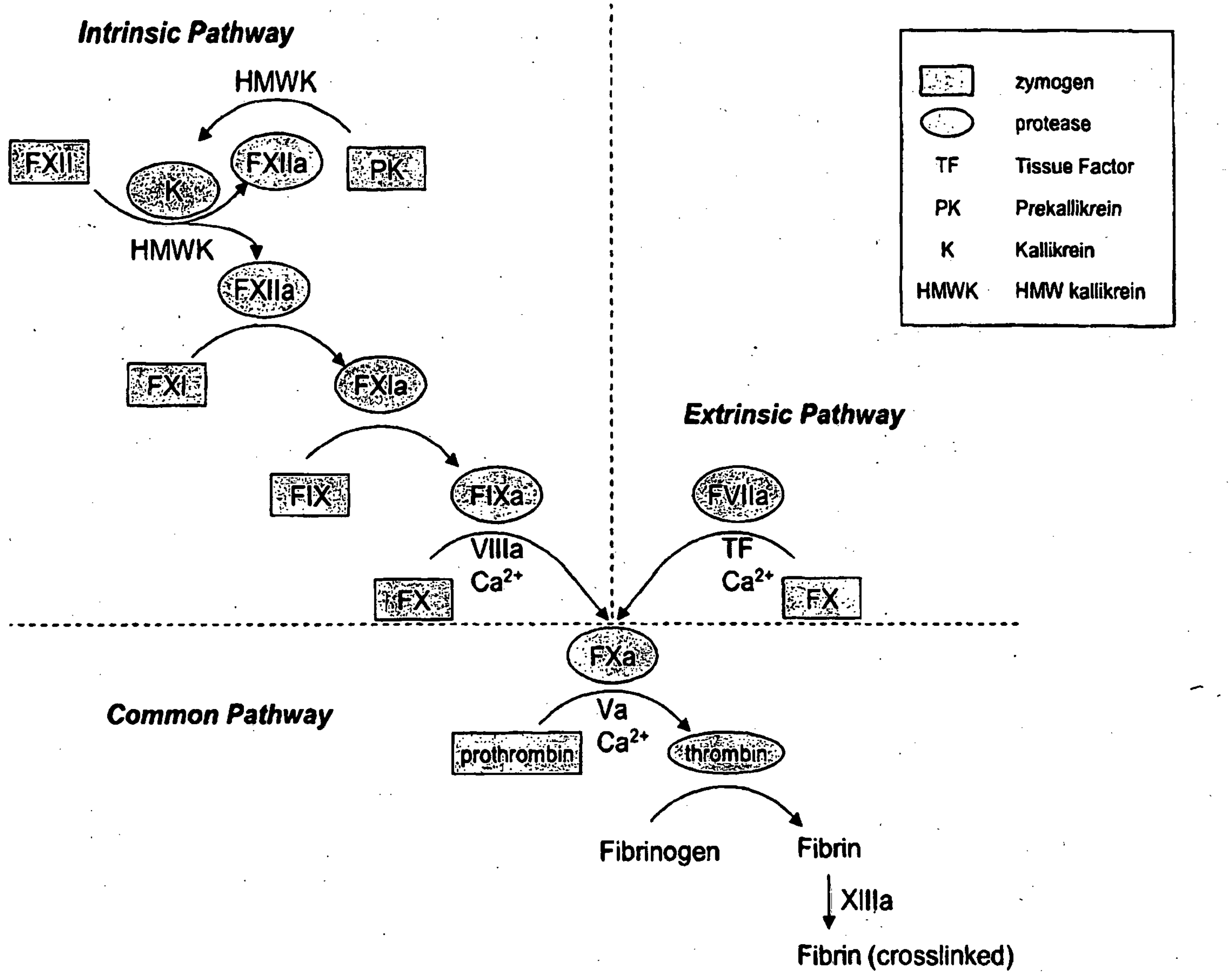


**Figure 1. Coagulation cascade**

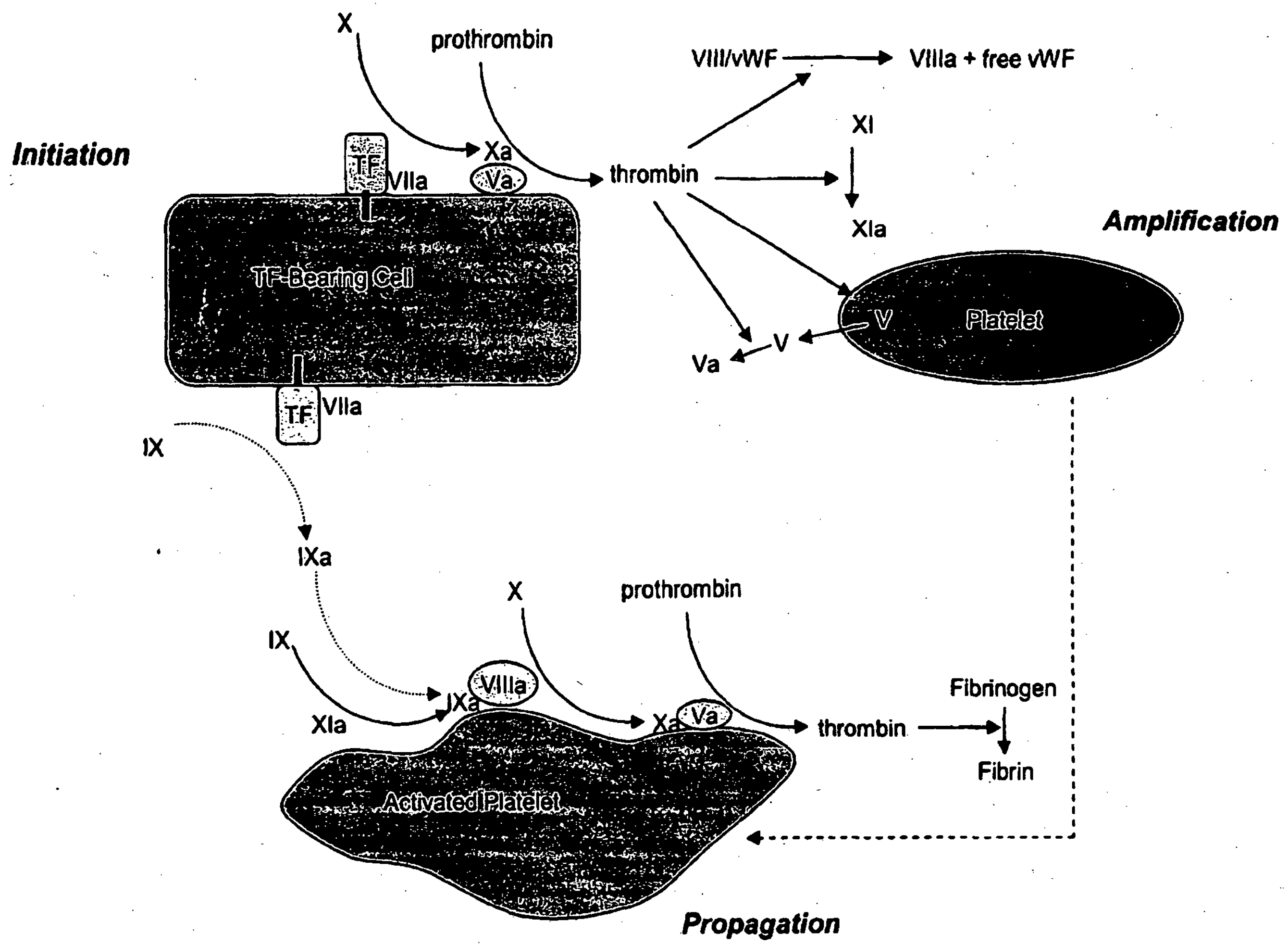


Figure 2. Cell-based model of coagulation

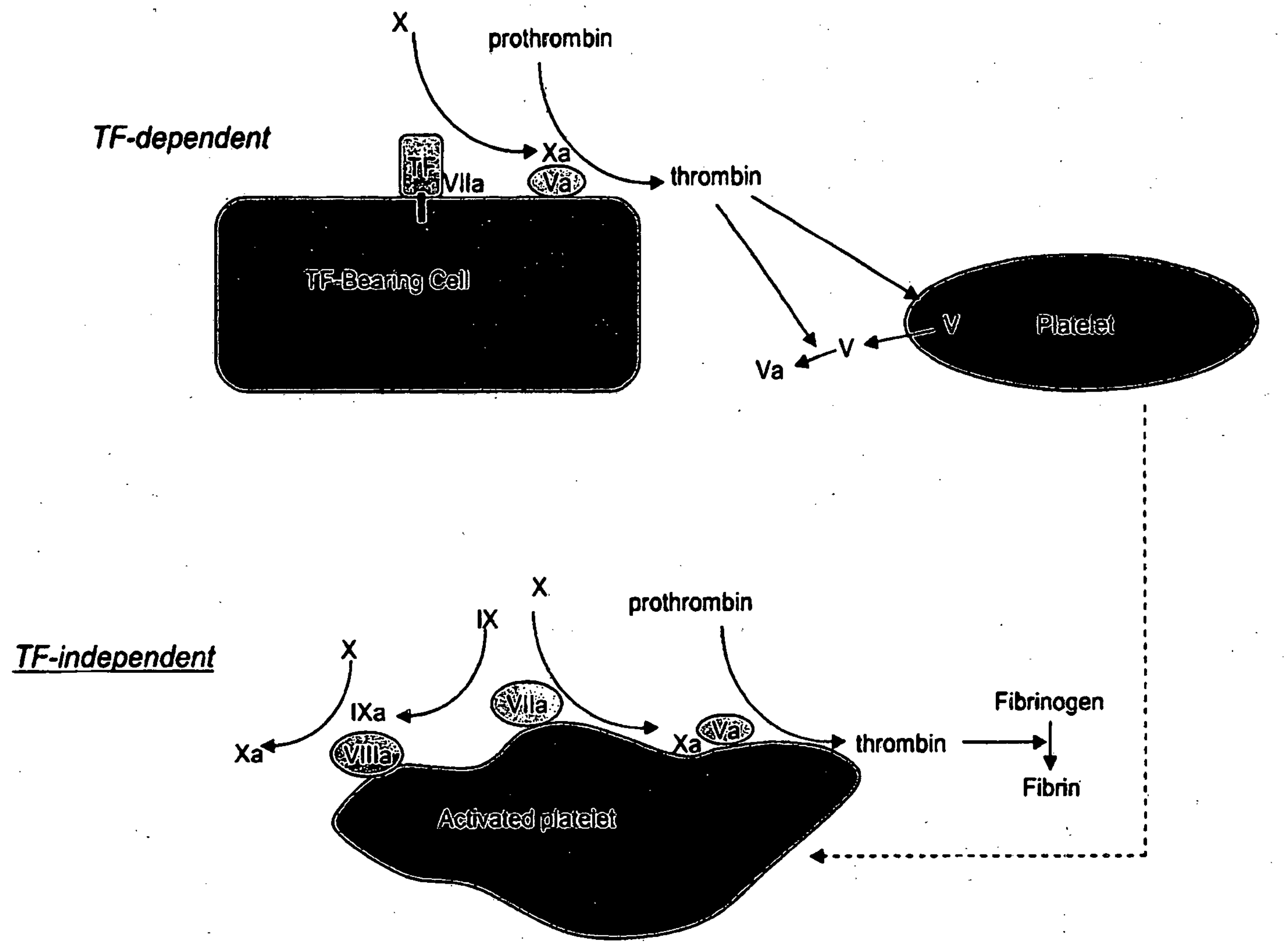


Figure 3. TF-dependent and -independent FVII initiation of thrombin production

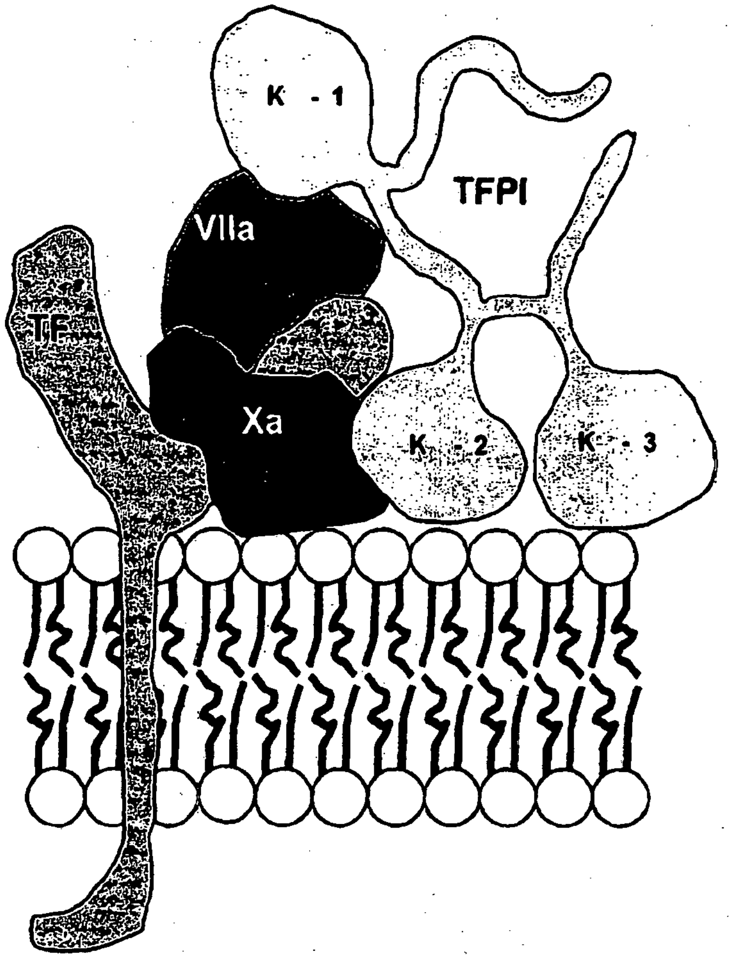


(adapted from : sanquinbbllh.nl/sanquin-eng/sqn_reagents.nsf/0/6dbd8d7ab5a0a4a5c1256f8c00540777/$FILE/highlight%20TFPI.doc

**Figure 4. Depiction of TFPI binding to FVIIa**

(a)

```
                  (1) 1        10        20        30        40            55
        BPTI 5L15 (1) APDFCLEPPYDGPCRALHLRYFYNAKAGLCQTFYYGGCLAKRNNFESAEDCMRTC
 TFPI-2 Kunitz 1  (1) ----CLLPLDYGPCRALLLRYYYDRYTQSCRQFLYGGCEGNANNFYTWEACDDAC
        Consensus (1)     CL P   GPCRAL LRYFY      C F YGGC A  NNF S E C   C
```

(b)

```
                  (1) 1        10        20        30        40        51
  TFPI-1 Kuntiz 1 (1) CAFKADDGPCKAIMKRFFFNIFTRQCEEFIYGGCEGNQNRFESLEECKKMC
  TFPI-2 Kunitz 1 (1) CLLPLDYGPCRALLLRYYYDRYTQSCRQFLYGGCEGNANNFYTWEACDDAC
        Consensus (1) C    D GPCKAIL RFFF  FT  C  FIYGGCEGN N F S E C   C
```

**Figure 5. Primary sequence alignment of the first Kunitz domain of TFPI-2 with BPTI$^{5L15}$ and TFPI-1**

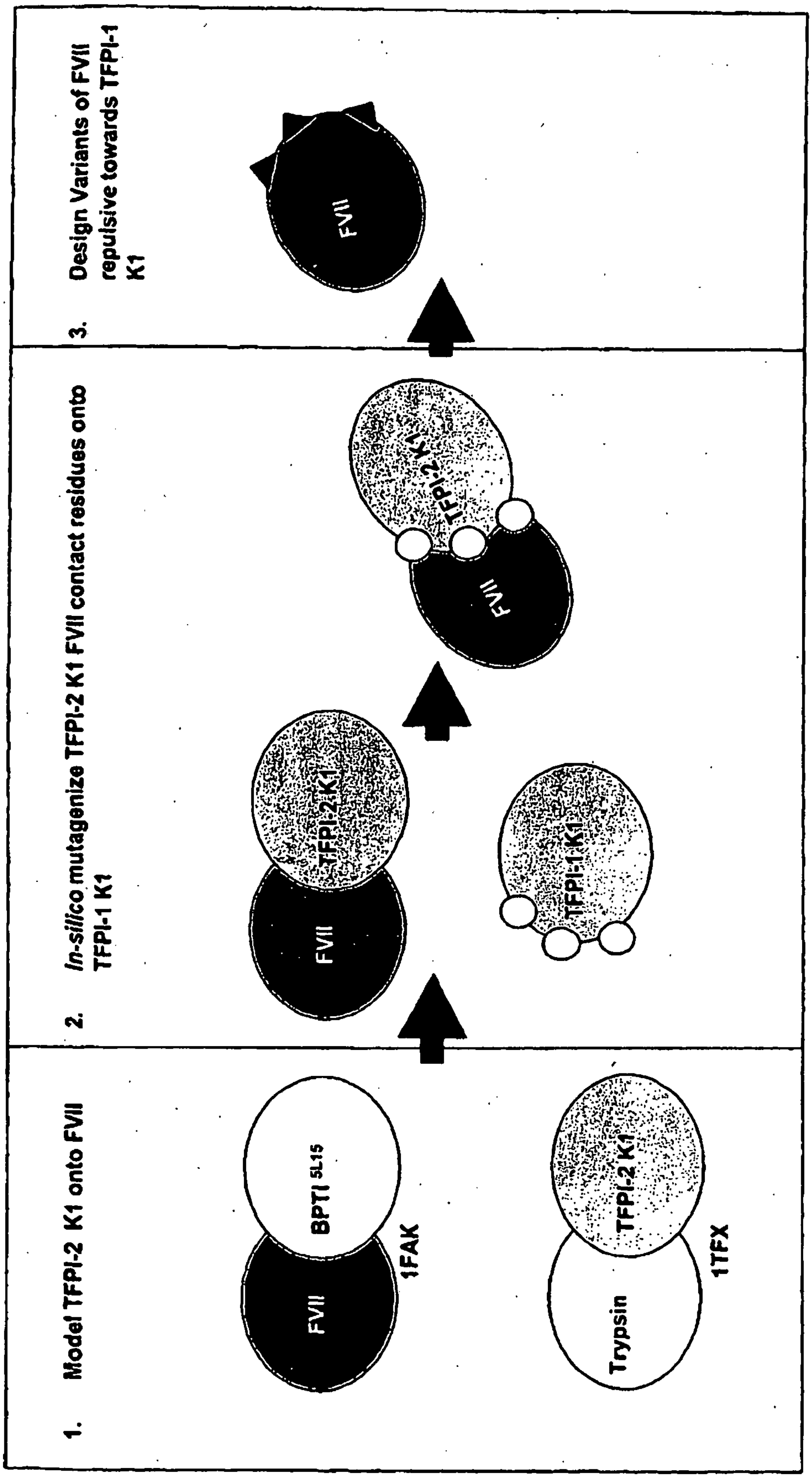


**Figure 6. Homology Model**

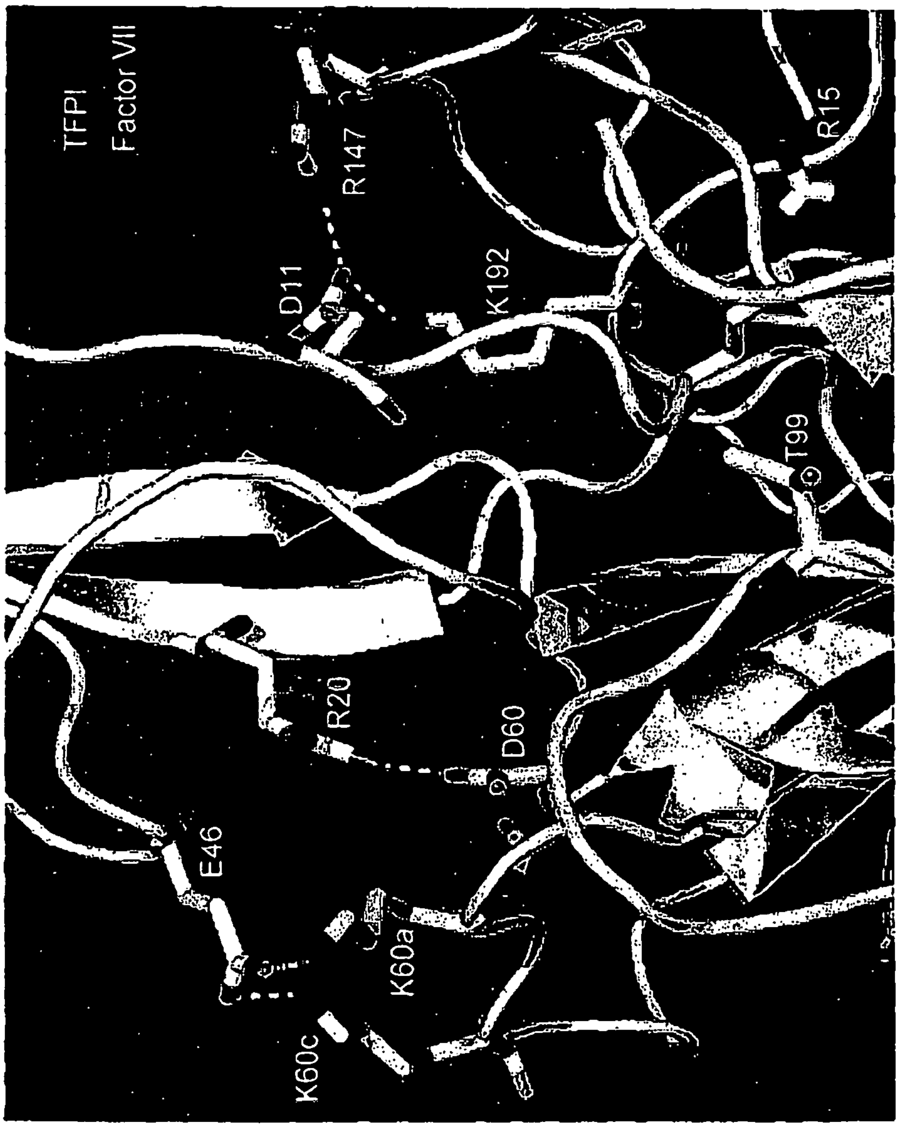


**Figure 7: Factor VII and TFPI Contact Residues**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004083661 A
- WO 2004111242 A
- US 5580560 A
- US 6017882 A
- US 6693075 B
- US 6762286 B
- US 6806063 B
- US 20030100506 A
- US 20040220106 A
- WO 1988010295 A
- WO 200183725 A
- WO 2003093465 A
- WO 200338162 A
- WO 2004083361 A
- WO 2004108763 A
- WO 2004029090 A
- WO 2004029091 A
- WO 2005123916 A
- US 20070037746 A
- WO 2002022776 A
- WO 2002038162 A
- WO 2003027147 A
- US 20060240526 A
- WO 200393465 A
- US 20030096338 A
- US 20060019336 A
- WO 200158935 A
- WO 2002077218 A
- US 20040146938 A
- US 2004146938 A
- US 20060115874 A
- US 5223409 A, Ladner
- WO 9218619 A
- WO 9117271 A
- WO 9220791 A
- WO 9215679 A
- WO 9301288 A
- WO 9201047 A
- WO 9209690 A
- WO 9002809 A
- US 20040235054 A
- WO 0140803 A
- WO 9951773 A
- US 20020192673 A1
- US 4952496 A
- US 20020166130 A
- US 20040133930 A
- WO 0132711 A
- US 6677440 B
- US 4522811 A
- US 4892538 A
- US 5283187 A
- US 5323907 A
- US 5052558 A
- US 5033352 A
- US 60923512 B


### Non-patent literature cited in the description


- **HOFFMAN et al.** *Thromb Haemost,* 2001, vol. 85, 958-965
- *J. Biol. Chem.,* 1969, vol. 243, 3552-3559
- **EISENBERG et al.** *Faraday Symp. Chem. Soc.,* 1982, vol. 17, 109-120
- Computational Molecular Biology. Oxford University Press, 1988
- Biocomputing: Informatics and Genome Projects. Academic Press, 1993
- Computer Analysis of Sequence Data. Humana Press, 1994
- **HEINJE, G.** Sequence Analysis in Molecular Biology. Academic Press, 1987
- Sequence Analysis Primer. M Stockton Press, 1991
- **CARILLO et al.** *SIAM J Applied Math,* 1988, vol. 48, 1073
- **PEARSON et al.** *Proc. Natl. Acad. Sci. USA,* 1988, vol. 85, 2444
- **DEVEREUX, J. et al.** *Nucleic Acids Research,* 1984, vol. 12 (I), 387
- **ATSCHUL, S.F. et al.** *J. Molec. Biol.,* 1990, vol. 215, 403
- Guide to Huge Computers. Academic Press, 1994
- **NEEDLEMAN et al.** *J. Mol. Biol.,* 1970, vol. 48, 443
- **SMITH ; WATERMAN.** *Adv. Appl. Math.,* 1981, vol. 2, 482
- **GRIBSKOV et al.** *Nucl. Acids Res.,* 1986, vol. 14, 6745
- Atlas of Protein Sequence and Structure. National Biomedical Research Foundation, 1979, 353-358

- *Biochem.,* 1972, vol. 11, 726
- **RUGGERI.** *Nat Med,* 2002, vol. 8, 1227-1234
- **SAVAGE et al.** *Curr Opin Hematol,* 2001, vol. 8, 270-276
- **WILDGOOSE et al.** *Blood,* 1992, vol. 80, 25-28
- **BUTENAS et al.** *Biochemistry,* 1996, vol. 35, 1904-1910
- **BANNER et al.** *Nature,* 1996, vol. 380, 41-46
- **OSTERBUND et al.** *Eur J Biochem,* 2000, vol. 267, 6204-6211
- **JIN et al.** *J Mol Biol,* 2001, vol. 307, 1503-1517
- **PERSSON et al.** *Biochem J.,* 2004, vol. 379, 497-503
- **PIKE et al.** *PNAS,* 1999, 8925-8930
- **HIMMELSPACH et al.** *Thromb Research,* 2000, vol. 97, 51-67
- **MARGARITAS et al.** *Clin Invest,* 2004, vol. 113 (7), 1025-1031
- **BUTENAS et al.** *Biochem,* 1996, vol. 35, 1904-1910
- **NAKAGAKI et al.** *Biochem,* 1991, vol. 30, 10819-10824
- **HIGASHI et al.** *J Biol Chem,* 1996, vol. 271, 26569-26574
- **HEMKER et al.** *Blood Cells,* 1983, vol. 9, 303-317
- **MONROE et al.** *Br J Haematol,* 1997, vol. 99, 542-7
- **HOFFMAN et al.** *Blood Coagul Fibrinolysis,* 1998, vol. 9, S61-S65
- **RATKO et al.** *P & T,* 2004, vol. 29, 712-720
- **WILLIAMSON et al.** *J Thromb Haemost,* 2005, vol. 3, 1250-6
- **WUN et al.** *J. Biol. Chem.,* 1988, vol. 263, 6001
- **PIRO et al.** *Circulation,* 2004, vol. 110, 3567-3572
- **PIRO et al.** *J Thromb Haemost,* 2005, vol. 3, 2677-2683
- **GIRARD et al.** *Nature,* 1989, vol. 338, 518-520
- **CHAND et al.** *J Biol Chem,* 2004, vol. 279, 17500-17507
- **CHAND et al.** *Thromb Haemost,* 2005, vol. 94, 1122-30
- **WARN-CRAMER et al.** *Arterioscl Thromb,* 1993, vol. 13, 1551-1557
- **RAGNI et al.** *Circulation,* 2000, vol. 102, 113-117
- **IAKHIAEV et al.** *J. Biol. Chem,* 1999, vol. 274, 36995-37003
- **NEUENSCHWANDER et al.** *Biochemistry,* 1995, vol. 34, 8701-8707
- **CHANG et al.** *Biochemistry,* 1999, vol. 38, 10940-10948
- **IAKHIAEV et al.** *Thromb. Haemost.,* 2001, vol. 85, 458-463
- **RAO et al.** *Blood,* 1993, vol. 81, 2600-2607
- **PERSSON et al.** *PNAS,* 2001, vol. 98, 13583-13588
- **PERSSON et al.** *Biochem J,* 2004, vol. 379, 497-503
- **NEUENSCHWANDER et al.** *J Biol Chem,* 1994, vol. 269, 8007-8013
- **HUANG et al.** *J Biol Chem,* 1996, vol. 271, 21752-21757
- **RUF et al.** *Biochemistry,* 1999, vol. 38, 1957-1966
- **MCDONALD et al.** *Biochemistry,* 1997, vol. 36, 5120-5127
- **SHEN et al.** *Biochemistry,* 1997, vol. 36, 16025-16031
- **HARVEY et al.** *J Biol Chem,* 2003, vol. 278, 8363-8369
- **SHAH et al.** *PNAS,* vol. 95, 4429-4234
- **GENG et al.** *Thromb Haemost,* 1997, vol. 77, 926-933
- **RUF et al.** *Biochem,* 1999, vol. 38, 1957-1966
- **MAUN et al.** *Prot Sci,* 2005, vol. 14, 1171-1180
- **PERSSON et al.** *Eur J Biochem,* 2002, vol. 269, 5950-5955
- **SOEJIMA et al.** *J Biol Chem,* 2001, vol. 276, 17229-17235
- **SOEJIMA et al.** *J Biol Chem,* 2002, vol. 277, 49027-49035
- **RUAN, H. et al.** *Gene,* 1997, vol. 188, 35-39
- **ZACCOLO, M. et al.** *J. Mol. Biol.,* 1996, vol. 255, 589-603
- **ZHENG et al.** *Nucl. Acids. Res.,* 2004, vol. 32, 115
- **KUNKLE.** Current Protocols in Molecular Biology. John Wiley and Sons, Inc,
- **CLACKSON et al.** Making Antibody Fragments Using Phage Display Libraries. *Nature,* 1991, vol. 352, 624-628
- **GLASER et al.** Antibody Engineering by Condon-Based Mutagenesis in a Filamentous Phage Vector System. *J. Immunol,* 1992, vol. 149, 3903-3913
- **HOOGENBOOM et al.** Multi-Subunit Proteins on the Surface of Filamentous Phage: Methodologies for Displaying Antibody (Fate) Heavy and 30 Light Chains. *Nucleic Acids Res.,* 1991, vol. 19, 4133-41370
- **BOUBLIK et al.** Eukaryotic Virus Display: Engineering the Major Surface Glycoproteins of the Autographa California Nuclear Polyhedrosis Virus (ACNPV) for the Presentation of Foreign Proteins on the Virus Surface. *Bio/Technology,* 1995, vol. 13, 1079-1084
- **RODI et al.** *Curr. Opin. Chem. Biol.,* 2002, vol. 6, 92-96
- **SMITH.** *Science,* 1985, vol. 228, 1315-1317
- **DE HAARD et al.** *J. Biol. Chem,* 1999, vol. 274, 18218-30

- **HOOGENBOOM et al.** *Immunotechnology,* 1998, vol. 4, 1-20
- **HOOGENBOOM et al.** *Immunol Today,* 2000, vol. 2, 371-8
- **FUCHS et al.** *Bio/Technology,* 1991, vol. 9, 1370-1372
- **HAY et al.** *Hum Antibod Hybridomas,* 1992, vol. 3, 81-85
- **HUSE et al.** *Science,* 1989, vol. 246, 1275-1281
- **GRIFFITHS et al.** *EMBO J,* 1993, vol. 12, 725-734
- **HAWKINS et al.** *J Mol Biol,* 1992, vol. 226, 889-896
- **CLACKSON et al.** *Nature,* 1991, vol. 352, 624-628
- **GRAM et al.** *PNAS,* 1992, vol. 89, 3576-3580
- **GARRARD et al.** *Bio/Technology,* 1991, vol. 9, 1373-1377
- **REBAR ET.** *Methods Enzymol.,* 1996, vol. 267, 129-49
- **HOOGENBOOM et al.** *Nuc Acid Res,* 1991, vol. 19, 4133-4137
- **BARBAS et al.** *PNAS,* 1991, vol. 88, 7978-7982
- **ANDRIS-WIDHOPF et al.** *J Immunol Methods,* 2000, vol. 28, 159-81
- **COREY et al.** *Gene,* 1993, vol. 128 (1), 129-34
- **CWIRLA et al.** *Proc Natl Acad Sci USA,* 1990, vol. 87 (16), 6378-82
- **FOWLKES et al.** *Biotechniques,* 1992, vol. 13 (3), 422-8
- **HOOGENBOOM et al.** *Nuc Acid Res,* 1991, vol. 19 (15), 4133-7
- **MCCAFFERTY et al.** *Nature,* 1990, vol. 348 (6301), 552-4
- **MCCONNELL et al.** *Gene,* 1994, vol. 151 (1-2), 115-8
- **SCOTT ; SMITH.** *Science,* 1990, vol. 249 (4967), 386-90
- **CHEN ; GEORGIOU.** *Biotechnol Bioeng,* 2002, vol. 79, 496-503
- **FRANCISCO J.A. ; GEORGIOU G.** *Ann N YAcad Sci.,* 1994, vol. 745, 372-382
- **GEORGIOU G. et al.** *Protein Eng.,* 1996, vol. 9, 239-247
- **SAMUELSON et al.** *J. Biotechnol.,* 2002, vol. 96, 129-154
- **HANES ; PLUCKTHUN.** *Proc. Natl. Acad. Sci. U.S.A.,* 1997, vol. 13, 4937-4942
- **LAM, K. S. et al.** *Nature,* 1991, vol. 354, 82-84
- **K. S. et al.** *Nature,* 1991, vol. 354, 82-84
- **HOUGHTEN, R. A. et al.** *Nature,* 1991, vol. 354, 84-86
- **FURKA, A. et al.** *Int. J. Peptide Protein Res.,* 1991, vol. 37, 487-493
- **LAM, K. S. et al.** *Chem. Rev.,* 1997, vol. 97, 411-448
- **CAHILL.** *J. Immunol. Meth.,* 2001, vol. 250, 81-91
- **FREEMAN et al.** *Biotechnol. Bioeng.,* 2004, vol. 86, 196-200
- **BEMOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. USA,* 1981, vol. 78, 1441-1445
- **BRINSTER et al.** *Nature,* 1982, vol. 296, 39-42
- **JAY et al.** *Proc. Natl. Acad. Sci. USA,* 1981, vol. 78, 5543
- **DEBOER et al.** *Proc. Natl. Acad. Sci. USA,* 1983, vol. 80, 21-25
- Useful Proteins from Recombinant Bacteria. *Scientific American,* 1980, vol. 242, 79-94
- **HERRAR-ESTRELLA et al.** *Nature,* 1984, vol. 303, 209-213
- **GARDER et al.** *Nucleic Acids Res.,* 1981, vol. 9, 2871
- **HERRERA-ESTRELLA et al.** *Nature,* 1984, vol. 310, 115-120
- **SWIFT et al.** *Cell,* 1984, vol. 38, 639-646
- **ORNITZ et al.** *Cold Spring Harbor Symp. Quant. Biol.,* 1986, vol. 50, 399-409
- **MACDONALD.** *Hepatology,* 1987, vol. 7, 425-515
- **HANAHAN et al.** *Nature,* 1985, vol. 315, 115-122
- **GROSSCHEDL et al.** *Cell,* 1984, vol. 38, 647-658
- **ADAMS et al.** *Nature,* 1985, vol. 318, 533-538
- **ALEXANDER et al.** *Mol. Cell Biol.,* 1987, vol. 7, 1436-1444
- **LEDER et al.** *Cell,* 1986, vol. 45, 485-495
- **PINCKERT et al.** *Genes and Devel.,* 1987, vol. 1, 268-276
- **KRUMLAUF et al.** *Mol. Cell. Biol.,* 1985, vol. 5, 1639-1648
- **HAMMER et al.** *Science,* 1987, vol. 235, 53-58
- **KELSEY et al.** *Genes and Devel.,* 1987, vol. 1, 161-171
- **MOGRAM et al.** *Nature,* 1985, vol. 315, 338-340
- **KOLLIAS et al.** *Cell,* 1986, vol. 46, 89-94
- **READHEAD et al.** *Cell,* 1987, vol. 48, 703-712
- **SANI.** *Nature,* 1985, vol. 314, 283-286
- **MASON et al.** *Science,* 1986, vol. 234, 1372-1378
- **WAJIH et al.** *J. Biol. Chem.,* vol. 280 (36), 31603-31607
- **PLATIS et al.** *Protein Exp. Purif.,* 2003, vol. 31 (2), 222-30
- **KHALIZZADEH et al.** *J. Ind. Microbiol. Biotechnol.,* 2004, vol. 31 (2), 63-69

- **SKOKO et al.** *Biotechnol. Appl. Biochem.,* 2003, vol. 38, 257-65
- **MUNETA et al.** *J. Vet. Med. Sci.,* 2003, vol. 65 (2), 219-23
- **PHAM et al.** *Biotechnol. Bioeng.,* 2003, vol. 84, 332-42
- **JURLANDER et al.** *Semin Throm Hemost,* 2003
- **MAYFIELD et al.** *PNAS,* 2003, vol. 100, 438-442
- **NELSESTUEN et al.** *J Biol Chem,* 2001, vol. 276, 39825-31
- **BROZE et al.** *J Biol Chem,* 1980, vol. 255, 1242-1247
- **HARVEY et al.** *J Biol Chem,* vol. 278, 8363-8369
- CURRENT PROTOCOLS IN MOLECULAR BIOLOGY. John Wiley & Sons, 1992
- **PRZYSIECKI et al.** *PNAS,* 1987, vol. 84, 7856-7860
- **MAUM et al.** *Prot Sci,* vol. 14, 1171-1180
- **LIN et al.** *J Biol Chem,* 2004, vol. 279, 6560-6566
- **LEIBMAN et al.** *PNAS,* 1985, vol. 82, 3879-3883
- **PUSATERI et al.** *Critical Care,* 2005, vol. 9, S15-S24
- **PETERSEN et al.** *Protein Science,* 2000, vol. 9, 859-866
- **SUN et al.** *Blood,* vol. 101, 2277-2284
- **BI et al.** *Nat Gen,* 1995, vol. 10, 119-121
- **TRANHOLM et al.** *Blood,* 2003, vol. 102, 3615-3620
- **MARGARITIS et al.** *J Clin Invest,* 2004, vol. 113, 1025-1031
- **DINESS et al.** *Thromb Res,* 1992, vol. 67, 233-241
- **ELG et al.** *Thromb Res,* 2001, vol. 101, 145-157
- **CHAN et al.** *J Thromb Haemost,* 2003, vol. 1, 760-765
- **BRINKHOUS et al.** *PNAS,* 1989, vol. 86, 1382-1386
- **TRANHOLM et al.** *Thromb Res,* 2003, vol. 109, 217-223
- **FATORUTTO et al.** *Can J Anaesth,* 2004, vol. 51, 672-679
- **LYNN et al.** *J Trauma,* 2002, vol. 52, 703-707
- **MARTINOWITZ et al.** *J Trauma,* 2001, vol. 50, 721-729
- **SONDEEM et al.** *Shock,* 2004, vol. 22, 163-168
- **JURLANDER et al.** *Semin Thromb Hemost,* 2001, vol. 27, 373-384
- **KEMBALL-COOK et al.** *J Biol Chem,* 1998, vol. 273, 8516-8521
- **WEINER et al.** *J Pharm Sci.,* 1985, vol. 74 (9), 922-5
- **LINDENBAUM et al.** *Nucleic Acids Res.,* 2004, vol. 32 (21), 172
- **KETNER et al.** *PNAS,* 1994, vol. 91, 6186-6190
- **SROUR et al.** *Thromb Haemost.,* 2003, vol. 90 (3), 398-405
- **BOGGIO et al.** *Br J Haematol,* 2001, vol. 112, 1074-1075
- **VON DEPKA et al.** *Blood Coagul Fibrin,* 2006, vol. 17, 311-316
- **VAN BUUREN et al.** *Dig Dis Sci,* 2002, vol. 47, 2134-2136
- **QEROTZIAFAS et al.** *Am J Hematol,* 2002, vol. 69, 219-222
- **SHAMI et al.** *Liver Transpl,* 2003, vol. 9, 138-143
- **MOSCARDO et al.** *Br J Haematol,* 2001, vol. 113, 174-176
- **OLOMU et al.** *J Perinatol,* 2002, vol. 22, 672-674
- **HICKS et al.** *Bone Marrow Transpl,* 2002, vol. 30, 975-978
- **DEVERAS et al.** *Ann Inten Med,* 2002, vol. 137, 884-888
- **FRIEDERICH et al.** *Lancet,* 2003, vol. 361, 201-205
- **AL DOURI et al.** *Blood Coag Fibrinol,* 2000, vol. 11, S121-S127
- **KASTRUP et al.** *Ann Thorac Surg,* 2002, vol. 74, 910-912
- **GERLACH et al.** *J Neurosurg,* 2002, vol. 96, 946-948
- **SAJDAK et al.** *Eur J Gynaecol Oncol,* 2002, vol. 23, 325-326
- **VLOT et al.** *Am J Med,* 2000, vol. 108, 421-423
- **KENET et al.** *Lancet,* 1999, vol. 354, 1879
- **KRISHNASWAMY et al.** *J. Biol. Chem.,* 1998, vol. 273, 8 4378-86
- **PAYNE et al.** *Biochemistry,* 1996, vol. 35, 7100-7106